# EUROPEAN PATENT SPECIFICATION

(11) **EP 4 058 146 B1**
(45) Date of publication and mention of the grant of the patent: **23.10.2024**
(21) Application number: 20820657.3
(22) Date of filing: 12.11.2020
(51) Int. Cl.: A61P 31/04, C07D 401/14, C07D 403/12, C07D 403/14, C07D 405/14, A61K 31/513

(54) **ANTIMICROBIAL COMPOUNDS AND METHODS**
ANTIMIKROBIELLE VERBINDUNGEN UND VERFAHREN
COMPOSÉS ANTIMICROBIENS ET PROCÉDÉS

(30) Priority: 13.11.2019 US 201962934853 P
(43) Date of publication of application: 21.09.2022
(73) Proprietor: Curza Global LLC, Salt Lake City, UT 84108 (US); The University of Utah Research Foundation, Salt Lake City, Utah 84108 (US)
(72) Inventor: SEBAHAR, Paul, R., Sandy, UT 84094 (US); LOOPER, Ryan, E., Salt Lake City, UT 84103 (US); TESTA, Charles, A., North Salt Lake, UT 84054 (US); TRESCO, Benisaac, C., Salt Lake City, UT 84109 (US); HAUSSENER, Travis, J., Salt Lake City, UT 84106 (US); REDDY, Hariprasada, R., Kanna, Salt Lake City, UT 84108-3512 (US); GRANT, Seth, West Jordan, UT 84088 (US)
(74) Representative: Arch, Peter Jonathan Sanders
(86) International application number: PCT/US2020/060185
(87) International publication number: WO 2021/097061

(56) References cited:
- WO-A1-2015/035421
- WO-A1-2015/035426
- WO-A1-2016/145417
- WO-A1-2017/193016
- WO-A1-2017/193017
- WO-A1-2017/193023
- WO-A1-2018/236692
- WO-A1-2019/013789
- WO-A1-2019/164880
- WO-A2-2011/047319
- WO-A2-2012/173689

## Description

### Cross-Reference to Related Applications

This application claims the benefit of priority to United States Provisional Application Serial Number 62/934,853, filed November 13, 2019.

### Statement of Government Interest

This invention was made with government support under 1R01AI132304-01 awarded by the National Institutes of Health (NIH). The government has certain rights in the invention.

### Field of the Invention

The present disclosure relates to compounds that are active as antibacterial agents. The present disclosure also relates to methods of treating bacterial infections with the present compounds. The invention is defined in the appended claims.

### Background of the Invention

Antibacterial resistance is a worldwide problem. Both gram-positive and gram-negative bacteria are increasingly becoming resistant to antibiotics.

Gram-positive bacteria such as methicillin resistant *Staphylococcus aureus* (MRSA) are resistant to most antibiotics that are related to penicillin. MRSA strains are commonly involved in infections acquired in health care facilities and can cause infections in greater communities.

Gram-negative bacteria are believed to be more resistant to antibiotics than Gram-positive bacteria, because of the impermeability of their cell walls. According to the National Institutes of Health (NIH), Gram-negative bacteria can cause many types of infections and are spread to humans in a variety of ways. Several species, including *Escherichia coli,* are common causes of foodborne disease. *Vibrio cholerae,* the bacteria responsible for cholera, is a waterborne pathogen. Gram-negative bacteria can also cause respiratory infections, such as certain types of pneumonia, and sexually transmitted diseases, including gonorrhea. *Yersinia pestis,* the Gram-negative bacterium responsible for plague, is transmitted to people through the bite of an infected insect or handling an infected animal. See www.niaid.nih.gov/research/gram-negative-bacteria (last visited November 6, 2020).

Some types of Gram-negative bacteria have become increasingly resistant to available antibiotic drugs. Some strains are now resistant to many, most, or all available treatments resulting in increased illness and death from bacterial infections and contributing to escalating healthcare costs. Examples of Gram-negative bacteria that have demonstrated drug resistance include: *E. coli,* which causes the majority of urinary tract infections; *Acinetobacter baumanii,* which causes disease mainly in healthcare settings; *Pseudomonas aeruginosa,* which causes bloodstream infections and pneumonia in hospitalized patients and is a common cause of pneumonia in patients with cystic fibrosis; *Klebsiella pneumoniae,* which causes many types of healthcare-associated infections, including pneumonia, urinary tract infections, and bloodstream infections; and *Neisseria gonorrhoeae,* which causes the sexually transmitted disease gonorrhea and is the second most commonly reported infectious disease in the United States.

As a result, new drugs to combat Gram-positive and Gram-negative bacterial infections are needed.

### Summary of the Invention

These and other needs are met by the present invention which provides in one aspect a compound of formula I: 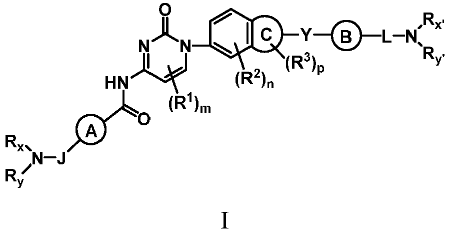 or a single stereoisomer or mixture of stereoisomers thereof, or a pharmaceutically acceptable salt thereof, wherein:
ring A is a 3-8 membered monocyclic heterocycloalkylene optionally substituted with up to three substituents selected from the group consisting of C₁-C₆ alkyl, C₁-C₆ alkoxy, halo, CN, C₁-C₆ haloalkyl, phenyl, OH, NH₂, NH(C₁-C₆ alkyl), N(C₁-C₆ alkyl)₂, COOH, COO(C₁-C₆ alkyl), CONH₂, CONH(C₁-C₆ alkyl), CON(C₁-C₆ alkyl)₂, and oxo;
J is C₁-C₆ alkylene or C₃-C₈ cycloalkylene, either of which is optionally substituted with halo, OH, or C₁-C₆ alkoxy, wherein up to two methylene units of the C₁-C₆ alkylene are optionally and independently replaced with O, S, SO, SO₂, or C=O;
Rₓ, R_{y}, R_{x'}, and R_{y'} are each independently H, C₁-C₆ alkyl, or an amino protecting group;
Y is a bond, or C₁-C₆ alkylene optionally substituted with OH, NH₂, CN, halo, or C₁-C₆ alkoxy, wherein up to two methylene units of the C₁-C₆ alkylene are optionally and independently replaced by O, NH, N-(C₁-C₆ alkyl), N-(C₁-C₆ hydroxyalkyl), N-(C₁-C₆ haloalkyl), N-(C₁₋₆ alkylene-C₃₋₈ cycloalkyl), NH(C=O), N-(C₁₋₆ alkyl)(C=O), or (C=O);
ring B is a 3-8 membered monocyclic cycloalkylene, 3-8 membered monocyclic heterocycloalkylene, 6-12 membered bicyclic cycloalkylene, or a 6-12 membered bicyclic heterocycloalkylene, each of which is optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and C₁-C₆ hydroxyalkyl;
L is a bond or C₁-C₆ alkylene, wherein up to two methylene units of the C₁-C₆ alkylene may be independently replaced with O, NH, (C=O), NH(C=O), N-(C₁₋₆ alkyl)(C=O), (C=NH), NH(C=N), or N-(C₁₋₆ alkyl);
ring C, together with the phenyl ring to which it is fused, forms an 8-12 membered bicyclic arylene or an 8-12 membered bicyclic heteroarylene, wherein the bicyclic heteroarylene has 1-3 heteroatoms independently selected from N, O, or S;
R¹, R², and R³ are each independently selected from the group consisting of C₁-C₆ alkyl, halo, CN, OH, NH₂, NH(C₁-C₆ alkyl), N(C₁-C₆ alkyl)₂, COO(C₁-C₆ alkyl), CONH₂, C₁-C₆ haloalkyl, oxo, and C₁-C₆ alkoxy; and
m, n, and p are each independently 0, 1, 2, or 3.

In another aspect, disclosed but not claimed are methods of using compounds of formula I or a pharmaceutically acceptable salt thereof for the treatment of bacterial infections.

In another aspect, the invention provides pharmaceutical compositions comprising a compound of formula I or a pharmaceutically acceptable salt thereof and a pharmaceutically acceptable carrier.

In a further aspect, the invention provides processes for making compounds of formula I or a pharmaceutically acceptable salt thereof as depicted in the synthetic schemes.

### Detailed Description of the Invention

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. In case of conflict, the present specification, including these definitions, will control.

The terms "a," "an," and "the" as used herein not only include aspects with one member, but also include aspects with more than one member.

The term "about" as used herein means "approximately" and is used to modify a numerical value indicating a defined range around that value. If "X" were the value, "about X" would generally indicate a value from 0.95X to 1.05X. Any reference to "about X" specifically indicates at least the values X, 0.95X, 0.96X, 0.97X, 0.98X, 0.99X, 1.01X, 1.02X, 1.03X, 1.04X, and 1.05X. Thus, "about X" is intended to teach and provide written description support for a claim limitation of, *e.g*., "0.98X." When the quantity "X" only includes whole-integer values (*e.g*., "X carbons"), "about X" indicates from (X-1) to (X+1). In this case, "about X" as used herein specifically indicates at least the values X, X-1, and X+1.

When "about" is applied to the beginning of a numerical range, it applies to both ends of the range. Thus, "from about 5 to 20%" is equivalent to "from about 5% to about 20%." When "about" is applied to the first value of a set of values, it applies to all values in that set. Thus, "about 7, 9, or 11%" is equivalent to "about 7%, about 9%, or about 11%."

As used herein, a wavy line drawn on a structure can be used to show the attachment point of the structure, such as 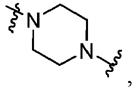 wherein 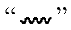 indicates points of attachment.

The term "acyl" as used herein includes an alkanoyl, aroyl, heterocycloyl, or heteroaroyl group as defined herein. Examples of acyl groups include, but are not limited to, acetyl, benzoyl, and nicotinoyl.

The term "alkanoyl" as used herein includes an alkyl-C(O)- group wherein the alkyl group is as defined herein. Examples of alkanoyl groups include, but are not limited to, acetyl and propanoyl.

The term "agent" as used herein includes a compound or mixture of compounds that, when added to a composition, tend to produce a particular effect on the composition's properties. For example, a composition comprising a thickening agent is likely to be more viscous than an otherwise identical comparative composition that lacks the thickening agent.

The term "alkyl" as used herein includes an aliphatic hydrocarbon chain that may be straight chain or branched. The chain may contain an indicated number of carbon atoms: For example, C₁-C₁₀ indicates that the group may have from 1 to 10 (inclusive) carbon atoms in it. If not otherwise indicated, an alkyl group contains from 1 to about 20 carbon atoms. In some aspects, alkyl groups have 1 to about 10 carbon atoms. In some aspects, alkyl groups ("lower alkyl") have 1 to 8, 1 to 6, or 1 to 3 carbon atoms in the chain. Examples may include, but are not limited to, methyl, ethyl, propyl, isopropyl (iPr), 1-butyl, 2-butyl, isobutyl (iBu), tert-butyl, pentyl, 2-methylbutyl, 1,1-dimethylpropyl, hexyl, heptyl, octyl, nonyl, decyl, docecyl, cyclopentyl, or cyclohexyl.

An alkyl group can be unsubstituted or optionally substituted. When optionally substituted, one or more hydrogen atoms of the alkyl group (e.g., from 1 to 4, from 1 to 2, or 1) may be replaced with a moiety independently selected from the group consisting of fluoro, hydroxy, alkoxy, amino, alkylamino, acylamino, thio, and alkylthio. In some aspects, the alkyl group is unsubstituted or not optionally substituted.

"Alkylene" as used herein includes an alkyl group that is substituted at two points. An example is methylene (-CH₂-), propylene (-CH₂CH₂CH₂-), and the like.

The term "alkenyl" as used herein includes a straight or branched chain hydrocarbon containing at least one carbon-carbon double bond. The chain may contain an indicated number of carbon atoms. For example, "C₁-C₁₂ alkenyl" indicates that the group may have from 1 to 12 (inclusive) carbon atoms and at least one carbon-carbon double bond. When the indicated number of carbon atoms is 1, then the Cᵢ alkenyl is double bonded to a carbon *(i.e.,* a carbon equivalent to an oxo group). In certain aspects, the chain includes 1 to 12, about 2 to 15, about 2 to 12, about 2 to 8, or about 2 to 6 carbon atoms. An alkenyl group can be preferably one stereoisomer *(i.e., cis-* or, alternatively, *trans-).* Examples of an alkenyl group may include, but are not limited to, ethenyl (i.e., vinyl), allyl, propenyl, butenyl, crotyl, pentenyl, hexenyl, heptenyl, octenyl, nonenyl, decenyl, dodecenyl, cyclopentenyl, cyclohexenyl, 2-isopentenyl, allenyl, butadienyl, pentadienyl, 3-(l,4-pentadienyl), and hexadienyl.

An alkenyl group can be unsubstituted or optionally substituted. When optionally substituted, one or more hydrogen atoms of the alkenyl group (e.g., from 1 to 4, from 1 to 2, or 1) may be replaced with a moiety independently selected from the group consisting of fluoro, hydroxy, alkoxy, amino, alkylamino, acylamino, thio, and alkylthio, with the proviso that no hydrogen atom substituent on the carbon-carbon double bond is replaced by a hydroxy, amino, or thio group. In some aspects, the alkenyl group is unsubstituted or not optionally substituted.

"Alkenylene" as used herein includes an alkenyl group that is substituted at two points. An example is but-2-enylene (-CH₂CH=CHCH₂-) and the like.

The term "alkoxy" as used herein includes a straight or branched chain saturated or unsaturated hydrocarbon containing at least one oxygen atom in an ether group (e.g., EtO-). The chain may contain an indicated number of carbon atoms. For example, "C₁-C₁₂ alkoxy" indicates that the group may have from 1 to 12 (inclusive) carbon atoms and at least one oxygen atom. Examples of a C₁-C₁₂ alkoxy group include, but are not limited to, methoxy, ethoxy, isopropoxy, butoxy, n-pentoxy, isopentoxy, neopentoxy, and hexoxy.

An alkoxy group can be unsubstituted or optionally substituted. When optionally substituted, one or more hydrogen atoms of the alkoxy group (e.g., from 1 to 4, from 1 to 2, or 1) may be replaced with a moiety independently selected from the group consisting of fluoro, hydroxy, alkoxy, amino, alkylamino, acylamino, thio, and alkylthio, with the proviso that no hydrogen atom alpha to the ether oxygen is replaced by a hydroxy, amino, or thio group. In some aspects, the alkoxy group is unsubstituted or not optionally substituted.

The term "alkynyl" as used herein includes a straight, branched, or cyclic hydrocarbon containing at least one carbon-carbon triple bond. Examples may include, but are not limited to, ethynyl, propargyl, propynyl, butynyl, pentynyl, hexynyl, heptynyl, octynyl, nonynyl, decynyl, or decynyl.

"Alkynylene" as used herein includes an alkynyl group that is substituted at two points. An example is 2-butynylene (-CH₂CCCH₂-) and the like.

An alkynyl group can be unsubstituted or optionally substituted. When optionally substituted, one or more hydrogen atoms of the alkynyl group (e.g., from 1 to 4, from 1 to 2, or 1) may be replaced with a moiety independently selected from the group consisting of fluoro, hydroxy, alkoxy, amino, alkylamino, acylamino, thio, and alkylthio, with the proviso that no sp-hybridized hydrogen atom substituent is replaced by a hydroxy, amino, or thio group. In some aspects, the alkynyl group is unsubstituted or not optionally substituted.

As used herein, an "aryl" group refers to monocyclic (e.g., phenyl); bicyclic (e.g., indenyl, naphthalenyl, tetrahydronaphthyl, dihydroindenyl); and tricyclic (e.g., fluorenyl tetrahydrofluorenyl, tetrahydroanthracenyl, or anthracenyl) ring systems in which the monocyclic ring system is aromatic or at least one of the rings in the bicyclic or tricyclic ring system is aromatic. The other ring(s) in the bicyclic or tricyclic ring system may be saturated, partially unsaturated, or fully unsaturated. The bicyclic and tricyclic groups include benzofused carbocyclic rings. For example, a benzofused group includes a benzene ring fused with one or two 4 to 8 membered carbocyclic moieties (e.g., a 1,2,3,4-tetrahydronaphthalene or a 2,3-dihydro-1H-indene ring system ).

The term "arylene" as used herein includes an aryl group that is substituted at two points.

An aryl group can be unsubstituted or optionally substituted. When optionally substituted, one or more hydrogen atoms of the aryl group (e.g., from 1 to 5, from 1 to 2, or 1) may be replaced with a moiety independently selected from the group consisting of alkyl, cyano, acyl, halo, hydroxy, alkoxy, amino, alkylamino, acylamino, thio, and alkylthio. In some aspects, the alkoxy group is unsubstituted or not optionally substituted.

The term "arylalkyl" or "aralkyl" as used herein includes an alkyl group as defined herein where at least one hydrogen substituent has been replaced with an aryl group as defined herein. Examples include, but are not limited to, benzyl, 1-phenylethyl, 4-methylbenzyl, and 1,1,-dimethyl-1-phenylmethyl.

An arylalkyl or aralkyl group can be unsubstituted or optionally substituted as per its component groups. For example, but without limitation, the aryl group of an arylalkyl group can be substituted, such as in 4-methylbenzyl. In some aspects, the group is unsubstituted or not optionally substituted, especially if including a defined substituent, such as a hydroxyalkyl or alkylaminoalkoxy group.

The term "cycloalkyl" as used herein includes non-aromatic saturated monocyclic or multicyclic ring system that may contain an indicated number of carbon atoms, wherein none of the rings in the multicyclic ring system is aromatic. For example, C₃-C₁₂ indicates that the group may have from 3 to 12 (inclusive) carbon atoms in it. If not otherwise indicated, a cycloalkyl group includes about 3 to about 20 carbon atoms. In some aspects, cyclo alkyl groups have 3 to about 12 carbon atoms in the group. In some aspects, cycloalkyl groups have 3 to about 7 carbon atoms in the group. Examples may include, but are not limited to, cyclopropyl, cyclobutyl, cyclopentyl, cyclohexyl, 4,4-dimethylcyclohexyl, and cycloheptyl. The term "cycloalkyl" also includes multicyclic rings such as a bicyclic cycloalkyl, or a tricyclic cycloalkyl which may be in a fused, bridged, or spiro orientation.

A cycloalkyl group can be unsubstituted or optionally substituted. When optionally substituted, one or more hydrogen atoms of the cycloalkyl group (*e.g.,* from 1 to 4, from 1 to 2, or 1) may be replaced with a moiety independently selected from the group consisting of fluoro, hydroxy, alkoxy, amino, alkylamino, acylamino, thio, and alkylthio. In some aspects, a substituted cycloalkyl group can incorporate an exo- or endocyclic alkene (e.g., cyclohex-2-en-1-yl). In some aspects, a cycloalkyl group is unsubstituted or not optionally substituted.

The term "cycloalkylene" as used herein includes a cycloalkyl group that is substituted at two points.

The terms "disorder" and "disease" are used herein interchangeably for a condition in a subject. A disorder is a disturbance or derangement that affects the normal function of the body of a subject. A disease is a pathological condition of an organ, a body part, or a system resulting from various causes, such as infection, genetic defect, or environmental stress that is characterized by an identifiable group of symptoms. A disorder or disease can refer to a biofilm-related disorder or disorder caused by a planktonic bacterial phenotype that is characterized by a disease-related growth of bacteria.

The term "effective amount" or "effective dose" as used herein includes an amount sufficient to achieve the desired result and accordingly will depend on the ingredient and its desired result. Nonetheless, once the desired effect is identified, determining the effective amount is within the skill of a person skilled in the art.

As used herein, "fluoroalkyl" includes an alkyl group wherein the alkyl group includes one or more fluoro- substituents. Examples include, but are not limited to, trifluoromethyl.

As used herein, "geminal" substitution includes two or more substituents that are directly attached to the same atom. An example is 3,3-dimethyl substitution on a cyclohexyl or spirocyclohexyl ring.

As used herein, "halo" or "halogen" includes fluoro, chloro, bromo, and iodo.

A "heteroaryl" group, as used herein, refers to a monocyclic, bicyclic, or tricyclic ring system having 4 to 15 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic. The other ring(s) in the bicyclic or tricyclic ring system may be saturated, partially unsaturated, or fully unsaturated. A heteroaryl group may include a benzofused ring system having 2 to 3 rings. For example, a benzofused group includes benzo fused with one or two 4 to 8 membered heterocyclic moieties. Some examples of heteroaryl are azetidinyl, pyridyl, 1H-indazolyl, furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl, cinnolyl, phthalazyl, quinazolyl, quinoxalyl, isoquinolyl, 4H-quinolizyl, benzo-1,2,5-thiadiazolyl, 1,8-naphthyridyl, indolizyl, indolyl, isoindolyl, 3H-indolyl, indolinyl, isoindolinyl, benzo[*b*]thiophene-yl, quinolinyl, dihydroisoquinolinyl, tetrahydroisoquinolinyl, benzodihydropyranyl or chromanyl. In some embodiments, the heteroaryl group may include chromanyl, isoindolinyl, and tetrahydroisoquinolinyl.

The term "heteroarylene" as used herein includes a heteroaryl group that is substituted at two points.

A heteroaryl group can be unsubstituted or optionally substituted. When optionally substituted, one or more hydrogen atoms of the heteroaryl group (e.g., from 1 to 5, from 1 to 2, or 1) may be replaced with a moiety independently selected from the group consisting of alkyl, cyano, acyl, halo, hydroxy, alkoxy, amino, alkylamino, acylamino, thio, and alkylthio. In some aspects, the heteroaryl group is unsubstituted or not optionally substituted.

As used herein, "heterocycloalkyl" includes a non-aromatic ring of about 3 to about 15 ring atoms (e.g., 5 to about 10 ring atoms, or 3 to about 6 ring atoms), in which one or more of the atoms in the ring system is an element or elements other than carbon, *e.g.,* nitrogen, oxygen or sulfur. A heterocycloalkyl group optionally comprises at least one sp²-hybridized atom (*e.g.,* a ring incorporating a carbonyl, endocyclic olefin, or exocyclic olefin). In some embodiments, a nitrogen or sulfur atom of the heterocycloalkyl is optionally oxidized to the corresponding N-oxide, S-oxide or S,S-dioxide. The monocyclic heterocycle means a three-, four-, five-, six-, seven-, or eight-membered ring containing at least one heteroatom independently selected from the group consisting of O, N, and S. The three- or four-membered ring contains zero or one double bond, and one heteroatom selected from the group consisting of O, N, and S. The five-membered ring contains zero or one double bond and one, two or three heteroatoms selected from the group consisting of O, N and S. The six-membered ring contains zero, one or two double bonds and one, two, or three heteroatoms selected from the group consisting of O, N, and S. The seven- and eight-membered rings contains zero, one, two, or three double bonds and one, two, or three heteroatoms selected from the group consisting of O, N, and S. Representative examples of monocyclic heterocycles include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyranyl, pyrazolinyl, pyrazolidinyl, pyridazin-3(2H)-onyl, pyridin-2(1H)-onyl, pyrrolinyl, pyrrolidinyl, tetrahydrofuranyl, tetrahydropyranyl, tetrahydropyridinyl, tetrahydropyrimidinyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl.

The term "heterocycloalkylene" as used herein includes a heterocycloalkyl group that is substituted at two points.

The term "heterocycloalkyl" also includes multicyclic ring system such as a bicyclic heterocycle, or a tricyclic heterocycle which may be in a fused, bridged, or spiro orientation, wherein none of the rings in the multicyclic ring system is aromatic. The bicyclic heterocycle may be a monocyclic heterocycle fused to a monocyclic cycloalkyl, or a monocyclic heterocycle fused to a monocyclic cycloalkenyl, or a monocyclic heterocycle fused to a monocyclic heterocycle, or a bridged monocyclic heterocycle ring system in which two non-adjacent atoms of the ring are linked by an alkylene bridge of 1, 2, 3, or 4 carbon atoms, or an alkenylene bridge of two, three, or four carbon atoms. Representative examples of bicyclic heterocycles include, but are not limited to, 3-azabicyclo[3.1.0]hexane, 3-azabicyclo[4.1.0]heptane, 3-azabicyclo[3.2.0]heptane, (3aR,6aS)-hexahydro-1H-2λ₂-cyclopenta[c]pyrrole, (3aR,7aS)-octahydro-2λ₂-isoindole.

A heterocycloalkyl group can be unsubstituted or optionally substituted. When optionally substituted, one or more hydrogen atoms of the group (*e*.*g*., from 1 to 4, from 1 to 2, or 1) may be replaced with a moiety independently selected from the group consisting of fluoro, hydroxy, alkoxy, amino, alkylamino, acylamino, thio, and alkylthio. In some aspects, a substituted heterocycyl group can incorporate an exo- or endocyclic alkene (e.g., cyclohex-2-en-1-yl). In some aspects, the heterocyclyl group is unsubstituted or not optionally substituted.

The monocyclic, bicyclic, and tricyclic heterocycles are connected to the parent molecular moiety through any carbon atom or any nitrogen atom contained within the rings, and can be unsubstituted or substituted.

As used herein, the term "hydrophilic moiety" or "hydrophilic group" includes a moiety or a functional group that has a strong affinity to water. Examples may include, but are not limited to, a charged moiety, such as a cationic moiety or an anionic moiety, or a polar uncharged moiety, such as an alkoxy group or an amine group.

As used herein, the term "hydroxyalkyl" includes an alkyl group where at least one hydrogen substituent has been replaced with an alcohol (-OH) group. In certain aspects, the hydroxyalkyl group has one alcohol group. In certain aspects, the hydroxyalkyl group has one or two alcohol groups, each on a different carbon atom. In certain aspects, the hydroxyalkyl group has 1, 2, 3, 4, 5, or 6 alcohol groups. Examples may include, but are not limited to, hydroxymethyl, 2-hydroxyethyl, and 1-hydroxyethyl.

When any two substituent groups or any two instances of the same substituent group are "independently selected" from a list of alternatives, the groups may be the same or different. For example, if R^{a} and R^{b} are independently selected from the group consisting of alkyl, fluoro, amino, and hydroxyalkyl, then a molecule with two R^{a} groups and two R^{b} groups could have all groups be an alkyl group (e.g., four different alkyl groups). Alternatively, the first R^{a} could be alkyl, the second R^{a} could be fluoro, the first R^{b} could be hydroxyalkyl, and the second R^{b} could be amino (or any other substituents taken from the group). Alternatively, both R^{a} and the first R^{b} could be fluoro, while the second R^{b} could be alkyl (*i.e*., some pairs of substituent groups may be the same, while other pairs may be different).

"Amino protecting group" is a protecting group that is suitable for preventing undesired reactions at an amino nitrogen. Representative amino-protecting groups include, but are not limited to, formyl; acyl groups, for example alkanoyl groups, such as acetyl and trifluoroacetyl; alkoxycarbonyl groups, such as tert-butoxycarbonyl (Boc) and methoxylcarbonyl; arylmethoxycarbonyl groups, such as benzyloxycarbonyl (Cbz) and 9-fluorenylmethoxycarbonyl (Fmoc); arylmethyl groups, such as benzyl (Bn), trityl (Tr), and 1,1-di-(4'-methoxyphenyl)methyl; and the like.

"Hydroxyl protecting group" is a protecting group that is suitable for preventing undesired reactions at a hydroxyl oxygen. Representative hydroxy-protecting groups include, but are not limited to, acyl groups, for example alkanoyl groups, such as acetyl; arylmethyl groups, such as benzyl (Bn), trityl (Tr), and 1,1-di-(4'-methoxyphenyl)methyl; silyl groups, such as trimethylsilyl (TMS) and tert-butyldimethylsilyl (TBDMS or TBS); and the like.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

"Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid, and the like, as well as organic acids such as acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, orotic acid, methanesulfonic acid, ethanesulfonic acid, p-toluenesulfonic acid, salicylic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Exemplary salts are the ammonium, potassium, sodium, calcium, and magnesium salts. Salts derived from pharmaceutically acceptable organic non-toxic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins, and the like. Exemplary organic bases are isopropylamine, diethylamine, ethanolamine, trimethylamine, dicyclohexylamine, choline, and caffeine. (See, for example, S. M. Berge, et al., "Pharmaceutical Salts," J. Pharm. Sci., 1977;66 1-19).

As used herein, "or" should in general be construed non-exclusively. For example, an embodiment of "a composition comprising A or B" would typically present an aspect with a composition comprising both A and B. "Or" should, however, be construed to exclude those aspects presented that cannot be combined without contradiction (*e.g*., a composition pH that is between 9 and 10 or between 7 and 8).

As used herein, "spiro bicyclic cycloalkyl" includes a cycloalkyl in which geminal substituents on a carbon atom are replaced to join in forming a 1,1-substituted ring. For example, but without limitation, for a -C(R¹)(R²)- group that was part of a longer carbon chain, if R¹ and R² joined to form a cyclopropyl ring incorporating the carbon to which R¹ and R² were bonded, this would be a spiro bicyclic cycloalkyl group (i.e. spirocyclopropyl).

The term "spiro bicyclic cycloalkylene" as used herein includes a spiro bicyclic cycloalkyl group that is substituted at two points.

As used herein, "spiro bicyclic heterocycloalkyl" includes a heterocycloalkyl in which geminal substituents on a carbon atom are replaced to join in forming a 1,1 -substituted ring. For example, but without limitation, for a -C(R¹)(R²)- group that was part of a longer carbon chain, if R¹ and R² are joined to form a pyrrolidine ring incorporating the carbon to which R¹ and R² were bonded, this would be a spiro bicyclic heterocycloalkyl group.

The term "spiro bicyclic heterocycloalkylene" as used herein includes a spiro bicyclic heterocycloalkyl group that is substituted at two points.

The compounds disclosed herein are characterized by the presence of amino functional groups. One of ordinary skill would therefore understand that compounds can be isolated as salts wherein the amino functional group nitrogen is quarternized.

As used herein, the term "treat," "treating," or "treatment" includes administering or applying a composition (e.g., a composition described herein) in an amount, manner (*e.g*., schedule of administration), and mode *(e.g.,* route of administration) that is effective to improve a disorder or a symptom thereof, or to retard, or to slow the progression of a disorder or a symptom thereof. Such improvements can include, but are not limited to, alleviation or amelioration of one or more symptoms or conditions, diminishment of the extent of a disease, stabilizing (*i.e.,* not worsening) the state of disease, delaying or slowing of disease progression, amelioration or palliation of the disease state, diminishment of the reoccurrence of disease, and remission, whether partial or total and whether detectable or undetectable.

### Embodiments

### Compounds

In a first aspect, the disclosure provides a compound of formula I: 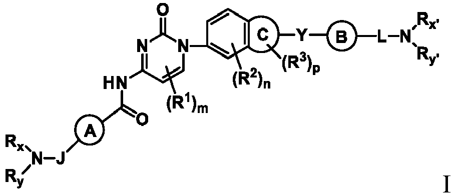 or a single stereoisomer or mixture of stereoisomers thereof, or a pharmaceutically acceptable salt thereof, wherein:
ring A is a 3-8 membered monocyclic heterocycloalkylene optionally substituted with up to three substituents selected from the group consisting of C₁-C₆ alkyl, C₁-C₆ alkoxy, halo, CN, C₁-C₆ haloalkyl, phenyl, OH, NH₂, NH(C₁-C₆ alkyl), N(C₁-C₆ alkyl)₂, COOH, COO(C₁-C₆ alkyl), CONH₂, CONH(C₁-C₆ alkyl), CON(C₁-C₆ alkyl)₂, and oxo;
J is C₁-C₆ alkylene or C₃-C₈ cycloalkylene, either of which is optionally substituted with halo, OH, or C₁-C₆ alkoxy, wherein up to two methylene units of the C₁-C₆ alkylene are optionally and independently replaced with O, S, SO, SO₂, or C=O;
Rₓ, R_{y}, R_{x'}, and R_{y'} are each independently H, C₁-C₆ alkyl, or an amino protecting group;
Y is a bond or C₁-C₆ alkylene optionally substituted with OH, NH₂, CN, halo, or C₁-C₆ alkoxy, wherein up to two methylene units of the C₁-C₆ alkylene are optionally and independently replaced by O, NH, N-(C₁-C₆ alkyl), N-(C₁-C₆ hydroxyalkyl), N-(C₁-C₆ haloalkyl), N-(C₁₋₆alkylene-C₃₋₈cycloalkyl), NH(C=O), N-(C₁₋₆ alkyl)(C=O), or (C=O);
ring B is a 3-8 membered monocyclic cycloalkylene, 3-8 membered monocyclic heterocycloalkylene, 6-12 membered bicyclic cycloalkylene, or a 6-12 membered bicyclic heterocycloalkylene, each of which is optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), COONH₂, COONH(C₁-C₆ alkyl), COON(C₁-C₆ alkyl)₂, and C₁-C₆ hydroxyalkyl;
L is a bond or C₁-C₆ alkylene, wherein up to two methylene units of the C₁-C₆ alkylene may be independently replaced with O, NH, (C=O), NH(C=O), N-(C₁₋₆ alkyl)(C=O), (C=NH), NH(C=N), or N-(C₁₋₆ alkyl);
ring C, together with the phenyl ring to which it is fused, forms an 8-12 membered bicyclic arylene or an 8-12 membered bicyclic heteroarylene, wherein the bicyclic heteroarylene has 1-3 heteroatoms independently selected from N, O, or S;
R¹, R², and R³ are each independently selected from the group consisting of C₁-C₆ alkyl, halo, CN, OH, NH₂, NH(C₁-C₆ alkyl), N(C₁-C₆ alkyl)₂, COO(C₁-C₆ alkyl), COONH₂, C₁-C₆ haloalkyl, oxo, and C₁-C₆ alkoxy; and
m, n, and p are each independently 0, 1, 2, or 3.

In one embodiment of a compound of formula I, or a single stereoisomer or mixture of stereoisomers thereof, or a pharmaceutically acceptable salt thereof, ring A is a 5-6 membered monocyclic heterocycloalkylene optionally substituted with up to three substituents selected from the group consisting of C₁-C₆ alkyl, C₁-C₆ alkoxy, halo, CN, C₁-C₆ haloalkyl, phenyl, OH, NH₂, and oxo.

In another embodiment of a compound of formula I, or a single stereoisomer or mixture of stereoisomers thereof, or a pharmaceutically acceptable salt thereof, ring A is 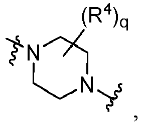 wherein each R⁴ is independently selected from the group consisting of C₁-C₆ alkyl, C₁-C₆ alkoxy, halo, CN, C₁-C₆ haloalkyl, phenyl, OH, NH₂, and oxo, wherein q is 0, 1, or 2.

In another embodiment of a compound of formula I, or a single stereoisomer or mixture of stereoisomers thereof, or a pharmaceutically acceptable salt thereof, ring A is

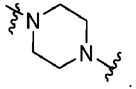

In another embodiment of a compound of formula I, or a single stereoisomer or mixture of stereoisomers thereof, or a pharmaceutically acceptable salt thereof, J is C₁-C₆ alkylene optionally substituted with halo, OH, or C₁-C₆ alkoxy, wherein up to two methylene units of the C₁-C₆ alkylene are optionally and independently replaced with O, S, SO, SO₂, or C=O.

In another embodiment of a compound of formula I, or a single stereoisomer or mixture of stereoisomers thereof, or a pharmaceutically acceptable salt thereof, J is C₁-C₆ alkylene optionally substituted with OH, wherein one methylene unit of the C₁-C₆ alkylene is replaced with C=O.

In another embodiment of a compound of formula I, or a single stereoisomer or mixture of stereoisomers thereof, or a pharmaceutically acceptable salt thereof, J is

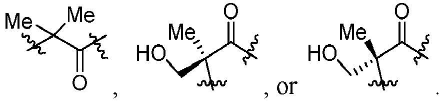

In another embodiment of a compound of formula I, or a single stereoisomer or mixture of stereoisomers thereof, or a pharmaceutically acceptable salt thereof, Rₓ and R_{y} are H. In another embodiment, Rₓ is H and R_{y} is C₁-C₆ alkyl. In another embodiment, Rₓ is H and R_{y} is an amino protecting group. In another embodiment, Rₓ is H and R_{y} is a Boc group or a methoxycarbonyl group. In another embodiment, Rₓ and R_{y} are each independently C₁-C₆ alkyl. In another embodiment, Rₓ and R_{y} are each independently selected from the group consisting of H, Boc, and methoxycarbonyl.

In another embodiment of a compound of formula I, or a single stereoisomer or mixture of stereoisomers thereof, or a pharmaceutically acceptable salt thereof, 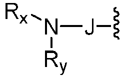 is

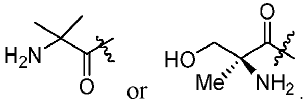

In another embodiment of a compound of formula I, or a single stereoisomer or mixture of stereoisomers thereof, or a pharmaceutically acceptable salt thereof,

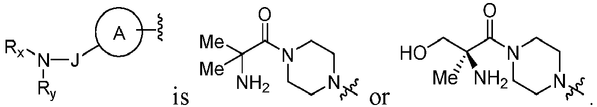

In another embodiment of a compound of formula I, or a single stereoisomer or mixture of stereoisomers thereof, or a pharmaceutically acceptable salt thereof, ring C, together with the phenyl ring to which it is fused, forms a 9-10 membered bicyclic arylene or a 9-10 membered bicyclic heteroarylene, wherein the bicyclic heteroarylene has 1-3 heteroatoms independently selected from N, O, and S.

In another embodiment, ring C, together with the phenyl ring to which it is fused, forms a 9-10 membered bicyclic arylene or a 9-10 membered bicyclic heteroarylene, wherein the bicyclic heteroarylene has 1-3 heteroatoms independently selected from N and O.

In another embodiment of a compound of formula I, or a single stereoisomer or mixture of stereoisomers thereof, or a pharmaceutically acceptable salt thereof, 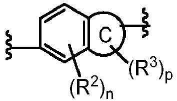 is selected from the group consisting of 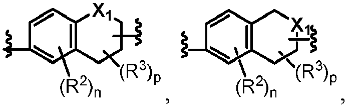 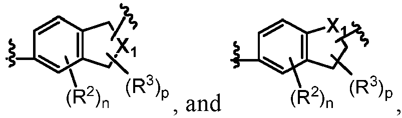 wherein each X₁ is independently selected from the group consisting of CH₂, CH, O, S, SO, SO₂, N, and NH; R² and R³ are each independently C₁-C₆ alkyl, halo, or C₁-C₆ haloalkyl; and n and p are each independently 0, 1, or 2. In another embodiment, each X₁ is independently selected from the group consisting of CH₂, CH, O, N, and NH; R² and R³ are each independently C₁-C₆ alkyl, halo, or C₁-C₆ haloalkyl; and n and p are each independently 0 or 1.

In another embodiment of a compound of formula I, or a single stereoisomer or mixture of stereoisomers thereof, or a pharmaceutically acceptable salt thereof, 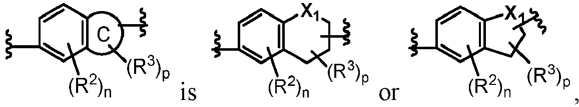 wherein X₁ is CH₂, O, S, SO, SO₂, or NH, and wherein R² and R³ are each independently C₁-C₆ alkyl, halo, or C₁-C₆ haloalkyl, wherein n and p are each independently 0, 1, or 2.

In another embodiment of a compound of formula I, or a single stereoisomer or mixture of stereoisomers thereof, or a pharmaceutically acceptable salt thereof, 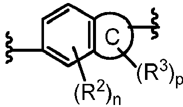 is selected from the group consisting of

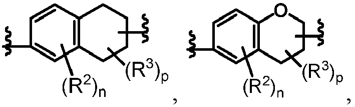

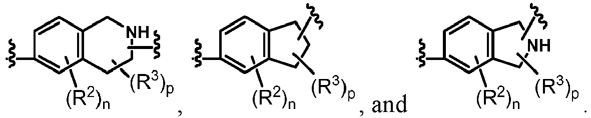

In another embodiment,

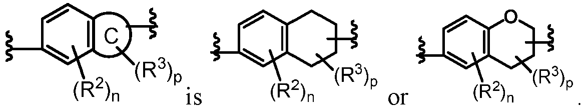

In another embodiment of a compound of formula I, or a single stereoisomer or mixture of stereoisomers thereof, or a pharmaceutically acceptable salt thereof, 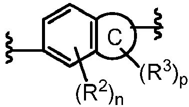 is selected from the group consisting of

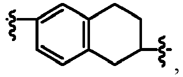

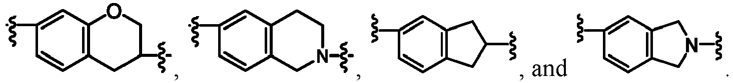

In another embodiment,

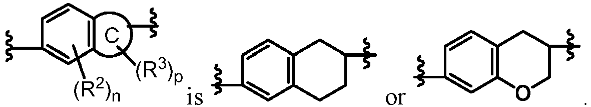

In another embodiment of a compound of formula I, or a single stereoisomer or mixture of stereoisomers thereof, or a pharmaceutically acceptable salt thereof, Y is a bond.

In another embodiment of a compound of formula I, or a single stereoisomer or mixture of stereoisomers thereof, or a pharmaceutically acceptable salt thereof, Y is C₁-C₃ alkylene optionally substituted with OH, NH₂, halo, or C₁-C₆ alkoxy, and wherein one methylene unit of the C₁-C₃ alkylene is optionally replaced by O, NH, N(C₁-C₆ alkyl), N(C₁-C₆ hydroxyalkyl), N(C₁-C₆ haloalkyl), N(C₁₋₆ alkylene-C₃₋₈ cycloalkyl), NH(C=O), N(C₁₋₆ alkyl)(C=O), or (C=O).

In another embodiment of a compound of formula I, or a single stereoisomer or mixture of stereoisomers thereof, or a pharmaceutically acceptable salt thereof, Y is selected from the group consisting of O, NH, NH-C₁₋₂ alkylene, N(C₁-C₆ alkyl), N(C₁-C₆ hydroxyalkyl), N(C₁-C₆ haloalkyl), N(C₁₋₆ alkylene-C₃₋₈cycloalkyl), NH(C=O), N(C₁₋₆ alkyl) (C=O), and (C=O).

In another embodiment, Y is O, NH, N(C₁-C₆ alkyl), N(C₁-C₆ hydroxyalkyl), N(C₁-C₆ haloalkyl), N(C₁₋₆ alkylene-C₃₋₈cycloalkyl), NH(C=O), N(C₁₋₆ alkyl) (C=O), or (C=O).

In another embodiment of a compound of formula I, or a single stereoisomer or mixture of stereoisomers thereof, or a pharmaceutically acceptable salt thereof, Y is -NH-, - N(C₁-C₆ alkyl)-, or -N(C₁₋₆ alkylene-C₃₋₈cycloalkyl)-.

In another embodiment of a compound of formula I, or a single stereoisomer or mixture of stereoisomers thereof, or a pharmaceutically acceptable salt thereof, Y is selected from the group consisting of -NH-, -N(C₁-C₃ alkyl)-, -NH-C₁₋₂ alkylene-, and -N(C₁₋₃ alkylene-C₃₋₆cycloalkyl)-. In another embodiment, Y is selected from the group consisting of -NH-, -NMe-, -NEt-, -NH-CH₂-, and -N(CH₂-cyclopropyl)-.

In another embodiment, Y is selected from the group consisting of a bond, -NH-, - N(C₁-C₃ alkyl)-, -NH-C₁₋₂ alkylene-, and -N(C₁₋₃ alkylene-C₃₋₆cycloalkyl)-. In another embodiment, Y is selected from the group consisting of a bond, -NH-, -NMe-, -NEt-, -NH-CH₂-, and -N(CH₂-cyclopropyl)-.

In another embodiment of a compound of formula I, or a single stereoisomer or mixture of stereoisomers thereof, or a pharmaceutically acceptable salt thereof, 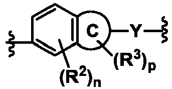 is selected from the group consisting of

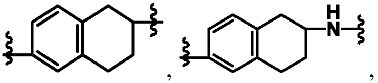

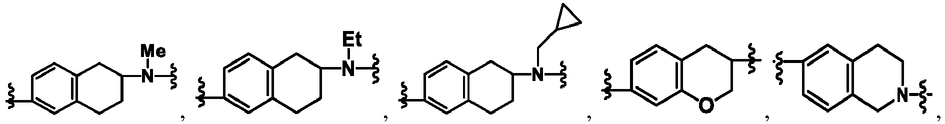

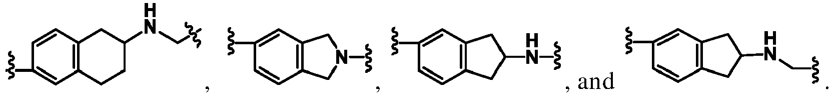

In another embodiment,

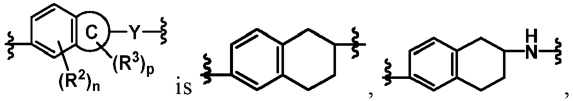

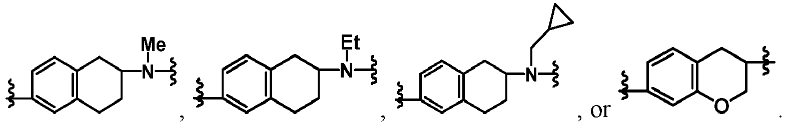

In another embodiment of a compound of formula I or a single stereoisomer or mixture of stereoisomers thereof, or a pharmaceutically acceptable salt thereof, ring B is a 4-7 membered monocyclic cycloalkylene, 4-7 membered monocyclic heterocycloalkylene, 6-10 membered bicyclic cycloalkylene, or a 6-10 membered bicyclic heterocycloalkylene, each of which is optionally substituted with up to three substituents independently selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and C₁-C₆ hydroxyalkyl.

In another embodiment, ring B is a 4-7 membered monocyclic cycloalkylene, 4-7 membered monocyclic heterocycloalkylene, 6-10 membered bicyclic cycloalkylene, or a 6-10 membered bicyclic heterocycloalkylene.

In another embodiment, ring B is a 4-6 membered monocyclic cycloalkylene, 4-7 membered monocyclic heterocycloalkylene, or 6-7 membered bicyclic cycloalkylene, or a 6-7 membered bicyclic heterocycloalkylene.

In another embodiment of a compound of formula I, or a single stereoisomer or mixture of stereoisomers thereof, or a pharmaceutically acceptable salt thereof, ring B is a 3-8 membered monocyclic cycloalkylene optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and C₁-C₆ hydroxyalkyl.

In another embodiment of a compound of formula I, or a single stereoisomer or mixture of stereoisomers thereof, or a pharmaceutically acceptable salt thereof, ring B is a 4-6 membered monocyclic cycloalkylene optionally substituted with up to three substituents selected from the group consisting of C₁-C₆ alkyl, C₁-C₆ alkoxy, halo, CN, C₁-C₆ haloalkyl, OH, and C₁-C₆ hydroxyalkyl.

In another embodiment, ring B is a 3-8 membered monocyclic cycloalkylene. In another embodiment, ring B is a 4-7 membered monocyclic cycloalkylene. In another embodiment, ring B is selected from the group consisting of cyclobutylene, cyclopentylene, and cyclohexylene.

In another embodiment of a compound of formula I, or a single stereoisomer or mixture of stereoisomers thereof, or a pharmaceutically acceptable salt thereof, ring B is a 3-8 membered monocyclic heterocycloalkylene optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and C₁-C₆ hydroxyalkyl.

In another embodiment of a compound of formula I, or a single stereoisomer or mixture of stereoisomers thereof, or a pharmaceutically acceptable salt thereof, ring B is a 4-7 membered monocyclic heterocycloalkylene optionally substituted with up to three substituents selected from the group consisting of C₁-C₆ alkyl, C₁-C₆ alkoxy, halo, CN, C₁-C₆ haloalkyl, OH, and C₁-C₆ hydroxyalkyl, and ring B contains up to 2 nitrogen atoms.

In another embodiment, ring B is a 3-8 membered monocyclic heterocycloalkylene. In another embodiment, ring B is a 5-7 membered monocyclic heterocycloalkylene, and ring B contains 2 nitrogen atoms. In another embodiment, ring B is a 4-7 membered monocyclic heterocycloalkylene, wherein ring B contains 1 nitrogen atom. In another embodiment, ring B is a 5-7 membered monocyclic heterocycloalkylene, wherein ring B contains 1 nitrogen atom.

In another embodiment of a compound of formula I, or a single stereoisomer or mixture of stereoisomers thereof, or a pharmaceutically acceptable salt thereof, ring B is a 6-10 membered bicyclic cycloalkylene optionally substituted with up to three substituents selected from the group consisting of C₁-C₆ alkyl, C₁-C₆ alkoxy, halo, C₁-C₆ haloalkyl, OH, and C₁-C₆ hydroxyalkyl.

In another embodiment of a compound of formula I, or a single stereoisomer or mixture of stereoisomers thereof, or a pharmaceutically acceptable salt thereof, ring B is a 6-10 membered fused, spiro, or bridged bicyclic cycloalkylene.

In another embodiment of a compound of formula I, or a single stereoisomer or mixture of stereoisomers thereof, or a pharmaceutically acceptable salt thereof, ring B is a 6-10 membered fused bicyclic cycloalkylene.

In another embodiment of a compound of formula I, or a single stereoisomer or mixture of stereoisomers thereof, or a pharmaceutically acceptable salt thereof, ring B is a 6-10 membered bridged bicyclic cycloalkylene.

In another embodiment of a compound of formula I, or a single stereoisomer or mixture of stereoisomers thereof, or a pharmaceutically acceptable salt thereof, ring B is a 6-10 membered spiro bicyclic cycloalkylene. In another embodiment, ring B is a 7 membered spiro bicyclic cycloalkylene.

In another embodiment of a compound of formula I, or a single stereoisomer or mixture of stereoisomers thereof, or a pharmaceutically acceptable salt thereof, ring B is a 6-12 membered bicyclic heterocycloalkylene optionally substituted with up to three substituents selected from the group consisting of C₁-C₆ alkyl, C₁-C₆ alkoxy, halo, C₁-C₆ haloalkyl, OH, and C₁-C₆ hydroxyalkyl. In another embodiment, ring B is a 6-12 membered bicyclic heterocycloalkylene.

In another embodiment of a compound of formula I, or a single stereoisomer or mixture of stereoisomers thereof, or a pharmaceutically acceptable salt thereof, ring B is a 6-12 membered fused, spiro, or bridged bicyclic heterocycloalkylene containing up to 3 nitrogen atoms.

In another embodiment of a compound of formula I, or a single stereoisomer or mixture of stereoisomers thereof, or a pharmaceutically acceptable salt thereof, ring B is a 6-12 membered fused bicyclic heterocycloalkylene containing up to 2 nitrogen atoms. In another embodiment, ring B is a 6-10 membered fused bicyclic heterocycloalkylene containing 2 nitrogen atoms. In another embodiment, ring B is a 6-10 membered fused bicyclic heterocycloalkylene containing 1 nitrogen atom. In another embodiment, ring B is a 6 membered fused bicyclic heterocycloalkylene containing 1 nitrogen atom.

In another embodiment of a compound of formula I, or a single stereoisomer or mixture of stereoisomers thereof, or a pharmaceutically acceptable salt thereof, ring B is a spiro bicyclic heterocycloalkylene containing up to 2 nitrogen atoms.

In another embodiment of a compound of formula I, or a single stereoisomer or mixture of stereoisomers thereof, or a pharmaceutically acceptable salt thereof, ring B is a bridged bicyclic heterocycloalkylene containing up to 2 nitrogen atoms.

In another embodiment of a compound of formula I, or a single stereoisomer or mixture of stereoisomers thereof, or a pharmaceutically acceptable salt thereof, ring B is selected from the group consisting of:

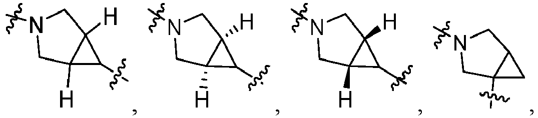

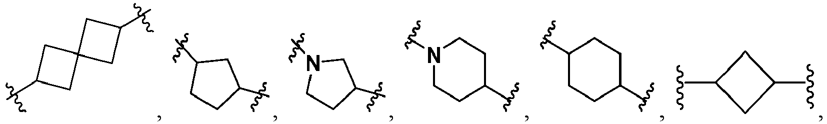

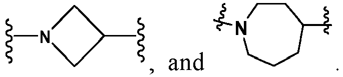

In another embodiment, ring B is selected from the group consisting of:

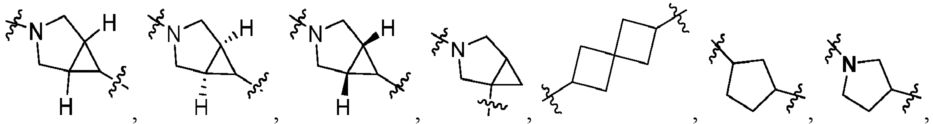

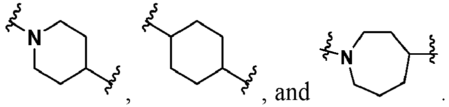

In another embodiment of a compound of formula I, or a single stereoisomer or mixture of stereoisomers thereof, or a pharmaceutically acceptable salt thereof, L is a bond.

In another embodiment of a compound of formula I, or a single stereoisomer or mixture of stereoisomers thereof, or a pharmaceutically acceptable salt thereof, L is C₁-C₆ alkylene, wherein up to two methylene units of the C₁-C₆ alkylene are optionally and independently replaced with O, NH, (C=O), NH(C=O), N-(C₁₋₆ alkyl)(C=O), (C=NH), NH(C=N), or N-(C₁₋₆ alkyl).

In another embodiment of a compound of formula I, or a single stereoisomer or mixture of stereoisomers thereof, or a pharmaceutically acceptable salt thereof, L is C₁-C₆ alkylene.

In another embodiment, L is -CH₂- or -CH₂CH₂-. In another embodiment, L is - CH₂-. In another embodiment, L is -CH₂CH₂-.

In another embodiment, L is a bond or C₁-C₃ alkylene. In another embodiment, L is a bond, -CH₂-, or -CH₂-CH₂-.

In another embodiment of a compound of formula I, or a single stereoisomer or mixture of stereoisomers thereof, or a pharmaceutically acceptable salt thereof, R_{x'} and R_{y'} are H. In another embodiment, R_{x'} is H and R_{y'} is C₁-C₆ alkyl. In another embodiment, R_{x'} is H and R_{y'} is an amino protecting group. In another embodiment, R_{x'} and R_{y'} are each independently C₁-C₆ alkyl. In another embodiment, R_{x'} and R_{y'} are each independently selected from the group consisting of H, Boc, and methoxycarbonyl. In another embodiment, R_{x'} and R_{y'} are each independently selected from the group consisting of H and methoxycarbonyl.

In another embodiment, Y is C₁-C₃ alkylene, wherein one methylene unit of the C₁-C₃ alkylene is optionally replaced by -NH-, -N(C₁-C₆ alkyl)-, or -N(C₁₋₆ alkylene-C₃₋scycloalkyl)-; ring B is a 4-6 membered monocyclic cycloalkylene, 4-7 membered monocyclic heterocycloalkylene, 6-9 membered bicyclic cycloalkylene, or 6-9 membered bicyclic heterocycloalkylene; L is a bond or C₁-C₃ alkylene; and R_{x'} and R_{y'} are each independently H or an amino protecting group.

In another embodiment, Y is selected from the group consisting of -NH-, -NMe-, - NEt-, -NH-CH₂-, and -N(CH₂-cyclopropyl)-; ring B is selected from the group consisting of 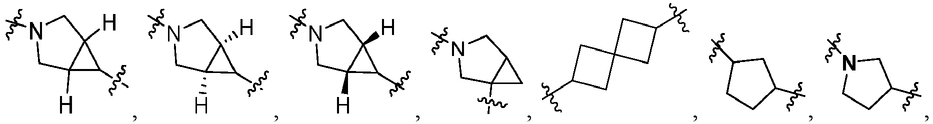 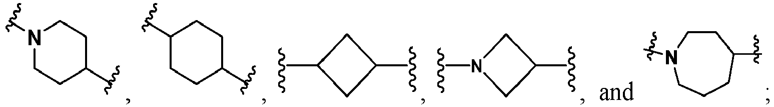 L is a bond, - CH₂-, or -CH₂-CH₂-; and R_{x'} and R_{y'} are each independently selected from the group consisting of H, Boc, and methoxycarbonyl.

In another embodiment of a compound of formula I, or a single stereoisomer or mixture of stereoisomers thereof, or a pharmaceutically acceptable salt thereof, 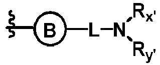 is selected from the group consisting of

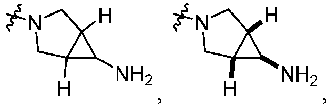

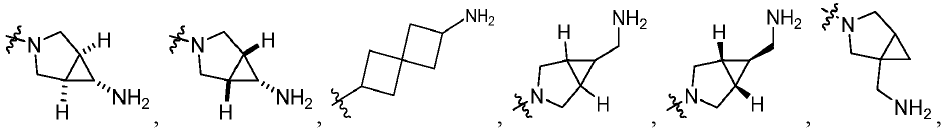

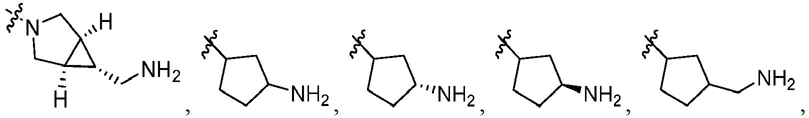

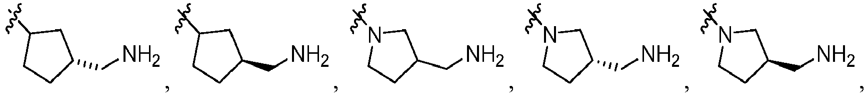

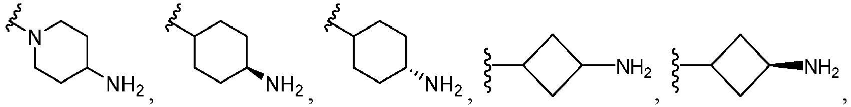

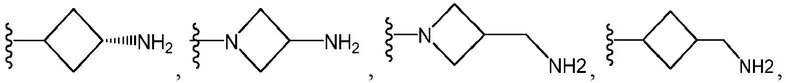

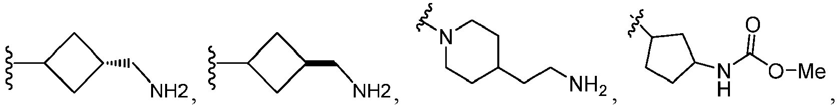

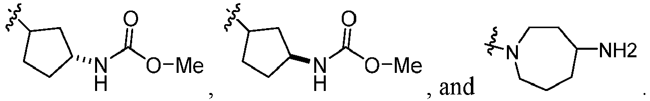

In another embodiment, 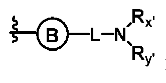 is selected from the group consisting of

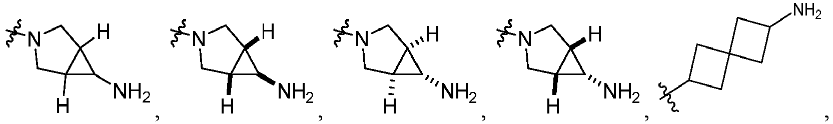

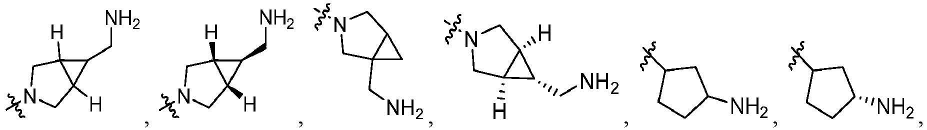

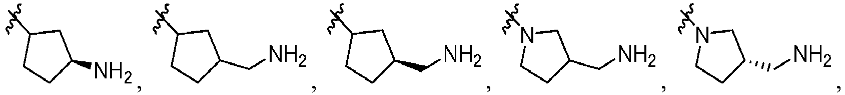

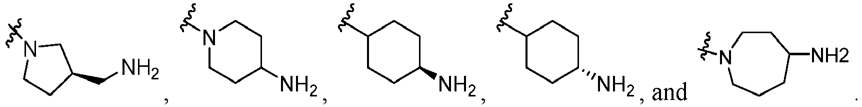

In another embodiment of a compound of formula I, or a single stereoisomer or mixture of stereoisomers thereof, or a pharmaceutically acceptable salt thereof, wherein R¹, R², and R³ are each independently selected from the group consisting of C₁-C₆ alkyl, halo, C₁-C₆ haloalkyl, oxo, and C₁-C₆ alkoxy, and m, n, and p are each independently 0, 1, or 2.

In another embodiment of a compound of formula I, or a single stereoisomer or mixture of stereoisomers thereof, or a pharmaceutically acceptable salt thereof, wherein R¹, R², and R³ are each independently C₁-C₆ alkyl, halo, oxo, or C₁-C₆ haloalkyl, and m, n, and p are each independently 0 or 1. In another embodiment, m, n, and p are 0.

In another embodiment, the compound of formula I is a compound of formula IA: 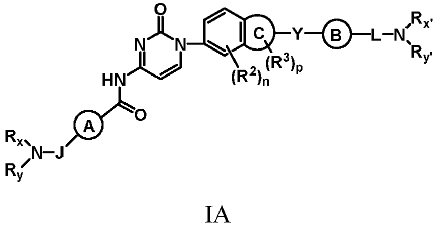 or a single stereoisomer or mixture of stereoisomers thereof, or a pharmaceutically acceptable salt thereof, wherein ring A, ring B, ring C, J, L, Y, R², R³, Rₓ, R_{y}, R_{x'}, R_{y'}, n, and p have the definitions as provided in the preceding paragraphs.

In another embodiment, the compound of formula I or formula IA is a compound of formula IA-1: 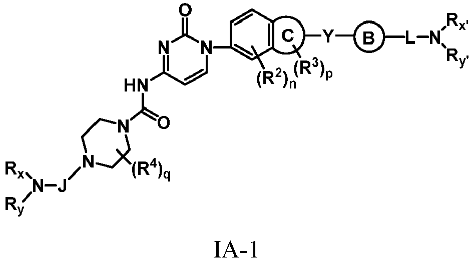 or a single stereoisomer or mixture of stereoisomers thereof, or a pharmaceutically acceptable salt thereof, wherein ring B, ring C, J, L, Y, R², R³, Rₓ, R_{y}, R_{x'}, R_{y'}, n, and p have the definitions as provided in the preceding paragraphs; each R⁴ is independently selected from the group consisting of H, C₁-C₆ alkyl, C₁-C₆ alkoxy, halo, CN, C₁-C₆ haloalkyl, OH, NH₂, and oxo; and q is 0, 1, 2, or 3.

In another embodiment, the compound of formula I, IA, or IA-1 is a compound of formula IA-2: 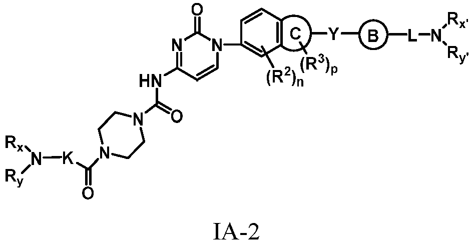 or a single stereoisomer or mixture of stereoisomers thereof, or a pharmaceutically acceptable salt thereof, wherein ring B, ring C, L, Y, R², R³, Rₓ, R_{y}, R_{x'}, R_{y'}, n, and p have the definitions as provided in the preceding paragraphs, wherein K is C₁-C₄ alkylene optionally substituted with halo, hydroxyl or C₁-C₆ alkoxy group.

In another embodiment, the compound of formula I, IA, IA-1, or IA-2 is a compound of formula IA-3: 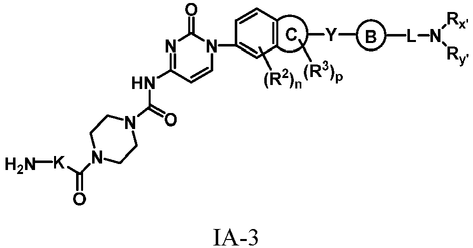 or a single stereoisomer or mixture of stereoisomers thereof, or a pharmaceutically acceptable salt thereof, wherein ring B, ring C, L, Y, R², R³, Rₓ, R_{y}, R_{x'}, R_{y'}, n, and p have the definitions as provided in the preceding paragraphs, wherein K is C₁-C₃ alkylene optionally substituted with hydroxyl.

In another embodiment, the compound of formula I, IA, IA-1, IA-2, or IA-3 is a compound of formula IA-4a or IA-4b: 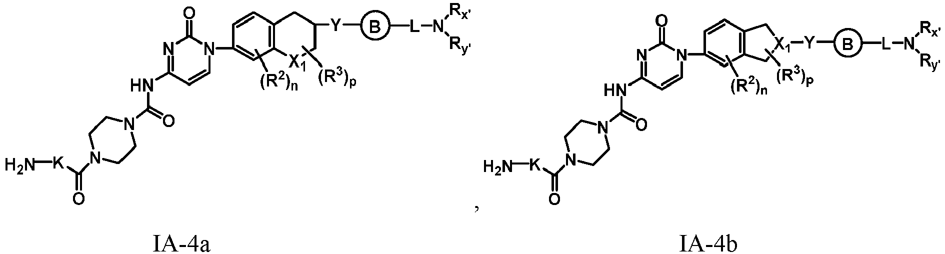 or a single stereoisomer or mixture of stereoisomers thereof, or a pharmaceutically acceptable salt thereof, wherein ring B, L, K, Y, R², R³, R_{x'}, R_{y'}, n, and p have the definitions as provided in the preceding paragraphs, wherein each X₁ is independently selected from the group consisting of CH₂, CH, O, S, N, and NH.

In another embodiment, the compound of formula I, IA, IA-1, IA-2, IA-3, IA-4a, or IA-4b is a compound of formula IA-5a or IA-5b: 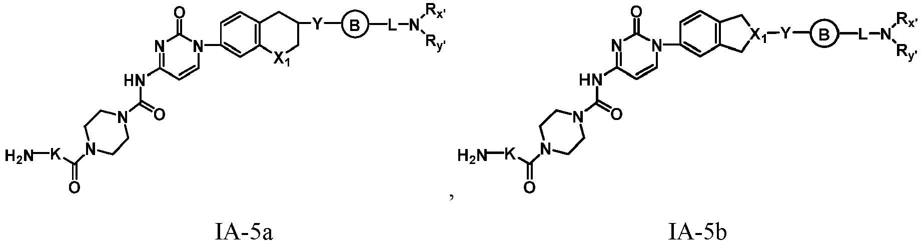 or a single stereoisomer or mixture of stereoisomers thereof, or a pharmaceutically acceptable salt thereof, wherein ring B, L, K, Y, R_{x'}, and R_{y'} have the definitions as provided in the preceding paragraphs, wherein each X₁ is independently selected from the group consisting of CH₂, CH, N, NH, and O. In another embodiment, each X₁ is independently selected from the group consisting of CH₂ and O.

In another embodiment, the compound of formula I, IA, IA-1, IA-2, IA-3, IA-4a, IA-4b, IA-5a, or IA-5b is a compound of formula IA-6a or IA-6b: 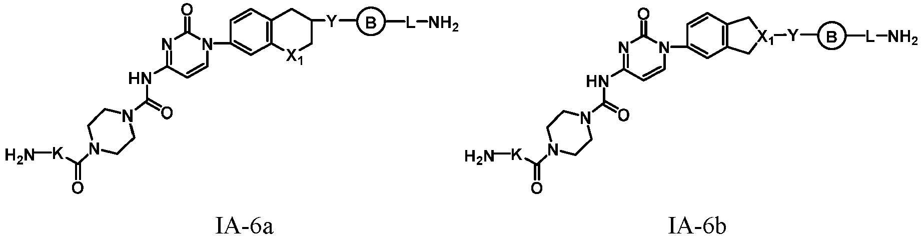 or a single stereoisomer or mixture of stereoisomers thereof, or a pharmaceutically acceptable salt thereof, wherein ring B, L, K, Y, and X₁ have the definitions as provided in the preceding paragraphs.

In another embodiment, the compound of formula I, IA, IA-1, IA-2, IA-3, IA-4a, IA-4b, IA-5a, IA-5b, IA-6a, or IA-6b is a compound of formula IA-7a, formula IA-7b, formula IA-7c, formula IA-7d, formula IA-7e, or formula IA-7f: 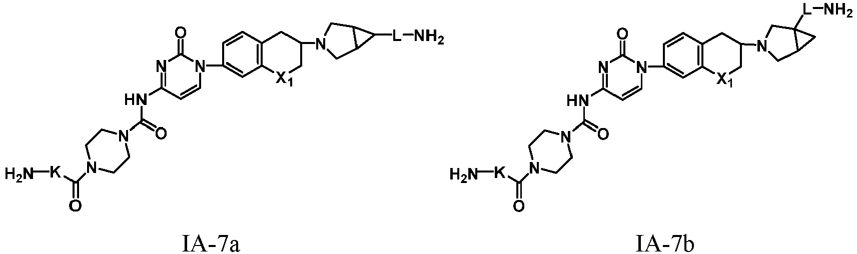 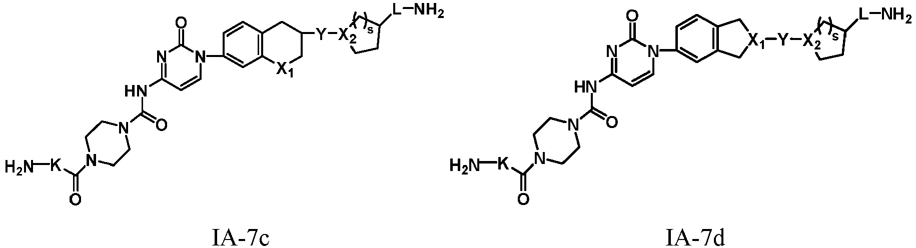 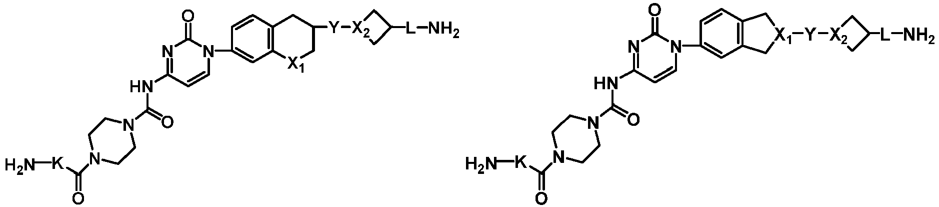 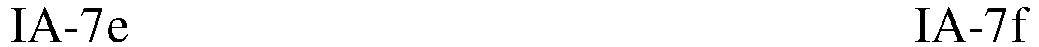 or a single stereoisomer or mixture of stereoisomers thereof, or a pharmaceutically acceptable salt thereof, wherein L, K, Y, and X₁ have the definitions as provided in the preceding paragraphs; each X₂ is independently CH or N; and each s is independently 1, 2, or 3.

In one embodiment, K is

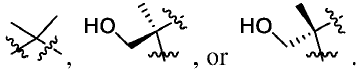

In one embodiment, Y is a bond, -NH-, -NH-(C₁-C₅ alkylene)-, -N(C₁-C₆ alkyl)-, or -N(C₁-C₆ alkylene-C₃-C₈ cycloalkyl)-.

In one embodiment, Y is -NH-, -N(C₁-C₆ alkyl)-, or -N(C₁-C₆ alkylene-C₃-C₈ cycloalkyl)-.

In another embodiment, L is a bond or C₁-C₆ alkylene. In another embodiment, L is C₁-C₆ alkylene.

In another embodiment, the compound of formula I, IA, IA-1, IA-2, IA-3, IA-4a, IA-5a, IA-6a, or IA-7a is a compound of formula IA-7a-1, formula IA-7a-2, formula IA-7a-3, formula IA-7a-4, formula IA-7a-5, formula IA-7a-6, formula IA-7a-7, or formula IA-7a-8: 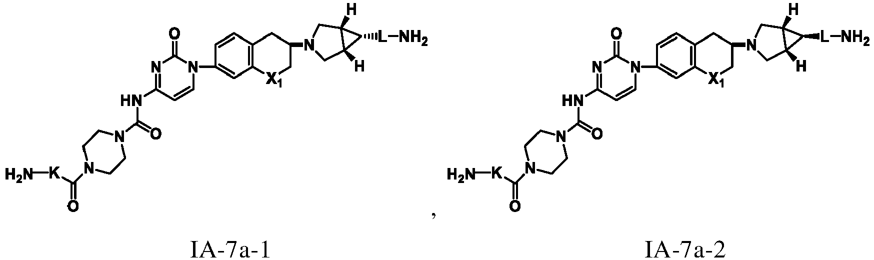 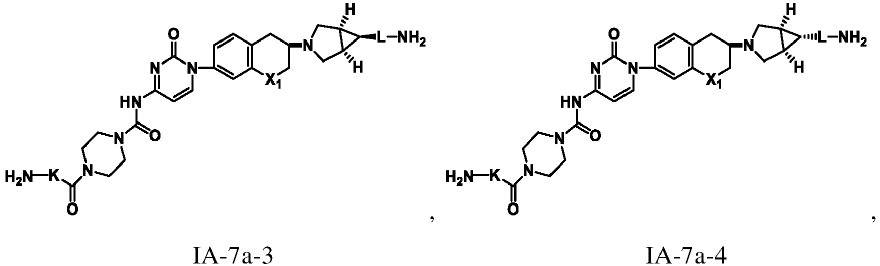 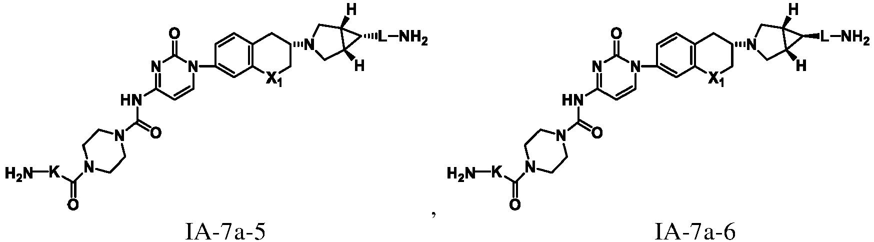 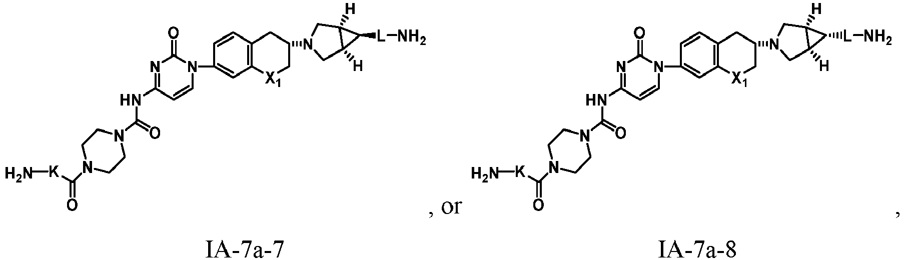 or a pharmaceutically acceptable salt thereof, wherein K, X₁, and L are as defined herein.

In another embodiment, the compound of formula I, IA, IA-1, IA-2, IA-3, IA-4a, IA-5a, IA-6a, or IA-7c is a compound of formula IA-7c-1, formula IA-7c-2, formula IA-7c-3, formula IA-7c-4, formula IA-7c-5, formula IA-7c-6, formula IA-7c-7, or formula IA-7c-8: 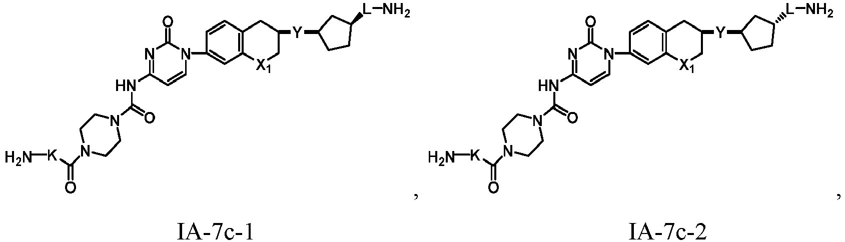 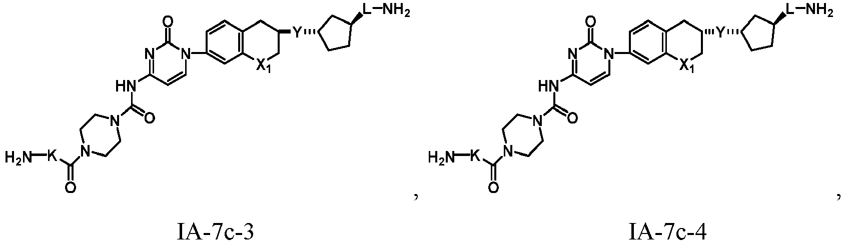 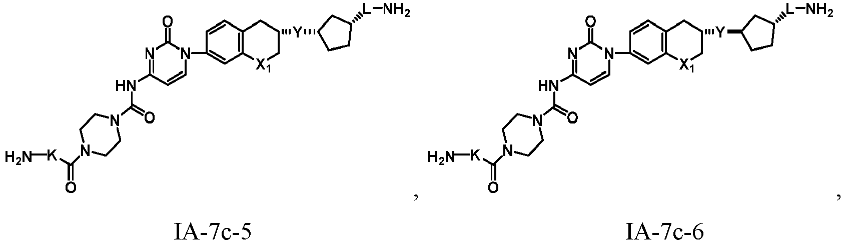 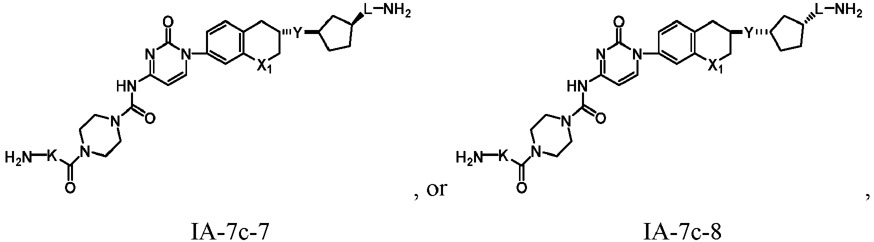 or a pharmaceutically acceptable salt thereof, wherein K, X₁, Y, and L are as defined herein.

In another embodiment, the compound of formula I, IA, IA-1, IA-2, IA-3, IA-4a, IA-5a, IA-6a, or IA-7c is a compound of formula IA-7c-9, formula IA-7c-10, formula IA-7c-11, or formula IA-7c-12: 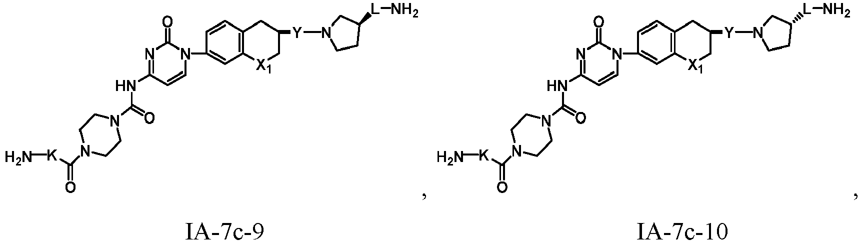 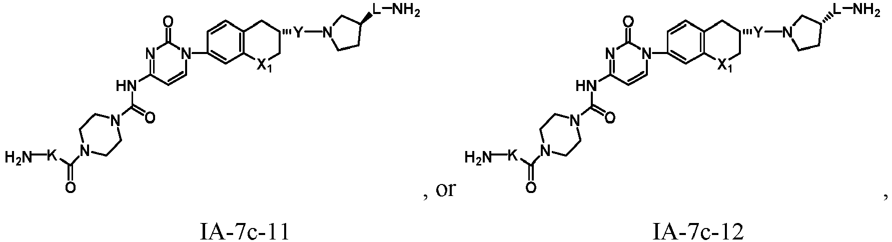 or a pharmaceutically acceptable salt thereof, wherein K, X₁, Y, and L are as defined herein.

In another embodiment, the compound of formula I, IA, IA-1, IA-2, IA-3, IA-4a, IA-5a, IA-6a, or IA-7c is a compound of formula IA-7c-13, formula IA-7c-14, formula IA-7c-15, or formula IA-7c-16: 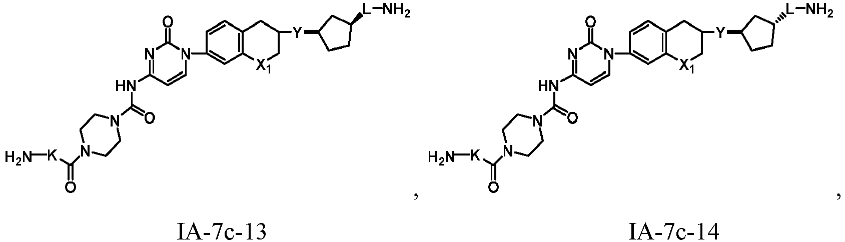 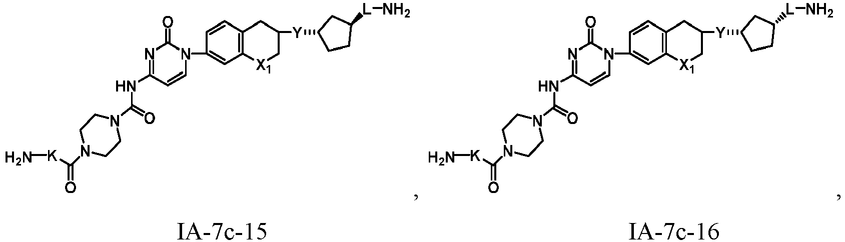 or a pharmaceutically acceptable salt thereof, wherein K, X₁, Y, and L are as defined herein.

In another embodiment, the compound of formula I, IA, IA-1, IA-2, IA-3, IA-4a, IA-5a, IA-6a, or IA-7c is a compound of formula IA-7c-17, formula IA-7c-18, formula IA-7c-19, formula IA-7c-20, formula IA-7c-21, formula IA-7c-22, formula IA-7c-23, or formula IA-7c-24: 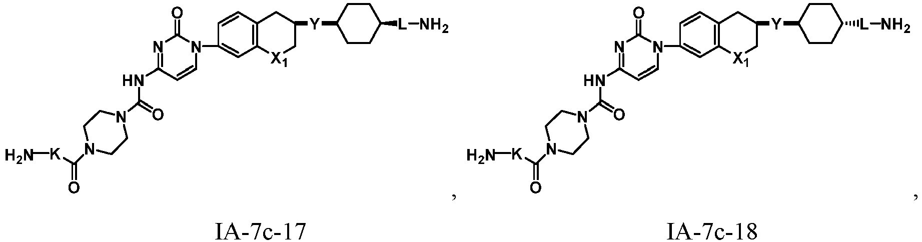 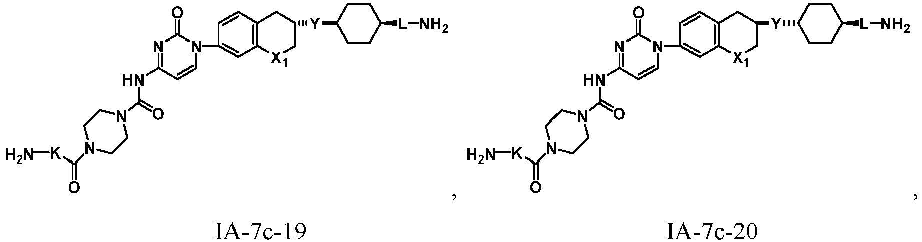 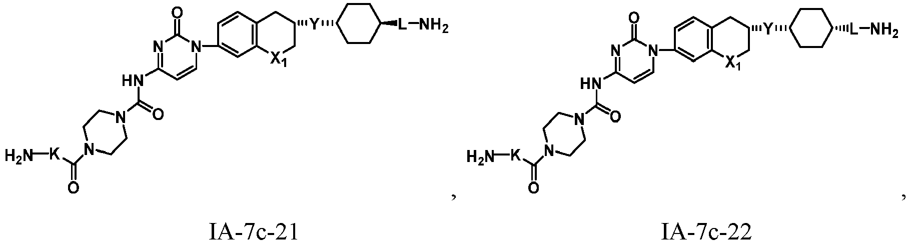 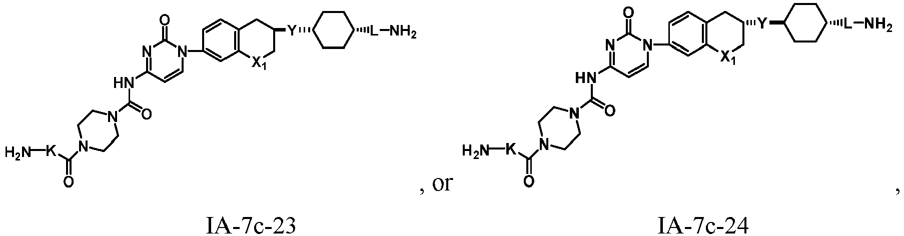 or a pharmaceutically acceptable salt thereof, wherein K, X₁, Y, and L are as defined herein.

In another embodiment, the compound of formula I, IA, IA-1, IA-2, IA-3, IA-4a, IA-5a, IA-6a, or IA-7c is a compound of formula IA-7c-25, formula IA-7c-26, formula IA-7c-27, or formula IA-7c-28: 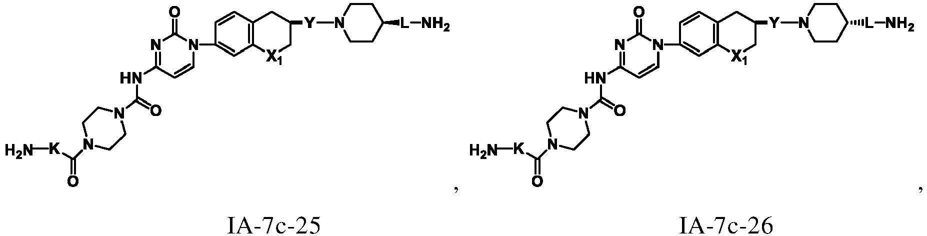 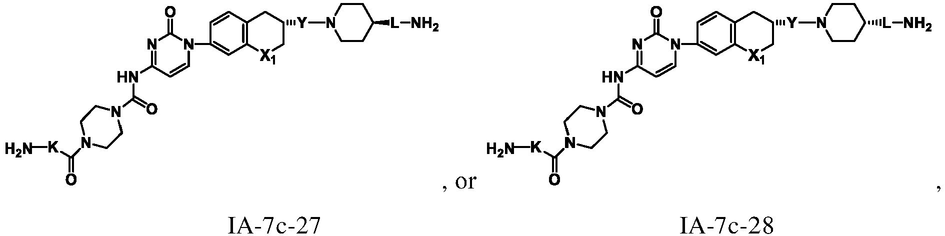 or a pharmaceutically acceptable salt thereof, wherein K, X₁, Y, and L are as defined herein.

In one embodiment, K is C₁-C₃ alkylene optionally substituted with hydroxyl; each X₁ is independently selected from the group consisting of CH₂, CH, NH, N, and O; Y is a bond, NH, -NH(C₁-C₆ alkylene)-, -N(C₁-C₆ alkyl)-, or -N(C₁-C₆ alkylene-C₃-C₈ cycloalkyl)-; L is a bond or C₁-C₃ alkylene.

In one embodiment, K is 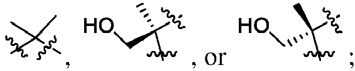 each X₁ is independently selected from the group consisting of CH₂, CH, NH, N, and O; Y is selected from the group consisting of a bond, -NH-, -NMe-, -NEt-, -NH-CH₂-, and -N(CH₂-cyclopropyl)-; and L is a bond, -CH₂- or -CH₂CH₂-.

In one embodiment, K is 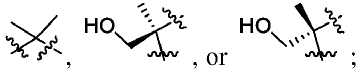 each X₁ is independently selected from the group consisting of CH₂, CH, NH, N, and O; Y is selected from the group consisting of a bond, -NH-, -NMe-, -NEt-, -NH-CH₂-, and -N(CH₂-cyclopropyl)-; L is a bond, -CH₂- or -CH₂CH₂-; and ring B is selected from the group consisting of: 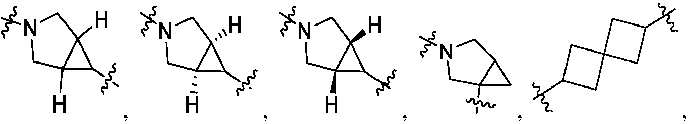 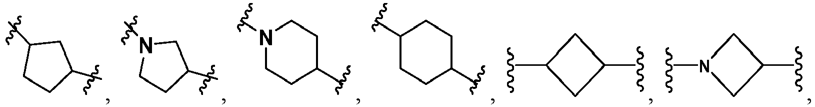 and

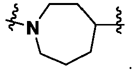

In another aspect, the disclosure provides a compound or a pharmaceutically acceptable salt thereof, which is listed in Table 1. In Table 1, both free base and salt structures of the compounds are depicted.

**Table 1. Compounds of Formula I**

| **No.** | **Free Base Structure** | **Salt Structure** |
|---|---|---|
| 1 | 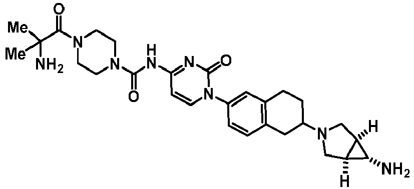 | 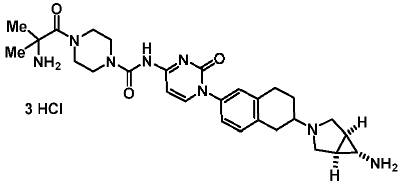 |
| 2 | 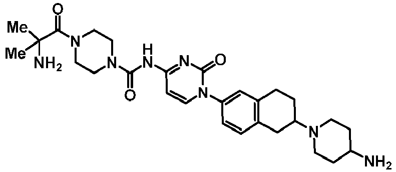 | 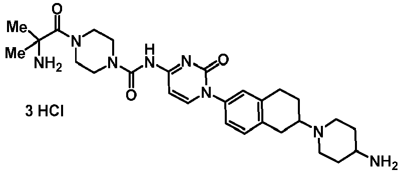 |
| 3 | 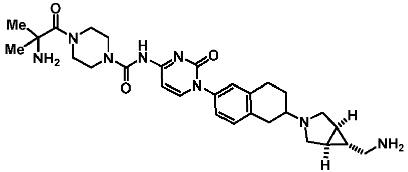 | 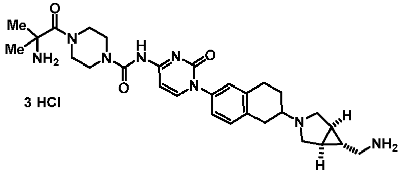 |
| 4 | 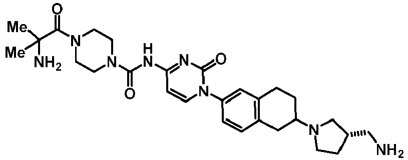 | 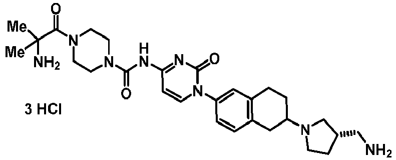 |
| 5 | 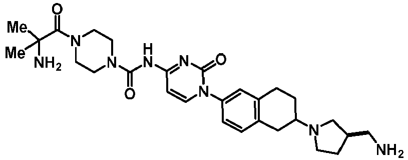 | 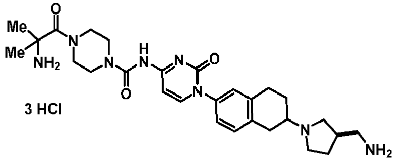 |
| 6 | 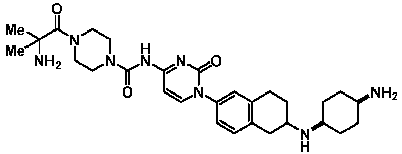 | 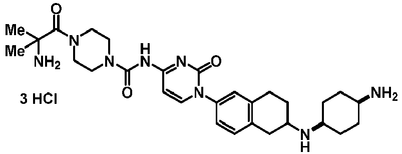 |
| 7 | 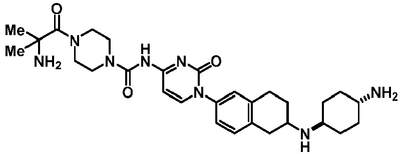 | 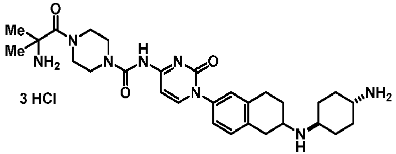 |
| 8 | 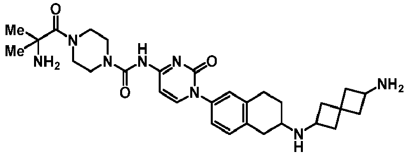 | 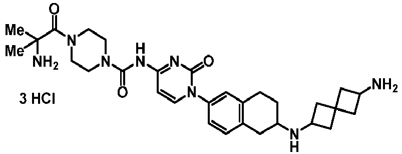 |
| 9 | 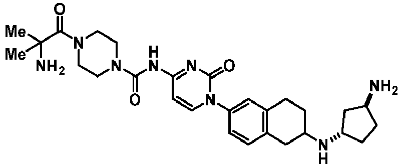 | 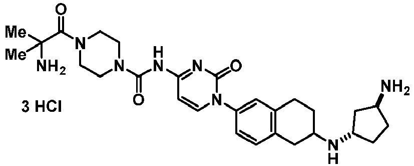 |
| 10 | 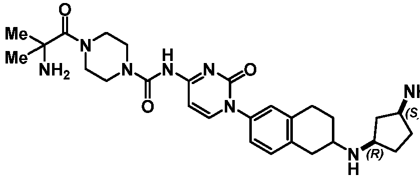 | 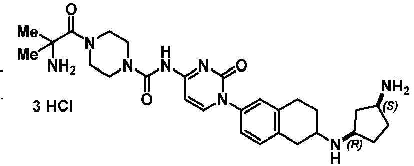 |
| 11 | 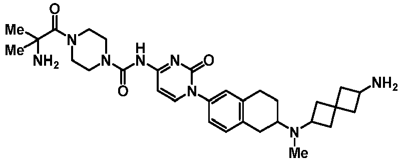 | 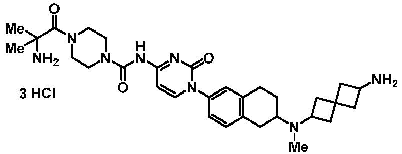 |
| 12 | 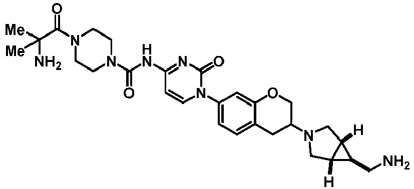 | 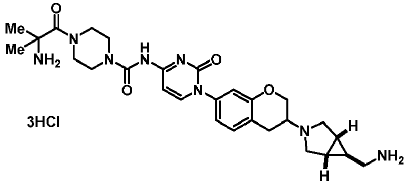 |
| 13 | 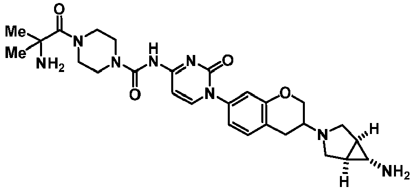 | 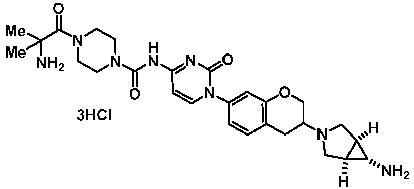 |
| 14 | 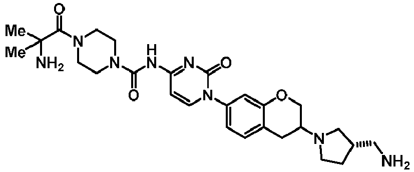 | 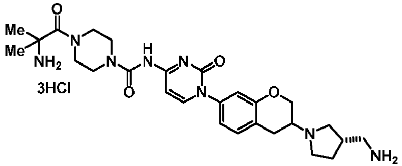 |
| 15 | 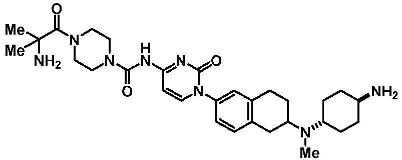 | 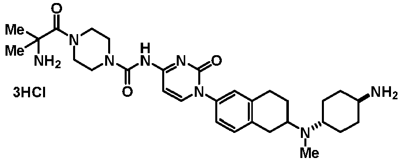 |
| 16 | 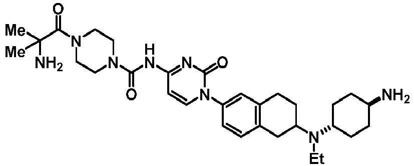 | 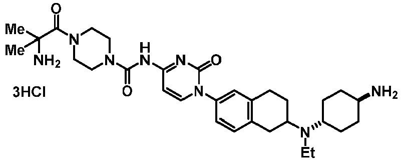 |
| 17 | 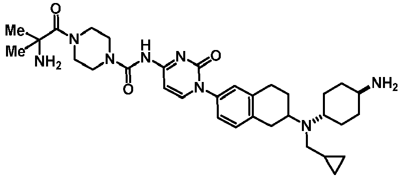 | 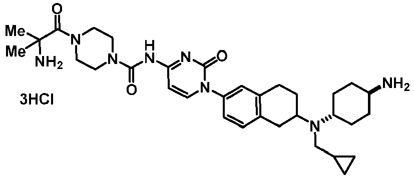 |
| 18 | 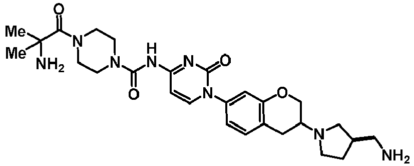 | 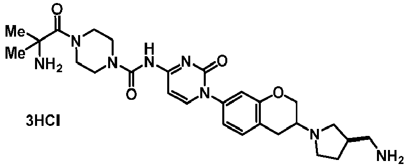 |
| 19 | 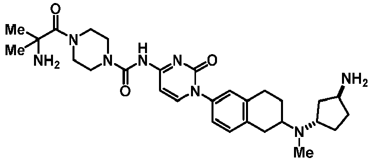 | 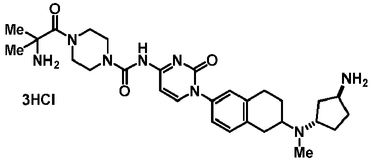 |
| 20 | 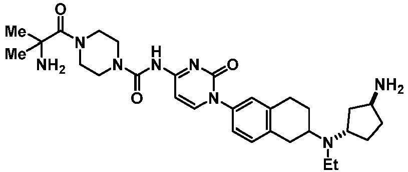 | 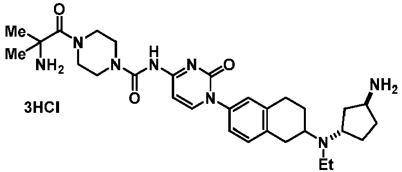 |
| 21 | 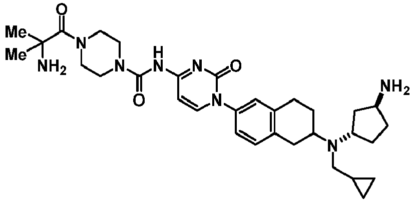 | 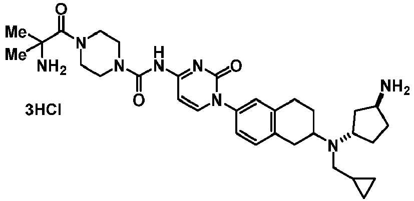 |
| 22 | 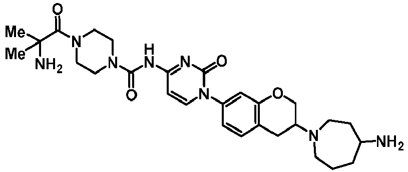 | 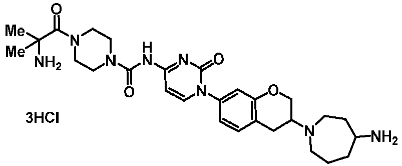 |
| 23 | 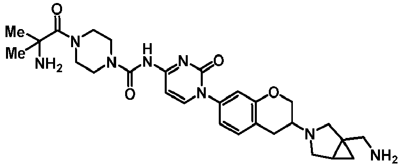 | 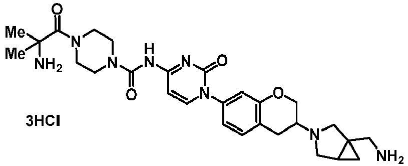 |
| 24 | 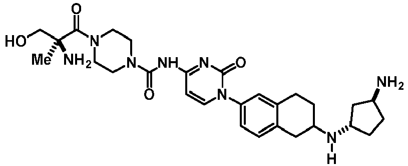 | 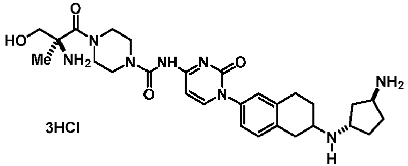 |
| 25 | 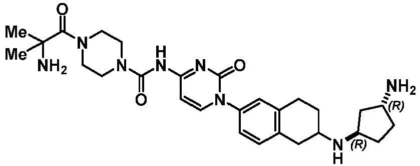 | 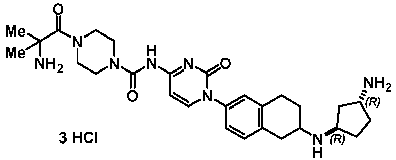 |
| 26 | 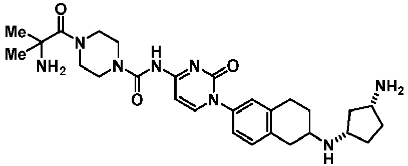 | 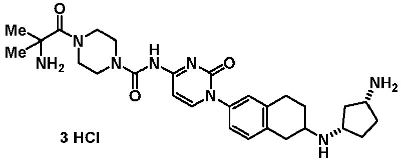 |
| 27 | 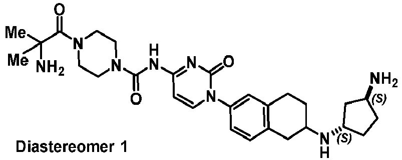 | 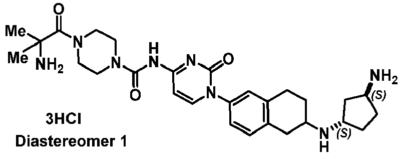 |
| 28 | 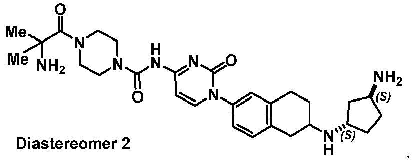 | 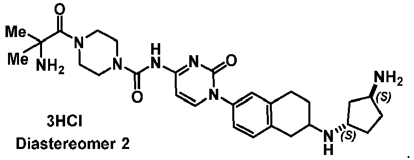 |

In another aspect, the disclosure provides a compound or a pharmaceutically acceptable salt thereof, which is listed in Table 2. In Table 2, both free base and salt structures of the compounds are depicted.

**Table 2. Compounds of Formula I Continued**

| **No.** | **Free Base Structure** | **Salt Structure** |
|---|---|---|
| 29 | 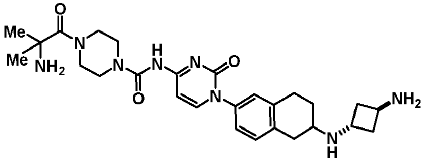 | 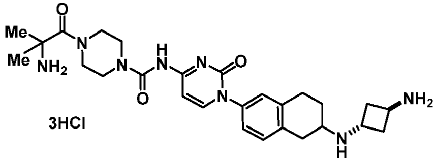 |
| 30 | 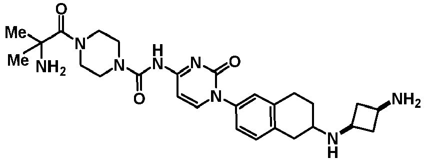 | 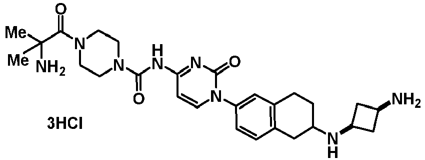 |
| 31 | 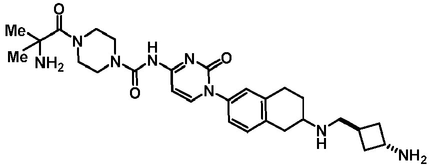 | 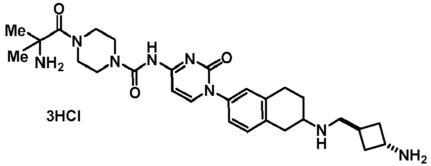 |
| 32 | 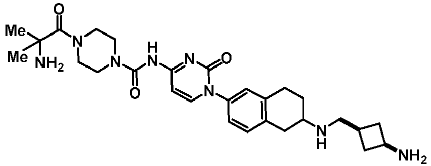 | 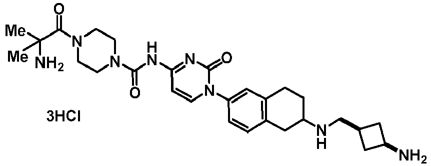 |
| 33 | 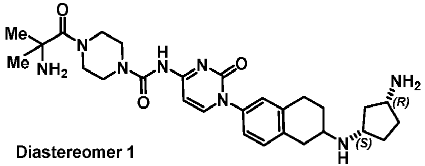 | 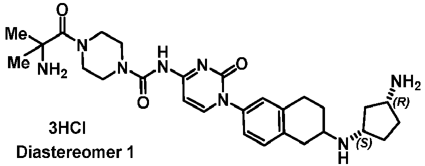 |
| 34 | 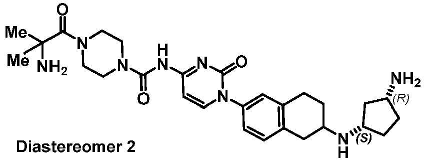 | 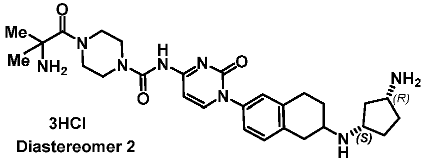 |
| 35 | 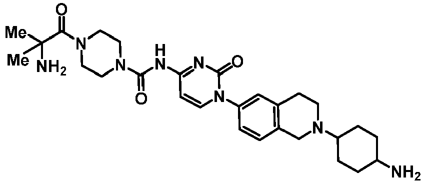 | 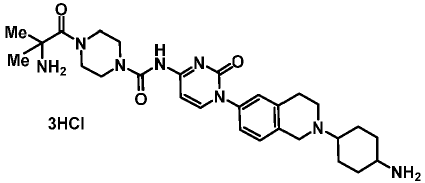 |
| 36 | 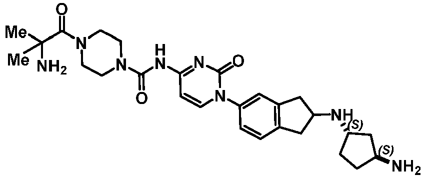 | 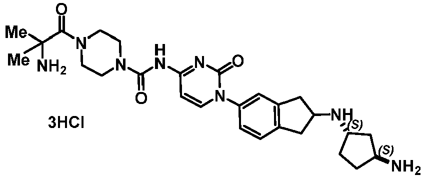 |
| 37 | 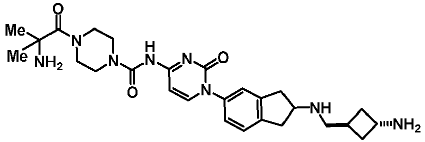 | 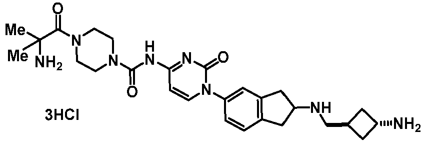 |
| 38 | 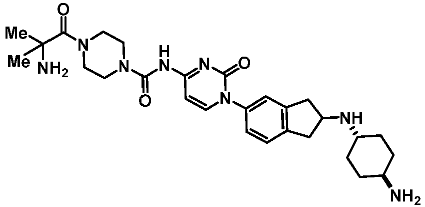 | 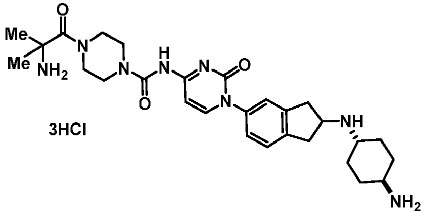 |
| 39 | 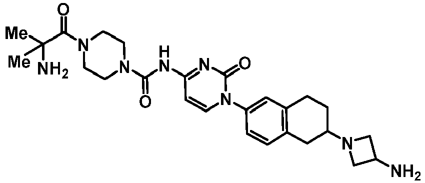 | 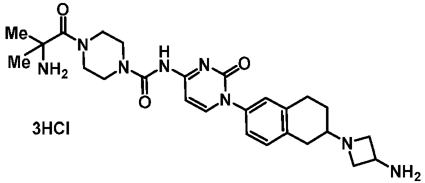 |
| 40 | 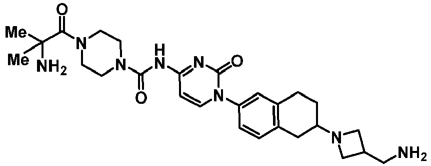 | 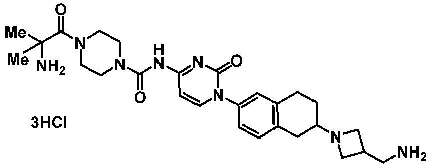 |
| 41 | 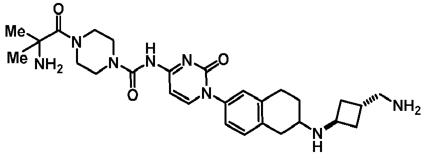 | 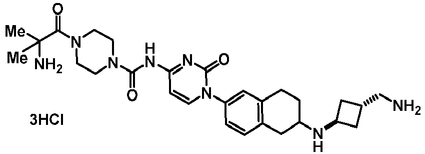 |
| 42 | 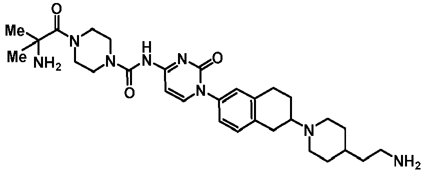 | 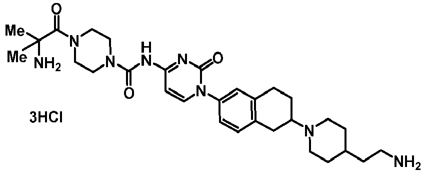 |
| 43 | 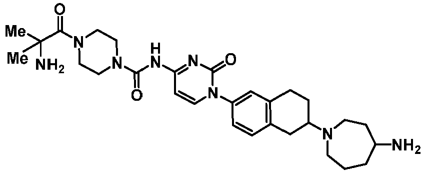 | 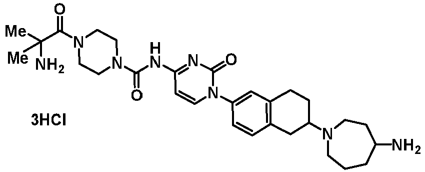 |
| 44 | 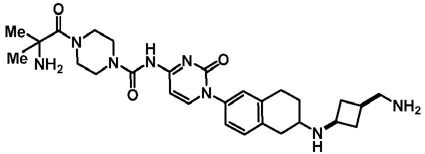 | 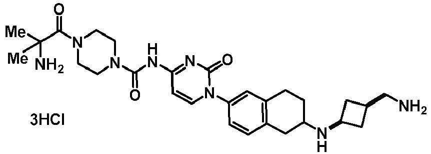 |
| 45 | 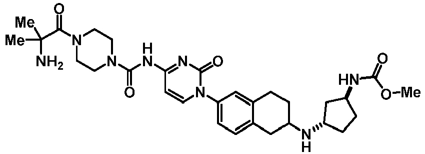 | 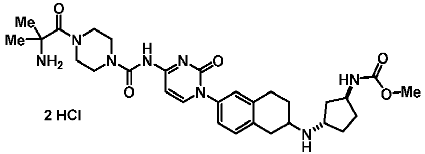 |
| 46 | 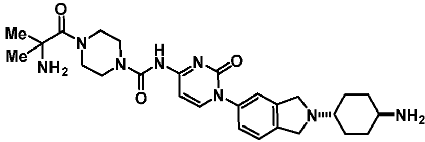 | 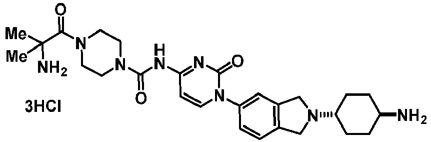 |

In another embodiment, the compound of formula I, or a single stereoisomer or mixture of stereoisomers thereof, or a pharmaceutically acceptable salt thereof is selected from the compounds listed in any one of Table 1 and Table 2.

In another aspect, the disclosure provides a compound of formula II: 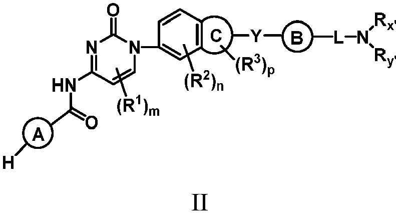 or a single stereoisomer or mixture of stereoisomers thereof, or a pharmaceutically acceptable salt thereof, wherein ring A, ring B, ring C, L, Y, R¹, R², R³, R_{x'}, R_{y'}, m, n, and p have the definitions as provided in the preceding paragraphs.

In one embodiment, the compound of formula II or a single stereoisomer or mixture of stereoisomers thereof, or a pharmaceutically acceptable salt thereof is selected from the compounds as depicted in Table 3 below.

**Table 3. Compounds of Formula II**

| **Compound Structure** | **Compound Structure** |
|---|---|
| 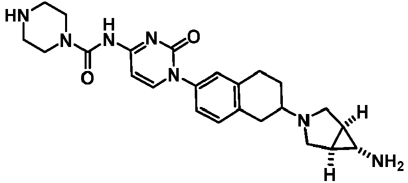 | 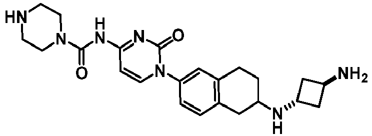 |
| 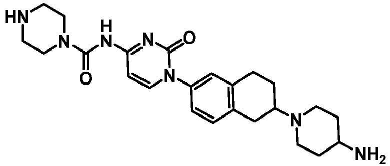 | 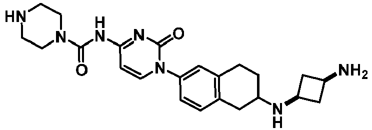 |
| 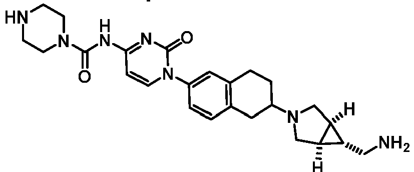 | 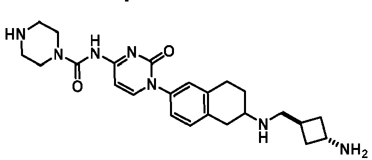 |
| 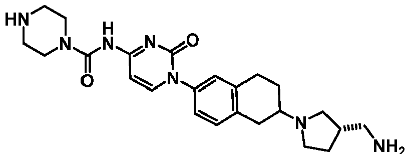 | 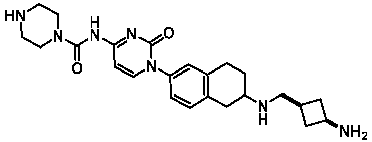 |
| 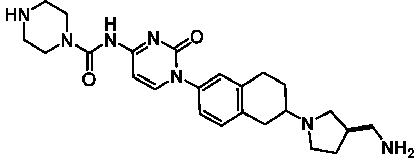 | 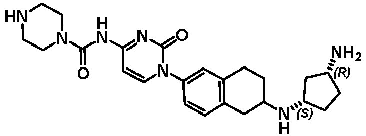 |
| 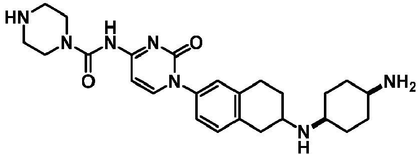 | 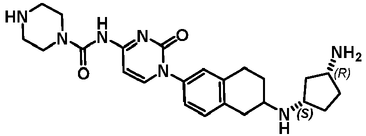 |
| 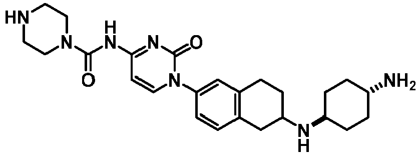 | 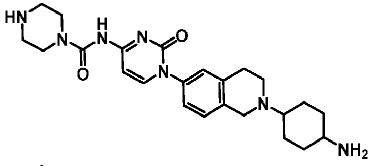 |
| 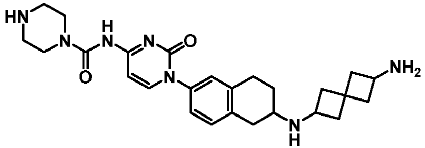 | 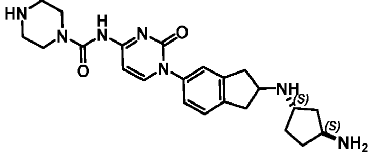 |
| 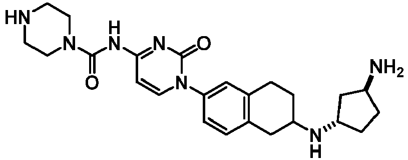 | 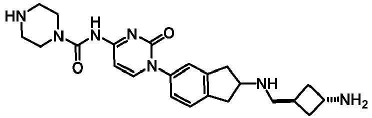 |
| 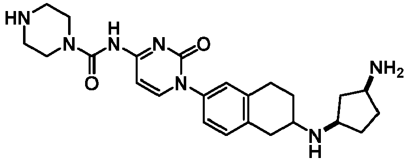 | 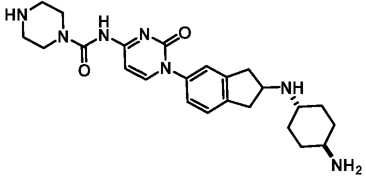 |
| 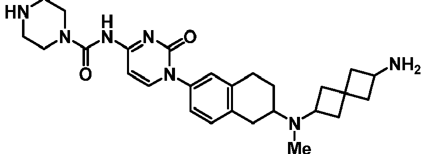 | 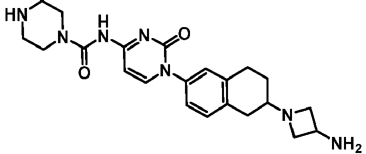 |
| 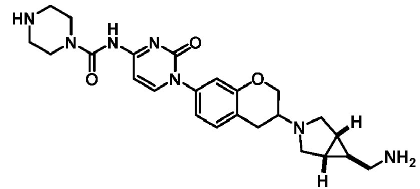 | 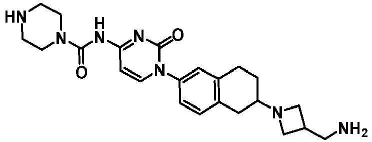 |
| 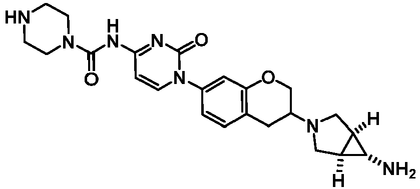 | 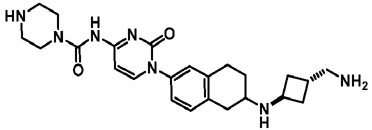 |
| 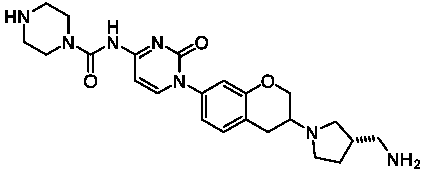 | 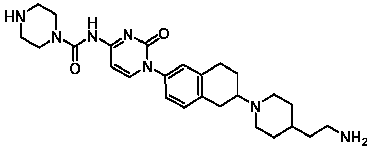 |
| 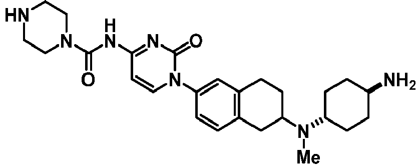 | 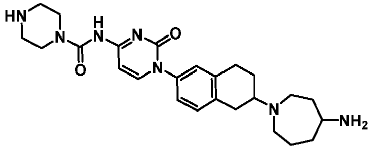 |
| 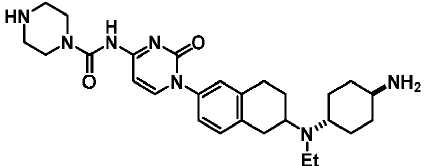 | 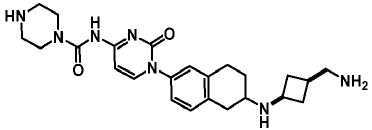 |
| 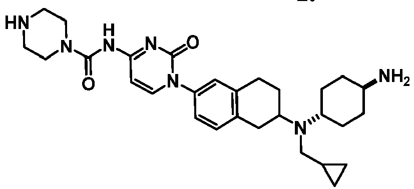 | 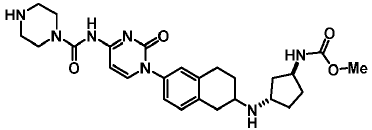 |
| 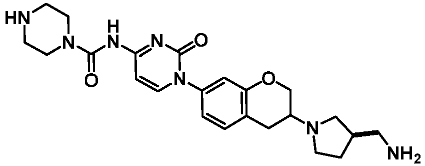 | 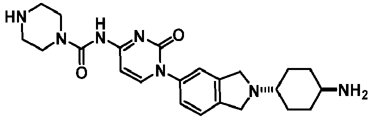 |
| 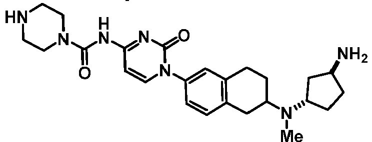 | 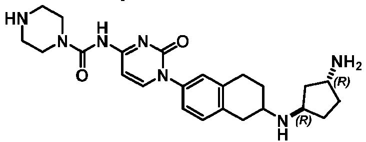 |
| 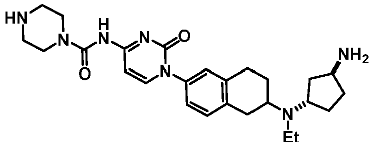 | 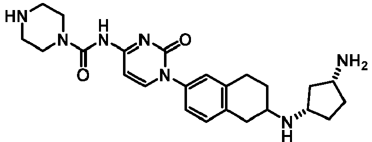 |
| 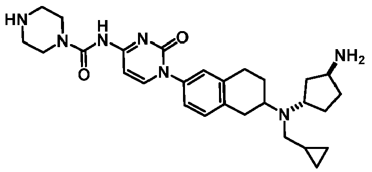 | 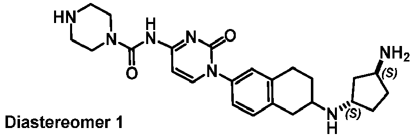 |
| 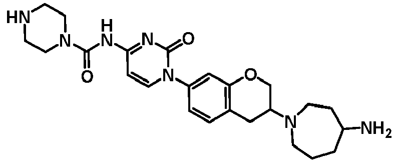 | 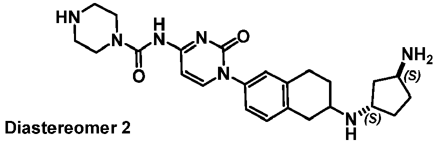 |
| 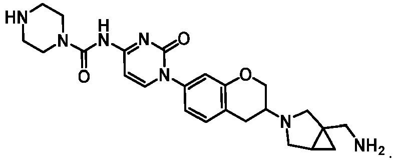 | |

In another aspect, the disclosure provides a compound of formula III 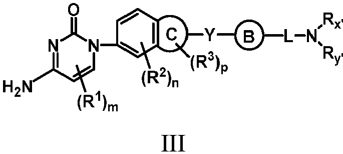 or a single stereoisomer or mixture of stereoisomers thereof, or a pharmaceutically acceptable salt thereof, wherein ring B, ring C, L, Y, R¹, R², R³, R_{x'}, R_{y'}, m, n, and p have the definitions as provided in the preceding paragraphs.

In one embodiment, the compound of formula III or a single stereoisomer or mixture of stereoisomers thereof, or a pharmaceutically acceptable salt thereof is selected from the compounds as depicted in Table 4 below.

**Table 4. Compounds of formula III**

| **Compound Structure** | **Salt Structure** |
|---|---|
| 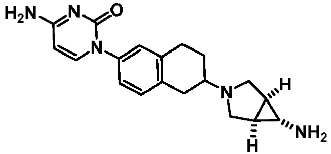 | 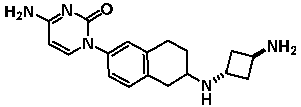 |
| 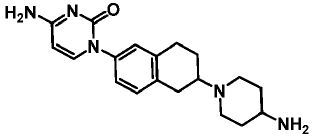 | 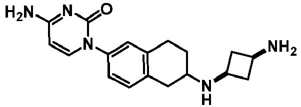 |
| 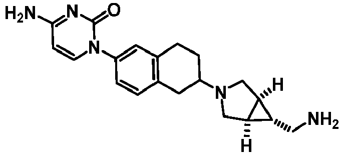 | 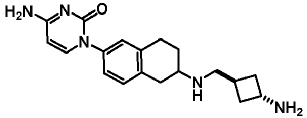 |
| 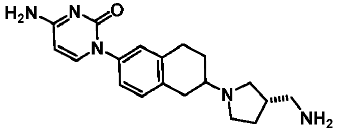 | 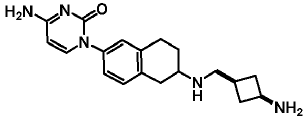 |
| 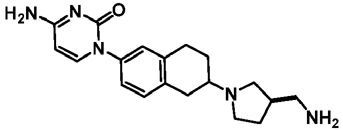 | 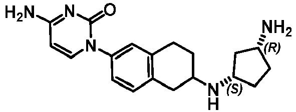 |
| 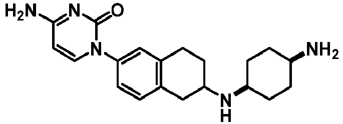 | 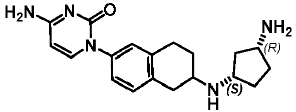 |
| 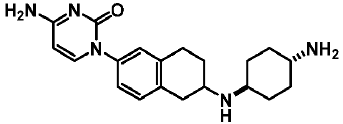 | 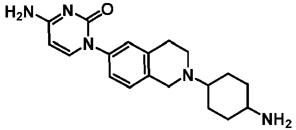 |
| 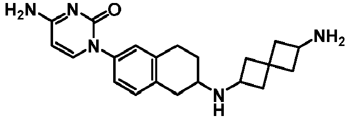 | 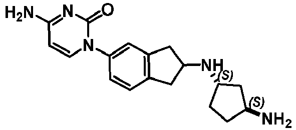 |
| 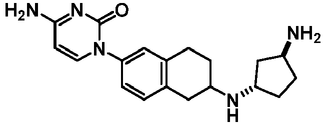 | 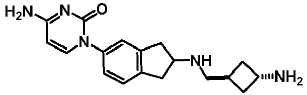 |
| 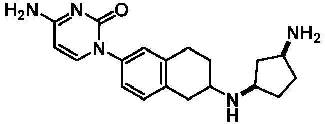 | 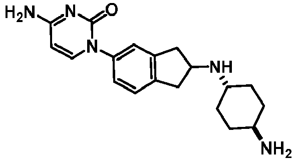 |
| 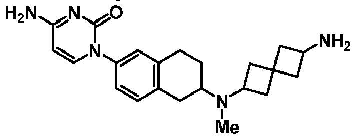 | 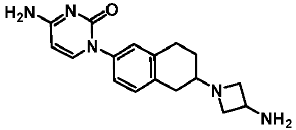 |
| 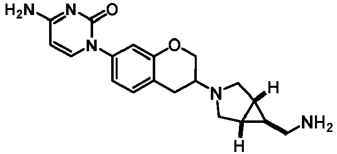 | 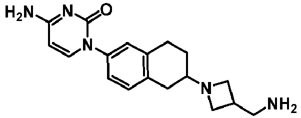 |
| 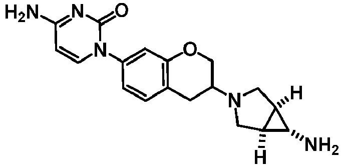 | 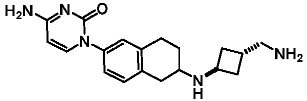 |
| 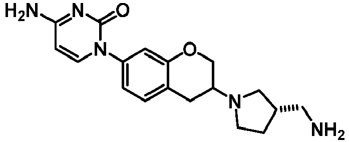 | 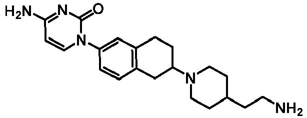 |
| 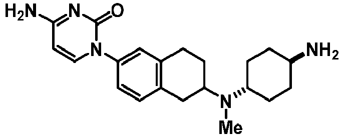 | 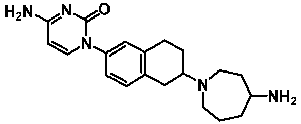 |
| 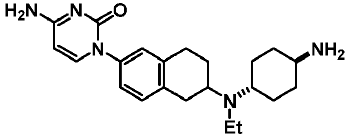 | 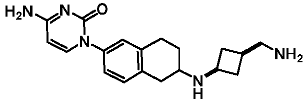 |
| 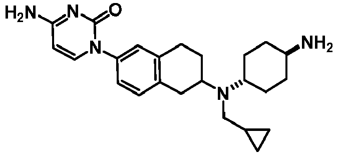 | 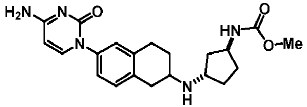 |
| 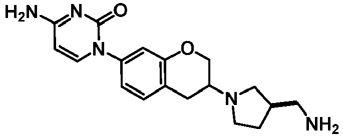 | 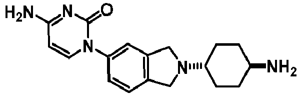 |
| 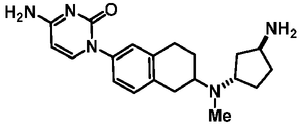 | 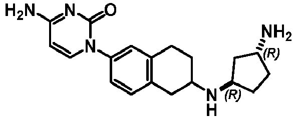 |
| 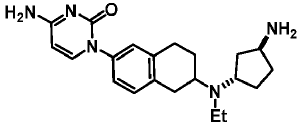 | 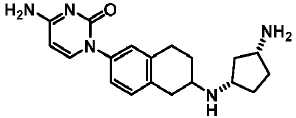 |
| 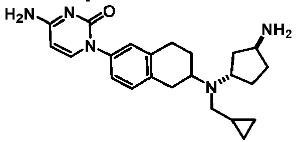 | 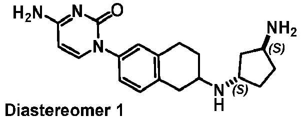 |
| 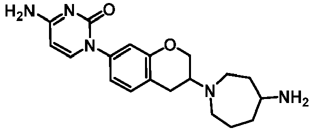 | 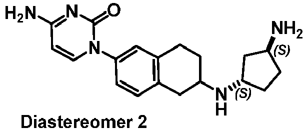 |
| 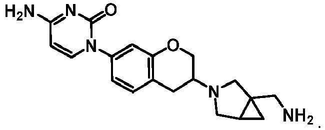 | |

### Pharmaceutical Compositions and Administration

The present invention provides pharmaceutical compositions comprising a compound of the present invention and a pharmaceutically acceptable excipient. In certain embodiments, the compound of the present invention is provided in an effective amount in the pharmaceutical composition. In certain embodiments, the effective amount is a therapeutically effective amount. In certain embodiments, the effective amount is a prophylactically effective amount.

Pharmaceutically acceptable excipients include any and all solvents, diluents, or other liquid vehicles, dispersions, suspension aids, surface active agents, isotonic agents, thickening or emulsifying agents, preservatives, solid binders, lubricants and the like, as suited to the particular dosage form desired. General considerations in formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

Pharmaceutical compositions described herein can be prepared by any method known in the art of pharmacology. In general, such preparatory methods include the steps of bringing the compound of the present invention (the "active ingredient") into association with a carrier and/or one or more other accessory ingredients, and then, if necessary and/or desirable, shaping and/or packaging the product into a desired single- or multi-dose unit.

Pharmaceutical compositions can be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered. By way of example, the composition may comprise between 0.1% and 100% (w/w) active ingredient.

Pharmaceutically acceptable excipients used in the manufacture of provided pharmaceutical compositions include inert diluents, dispersing and/or granulating agents, surface active agents and/or emulsifiers, disintegrating agents, binding agents, preservatives, buffering agents, lubricating agents, and/or oils. Excipients such as cocoa butter and suppository waxes, coloring agents, coating agents, sweetening, flavoring, and perfuming agents may also be present in the composition.

Exemplary diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and mixtures thereof.

Exemplary granulating and/or dispersing agents include potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and mixtures thereof.

Exemplary surface active agents and/or emulsifiers include natural emulsifiers (e.g. acacia, agar, alginic acid, sodium alginate, tragacanth, chondrux, cholesterol, xanthan, pectin, gelatin, egg yolk, casein, wool fat, cholesterol, wax, and lecithin), colloidal clays (e.g. bentonite [aluminum silicate] and Veegum [magnesium aluminum silicate]), long chain amino acid derivatives, high molecular weight alcohols (e.g. stearyl alcohol, cetyl alcohol, oleyl alcohol, triacetin monostearate, ethylene glycol distearate, glyceryl monostearate, and propylene glycol monostearate, polyvinyl alcohol), carbomers (e.g. carboxy polymethylene, polyacrylic acid, acrylic acid polymer, and carboxyvinyl polymer), carrageenan, cellulosic derivatives (e.g. carboxymethylcellulose sodium, powdered cellulose, hydroxymethyl cellulose, hydroxypropyl cellulose, hydroxypropyl methylcellulose, methylcellulose), sorbitan fatty acid esters (e.g. polyoxyethylene sorbitan monolaurate [Tween 20], polyoxyethylene sorbitan [Tween 60], polyoxyethylene sorbitan monooleate [Tween 80], sorbitan monopalmitate [Span 40], sorbitan monostearate [Span 60], sorbitan tristearate [Span 65], glyceryl monooleate, sorbitan monooleate [Span 80]), polyoxyethylene esters (e.g. polyoxyethylene monostearate [Myrj 45], polyoxyethylene hydrogenated castor oil, polyethoxylated castor oil, polyoxymethylene stearate, and Solutol), sucrose fatty acid esters, polyethylene glycol fatty acid esters (e.g. Cremophor), polyoxyethylene ethers, (e.g. polyoxyethylene lauryl ether [Brij 30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic F 68, Poloxamer 188, cetrimonium bromide, cetylpyridinium chloride, benzalkonium chloride, docusate sodium, and/or mixtures thereof.

Exemplary binding agents include starch (e.g. cornstarch and starch paste), gelatin, sugars (e.g. sucrose, glucose, dextrose, dextrin, molasses, lactose, lactitol, mannitol, etc.), natural and synthetic gums (e.g. acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (Veegum), and larch arabogalactan), alginates, polyethylene oxide, polyethylene glycol, inorganic calcium salts, silicic acid, polymethacrylates, waxes, water, alcohol, and/or mixtures thereof.

Exemplary preservatives include antioxidants, chelating agents, antimicrobial preservatives, antifungal preservatives, alcohol preservatives, acidic preservatives, and other preservatives.

Exemplary antioxidants include alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite.

Exemplary chelating agents include ethylenediaminetetraacetic acid (EDTA) and salts and hydrates thereof (e.g., sodium edetate, disodium edetate, trisodium edetate, calcium disodium edetate, dipotassium edetate, and the like), citric acid and salts and hydrates thereof (e.g., citric acid monohydrate), fumaric acid and salts and hydrates thereof: malic acid and salts and hydrates thereof: phosphoric acid and salts and hydrates thereof: and tartaric acid and salts and hydrates thereof. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal.

Exemplary antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid.

Exemplary alcohol preservatives include ethanol, polyethylene glycol, phenol, phenolic compounds, bisphenol, chlorobutanol, hydroxybenzoate, and phenylethyl alcohol.

Exemplary acidic preservatives include vitamin A, vitamin C, vitamin E, beta-carotene, citric acid, acetic acid, dehydroacetic acid, ascorbic acid, sorbic acid, and phytic acid.

Other preservatives include tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl. In certain embodiments, the preservative is an anti-oxidant. In other embodiments, the preservative is a chelating agent.

Exemplary buffering agents include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and mixtures thereof.

Exemplary lubricating agents include magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and mixtures thereof.

Exemplary natural oils include almond, apricot kernel, avocado, babassu, bergamot, black current seed, borage, cade, chamomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary synthetic oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and mixtures thereof.

Liquid dosage forms for oral and parenteral administration include pharmaceutically acceptable emulsions, microemulsions, solutions, suspensions, syrups and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (e.g., cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring, and perfuming agents. In certain embodiments for parenteral administration, the conjugates of the invention are mixed with solubilizing agents such as Cremophor, alcohols, oils, modified oils, glycols, polysorbates, cyclodextrins, polymers, and mixtures thereof.

Injectable preparations, for example, sterile injectable aqueous or oleaginous suspensions can be formulated according to the known art using suitable dispersing or wetting agents and suspending agents. The sterile injectable preparation can be a sterile injectable solution, suspension or emulsion in a nontoxic parenterally acceptable diluent or solvent, for example, as a solution in 1,3-butanediol. Among the acceptable vehicles and solvents that can be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of inj ectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purposes of formulation.

In order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Compositions for rectal or vaginal administration are typically suppositories which can be prepared by mixing the conjugates of this invention with suitable non-irritating excipients or carriers such as cocoa butter, polyethylene glycol or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethyl cellulose, alginates, gelatin, polyvinylpyrrolidinone, sucrose, and acacia, c) humectants such as glycerol, d) disintegrating agents such as agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may comprise buffering agents.

Solid compositions of a similar type can be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type can be employed as fillers in soft and hard-filled gelatin capsules using such excipients as lactose or milk sugar as well as high molecular weight polyethylene glycols and the like.

The active ingredient can be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active ingredient can be admixed with at least one inert diluent such as sucrose, lactose or starch. Such dosage forms may comprise, as is normal practice, additional substances other than inert diluents, e.g., tableting lubricants and other tableting aids such a magnesium stearate and microcrystalline cellulose. In the case of capsules, tablets and pills, the dosage forms may comprise buffering agents. They may optionally comprise opacifying agents and can be of a composition that they release the active ingredient(s) only, or preferentially, in a certain part of the intestinal tract, optionally, in a delayed manner Examples of embedding compositions which can be used include polymeric substances and waxes.

Dosage forms for topical and/or transdermal administration of a compound of this invention may include ointments, pastes, creams, lotions, gels, powders, solutions, sprays, inhalants and/or patches. Generally, the active ingredient is admixed under sterile conditions with a pharmaceutically acceptable carrier and/or any needed preservatives and/or buffers as can be required. Additionally, the present invention contemplates the use of transdermal patches, which often have the added advantage of providing controlled delivery of an active ingredient to the body. Such dosage forms can be prepared, for example, by dissolving and/or dispensing the active ingredient in the proper medium. Alternatively or additionally, the rate can be controlled by either providing a rate controlling membrane and/or by dispersing the active ingredient in a polymer matrix and/or gel.

Suitable devices for use in delivering intradermal pharmaceutical compositions described herein include short needle devices such as those described in U.S. Pat. Nos. 4,886,499; 5,190,521; 5,328,483; 5,527,288; 4,270,537; 5,015,235; 5,141,496; and 5,417,662. Intradermal compositions can be administered by devices which limit the effective penetration length of a needle into the skin, such as those described in PCT publication WO 99/34850 and functional equivalents thereof. Jet injection devices which deliver liquid vaccines to the dermis via a liquid jet injector and/or via a needle which pierces the stratum corneum and produces a jet which reaches the dermis are suitable. Jet injection devices are described, for example, in U.S. Pat. Nos. 5,480,381; 5,599,302; 5,334,144; 5,993,412; 5,649,912; 5,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537. Ballistic powder/particle delivery devices which use compressed gas to accelerate vaccine in powder form through the outer layers of the skin to the dermis are suitable. Alternatively or additionally, conventional syringes can be used in the classical mantoux method of intradermal administration.

A pharmaceutical composition of the invention can be prepared, packaged, and/or sold in a formulation suitable for pulmonary administration via the buccal cavity. Such a formulation may comprise dry particles which comprise the active ingredient and which have a diameter in the range from about 0.5 to about 7 nanometers or from about 1 to about 6 nanometers. Such compositions are conveniently in the form of dry powders for administration using a device comprising a dry powder reservoir to which a stream of propellant can be directed to disperse the powder and/or using a self-propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 nanometers and at least 95% of the particles by number have a diameter less than 7 nanometers. Alternatively, at least 95% of the particles by weight have a diameter greater than 1 nanometer and at least 90% of the particles by number have a diameter less than 6 nanometers. Dry powder compositions may include a solid fine powder diluent such as sugar and are conveniently provided in a unit dose form.

Low boiling propellants generally include liquid propellants having a boiling point of below 65 °F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic and/or solid anionic surfactant and/or a solid diluent (which may have a particle size of the same order as particles comprising the active ingredient).

Pharmaceutical compositions of the invention formulated for pulmonary delivery may provide the active ingredient in the form of droplets of a solution and/or suspension. Such formulations can be prepared, packaged, and/or sold as aqueous and/or dilute alcoholic solutions and/or suspensions, optionally sterile, comprising the active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. The droplets provided by this route of administration may have an average diameter in the range from about 0.1 to about 200 nanometers.

Formulations described herein as being useful for pulmonary delivery are useful for intranasal delivery of a pharmaceutical composition of the invention. Another formulation suitable for intranasal administration is a coarse powder comprising the active ingredient and having an average particle from about 0.2 to 500 micrometers. Such a formulation is administered by rapid inhalation through the nasal passage from a container of the powder held close to the nares.

Formulations for nasal administration may, for example, comprise from about as little as 0.1% (w/w) and as much as 100% (w/w) of the active ingredient, and may comprise one or more of the additional ingredients described herein. A pharmaceutical composition of the invention can be prepared, packaged, and/or sold in a formulation for buccal administration. Such formulations may, for example, be in the form of tablets and/or lozenges made using conventional methods, and may contain, for example, 0.1 to 20% (w/w) active ingredient, the balance comprising an orally dissolvable and/or degradable composition and, optionally, one or more of the additional ingredients described herein. Alternately, formulations for buccal administration may comprise a powder and/or an aerosolized and/or atomized solution and/or suspension comprising the active ingredient. Such powdered, aerosolized, and/or aerosolized formulations, when dispersed, may have an average particle and/or droplet size in the range from about 0.1 to about 200 nanometers, and may further comprise one or more of the additional ingredients described herein.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation.

Compounds provided herein are typically formulated in dosage unit form for ease of administration and uniformity of dosage. It will be understood, however, that the total daily usage of the compositions of the present invention will be decided by the attending physician within the scope of sound medical judgment. The specific therapeutically effective dose level for any particular subject or organism will depend upon a variety of factors including the disease, disorder, or condition being treated and the severity of the disorder; the activity of the specific active ingredient employed; the specific composition employed; the age, body weight, general health, sex and diet of the subject; the time of administration, route of administration, and rate of excretion of the specific active ingredient employed; the duration of the treatment; drugs used in combination or coincidental with the specific active ingredient employed; and like factors well known in the medical arts.

To practice the method of this invention, the above-described compound or its pharmaceutical composition can be administered orally, parenterally, by inhalation spray, topically, rectally, nasally, buccally, vaginally, rectally, or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In general the most appropriate route of administration will depend upon a variety of factors including the nature of the agent (e.g., its stability in the environment of the gastrointestinal tract), and/or the condition of the subject (e.g., whether the subject is able to tolerate oral administration).

The exact amount of a compound required to achieve an effective amount will vary from subject to subject, depending, for example, on species, age, and general condition of a subject, severity of the side effects or disorder, identity of the particular compound(s), mode of administration, and the like. The desired dosage can be delivered three times a day, two times a day, once a day, every other day, every third day, every week, every two weeks, every three weeks, or every four weeks. In certain embodiments, the desired dosage can be delivered using multiple administrations (e.g., two, three, four, five, six, seven, eight, nine, ten, eleven, twelve, thirteen, fourteen, or more administrations).

In certain embodiments, an effective amount of a compound for administration one or more times a day to a 70 kg adult human may comprise about 0.0001 mg to about 3000 mg, about 0.0001 mg to about 2000 mg, about 0.0001 mg to about 1000 mg, about 0.001 mg to about 1000 mg, about 0.01 mg to about 1000 mg, about 0.1 mg to about 1000 mg, about 1 mg to about 1000 mg, about 1 mg to about 100 mg, about 10 mg to about 1000 mg, or about 100 mg to about 1000 mg, of a compound per unit dosage form.

In certain embodiments, the compounds of the invention may be administered orally or parenterally at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day, one or more times a day, to obtain the desired therapeutic effect.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

It will be also appreciated that a compound or composition, as described herein, can be administered in combination with one or more additional therapeutically active agents. The compounds or compositions can be administered in combination with additional therapeutically active agents that improve their bioavailability, reduce and/or modify their metabolism, inhibit their excretion, and/or modify their distribution within the body. It will also be appreciated that the therapy employed may achieve a desired effect for the same disorder, and/or it may achieve different effects.

The compound or composition can be administered concurrently with, prior to, or subsequent to, one or more additional therapeutically active agents. In general, each agent will be administered at a dose and/or on a time schedule determined for that agent. In will further be appreciated that the additional therapeutically active agent utilized in this combination can be administered together in a single composition or administered separately in different compositions. The particular combination to employ in a regimen will take into account compatibility of the inventive compound with the additional therapeutically active agent and/or the desired therapeutic effect to be achieved. In general, it is expected that additional therapeutically active agents utilized in combination be utilized at levels that do not exceed the levels at which they are utilized individually. In some embodiments, the levels utilized in combination will be lower than those utilized individually. Additional therapeutically active agents include antibiotic agents, e.g., antibiotics useful for treating tuberculosis. Exemplary antibiotics include, but are not limited to, isoniazid, rifampin, pyrazinamide, ethambutol, and streptomycin.

Also encompassed by the invention are kits (e.g., pharmaceutical packs). The kits provided may comprise an inventive pharmaceutical composition or compound and a container (e.g., a vial, ampule, bottle, syringe, and/or dispenser package, or other suitable container). In some embodiments, provided kits may optionally further include a second container comprising a pharmaceutical excipient for dilution or suspension of an inventive pharmaceutical composition or compound. In some embodiments, the inventive pharmaceutical composition or compound provided in the container and the second container are combined to form one unit dosage form.

### Uses and Methods of Treatment

In another aspect, disclosed but not claimed is a method of treating a bacterial infection in a patient in need of such treatment, comprising administering an effective amount of a compound of formula I, or a single stereoisomer or mixture of stereoisomers thereof, or a pharmaceutically acceptable salt thereof or a composition comprising a compound of formula I, or a single stereoisomer or mixture of stereoisomers thereof, or a pharmaceutically acceptable salt thereof. In certain embodiments, the effective amount is a therapeutically effective amount. In certain other embodiments, the effective amount is a prophylactically effective amount.

In some embodiments, the compounds of the invention can be active against a wide range of both Gram-positive and Gram-negative organisms. In these and other embodiments, the compounds of the invention can be used to treat infections and to inhibit microbial growth. Thus, the compounds of the invention can be used to treat humans and animals having a broad spectrum of bacterial infections such as impetigo, pneumonia, bronchitis, pharyngitis, endocarditis, urinary tract infections, diabetes foot ulcers, gastro-intestinal infections and bacteremia. These bacterial infections could be caused by any of the following bacteria--Staphylococcus aureus, coagulase negative staphylococci, methicillin-resistant Staphylococcus aureus, methicillin-resistant coagulase negative staphylococci, enterococci, beta-haemolytic streptococci, viridans group of streptococci, Bacillus mycobacterial infections due to multi-drug resistant M. tuberculosis and other atypical mycobacteria such as M. intracellulare and M. avium, as well as newly emerging Gram-negative pathogens such as Chryseobacterium meningosepticum, Chryseobacterium indologense and other Gram-negative pathogens such as E. coli, Klebsiella, Proteus, Serratia, Citrobacter, Pseudomonas, Burkholderia, Brucella, Yersinia, Francisella, Coxiella, Chlamydia, Salmonella, Rickettsia, Shigella and Campylobacter.

In one embodiment, the bacterial infection is tuberculosis. In certain embodiments, the tuberculosis infection is a Mycobacterium tuberculosis infection. In certain embodiments, the tuberculosis infection is multi-drug-resistant tuberculosis (MDR-TB) infection, e.g., resistant to first-line TB drugs rifampicin and/or isoniazid. In certain embodiments, the tuberculosis infection is extensively-drug-resistant tuberculosis (XDR-TB) infection, e.g., also resistant to three or more of the six classes of second-line drugs (see, e.g., Centers for Disease Control and Prevention (CDC) (2006). "Emergence of Mycobacterium tuberculosis with extensive resistance to second-line drugs worldwide, 2000-2004". MMWR Morb Mortal Wkly Rep 55 (11): 301-5).

### Processes

In some aspects, the compounds and intermediates of the present disclosure can be prepared according to General Synthetic Scheme I and II below. In the general schemes, variables such as R¹, R², R³, Rₓ, R_{y}, R_{x'}, R_{y'}, ring A, ring B, ring C, J, L, Y, m, n, and p have the same definitions in the preceding paragraphs; X is a leaving group such as halo, mesylate, tosylate, triflate; and P is a hydroxyl protecting group. In some embodiments, X is halo; and P is TBS.

### General Synthetic Scheme I

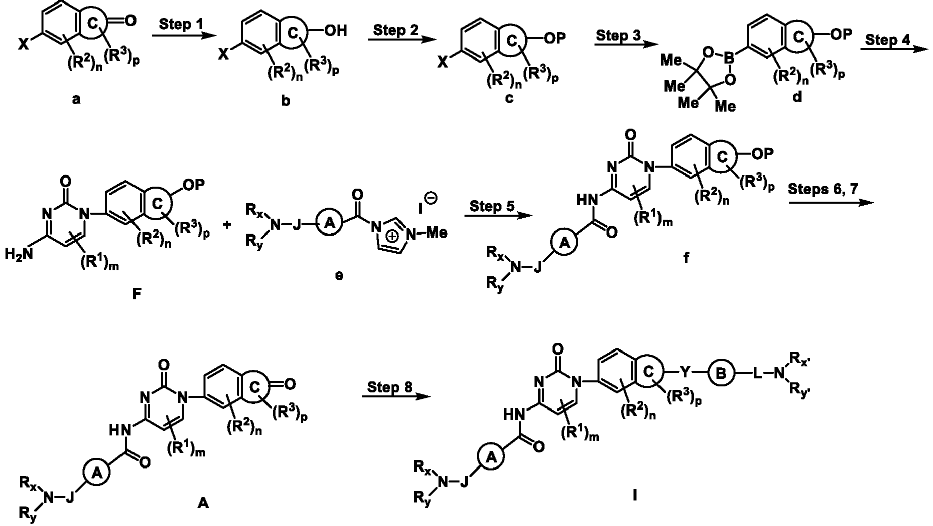

In General Synthetic Scheme I, the ketone (a) undergoes a reduction to yield an alcohol (b), and the protection of the hydroxyl group affords the intermediate (c).

In step 3 of General Synthetic Scheme I, the intermediate (c) is reacted with triisopropyl borate in the presence of a base such as butyl lithium to afford the boronate (d). In step 4, the boronate (d) is cross-coupled with cytosine in the presence of a base such as a tertiary amine and a copper reagent such as a copper (II) reagent to afford the compound of formula F.

In step 5 of General Synthetic Scheme I, the compound of formula F and a compound of formula (e) undergoes an amide coupling to yield the intermediate (f). In some processes, about 1.1 to 2.0 molar equivalents of the compound of formula (e) are combined with 1 molar equivalent of the compound of formula F in a suitable solvent, such as a polar aprotic solvent. Polar aprotic solvents include solvents such as dichloromethane, dimethylformamide, acetonitrile, and the like. The mixture in the polar aprotic solvent is then allowed to undergo reaction at a temperature of from about 0 °C to 100 °C for a sufficient time. In some embodiments, the temperature is from about 25 °C to 95 °C or from about 50 °C to 95 °C and the reaction time is from about 1 to 24 hours, or from 2 to 20 hours, or from about 5 to 18 hours.

In steps 6 and 7, the compound of formula (f) is deprotected to yield a free alcohol and then oxidized to a ketone, a compound of formula A.

In step 8 of General Synthetic Scheme I, the compound of formula A is reacted with an amine under a reductive amination condition to afford the compound of formula I. The reductive amination can be performed in the presence of a reducing agent and a suitable solvent. A suitable solvent includes protic solvents or aprotic solvents. Protic solvents include but are not limited to water and alcohols such as methanol, ethanol, propanol, and the like. Aprotic solvents include but are not limited to solvents such as dichloromethane, dimethylformamide, acetonitrile, and the like. The suitable solvent may also be a combination of two or three solvents. The reducing agent includes but is not limited to a borohydride reagent or a metal hydride reagent. Non-limiting examples are lithium borohydride, sodium borohydride, sodium cyanoborohydride.

### General Synthetic Scheme II

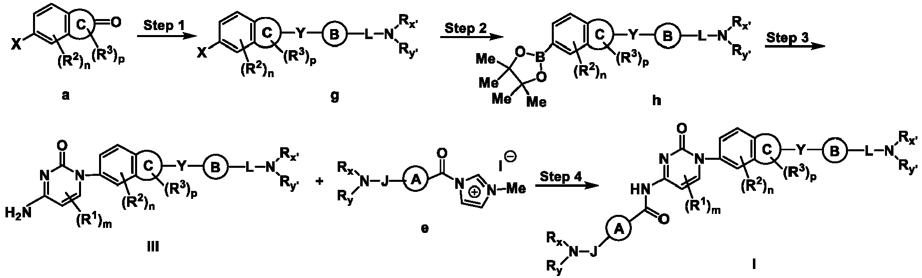

In step 1 of General Synthetic Scheme II, the ketone (a) undergoes reductive amination with an amine to yield the intermediate (g). The conditions to carry out the reductive amination are provided in the previous paragraph.

In step 2 of General Synthetic Scheme II, the intermediate (g) is reacted with triisopropyl borate in the presence of a base such as butyl lithium to afford the boronate (h). In step 3, the boronate (d) is cross-coupled with cytosine in the presence of a base such as a tertiary amine and a copper reagent such as a copper (II) reagent to afford the compound of formula III.

In step 4 of General Synthetic Scheme II, the compound of Formula III and a compound of formula (e) undergo an amide coupling to yield the compound of formula (I). The conditions to carry out the amide coupling are similar to the conditions in step 5 of the General Synthetic Scheme I.

In one aspect, the disclosure provides processes for preparing a compound of formula I: 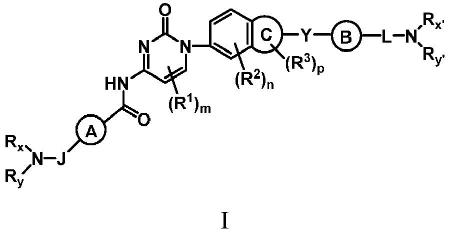 or a single stereoisomer or mixture of stereoisomers thereof, or a pharmaceutically acceptable salt thereof, the process comprising:
combining a compound of formula A: 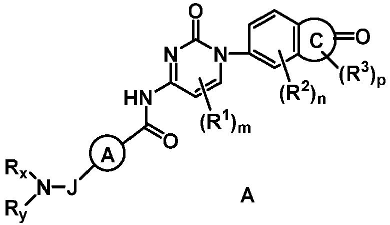 with a compound of: 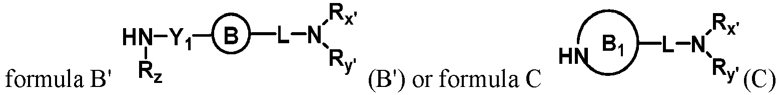 under a reductive amination condition to provide the compound of formula I,
wherein ring A, ring B, ring C, J, L, R¹, R², R³, Rₓ, R_{y}, R_{x'}, R_{y'}, m, n, and p are as defined in the preceding paragraphs;
ring Bi is a nitrogen containing 3-8 membered monocyclic heterocycloalkylene, or a nitrogen containing 6-12 membered bicyclic heterocycloalkylene, each of which is optionally and independently substituted with up to three substituents selected from the group consisting of C₁-C₆ alkyl, C₁-C₆ alkoxy, halo, CN, C₁-C₆ haloalkyl, OH, COO(C₁-C₆ alkyl), CONH₂, CONH(C₁-C₆ alkyl), CON(C₁-C₆ alkyl)₂, and C₁-C₆ hydroxyalkyl;
Y is a bond or C₁-C₆ alkylene optionally substituted with OH, NH₂, CN, halo, or C₁-C₆ alkoxy, wherein one methylene unit of the C₁-C₆ alkylene is optionally replaced by NH, N-(C₁-C₆ alkyl), N-(C₁-C₆ hydroxyalkyl), N-(C₁-C₆ haloalkyl), or N-(C₁-C₆ alkylene-C₃-C₈ cycloalkyl);
Yi is a bond or C₁-C₅ alkylene optionally substituted with OH, NH₂, CN, halo, or C₁-C₆ alkoxy; and
R_{z} is H, C₁-C₆ alkyl, C₁-C₆ hydroxyalkyl, C₁-C₆ haloalkyl, or C₁-C₆ alkylene-C₃-C₈ cycloalkyl.

In some embodiments, ring Bi is a nitrogen containing 4-7 membered monocyclic heterocycloalkylene or a nitrogen containing 6-9 membered bicyclic heterocycloalkylene, each of which is optionally substituted with up to three substituents independently selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and C₁-C₆ hydroxyalkyl.

In some embodiments, ring Bi is a nitrogen containing 4-7 membered monocyclic heterocycloalkylene or a nitrogen containing 6-9 membered bicyclic heterocycloalkylene. In some embodiments, ring Bi is a nitrogen containing 4-7 membered monocyclic heterocycloalkylene or a nitrogen containing 6 membered bicyclic heterocycloalkylene.

In some embodiments, ring B in formula B' is selected from the group consisting of

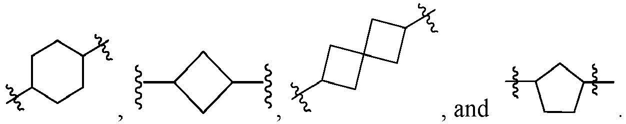

In some embodiments, ring Bi is selected from the group consisting of 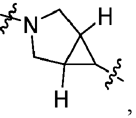 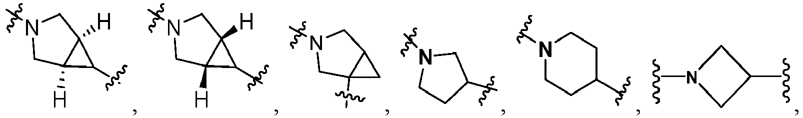 and

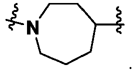

In some embodiments, L is a bond, -CH₂-, or -CH₂-CH₂-; and R_{x'} and R_{y'} are each independently H, Boc or methoxycarbonyl.

In some embodiments, Yi is a bond or C₁-C₃ alkylene optionally substituted with OH, NH₂, CN, halo, or C₁-C₆ alkoxy. In some embodiments, Yi is a bond or C₁-C₃ alkylene. In some embodiment, Y₁ is a bond or -CH₂-.

In some embodiments, R_{z} is H, C₁-C₃ alkyl, C₁-C₃ hydroxyalkyl, C₁-C₃ haloalkyl, or C₁-C₃ alkylene-C₃-C₆ cycloalkyl. In some embodiments, R_{z} is H, Me, Et, or CH₂-cyclopropyl.

In some embodiments, the compound of formula I is a compound of formula IB, formula IB', or formula IC: 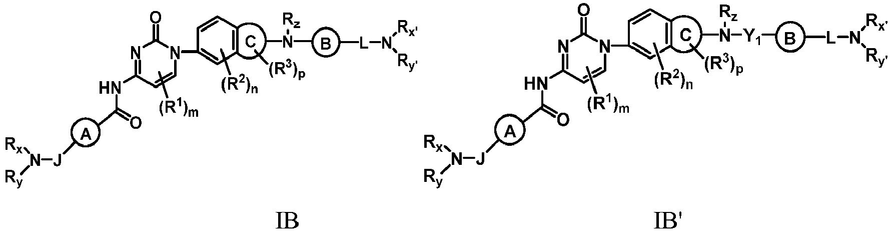 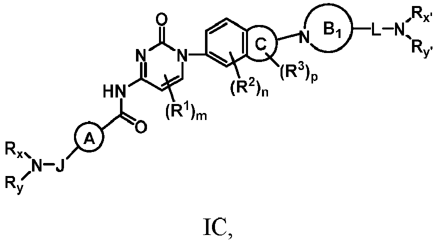 wherein the variables are as defined herein.

In some embodiments, the compound of formula I is a compound of formula IB or formula IC.

In some embodiments, the compound of formula IB, formula IB', or formula IC is selected from the compounds listed in Table 1 and Table 2.

In one aspect, the disclosure provides processes for preparing a compound of formula IB: 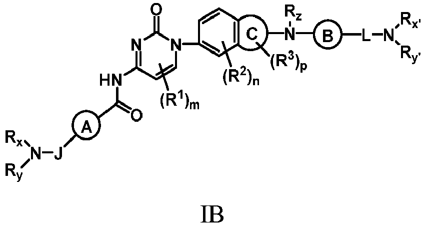 or a single stereoisomer or mixture of stereoisomers thereof, or a pharmaceutically acceptable salt thereof, the process comprising:
combining a compound of formula A with a compound of formula B under a reductive amination condition to provide a compound of formula IB 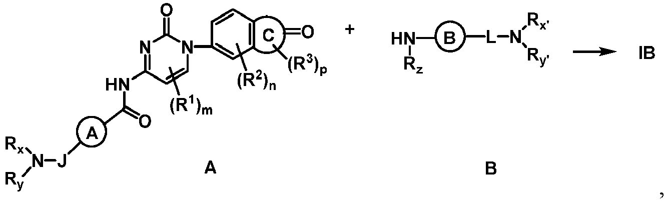
wherein ring A, ring B, ring C, J, L, R¹, R², R³, Rₓ, R_{y}, R_{z}, R_{x'}, R_{y'}, m, n, and p have the definitions as provided in the preceding paragraphs.

In one embodiment, the process further comprises the step of removing the amino protecting group when any of Rₓ, R_{y}, R_{x'}, and R_{y'} is an amino protecting group.

In another aspect, the disclosure provides processes for preparing a compound of formula IC: 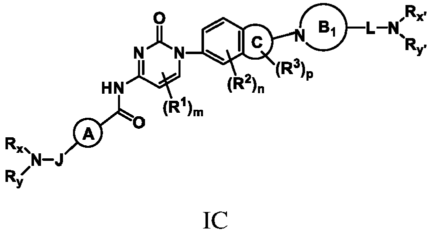 or a pharmaceutically acceptable salt thereof, the process comprising:
combining a compound of formula A with a compound of formula C under a reductive amination condition to provide a compound of formula IC 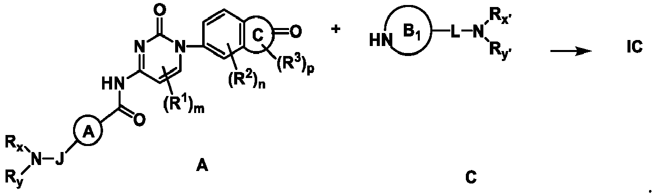
wherein ring A, ring B₁, ring C, J, L, R¹, R², R³, Rₓ, R_{y}, R_{x'}, R_{y'}, m, n, and p have the definitions as provided in the preceding paragraphs.

In one embodiment, the process further comprises the step of removing the amino protecting group when at least one of Rₓ, R_{y}, R_{x'}, and R_{y'} is an amino protecting group.

Processes and conditions for performing the reductive amination of the compound of formula E are as in the general synthetic scheme.

In some embodiments, the reductive amination between a compound of formula A and a compound of formula B (or formula B') or between a compound of formula A and a compound of formula C can be performed in the presence of a reducing agent and a suitable solvent. A suitable solvent includes protic solvents or aprotic solvents. Protic solvents include but are not limited to water and alcohols such as methanol, ethanol, propanol, and the like. Aprotic solvents include but are not limited to solvents such as dichloromethane, dimethylformamide, acetonitrile, and the like. The suitable solvent may also be a combination of two or three solvents. The reducing agent includes but is not limited to a borohydride reagent or a metal hydride reagent. Non-limiting examples are lithium borohydride, sodium borohydride, sodium cyanoborohydride. In some processes, about 1.1 to 2.0 molar equivalents of the compound of formula B (or formula B') or C are combined with 1 molar equivalent of a compound of formula A and 1.0 to 2.0 molar equivalents of the reducing agent in a suitable solvent. The mixture is then allowed to undergo reaction at a temperature of from about 0 °C to 100 °C for a sufficient time. In some embodiments, the temperature is from about 10 °C to 95 °C, or form about 10 °C to 50 °C, or at room temperature, and the reaction time is from about 1 to 24 hours, or from 2 to 20 hours, or from about 5 to 18 hours.

In some embodiments, the compound of formula A is a compound of formula ID

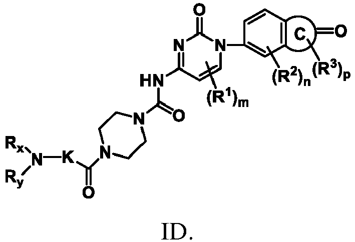

In another aspect, the disclosure provides processes for preparing a compound of formula ID or a pharmaceutically acceptable salt thereof, the process comprising:
coupling a compound of formula E 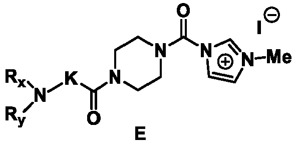
with a compound of formula F 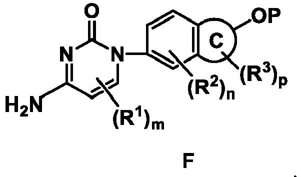
wherein ring C, K, R¹, R², R³, Rₓ, R_{y}, m, n, and p are as defined herein; and
P is a hydroxyl protecting group.

Processes and conditions for performing the amide coupling of a compound of formula E to a compound of formula F are as described in step 5 of the General Synthetic Scheme I.

In one embodiment, the process further comprises the step of removing the hydroxyl protecting group to provide a compound of formula IE

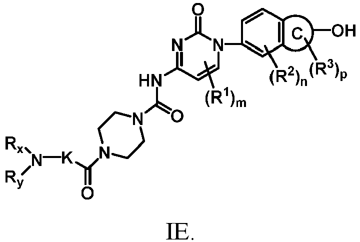

In some embodiments, the compound of formula IE is selected from the group consisting of:

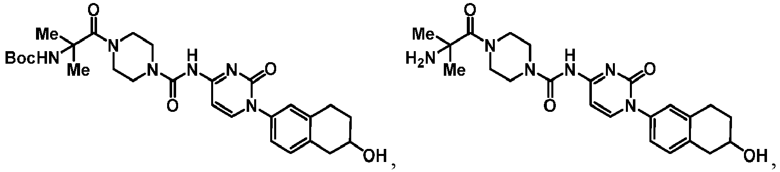

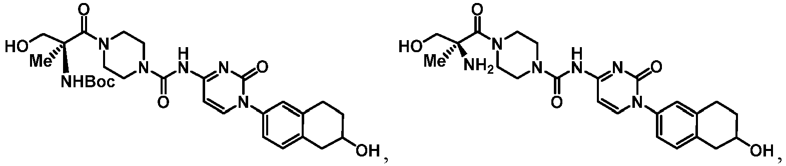

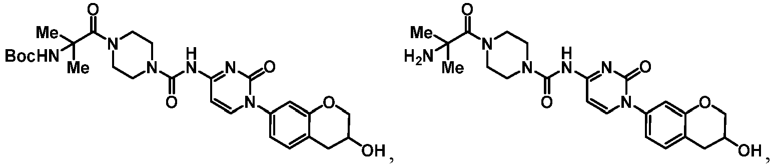

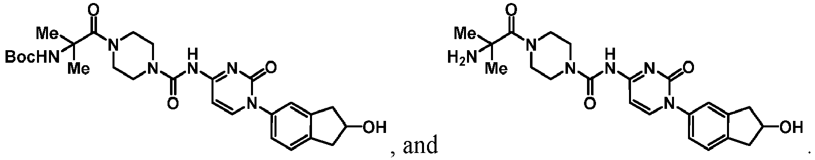

In another embodiment, the process further comprises the step of oxidizing the hydroxyl group in formula IE to provide the compound of formula ID. Exemplary oxidizing agents include Dess-Martin periodinane, chromium trioxide, pyridinium chlorochromate, dimethylsulfoxide, oxalyl chloride, etc.

In one embodiment, the compound of formula ID is a compound of formula ID-1a or ID-1b: 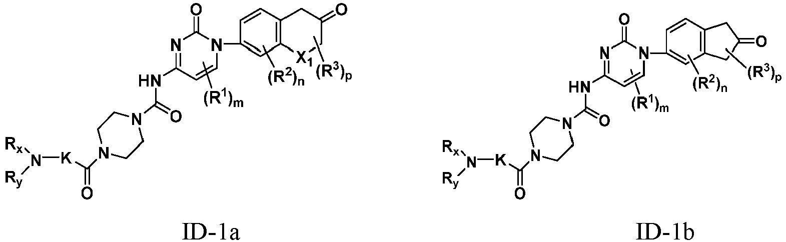 or a pharmaceutically acceptable salt thereof, wherein X₁, K, R¹, R², R³, Rₓ, R_{y}, m, n, and p have the definitions as provided in any of the preceding paragraphs.

In another embodiment, the compound of formula ID is a compound of formula ID-2a or ID-2b: 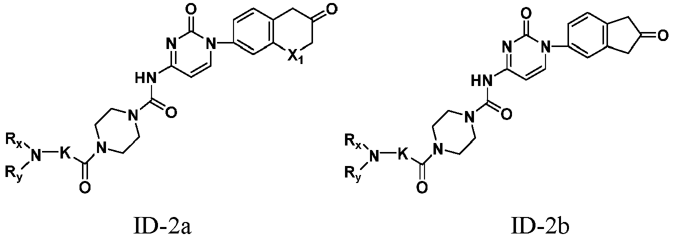 or a pharmaceutically acceptable salt thereof, wherein Rₓ, R_{y}, X₁ and K have the definitions as provided in the preceding paragraphs.

In another embodiment, the compound of formula ID is selected from the group consisting of:

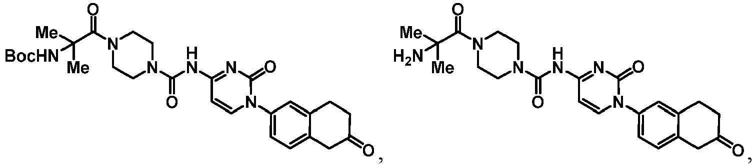

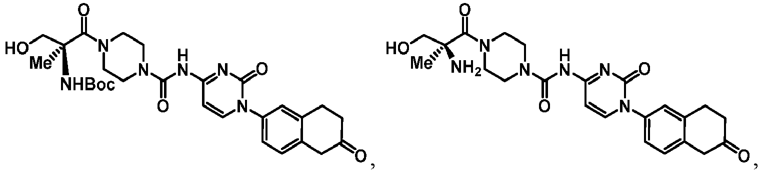

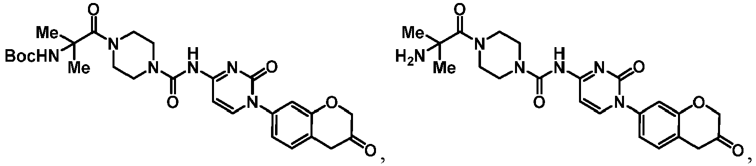

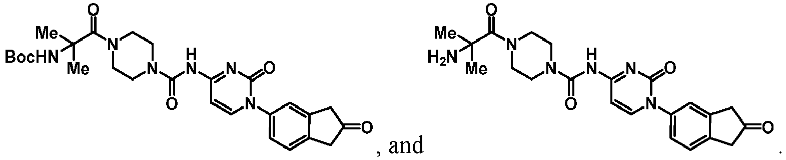

### Compound Preparation

The preparation of starting materials that are commercially available, described in the literature, or readily obtainable by those skilled in the art is not described. It will be appreciated by the skilled person that where it is stated that compounds were prepared analogously to earlier examples or intermediates, the reaction time, number of equivalents of reagents, and temperature, can be modified for each specific reaction and that it may be necessary or desirable to employ different work-up or purification techniques. Where reactions are carried out using microwave irradiation, the microwave oven used was a Biotage Initiator or a CEM Discover System Model 908005. The actual power supplied varies during the course of the reaction in order to maintain a constant temperature.

### General Methods

Reactions requiring anhydrous conditions were conducted in oven-dried glassware under a positive pressure of either nitrogen or argon. Commercially available reagents were used as received; otherwise, materials were purified according to *Purification of Laboratory Chemicals.* Dichloromethane (CH₂Cl₂), *N,N*'-dimethylformamide (DMF), toluene and tetrahydrofuran (THF) were degassed with nitrogen and passed through a solvent purification system (Innovative Technologies Pure Solv). Dry 1,4-dioxane was purchased from Acros Organics in a Acros Seal^{™} bottle. Triethylamine (Et₃N) and *N*,*N*-diisopropylethylamine (DIPEA) were stored over 4 Å molecular sieves or distilled over 4 Å molecular sieves prior to usage. Microwave reactions were done in CEM Discover System Model 908005. Reactions were monitored by TLC or LCMS and visualized by a dual short wave/long wave UV lamp and/or stained with ethanolic solutions of either KMnO₄, 12-phosphomolybdic acid or other commonly used stains. Flash chromatography was performed on Merck silica gel Kieselgel 60 (230-400 mesh) from EM Science with the indicated HPLC grade solvent or an automated medium pressure column chromatography system (Teledyne ISCO CombiFlash RF75 or CombiFlash Rf+). Reverse phase HPLC was conducted on a Waters HPLC Semi Prep 150B system with Sunfire C18 Prep Column or Atlantis T3 Prep Column with isocratic or gradient conditions with H₂O (0.1% TFA) and 10%H₂O:90 CH₃CN (0.1% TFA) as eluents

Melting points were determined using Mel-Temp^{®} Capillary Melting Point Apparatus. Infrared spectra were obtained using Nicolet 380-FT IR spectrometer fitted with a Smart Orbit sample system. Optical rotations were obtained at ambient temperature on a Perkin Elmer Model 343 polarimeter (Na D line) using a microcell with a 1 decimeter path length. Mass spectra determined by LCMS were collected on Thermo Scientific^{™} UltiMate^{™} 3000 UHPLC with electrochemical detector with a fluorescence detector monitored at either 214 or 254 nm, or a Waters Aquity UPLC H-Class Series with photodiode array detector and QDa mass detector. ¹H NMR spectra were recorded at 500 MHz, 400 MHz, and 300 MHz, and ¹³C at 125 MHz. Proton resonances were reported relative to the deuterated solvent peak: 7.27 ppm for CDCl₃, 3.31 ppm (center line signal) for CD₃OD, 2.50 for D6-DMSO and 4.79 for D₂O using the following format: chemical shift (δ) [multiplicity (s= singlet, br s= broad singlet, d= doublet, t= triplet, q= quartet, m= multiplet)]. Carbon resonances were reported as chemical shifts (δ) in parts per million, relative to the center line signal of the respective solvent peak: 77.23 ppm for CDCl₃ and 49.15 ppm for CD₃OD. Commercially available chemicals are purchased from multiple vendors including Sigma-Aldrich, Acros, Enamine, TCI America, Combi-Blocks, Alfa-Aesar, Angene, Ark Pharma, PharmaBlock, Strem Chemicals, Frontier Scientific, and AstaTech, Inc.

### Liquid Chromatography-Mass Spectrometry Methods

### Liquid Chromatography-Mass Spectrometry Method A

Total ion current (TIC) and DAD UV chromatographic traces together with MS and UV spectra associated with the peaks were taken on a UPLC/MS Acquity^{™} system equipped with PDA detector and coupled to a Waters single quadrupole mass spectrometer operating in alternated positive and negative electrospray ionization mode. [LC/MS-ES (+/-): analyses performed using an Acquity UPLC^{™} CSH, C18 column (50 × 2.1mm, 1.7 µm particle size), column temperature 40 °C, mobile phase: A-water + 0.1% HCOOH/ B- CH₃CN + 0.1% HCOOH, flow rate: 1.0 mL/min, runtime = 2.0 min, gradient: t=0 min 3%B, t= 1.5 min 99.9% B, t = 1.9 min 99.9% B, t= 2.0 min 3% B, stop time 2.0 min. Positive ES 100-1000, Negative ES 100-1000, UV detection DAD 210-350 nm.

### Liquid Chromatography-Mass Spectrometry Method B

Total ion current (TIC) and DAD UV chromatographic traces together with MS and UV spectra associated with the peaks were taken on a UPLC/MS Acquity^{™} system equipped with PDA detector and coupled to a Waters single quadrupole mass spectrometer operating in alternated positive and negative electrospray ionization mode. [LC/MS-ES (+/-): analyses performed using an Acquity UPLC^{™} BEH, C18 column (50 × 2.1mm, 1.7 µm particle size), column temperature 40 °C, mobile phase: A- 0.1% v/v aqueous (aq) ammonia solution pH 10/ B- CH₃CN, flow rate: 1.0 mL/min, runtime = 2.0 min, gradient: t=0 min 3%B, t= 1.5 min 99.9% B, t = 1.9 min 99.9% B, t= 2.0 min 3% B, stop time 2.0 min. Positive ES 100-1000, Negative ES 100-1000, UV detection DAD 210-350 nm.

### Liquid Chromatography-Mass Spectrometry Method C

LC/MS-ES (+/-): analyses performed using an AQUITY with PDA detector and QDA Performance, C18 column (50 × 2.1mm, 1.6 µm particle size), column temperature 35 °C, mobile phase: A- 0.1 % Formic acid in Milli Q water (pH= 2.70)/ B- 0.1%Formic acid in water : Acetonitrile (10:90), flow rate: 0.8-1.0 mL/min, runtime = 4.0 min, gradient: t=0 min 3%B, t= 2.7 min 98% B, t = 3.0 min 100% B, t= 3.51 min 3% B, stop time 4.0 min.

### Liquid Chromatography-Mass Spectrometry Method D

LC/MS-ES (+/-): analyses performed using AQUITY H-Class with PDA detector and QDA, C18 column (50 × 2.1mm, 1.6 µm particle size), column temperature 35 °C, mobile phase: A- 0.1 % Formic acid in Milli Q water (pH= 2.70)/ B- 0.1%Formic acid in water : Acetonitrile (10:90), flow rate: 0.8-1.0 mL/min, runtime = 4.0 min, gradient: t=0 min 3%B, t= 2.7 min 98% B, t = 3.0 min 100% B, t= 3.51 min 3% B, stop time 4.0 min.

### Liquid Chromatography-Mass Spectrometry Method E

LC/MS-ES (+/-): analyses performed using AQUITY H-Class with PDA detector and QDA, C18 column (50 × 2.1mm, 1.6 µm particle size), column temperature 35 °C, mobile phase: A- 0.1 % Formic acid in water (pH= 2.70)/ B- 0.1%Formic acid in water : Acetonitrile (10:90), runtime = 9.0 min, gradient: t=0 min 1%B, t= 2.5 min 50% B, t = 4.5 min 97.5% B, t= 6.5 min 1% B, stop time 9.0 min.

### Liquid Chromatography-Mass Spectrometry Method F

LC/MS-ES (+/-): analyses performed using Agilent Infinity II G6125C LCMS, C18 column (50 × 4.6mm, 3.5 µm particle size), column temperature 35 °C, mobile phase: A-5mM Ammonium Bicarbonate in Milli-Qwater (pH = 7.35)/ B- Methanol, runtime = 7.0 min, gradient: t=0 min 8%B, t= 3.0 min 70% B, t = 3.7 min 95% B, t= 4.2 min 100% B, t= 5.21 min 8% B, stop time 7.0 min.

### Liquid Chromatography-Mass Spectrometry Method G

LC/MS-ES (+/-): analyses performed using Waters Alliance 2690 and 996 PDA detector with Micromass ZQ, C18 column (150 × 4.6mm, 3.5 µm particle size), column temperature 35 °C, mobile phase: A- 5mM Ammonium Acetate + 0.1% FA in Water / B-Methanol, runtime = 17.0 min, gradient: t=0 min 10%B, t= 7.0 min 90% B, t = 9.0 min 100% B, t= 14.01 min 10% B, stop time 17.0 min.

### Liquid Chromatography-Mass Spectrometry Method H

LC/MS-ES (+/-): analyses performed using AQUITY with PDA detector and QDA Performance, C18 column (50 × 2.1mm, 1.6 µm particle size), column temperature 35 °C, mobile phase: A- 0.1 % Formic acid inMilli Q water (pH= 2.70)/ B- 0.1%Formic acid in water : Acetonitrile (10:90), flow rate: 0.9 mL/min, runtime = 3.0 min, gradient: t=0 min 5%B, t= 1.8 min 98% B, t = 2.0 min 100% B, t= 2.51 min 5% B, stop time 17.0 min.

### Analytical Methods

¹H Nuclear magnetic resonance (NMR) spectroscopy was carried out using one of the following instruments: a Bruker Avance 400 instrument equipped with probe DUAL 400MHz S1, a Bruker Avance 400 instrument equipped with probe 6 S1 400 MHz 5mm ¹H-¹³C ID, a Bruker Avance III 400 instrument with nanobay equipped with probe Broadband BBFO 5 mm direct, a 400 MHz Agilent Direct Drive instrument with ID AUTO-X PFG probe, all operating at 400 MHz, or an Agilent VNMRS500 Direct Drive instrument equipped with a 5 mm Triple Resonance ¹H{¹³C/¹⁵N} cryoprobe operating at 500 MHz . The spectra were acquired in the stated solvent at around room temperature unless otherwise stated. In all cases, NMR data were consistent with the proposed structures. Characteristic chemical shifts (δ) are given in parts-per-million using conventional abbreviations for designation of major peaks: e.g. s, singlet; d, doublet; t, triplet; q, quartet; dd, doublet of doublets; dt, doublet of triplets; br, broad.

Thin layer chromatography (TLC) refers to silica gel TLC using silica gel F254 (Merck) plates, R_{f} is the distance travelled by the compound divided by the distance travelled by the solvent on a TLC plate. Column chromatography was performed using an automatic flash chromatography (Biotage SP1 or Isolera) system over Biotage silica gel cartridges (KP-Sil or KP-NH) or in the case of reverse phase chromatography over Biotage C18 cartridges (KP-C18).

Prep HPLC were performed on Shimadzu LC-20AP, Waters 2545 and Agilent 1260 infinity. Purity was determined on Waters Alliance e2695- PDA detector 2998 and Agilent 1260 Infinity-II. (Mobile phase: 0.05% HCl in Water/Methanol in gradient elution method).

**Table 5. Abbreviations and Names of Reagents**

| **Abbreviations/Acronyms** | **Full Name/Description** |
|---|---|
| AcOH | Acetic acid |
| aq. | Aqueous |
| CH₃CN | Acetonitrile |
| B₂pin₂ | Bis(pinacolato)diboron |
| Boc₂O | Di-tert-butyl dicarbonate |
| BH₃-SMe₂ | Borane dimethyl sulfide complex |
| *i*-BuMgBr | Isobutyl magnesium bromide |
| *n*-BuLi | *n*-Butyllithium |
| B(O-*i*Pr)₃. | Triisopropyl borate |
| CBzCl | Benzyl chloroformate |
| CDI | 1,1'-Carbonyldiimidazole |
| DAST | Diethylaminosulfur trifluoride |
| DCE | 1,2-Dichloethane |
| DCM | Dichloromethane |
| DIAD | Diisopropyl azodicarboxylate |
| DIBAL | Diisobutylaluminium hydride |
| DIPEA | *N*,*N*-Diisopropylethylamine |
| DMAP | *N*,*N*-dimethylaminopyridine |
| DMF | *N*,*N*'-dimethylformamide |
| DMP | Dess-Martin periodinane |
| DMSO | Dimethylsulfoxide |
| EDCI | 1-Ethyl-3-(3-dimethylaminopropyl)carbodiimide |
| DPPA | Diphenylphosphoryl azide |
| Et₂O | Diethyl ether |
| Et₃N | Triethylamine |
| EtOAc | Ethyl acetate |
| EtOH | Ethanol |
| EtMgBr | Ethylmagnesium bromide |
| HATU | Hexafluorophosphate azabenzotriazole tetramethyl uronium |
| HPLC | High performance liquid chromatography |
| KHMDS | Potassium bis(trimethylsilyl)amide |
| LCMS | Liquid chromatography mass spectrometry |
| LDA | Lithium diisopropylamide |
| Li(ALH)₄ | Lithium aluminum hydride |
| LiAlH(Ot-Bu)₃ | Lithium tri-tert-butoxyaluminum hydride |
| LHNMS | Lithium bis(trimethylsilyl)amide |
| MeOH | Methanol |
| MeI | Methyl Iodide |
| min. | minutes |
| NMR | Nuclear magnetic resonance |
| rt | Room temperature |
| NaBH₄ | Sodium borohydride |
| NaBH(OAc)₃ | Sodium triacetoxyborohydride |
| NaOAc | Sodium acetate |
| NaBH₃CN | Sodium cyanoborohydride |
| PCC | Pyridinium chlorochromate |
| Pd(dba)₂ | Bis(dibenzylideneacetone)palladium |
| Pd(dppf)Cl₂ | [1,1-Bis(diphenylphosphino)ferrocene]dichloropalladium |
| PPh₃ | Triphenylphosphine |
| *i*-PrBr | 2-Bromopropane |
| Sat. | Saturated |
| TBAF | Tetrabutlyammonium fluoride |
| TBSCl/TBDMSCl | *t*-butyldimethylsilyl chloride |
| Ti(O-*i*Pr)₄ | Titanium isopropoxide |
| TEA | Triethylamine |
| TFA | Trifluoroacetic acid |
| TFAA | Trifluoroacetic anhydride |
| THF | Tetrahydrofuran |
| TMEDA | *N*,*N*,*N*',*N'*-Tetramethylethylenediamine |
| TMSCN | Trimethylsilyl cyanide |
| Tf₂O | Trifluoromethanesulfonic anhydride |
| TsOH | p-Toluenesulfonic acid |

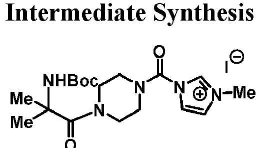

### 1-(4-(2-((tert-Butoxycarbonyl)amino)-2-methylpropanoyl)piperazine-1-carbonyl)-3-methyl-1H-imidazol-3-ium iodide (Intermediate 1)

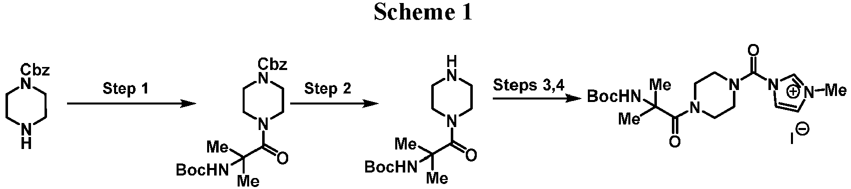

### Reagents: 1) 2-((tert-butoxycarbonyl)amino)-2-methylpropanoic acid, HATU, DIPEA, DMF, rt, 16h 2) 10% Pd/C, CH₃OH, rt, 16h 3) CDI, CH₂Cl₂, rt, 16h 4) MeI, CH₃CN, rt, 16h.

**Step 1: benzyl 4-(2-((t-butoxycarbonyl) amino)-2-methylpropanoyl) piperazine-1-carboxylate.** To a stirred solution of 2-((t-butoxycarbonyl) amino)-2-methylpropanoic acid (35.5 g, 175 mmol) in DMF (350 mL) were added DIPEA (51.2 g, 397mmol) and HATU (90.6 g, 238 mmol) at 0°C. The reaction mixture was stirred at 0°C for 45 min. Benzyl piperazine-1-carboxylate (35 g, 158.9 mmol) was added to the reaction mixture at 0°C and it was stirred at rt for 16 h. The resulting reaction mixture was poured into H₂O (1500 mL) and extracted with EtOAc (3 × 700 mL) and the combined organics were dried over Na₂SO₄, filtered and concentrated under reduced pressure. The resulting crude material was purified by flash chromatography (EtOAc:Hex) to afford the desired product (36 g, 55%) as an off-white solid LCMS[M+H] 406.

**Step 2: *t*-butyl (2-methyl-1-oxo-1(piperazn-1-yl) propan-2-yl)carbamate.** To a stirred solution of benzyl 4-(2-((*t*-butoxycarbonyl) amino)-2-methylpropanoyl) piperazine-1-carboxylate (35 g, 86.4 mmol) in CH₃OH (500 mL) was added 10% Pd/C (3.5 g). The reaction mixture was stirred under hydrogen atmosphere at rt for 16 h. The resulting reaction mixture was filtered through Celite^{®} and washed with CH₃OH (1500 mL). The resulting filtrate was concentrated under reduced pressure and dried to afford the desired product (25.0 g, 100 %) as a viscous oil. LCMS[M+H] 272.

**Step 3: *t*-butyl (1-(4-1*H*-imidazole-1-carbonyl) piperazin-1-yl)-2-methyl-1-oxopropan-2-yl) carbamate.** To a stirred solution of t-butyl (2-methyl-1-oxo-1(piperazn-1-yl) propan-2-yl) carbamate (25 g, 92.2 mmol) in CH₂Cl₂ (300 mL) was added CDI (17.78 g, 110 mmol) at rt. The reaction mixture was stirred at rt for 16 h. The reaction mixture was concentrated under reduced pressure. The resulting crude material was purified by flash chromatography (CH₃OH:CH₂Cl₂) to afford the desired product (30.0 g, 89%) as an off-white solid. ¹H NMR (DMSO-d₆, 400 MHz): δ 8.04 (s, 1H), 7.48 (s, 1H), 7.36 (s, 1H), 7.03 (s, 1H), 3.65-3.52 (m, 4H), 3.51-3.40 (m, 4H), 1.38 (s, 6H), 1.30 (s, 9H). LCMS[M+H] 366.3.

**Step 4: 1-(4-(2-((t-butoxycarbonyl) amino)-2-methylpropanoyl) piperazine-1-carbonyl)-3-methyl-1H-imidazol-3-ium iodide.** To a stirred solution of *t*-butyl (1-(4-1*H-*imidazole-1-carbonyl)piperazin-1-yl)-2-methyl-1-oxopropan-2-yl)carbamate (20 g, 55 mmol) in CH₃CN (250 mL) was added MeI (20.8 ml, 329 mmol) at 0°C. The reaction mixture was stirred at rt for 16 h then concentrated under reduced pressure to afford the desired product (30 g, quantitative) as a pale yellow solid. ¹H NMR (DMSO-d₆, 400 MHz): δ 9.57 9s, 1H), 8.05 (s, 1H), 7.87 (t, 1H), 7.40 (s, 1H), 3.93 (s, 3H), 3.78-3.65 (m, 4H), 3.59-3.45 (m, 4H), 1.40 (s, 6H), 1.32 (S, 9H). LCMS[M+H] 380.2.

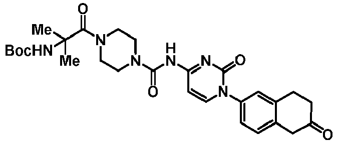

### tert-Butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate (Intermediate 2)

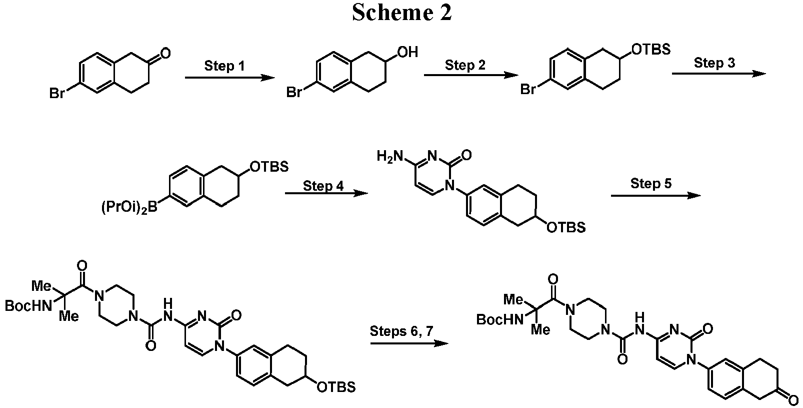

### Reagents: 1) NaBH₄, CH₃OH, rt, 3h 2) TBDMSCl, Imidazole, CH₂Cl₂, rt, 16 h 3) n-BuLi, B(OiPr)₃, THF, -78°C to rt, 3 h 4) Cytosine, TMEDA, Cu(OAc)₂·H₂O, CH₃OH:H₂O (4:1), O₂, rt, 16h 5) 1-(4-(2-((tert-butoxycarbonyl)amino)-2-methylpropanoyl)piperazine-1-carbonyl)-3-methyl-1H-imidazol-3-ium iodide, CH₃CN, 90°C, 16 h 6) MeOH, TsOH, rt, 16 h 7) DMP, CH₂Cl₂, rt, 3 h.

**Step 1: 6-bromo-1,2,3,4-tetrahydronaphthalen-2-ol.** To a stirred solution of 6-bromo-3,4-dihydronaphthalen-2(1H)-one (10 g, 44 mmol) in MeOH (250 mL) at 0°C was added NaBH₄ (1.7 g, 44 mmol). The reaction mixture was stirred at rt for 3h and the volatiles were removed under reduced pressure. The crude solid was dissolved in EtOAc (500 mL) and the reaction mixture was washed with H₂O (1x500 mL). The organics were dried over Na₂SO₄, filtered and concentrated under reduced pressure. The crude product was purified by flash chromatography to afford the desired product (9.0 g, 90%) as a brown oil.

**Step 2: ((6-bromo-1,2,3,4-tetrahydronaphthalen-2-yl)oxy)(*tert-*butyl)dimethylsilane.** To a stirred solution of 6-bromo-1,2,3,4-tetrahydronaphthalen-2-ol (49.0 g, 44 mmol) in CH₂Cl₂ (500 mL) at 0°C were added imidazole (3.2 g, 48 mmol) and t-butyldimethylsilyl chloride (7.2 g, 48 mmol). The reaction mixture was stirred at rt for 16 h. The resulting reaction mixture was poured into H₂O (500 mL) and extracted with CH₂Cl₂ (3 × 700 mL). The combined organics were dried over Na₂SO₄, filtered and concentrated under reduced pressure. The crude product was purified by flash chromatography (EtOAc:Hex) to afford the desired product (85%) as a yellow oil. ¹H NMR (500 MHz, CDCl₃) δ 7.24 (s, 1H), 7.22 (d, 1H), 6.93 (d, 1H), 4.15-4.06 (m, 1H), 2.99-2.88 (m, 2H), 2.80-2.73 (m, 1H), 2.69 (dd, 1H), 1.98-1.91 (m, 1H), 1.84-1.73 (m, 1H), 0.92 (s, 9H), 0.12 (d, 6H).

**Step 3: diisopropyl (6-((*tert*-butyldimethylsilyl)oxy)-5,6,7,8-tetrahydronaphthalen-2-yl)boronate.** To a stirred solution of ((6-bromo-1,2,3,4-tetrahydronaphthalen-2-yl)oxy)(*tert*-butyl)dimethylsilane (3.7 g, 9.5 mmol) in THF (300 mL) at -78 °C under N₂ atmosphere was added n-BuLi (2.5 M in hexanes, 4.1 mL, 10.4 mmol). The reaction mixture was stirred at -78 °C for 30 min. Triisopropyl borate (2.4 mL, 10.4 mmol) was added at -78 °C. The reaction mixture was warmed to rt and stirred for 3 h. The resulting reaction mixture was poured into NH₄Cl solution (100 mL) and extracted with EtOAc (3 × 300 ml). The combined organics were dried over Na₂SO₄, filtered and concentrated under reduced pressure to afford the desired product (90%) as a clear oil.

**Step 4: 4-amino-1-(6-((*tert*-butyldimethylsilyl)oxy)-5,6,7,8-tetrahydronaphthalen-2-yl)pyrimidin-2(1H)-one.** To a solution of diisopropyl (6-((*tert*butyldimethylsilyl)oxy)-5,6,7,8-tetrahydronaphthalen-2-yl)boronate (9.9 g, 25 mmol) and cytosine (3.1 g, 28 mmol) in MeOH:H₂O (500 mL, 4:1) was stirred at rt in open air for 30 min. TMEDA (5.1 mL, 34 mmol) and Cu(OAc)₂·H₂O (5.6 g , 28 mmol) were added and the reaction mixture stirred in open air at rt for 48 h. The reaction mixture was concentrated under reduced pressure and cold H₂O (100 mL) was added into the mixture. The solid was filtered and washed with H₂O (3 × 100 mL) and Et₂O (2 × 60 mL) under reduced pressure. The resulting solid was dried to afford the desired product (5.5 g, 60%) as a white solid. ¹H NMR (400 MHz, DMSO-d₆) δ 7.56 (d, 1H), 7.29-7.08 (m, 3H), 7.03 (d, 2H), 5.75 (d, 1H), 4.15 (s, 1H), 2.97 (dd, 1H), 2.92-2.82 (m, 1H), 2.81-2.71 (m, 1H), 2.66 (dd, 1H), 1.98-1.85 (m, 1H), 1.78-1.62 (m, 1H), 0.87 (s, 9H), 0.09 (d, 6H). LCMS[M+H] 372.2.

**Step 5: tert-butyl (1-(4-((1-(6-((*tert*-butyldimethylsilyl)oxy)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate.** A mixture of 4-amino-1-(6-((*tert-*butyldimethylsilyl)oxy)-5,6,7,8-tetrahydronaphthalen-2-yl)pyrimidin-2(1H)-one (1.5 g, 3.5 mmol) and 1-(4-(2-((*t*-butoxycarbonyl)amino)-2-methylpropanoyl)piperazine-1-carbonyl)-3-methyl-1*H*-imidazol-3-iumiodide (Intermediate 2, 3.1 g, 5.3 mmol) in CH₃CN (45 mL) was heated to 90°C for 16 h. The reaction mixture was concentrated under reduced pressure and the crude material was purified by column chromatography (MeOH:CH₂Cl₂) to afford the title compound (1.67 g, 80%) as a white solid. ¹H NMR (500 MHz, CDCl₃) δ 12.89 (s, 1H), 7.22 (d, 1H), 7.12 (d, 1H), 7.03 (d, 1H), 7.02 (s, 1H), 5.77 (d, 1H), 5.02 (br. s, 1H), 4.17-4.02 (m, 1H), 3.83 (br. s, 2H), 3.74 (br., 2H), 3.69 (br. s, 2H), 3.58 (br. s, 2H), 3.44 (s, 1H), 2.99-2.86 (m, 2H), 2.81-2.66 (m, 2H), 1.96-1.86 (m, 1H), 1.84-1.71 (m, 1H), 1.47 (s, 6H), 1.40 (s, 9H), 1.27-1.15 (m, 1H), 0.87 (s, 9H), 0.07 (s, 6H). LCMS[M+H] 669.4.

**Step 6: *tert*-butyl (1-(4-((1-(6-hydroxy-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate.** To a stirred solution of *tert*-butyl (1-(4-((1-(6-((*tert*-butyldimethylsilyl)oxy)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate (300 mg, 0.45 mmol) in MeOH (10 mL) at 0°C was added TsOH (172 mg, 0.90 mmol). The reaction mixture was stirred at rt for 16 h then poured into aq. NaHCO₃ (25 mL) and extracted with CH₂Cl₂ (3× 50 mL). The combined organics were dried over Na₂SO₄, filtered, concentrated under reduced pressure, and purified by column chromatography (MeOH: CH₂Cl₂) to afford the desired product as an off-white solid (290 mg, 93%). LCMS[M+H] 555.3.

**Step 7: *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate.** To a stirred solution of *tert*-butyl (1-(4-((1-(6-hydroxy-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate (290 mg 0.42 mmol) in DCM at rt (5 mL) was added Dess-Martin periodinane (259 mg, 0.63mmol). The reaction mixture was stirred at rt for 3 h, poured into saturated NaHCO₃ (20 mL) and extracted with CH₂Cl₂ (3 × 50 mL). The combined organics were dried over Na₂SO₄, filtered, and concentrated under reduced pressure below 35°C to afford the desired product (258 mg, quantitative) as an off-white solid. LCMS[M+H] 553.3.

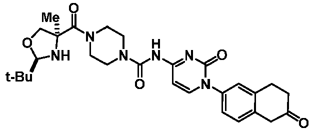

### 4-((2R,4S)-2-(tert-Butyl)-4-methyloxazolidine-4-carbonyl)-N-(2-oxo-1-(6-oxo-5,6,7,8-tetrahydronaphthalen-2-yl)-1,2-dihydropyrimidin-4-yl)piperazine-1-carboxamide (Intermediate 3)

Prepared as in Scheme 2 from 1-(4-((2R,4S)-2-(*tert*-butyl)-4-methyloxazolidine-4-carbonyl)piperazine-1-carbonyl)-3-methyl-1H-imidazol-3-ium iodide and 4-amino-1-(6-oxo-5,6,7,8-tetrahydronaphthalen-2-yl)pyrimidin-2(1H)-one. LCMS[M+H] 537.3.

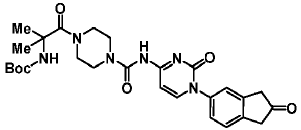

### tert-butyl (2-methyl-1-oxo-1-(4-((2-oxo-1-(2-oxo-2,3-dihydro-1H-inden-5-yl)-1,2-dihydropyrimidin-4-yl)carbamoyl)piperazin-1-yl)propan-2-yl)carbamate (Intermediate 4)

Prepared as in Scheme 2 from benzyl 4-(2-((*tert*-butoxycarbonyl) amino)-2-methylpropanoyl) piperazine-1-carboxylate and 4-amino-1-(2-((*tert*-butyldimethylsilyl)oxy)-2,3-dihydro-1H-inden-5-yl)pyrimidin-2(1H)-one. LCMS[M+H] 541.2.

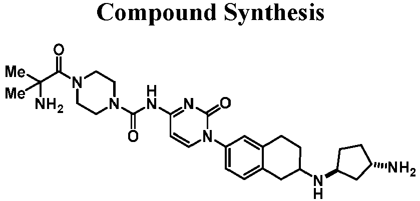

### 4-(2-Amino-2-methylpropanoyl)-N-(1-(6-(((1S,3S)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (Compound 9)

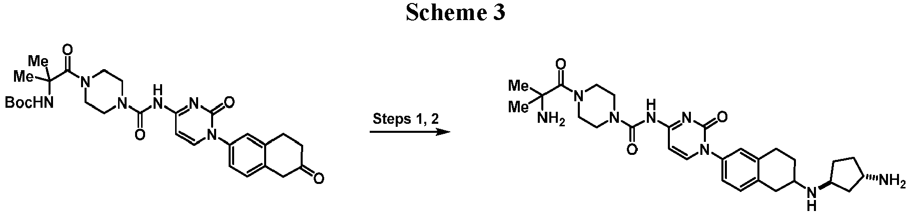

### Reagents: 1) tert-butyl ((1S,3S)-3-aminocyclopentyl)carbamate, Na(OAc)₃BH, DCE, rt, 16 h 2) HCl/MeOH, rt, 4 h.

**Step 1: *tert*-butyl (1-(4-((1-(6-(((1S,3S)-3-((*tert-*butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate.** To a solution of *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate (8.5 g, 15 mmol) in DCE (500 mL) was added *tert*-butyl ((1S,3S)-3-aminocyclopentyl)carbamate (3.4 g, 17 mmol), Na(OAc)₃BH (4.9 g, 23 mmol) and the reaction mixture was stirred at rt for 16h. The reaction mixture was treated with aq. NaHCO₃ (500 mL), the phases were separated, and the aqueous layer was extracted with CH₂Cl₂ (3 × 500 mL). The combined organics were dried over Na₂SO₄, concentrated under reduced pressure and purified by column chromatography (CH₂Cl₂ :MeOH:NH₄OH) to afford the desired compound as a gray solid (75%). ¹H NMR (500 MHz, CDCl₃) δ 7.22 (d, 1H), 7.15 (d, 1H), 7.05 (d,1H), 7.04 (s,1H), 5.79 (d, 1H), 4.86 (br. s, 1H), 4.49 (br. s, 1H), 4.08 (br. s, 1H), 3.86 (br. s, 2H), 3.76 (br. s, 2H), 3.71 (br. s, 2H), 3.61 (s, 2H), 3.48 (br. s, 1H), 3.13-2.98 (m, 2H), 2.96-2.75 (m, 2H), 2.71-2.56 (m, 1H), 2.24-1.98 (m, 3H), 1.91-1.74 (m, 2H), 1.64 (s, 1H), 1.51 (s, 6H), 1.43 (d, 20H), 1.28 (s, 1H). LCMS[M+H] 737.8.

**Step 2: 4-(2-amino-2-methylpropanoyl)-N-(1-(6-(((1S,3S)-3-aminocyclopentyl)amino)-5,6,7,8-tetrahydronaphthalen-2-yl)-2-oxo-1,2-dihydropyrimidin-4-yl)piperazine-1-carboxamide hydrochloride salt.** *tert*-Butyl (1-(4-((1-(6-(((1S,3S)-3-((*tert*-butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate was dissolved in a solution of HCl/MeOH (5 mL) and stirred for 4 h. The volatiles were removed under reduced pressure and the crude solid was purified by reverse phase HPLC (H₂O:CH₃CN:TFA) and concentrated under reduced pressure. Addition and evaporation under reduced pressure with HCl/MeOH (3 × 15 mL) afforded the desired product. ¹H NMR (400 MHz, D2O) δ 7.76 (d, 1H), 7.20 (d, 1H), 7.09 (d, 2H), 6.68 (d, 1H), 4.03-3.93 (m, 1H), 3.83-3.73 (m,1H), 3.63 (s, 4H), 3.58 (s, 6H), 3.23 (d, 1H), 2.88-2.77 (m, 3H), 2.33-2.12 (m, 5H), 1.83-1.62 (m, 2H), 1.59 (s, 6H). LCMS[M+H] 537.2.

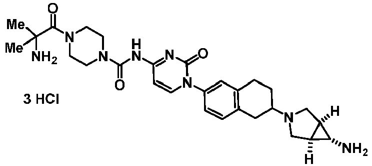

### 4-(2-Amino-2-methylpropanoyl)-N-(1-(6-((1R,5S,6s)-6-amino-3-azabicyclo[3.1.0]hexan-3-yl)-5,6,7,8-tetrahydronaphthalen-2-yl)-2-oxo-1,2-dihydropyrimidin-4-yl)piperazine-1-carboxamide hydrochloride salt (Compound 1)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((1R,5S,6s)-3-azabicyclo[3.1.0]hexan-6-yl)carbamate. ¹H NMR (400 MHz, D₂O) δ 7.87 (d, 1H), 7.30 (d, 1H), 7.21 (s, 2H), 6.80 (d, 1H), 4.01 (d, 2H), 3.71 (d, 10 H), 3.33 (s, 1H), 3.09-2.87 (m, 4H), 2.83 (s, 1H), 2.42 (s, 2H), 2.34 (s, 1H), 1.89 (m, 1H), 1.70 (d, 6H). LCMS[M+H] 535.2.

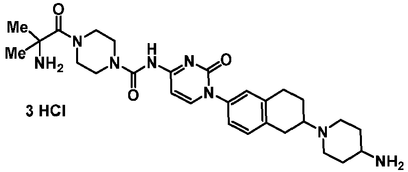

### 4-(2-Amino-2-methylpropanoyl)-N-(1-(6-(4-aminopiperidin-1-yl)-5,6,7,8-tetrahydronaphthalen-2-yl)-2-oxo-1,2-dihydropyrimidin-4-yl)piperazine-1-carboxamide hydrochloride salt (Compound 2)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl piperidin-4-ylcarbamate. ¹H NMR (400 MHz, D₂O) δ 8.02 (d, 1H), 7.39 (d, 1H), 7.28 (s, 1H),7.27 (d, 1H), 6.82 (d, 1H), 3.80 (s, 6H), 3.77-3.71 (m, 5H), 3.68-3.57 (m, 1H), 3.42-3.30 (m, 3H), 3.25-3.15 (m, 1H), 3.15-2.94 (m, 2H), 2.51-2.33 (m, 3H), 2.10-1.90 (m, 3H), 1.75 (s, 6H). LCMS[M+H] 537.3.

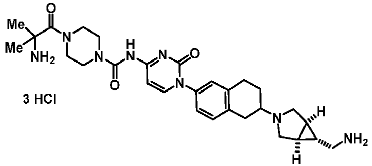

### 4-(2-Amino-2-methylpropanoyl)-N-(1-(6-((1R,SS,6s)-6-(aminomethyl)-3-azabicyclo[3.1.0]hexan-3-yl)-5,6,7,8-tetrahydronaphthalen-2-yl)-2-oxo-1,2-dihydropyrimidin-4-yl)piperazine-1-carboxamide hydrochloride salt (Compound 3)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (((1R,5S,6r)-3-azabicyclo[3.1.0]hexan-6-yl)methyl)carbamate. ¹H NMR (400 MHz, D₂O) δ 7.95 (d, 1H), 7.31 (d, 1H), 7.22 (s, 2H), 6.78 (d, 1H), 3.91 (d, 2H), 3.75 (s, 2H), 3.71 (s, 6H), 3.66-3.56 (m, 2H), 3.36 (d, 1H), 3.08-2.86 (m, 6H), 2.35 (s, 1H), 2.04 (d, 2H), 1.94-1.83 (m, 1H), 1.70 (d, 6H), 1.39-1.30 (m, 1H). LCMS[M+H] 549.3.

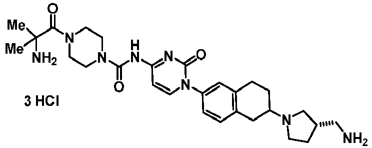

### 4-(2-amino-2-methylpropanoyl)-N-(1-(6-((S)-3-(aminomethyl)pyrrolidin-1-yl)-5,6,7,8-tetrahydronaphthalen-2-yl)-2-oxo-1,2-dihydropyrimidin-4-yl)piperazine-1-carboxamide hydrochloride salt (Compound 4)

Prepared as in scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (R)-(pyrrolidin-3-ylmethyl)carbamate. ¹H NMR (400 MHz, D₂O) δ 7.94 (d, 1H), 7.33 (d, 1H), 7.22 (d, 2H), 6.79 (d, 1H), 4.06-3.85 (m, 1H), 3.87-3.27 (m, 12H), 3.25-2.65 (m, 7H), 2.56-2.29 (m, 2H), 2.52-2.30 (m 2H), 1.71 (s, 6H). LCMS[M+H] 537.3.

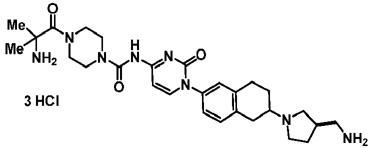

### 4-(2-Amino-2-methylpropanoyl)-N-(1-(6-((R)-3-(aminomethyl)pyrrolidin-1-yl)-5,6,7,8-tetrahydronaphthalen-2-yl)-2-oxo-1,2-dihydropyrimidin-4-yl)piperazine-1-carboxamide hydrochloride salt (Compound 5)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (S)-(pyrrolidin-3-ylmethyl)carbamate. ¹H NMR (400 MHz, D₂O) δ 8.00 (d, 1H), 7.34 (d, 1H), 7.23 (d, 2H), 6.78 (d, 1H), 4.05-3.87 (m, 1H), 3.86-3.27 (m, 12H), 3.27-2.83 (m, 7H), 2.81-2.24 (m, 2H), 2.07-1.72 (m, 2H), 1.70 (s, 6H). LCMS[M+H] 537.4.

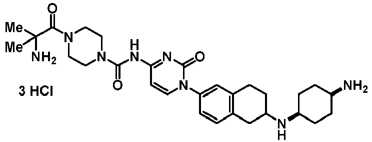

### 4-(2-Amino-2-methylpropanoyl)-N-(1-(6-(((cis)-4-aminocyclohexyl)amino)-5,6,7,8-tetrahydronaphthalen-2-yl)-2-oxo-1,2-dihydropyrimidin-4-yl)piperazine-1-carboxamide hydrochloride salt (Compound 6)

Prepared as in Scheme 3 from tert-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((cis)-4-aminocyclohexyl)carbamate. ¹H NMR (400 MHz, D₂O) δ 7.92-7.84 (m, 1H), 7.34 (d, 1H), 7.25 (s, 2H), 6.85 (d, 1H), 3.89-3.58 (m, 11H), 3.43-3.33 (m, 1H), 3.07-2.94 (m, 3H), 2.44-2.31 (m, 1H), 2.17-2.07 (m, 2H), 1.98 (s, 4H), 1.85-1.78 (m, 1H), 1.74 (s, 8H). LCMS[M+H] 551.3.

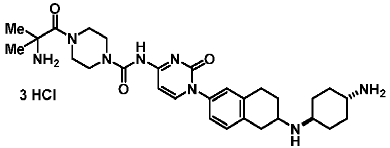

### 4-(2-Amino-2-methylpropanoyl)-N-(1-(6-(((trans)-4-aminocyclohexyl)amino)-5,6,7,8-tetrahydronaphthalen-2-yl)-2-oxo-1,2-dihydropyrimidin-4-yl)piperazine-1-carboxamide hydrochloride salt (Compound 7)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((*trans*)-4-aminocyclohexyl)carbamate. ¹H NMR (400 MHz, D₂O) 1H NMR (400 MHz, D2O) δ 7.91 (d, 1H), 7.35 (d, 1H), 7.25 (d, 2H), 6.84 (d, 1H), 3.89-3.69 (m, 9H), 3.50 (s, 1H), 3.40-3.23 (m, 2H), 3.09-2.92 (m, 3H), 2.32 (s, 3H), 2.23 (s, 2H), 1.98-1.85 (m, 1H), 1.75 (s, 6H), 1.68-1.53 (m, 4H). LCMS[M+H] 551.3.

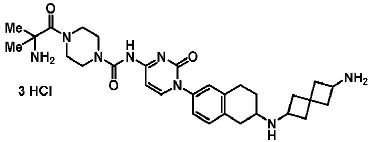

### 4-(2-Amino-2-methylpropanoyl)-N-(1-(6-((6-aminospiro[3.3]heptan-2-yl)amino)-5,6,7,8-tetrahydronaphthalen-2-yl)-2-oxo-1,2-dihydropyrimidin-4-yl)piperazine-1-carboxamide hydrochloride salt (Compound 8)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (6-aminospiro[3.3]heptan-2-yl)carbamate. ¹H NMR (400 MHz, D₂O) δ 7.90 (d, 1H), 7.34 (d, 1H), 7.25 (s, 2H), 6.84 (d, 1H), 3.99 (s, 1H), 3.86-3.68 (m, 8H), 3.64 (s, 1H), 3.33-3.23 (m, 1H), 2.99 (s, 3H), 2.71-2.56 (m, 2H), 2.56-2.40 (m, 2H), 2.39-2.21 (m, 6H), 1.91 (s, 1H), 1.75 (s, 6H). LCMS[M+H] 563.3.

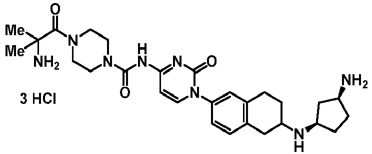

### 4-(2-Amino-2-methylpropanoyl)-N-(1-(6-(((1R,3S)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (Compound 10)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((1S,3R)-3-aminocyclopentyl)carbamate. ¹H NMR (400 MHz, D₂O) δ 7.89 (d, 1H), 7.36 (d, 1H), 7.25 (d, 2H), 6.85 (d, 1H), 4.06-3.95 (m, 1H), 3.85-3.68 (m, 10H), 3.39 (d, 1H), 3.07-2.93 (m, 3H), 2.82-2.71 (m, 1H), 2.42-2.21 (m, 3H), 2.05-1.89 (m, 3H), 1.89-1.78 (m, 1H), 1.75 (s, 6H). LCMS[M+H] 537.3.

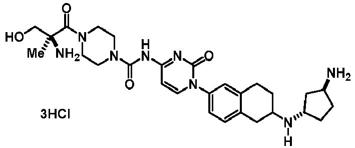

### 4-((S)-2-Amino-3-hydroxy-2-methylpropanoyl)-N-(1-(6-(((1S,3S)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (Compound 24)

Prepared as in Scheme 3 from 4-((2R,4S)-2-(*tert*-butyl)-4-methyloxazolidine-4-carbonyl)-N-(2-oxo-1-(6-oxo-5,6,7,8-tetrahydronaphthalen-2-yl)-1,2-dihydropyrimidin-4-yl)piperazine-1-carboxamide and *tert*-butyl ((1S,3S)-3-aminocyclopentyl)carbamate. ¹H NMR (400 MHz, D₂O) δ 7.89-7.84 (m, 1H) 7.36 (d, 1H), 7.25 (d, 2H), 6.86 (d, 1H), 4.16 (t, 2H), 3.92 (d, 2H), 3.79 (s, 4H), 3.73 (s, 5H), 3.37 (s, 1H), 3.02 (s, 3H), 2.41 (d, 2H), 2.37-2.28 (m, 3H), 1.99-1.86 (m, 1H) 1.87-1.85 (m, 2H), 1.70 (s, 3H). LCMS[M+H] 553.5.

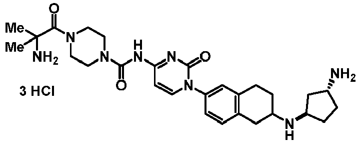

### 4-(2-Amino-2-methylpropanoyl)-N-(1-(6-(((1R,3R)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (Compound 25)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((1R,3R)-3-aminocyclopentyl)carbamate. ¹H NMR (400 MHz, D₂O) δ 7.97 (d, 1H), 7.37 (d, 1H), 7.26 (s, 1H), 7.26 (d, 1H), 6.83 (d, 1H), 4.22-4.06 (m, 1H), 4.01-3.86 (m, 1H), 3.79 (s, 4H), 3.74 (s, 6H), 3.44-3.33 (m, 1H), 3.07-2.93 (m, 3H), 2.53-2.27 (m, 5H), 2.01-1.76 (m, 2H), 1.75 (s, 6H). LCMS[M+H] 537.4.

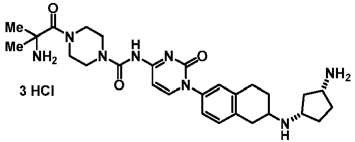

### 4-(2-Amino-2-methylpropanoyl)-N-(1-(6-(((1S,3R)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (Compound 26)

Prepared as in Scheme 3 from tert-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((1R,3S)-3-aminocyclopentyl)carbamate. ¹H NMR (400 MHz, D₂O) δ 8.03 (d, 1H), 7.37 (d, 1H), 7.26 (d, 2H), 6.82 (d, 1H), 4.06-3.96 (m, 1H), 3.80 (s, 5H), 3.75 (s, 6H), 3.44-3.33 (m, 1H), 3.08-2.95 (m, 3H), 2.85-2.72 (m, 1H), 2.42-2.19 (m, 3H), 2.05-1.92 (m, 2H), 1.89-1.79 (m, 1H), 1.75 (s, 6H). LCMS[M+H] 537.4.

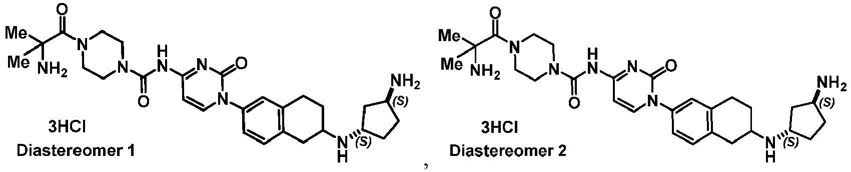

### 4-(2-Amino-2-methylpropanoyl)-N-(1-((S)-6-(((1S,3S)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (Compound 27 and Compound 28)

*tert-*Butyl (1-(4-((1-(6-(((1S,3S)-3-((*tert-*butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate was separated through Chiral HPLC to afford the optically pure stereoisomers.

| | |
|---|---|
| **Column** | Chromegachiral CCO |
| **Column size** | 250 × 4.6 mm 5 mm |
| **Mobile phase** | (A) 0.1% Triethyl Amine in n-Heptane |
| | (B) Ethanol : Methanol (50:50) |
| **Ratio of Mobile Phase (Isocratic)** | A: B = 85 : 15 |
| **Column Temperature** | Ambient |
| **Auto Sampler Temperature** | Ambient |
| **Run time** | 50 min |
| **Retention Time for optically pure stereoisomers** | 22.10 min; |
| | 24.22 min. |

First eluting Diastereomer 1: ¹H NMR (400 MHz, CDCl₃) δ 7.22 (d, 1H), 7.15 (d, 1H), 7.06 (d, 1H), 7.05 (s, 1H), 5.79 (d, 1H), 4.85 (br. s, 1H), 4.48 (br. s, 1H), 4.07 (br. s, 1H), 3.86 (br. s, 2H), 3.75 (br. s, 2H), 3.71 (br. s, 2H), 3.61 (s, 2H), 3.48-3.38 (m, 1H), 3.08-2.95 (m, 2H), 2.95-2.77 (m, 2H), 2.58 (dd, 1H), 2.22-1.99 (m, 3H), 1.85-1.69 (m, 2H), 1.63-1.54 (m, 1H), 1.51 (s, 6H), 1.44 (d, 18H), 1.41-1.30 (m, 2H), 1.25 (s, 1H). LCMS[M+H] 737.8.

Compound 27 was prepared from the optically pure first eluting Diastereomer 1 *tert*-butyl (1-(4-((1-(6-(((1S,3S)-3-((*tert*-butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate in a similar fashion as Step 2 in Scheme 3. ¹H NMR (500 MHz, D₂O) δ 7.97 (d, 1H), 7.38 (d, 1H), 7.28 (s, 1H), 7.26 (d, 2H), 6.85 (d, 1H), 4.23-4.11 (m, 1H), 4.01-3.90 (m, 1H), 3.88-3.68 (m, 9H), 3.40 (dd, 1H), 3.15-2.96 (m, 3H), 2.54-2.37 (m, 3H), 2.35 (t, 2H), 2.00-1.91 (m, 1H), 1.90-1.78 (m, 2H), 1.76 (s, 6H). LCMS[M+H] 537.4.

Second eluting Diastereomer 2: ¹H NMR (400 MHz, CDCl₃) δ 7.22 (d, 1H), 7.15 (d, 1H), 7.05 (dd, 2H), 5.78 (d, 1H), 4.86 (br. s, 1H), 4.48 (br. s, 1H), 4.07 (br. s, 1H), 3.85 (br. s, 2H), 3.75 (br. s, 2H), 3.71 (br. s, 2H), 3.61 (br. s, 2H), 3.47-3.39 (m, 1H), 3.07-2.95 (m, 2H), 2.95-2.77 (m, 2H), 2.57 (dd, 1H), 2.20-1.97 (m, 3H), 1.75 (t, 2H), 1.64-1.54 (m, 1H), 1.51 (s, 6H), 1.43 (d, 18H), 1.40-1.31 (m, 2H), 1.26 (s, 1H). LCMS[M+H] 737.8.

Compound 28 was prepared from the optically pure second eluting Diastereomer 2 *tert*-butyl (1-(4-((1-(6-(((1S,3S)-3-((*tert*-butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate in a similar fashion as Step 2 in Scheme 3. ¹H NMR (500 MHz, D₂O) δ 7.90 (d, 1H), 7.33 (d, 1H), 7.22 (s, 2H), 6.80 (d, 1H), 4.14-4.06 (m, 1H), 3.93-3.86 (m, 1H), 3.83-3.64 (m, 10H), 3.37 (dd, 1H), 3.06-2.91 (m, 3H), 2.45-2.31 (m, 4H), 2.28 (t, 2H), 1.96-1.85 (m, 1H), 1.86-1.73 (m, 1H), 1.71 (s, 6H). LCMS[M+H] 537.4.

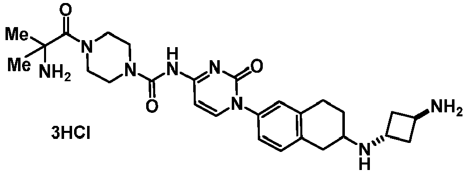

### 4-(2-Amino-2-methylpropanoyl)-N-(1-(6-((trans-3-aminocyclobutyl)amino)-5,6,7,8-tetrahydronaphthalen-2-yl)-2-oxo-1,2-dihydropyrimidin-4-yl)piperazine-1-carboxamide hydrochloride salt (Compound 29)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert-*butyl (*trans*-3-aminocyclobutyl)carbamate. ¹H NMR (400 MHz, D₂O) δ 7.86 (d, 1H), 7.35 (d, 1H), 7.25 (d, 1H), 7.24 (d, 1H), 6.86 (d, 1H), 4.41-4.29 (m, 1H), 4.17-4.06 (m, 1H), 3.79 (br. s, 3H), 3.73 (br. s, 6H), 3.31 (dd, 1H), 3.05-2.93 (m, 3H), 2.90-2.70 (m, 4H), 2.36-2.26 (m, 1H), 2.00-1.89 (m, 1H), 1.75 (s, 6H). LCMS[M+H] 523.4.

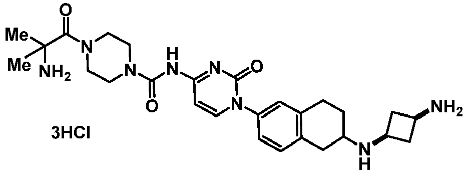

### 4-(2-Amino-2-methylpropanoyl)-N-(1-(6-(((cis-3-aminocyclobutyl)amino)-5,6,7,8-tetrahydronaphthalen-2-yl)-2-oxo-1,2-dihydropyrimidin-4-yl)piperazine-1-carboxamide hydrochloride salt (Compound 30)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (*cis*-3-aminocyclobutyl)carbamate. ¹H NMR (400 MHz, D₂O) δ 7.88 (d, 1H), 7.35 (d, 1H),7.25 (s, 1H), 7.24 (d, 1H), 6.85 (d, 1H), 4.06-3.90 (m, 1H), 3.86-3.66 (m, 10H), 3.36 (dd, 1H), 3.10-2.88 (m, 5H), 2.49 (q, 2H), 2.36-2.25 (m, 1H), 2.02-1.91 (m, 1H), 1.75 (s, 6H). LCMS[M+H] 523.31.

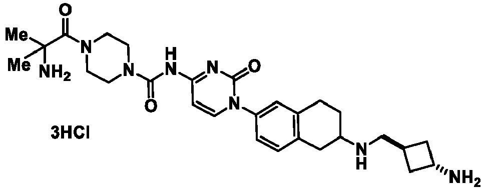

### 4-(2-Amino-2-methylpropanoyl)-N-(1-(6-((((trans)-3-aminocyclobutyl)methyl)amino)-5,6,7,8-tetrahydronaphthalen-2-yl)-2-oxo-1,2-dihydropyrimidin-4-yl)piperazine-1-carboxamide hydrochloride salt (Compound 31)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (*trans*-3-(aminomethyl)cyclobutyl)carbamate. ¹H NMR (500 MHz, D₂O) δ 7.93 (d, 1H), 7.39 (d, 1H), 7.29 (d, 1H), 7.28 (d, 1H), 6.87 (d, 1H), 4.06-3.97 (m, 1H), 3.82 (s, 4H), 3.76 (s, 5H), 3.71 (s, 1H), 3.44 (d, 2H), 3.12-2.95 (m, 3H),2.94-2.85(m, 1H), 2.54-2.45 (m, 2H), 2.45-2.36 (m, 3H), 2.00-1.90 (m, 1H), 1.78 (s, 6H). LCMS[M+H] 537.37.

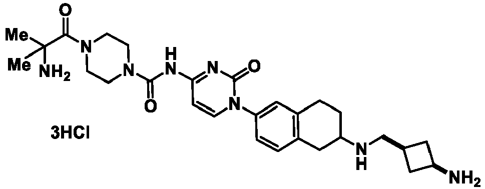

### 4-(2-Amino-2-methylpropanoyl)-N-(1-(6-((cis-3-aminocyclobutyl)methyl)amino)-5,6,7,8-tetrahydronaphthalen-2-yl)-2-oxo-1,2-dihydropyrimidin-4-yl)piperazine-1-carboxamide hydrochloride salt (Compound 32)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (*cis*-3-(aminomethyl)cyclobutyl)carbamate. ¹H NMR (500 MHz, D₂O) δ 7.94 (d, 1H), 7.39 (d, 1H), 7.29 (d, 1H), 7.28 (d, 1H), 6.87 (d, 1H), 3.89-3.72 (m, 9H), 3.72-3.63 (m, 1H), 3.43-3.31 (m, 3H), 3.10-2.94 (m, 3H), 2.73-2.56 (m, 3H), 2.43-2.32 (m, 1H), 2.13-2.03 (m, 2H), 2.00-1.88 (m, 1H), 1.78 (s, 6H). LCMS[M+H] 537.3.

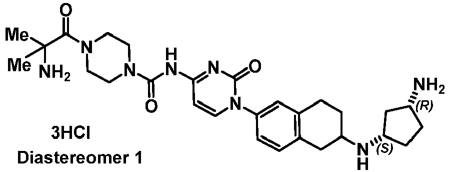

### 4-(2-Amino-2-methylpropanoyl)-N-(1-(6-(((1S,3R)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (first eluting diastereomer, Compound 33)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((1R,3S)-3-aminocyclopentyl)carbamate followed by separation by Chiral HPLC and deprotection of the optically pure stereoisomer. ¹H NMR (500 MHz, D₂O) δ 8.04 (d, 1H), 7.40 (d, 1H), 7.30 (s, 1H), 7.29 (d, 1H), 6.85 (d, 1H), 4.08-3.99 (m, 1H), 3.86- 3.74 (m, 10H), 3.42 (dd, 1H), 3.11-3.03 (m, 3H), 2.85-2.76 (m, 1H), 2.43-2.26 (m, 3H), 2.05 - 1.92 (m, 3H), 1.91-1.83 (m, 1H), 1.78 (s, 6H). LCMS[M+H] 537.4.

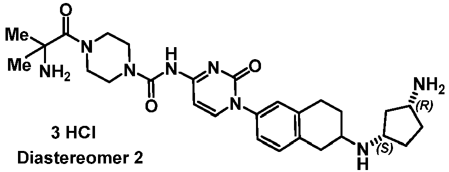

### 4-(2-Amino-2-methylpropanoyl)-N-(1-(6-(((1S,3R)-3-aminocyclopentyl)amino)-5,6,7,8-tetrahydronaphthalen-2-yl)-2-oxo-1,2-dihydropyrimidin-4-yl)piperazine-1-carboxamide hydrochloride salt (second eluting diastereomer, Compound 34)

Prepared as in Scheme 3 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl ((1R,3S)-3-aminocyclopentyl)carbamate followed by separation by Chiral HPLC and deprotection of the optically pure stereoisomer. ¹H NMR (500 MHz, D₂O) δ 8.04 (d, 1H), 7.40 (d, 1H), 7.30 (s, 1H), 7.29 (d, 1H), 6.85 (d, 1H), 4.08-3.99 (m, 1H), 3.91-3.71 (m, 10H), 3.42 (dd, 1H), 3.12-2.97 (m, 3H), 2.86-2.74 (m, 1H), 2.44-2.24 (m, 3H), 2.08-1.92 (m, 3H), 1.92-1.83 (m, 1H), 1.78 (s, 6H). LCMS[M+H] 537.4.

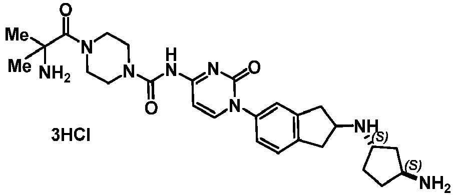

### 4-(2-Amino-2-methylpropanoyl)-N-(1-(2-(((1S,3S)-3-aminocyclopentyl)amino)-2,3-dihydro-1H-inden-5-yl)-2-oxo-1,2-dihydropyrimidin-4-yl)piperazine-1-carboxamide hydrochloride salt (Compound 36)

Prepared as in Scheme 3 from *tert*-butyl (2-methyl-1-oxo-1-(4-((2-oxo-1-(2-oxo-2,3-dihydro-1H-inden-5-yl)-1,2-dihydropyrimidin-4-yl)carbamoyl)piperazin-1-yl)propan-2-yl)carbamate and *tert*-butyl ((1S,3S)-3-aminocyclopentyl)carbamate. ¹H NMR (500 MHz, D₂O) δ 7.94 (d, 1H), 7.52 (d, 1H), 7.41 (s, 1H), 7.34 (d, 1H), 6.88 (d, 1H), 4.36-4.20 (m, 1H), 4.13-4.01 (m, 1H), 3.99-3.92 (m, 1H), 3.82 (br. s, 4H), 3.75 (br. s, 4H), 3.57 (dd, 2H), 3.26 (dd, 2H), 2.52-2.39 (m, 2H), 2.35 (t, 2H), 1.95-1.79 (m, 2H), 1.78 (s, 6H). LCMS[M+H] 523.3.

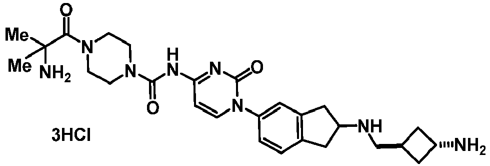

### 4-(2-Amino-2-methylpropanoyl)-N-(1-(2-(((trans-3-aminocyclobutyl)methyl)amino)-2,3-dihydro-1H-inden-5-yl)-2-oxo-1,2-dihydropyrimidin-4-yl)piperazine-1-carboxamide hydrochloride salt (Compound 37)

Prepared as in Scheme 3 from *tert*-butyl (2-methyl-1-oxo-1-(4-((2-oxo-1-(2-oxo-2,3-dihydro-1H-inden-5-yl)-1,2-dihydropyrimidin-4-yl)carbamoyl)piperazin-1-yl)propan-2-yl)carbamate and *tert*-butyl (*trans*-3-(aminomethyl)cyclobutyl)carbamate. ¹H NMR (400 MHz, D₂O) δ 7.97 (d, 1H), 7.49 (d, 1H), 7.38 (s, 1H), 7.31 (d, 1H), 6.84 (d, 1H), 4.24 (t, 1H), 4.02-3.92 (m, 1H), 3.79 (br. s, 3H), 3.74 (br. s, 5H), 3.55 (dd, 2H), 3.36 (d, 2H), 3.23 (dd, 2H), 2.92-2.75 (m, 1H), 2.52-2.41 (m, 2H), 2.41-2.31 (m, 2H), 1.75 (s, 6H). LCMS[M+H] 523.3.

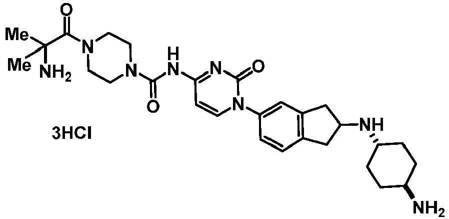

### 4-(2-Amino-2-methylpropanoyl)-N-(1-(2-((trans-4-aminocyclohexyl)amino)-2,3-dihydro-1H-inden-5-yl)-2-oxo-1,2-dihydropyrimidin-4-yl)piperazine-1-carboxamide hydrochloride salt (Compound 38)

Prepared as in Scheme 3 from *tert*-butyl (2-methyl-1-oxo-1-(4-((2-oxo-1-(2-oxo-2,3-dihydro-1H-inden-5-yl)-1,2-dihydropyrimidin-4-yl)carbamoyl)piperazin-1-yl)propan-2-yl)carbamate and *tert*-butyl (*trans*-4-aminocyclohexyl)carbamate. ¹H NMR (400 MHz, D₂O) δ 7.92 (d, 1H), 7.49 (d, 1H), 7.38 (s, 1H), 7.31 (d, 1H), 6.86 (d, 1H), 4.46-4.35 (m, 1H), 3.79 (br. s, 3H), 3.74 (br. s, 5H), 3.54 (dd, 2H), 3.34 (d, 2H), 3.21 (dd, 2H), 2.41-2.16 (m, 4H), 1.75 (s, 6H), 1.68-1.53 (m, 4H). LCMS[M+H] 537.3.

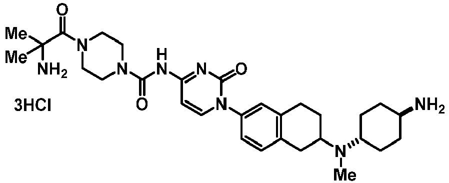

### 4-(2-Amino-2-methylpropanoyl)-N-(1-(6-(((trans)-4-aminocyclohexyl)(methyl)amino)-5,6,7,8-tetrahydronaphthalen-2-yl)-2-oxo-1,2-dihydropyrimidin-4-yl)piperazine-1-carboxamide hydrochloride salt (Compound 15)

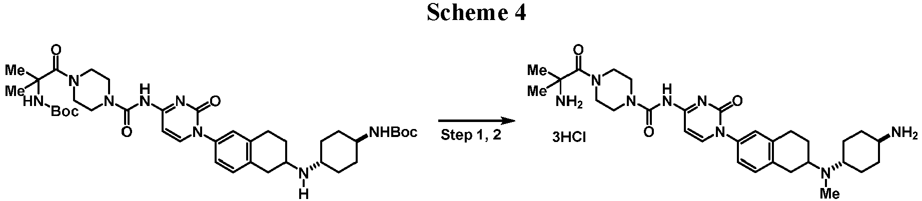

### Reagents: Step 1) 4 Å Molecular sieves, 37% aq. solution of formaldehyde, NaCNBH₃, MeOH, rt, 16h 2) 2M HCl in MeOH.

**Step 1: *tert*-butyl ((1r,4r)-4-((6-(4-(4-(2-((*tert*-butoxycarbonyl)amino)-2-methylpropanoyl)piperazine-1-carboxamido)-2-oxopyrimidin-b1(2H)-yl)-1,2,3,4-tetrahydronaphthalen-2-yl)(methyl)amino)cyclohexyl)carbamate.** To a stirring suspension of *tert*-butyl ((*trans*)-4-((6-(4-(4-(2-((*tert*-butoxycarbonyl)amino)-2-methylpropanoyl)piperazine-1-carboxamido)-2-oxopyrimidin-1(2H)-yl)-1,2,3,4-tetrahydronaphthalen-2-yl)amino)cyclohexyl)carbamate (0.04 g, 0.05 mmol), and 4Å molecular sieves (1.5g) in methanol, were added formaldehyde (30% solution in water, 1.5 mL) followed by sodium cyanoborohydride (5 mg, 0.08 mmol) under N₂ atmosphere and stirred for 16 h at rt. The reaction mixture was filtered through Celite^{®} and the solvent was evaporated. Saturated aq. NaHCO₃ was added and the compound was extracted with ethyl acetate (2x10 mL). The combined organic layers were dried over Na₂SO₄, filtered, concentrated under reduced pressure and the crude material was purified by flash column chromatography to afford the title compound (32 mg, 78% yield) as a white solid.

**Step 2: 4-(2-amino-2-methylpropanoyl)-N-(1-(6-(((1r,4r)-4-aminocyclohexyl)(methyl)amino)-5,6,7,8-tetrahydronaphthalen-2-yl)-2-oxo-1,2-dihydropyrimidin-4-yl)piperazine-1-carboxamide hydrochloride salt.** The compound *tert*-butyl ((1r,4r)-4-((6-(4-(4-(2-((*tert*-butoxycarbonyl)amino)-2-methylpropanoyl)piperazine-1-carboxamido)-2-oxopyrimidin-1(2H)-yl)-1,2,3,4-tetrahydronaphthalen-2-yl)(methyl)amino)cyclohexyl)carbamate (0.03 g, 0.04 mmol) was dissolved in 2M HCl in methanol and stirred for 4 h at rt. The solvent was evaporated and the crude material was purified by HPLC to afford the title compound (15 mg, 60% yield) as a brown solid. ¹H NMR (500 MHz, D₂O): δ 8.00 (d, 1H), 7.40-7.36 (m, 1H), 7.27 (s, 1H), 7.26 (s, 1H), 6.82 (d, 1H), 3.98-3.91 (m, 1H), 3.80-3.73 (m, 8H), 3.68-3.61 (m, 1H), 3.33-2.99 (m, 5H), 2.90 (s, 3H), 2.41-2.26 (m, 5H), 2.09-1.92 (m, 1H), 1.81-1.74 (m, 1H), 1.73 (s, 6H), 1.72-1.58 (m, 3H). LCMS [M+H] 565.4.

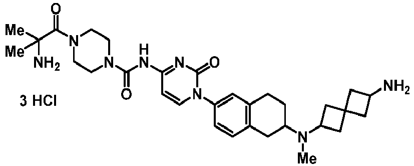

### 4-(2-Amino-2-methylpropanoyl)-N-(1-(6-((6-aminospiro[3.3]heptan-2-yl)(methyl)amino)-5,6,7,8-tetrahydronaphthalen-2-yl)-2-oxo-1,2-dihydropyrimidin-4-yl)piperazine-1-carboxamide hydrochloride salt (Compound 11)

Prepared as in scheme 4 from *tert*-butyl (1-(4-((1-(6-((6-((*tert-*butoxycarbonyl)amino)spiro[3.3]heptan-2-yl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate and formaldehyde. LCMS [M+H] 577.2.

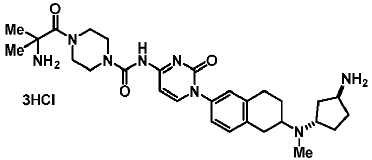

### 4-(2-Amino-2-methylpropanoyl)-N-(1-(6-(((1S,4S)-4-aminocyclohexyl)(methyl)amino)-5,6,7,8-tetrahydronaphthalen-2-yl)-2-oxo-1,2-dihydropyrimidin-4-yl)piperazine-1-carboxamide hydrochloride salt (Compound 19)

Prepared as in Scheme 4 from *tert*-butyl (1-(4-((1-(6-(((1S,3S)-3-((*tert-*butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate and formaldehyde. ¹H NMR (500 MHz, D₂O) δ: 7.99 (d, 1H), 7.38-7.34 (m, 1H), 7.25 (s, 2H), 6.80 (d, 1H), 4.22-4.15 (m, 1H), 3.99-3.87 (m, 2H), 3.83-3.65 (m, 8H), 3.28-3.16 (m, 2H), 3.15-2.92 (m, 2H), 2.88 (s, 3H), 2.45-2.29 (m, 4H), 2.26-2.18 (m, 1H), 2.17-2.00 (m, 1H), 1.99-1.84 (m, 1H), 1.83-1.71 (m, 1H), 1.72 (s, 6H). LCMS [M+H] 551.4.

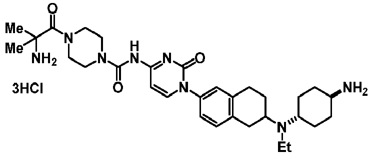

### 4-(2-Amino-2-methylpropanoyl)-N-(1-(6-(((trans)-4-aminocyclohexyl)(ethyl)amino)-5,6,7,8-tetrahydronaphthalen-2-yl)-2-oxo-1,2-dihydropyrimidin-4-yl)piperazine-1-carboxamide hydrochloride salt (Compound 16)

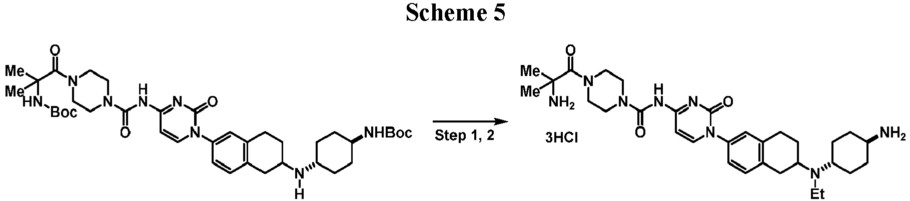

### Reagents: Step 1) Acetaldehyde, NaCNBH₃, MeOH, rt, 16 h 2) 2M HCl in MeOH.

**Step 1: tert-Butyl ((*trans*)-4-((6-(4-(4-(2-((*tert*-butoxycarbonyl)amino)-2-methylpropanoyl)piperazine-1-carboxamido)-2-oxopyrimidin-1(2H)-yl)-1,2,3,4-tetrahydronaphthalen-2-yl)(ethyl)amino)cyclohexyl)carbamate.** To a stirring solution of *tert*-butyl ((*trans*)-4-((6-(4-(4-(2-((*tert*-butoxycarbonyl)amino)-2-methylpropanoyl)piperazine-1-carboxamido)-2-oxopyrimidin-1(2H)-yl)-1,2,3,4-tetrahydronaphthalen-2-yl)amino)cyclohexyl)carbamate (0.04 g, 0.05 mmol) in methanol, were added acetaldehyde (0.5 mL) followed by sodium cyanoborohydride (5 mg, 0.08 mmol) under N₂ atmosphere and stirred for 16 h at rt. The reaction mixture was filtered through Celite^{®} and the solvent was evaporated. Saturated aq. NaHCO₃ was added to the residue and the compound was extracted with ethyl acetate (2x10 mL). The combined organic layers were dried over Na₂SO₄, filtered, concentrated under reduced pressure and the crude material was purified by flash column chromatography to afford the title compound (31 mg, 75% yield) as a white solid.

**Step 2: 4-(2-Amino-2-methylpropanoyl)-N-(1-(6-(((*trans*)-4-aminocyclohexyl)(ethyl)amino)-5,6,7,8-tetrahydronaphthalen-2-yl)-2-oxo-1,2-dihydropyrimidin-4-yl)piperazine-1-carboxamide hydrochloride salt.** *tert*-Butyl ((*trans*)-4-((6-(4-(4-(2-((*tert*-butoxycarbonyl)amino)-2-methylpropanoyl)piperazine-1-carboxamido)-2-oxopyrimidin-1 (2H)-yl)-1,2,3,4-tetrahydronaphthalen-2-yl)(ethyl)amino)cyclohexyl)carbamate (0.03 g, 0.04 mmol) was dissolved in 2 M HCl in methanol and stirred for 4 h at rt. The solvent was evaporated and the crude material was purified by HPLC to afford the title compound solid (18 mg, 65% yield) as a brown solid. ¹H NMR (500 MHz, D₂O): δ 8.04 (d, 1H), 7.37 (d, 1H), 7.27 (s, 1H), 7.26 (s, 1H), 6.82 (d, 1H), 3.98-3.91 (m, 1H), 3.81-3.73 (m, 8H), 3.66-3.59 (m, 1H), 3.44 (q, 2H), 3.29-2.99 (m, 5H), 2.41-2.26 (m, 5H), 2.09-1.96 (m, 1H), 1.81-1.76 (m, 1H), 1.75 (s, 6H), 1.72-1.58 (m, 3H), 1.43 (t, 3H). LCMS [M+H] 579.5.

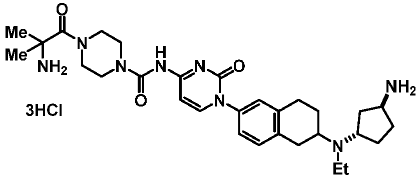

### 4-(2-Amino-2-methylpropanoyl)-N-(1-(6-(((1S,3S)-3-aminocyclopentyl)(ethyl)amino)-5,6,7,8-tetrahydronaphthalen-2-yl)-2-oxo-1,2-dihydropyrimidin-4-yl)piperazine-1-carboxamide hydrochloride salt (Compound 20)

Prepared as in Scheme 5 from *tert*-butyl (1-(4-((1-(6-(((1S,3S)-3-((*tert-*butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate and acetaldehyde. ¹H NMR (500 MHz, D₂O): δ 7.93 (d, 1H), 7.35 (d, 1H), 7.24 (s, 2H), 6.81 (d, 1H), 4.26-4.19 (m, 1H), 3.99-3.87 (m, 2H), 3.83-3.65 (m, 8H), 3.60-3.45 (m, 1H), 3.33-3.22 (m, 3H), 3.12-2.93 (m, 2H), 2.51-2.29 (m, 5H), 2.10-1.85 (m, 2H), 1.83-1.71 (m, 1H), 1.72 (s, 6H), 1.43 (t, 3H). LCMS [M+H] 565.4.

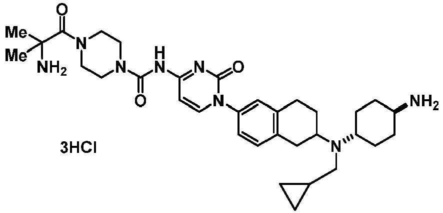

### 4-(2-Amino-2-methylpropanoyl)-N-(1-(6-(((trans)-4-aminocyclohexyl)(cyclopropylmethyl)amino)-5,6,7,8-tetrahydronaphthalen-2-yl)-2-oxo-1,2-dihydropyrimidin-4-yl)piperazine-1-carboxamide hydrochloride salt (Compound 17)

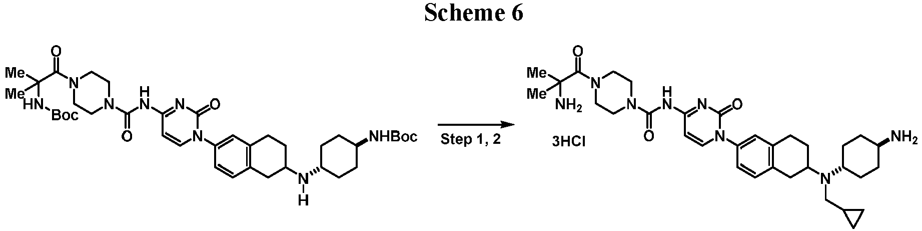

### Reagents: Step 1) Cyclopropanecarbaldehyde, NaCNBH₃, MeOH, rt, 16 h 2) 2M HCl in MeOH.

**Step 1: *tert*-Butyl ((*trans*)-4-((6-(4-(4-(2-((*tert*-butoxycarbonyl)amino)-2-methylpropanoyl)piperazine-1-carboxamido)-2-oxopyrimidin-1(2H)-yl)-1,2,3,4-tetrahydronaphthalen-2-yl)(cyclopropylmethyl)amino)cyclohexyl)carbamate.** To a stirred solution of *tert*-butyl ((*trans*)-4-((6-(4-(4-(2-((tert-butoxycarbonyl)amino)-2-methylpropanoyl)piperazine-1-carboxamido)-2-oxopyrimidin-1(2H)-yl)-1,2,3,4-tetrahydronaphthalen-2-yl)amino)cyclohexyl)carbamate (0.04 g, 0.05 mmol) in methanol, was added cyclopropanecarbaldehyde (0.05 mL) followed by sodium cyanoborohydride (5 mg, 0.08 mmol) under N₂ atmosphere and stirred for 16 h at rt. The reaction mixture was filtered through Celite^{®} and the solvent was evaporated. Saturated aq. NaHCO₃ was added to the residue and the compound was extracted with ethyl acetate (2x10 mL). The combined organic layers were dried over Na₂SO₄, filtered, concentrated under reduced pressure and the crude material was purified by flash column chromatography to afford the title compound (29 mg, 70% yield) as a white solid.

**Step 2: 4-(2-Amino-2-methylpropanoyl)-N-(1-(6-(((*trans*)-4-aminocyclohexyl)( cyclopropylmethyl)amino)-5,6,7,8-tetrahydronaphthalen-2-yl)-2-oxo-1,2-dihydropyrimidin-4-yl)piperazine-1-carboxamide hydrochloride salt.** *tert*-Butyl ((*trans*)-4-((6-(4-(4-(2-((*tert*-butoxycarbonyl)amino)-2-methylpropanoyl)piperazine-1-carboxamido)-2-oxopyrimidin-1(2H)-yl)-1,2,3,4-tetrahydronaphthalen-2-yl)( cyclopropylmethyl)amino)cyclohexyl)carbamate (0.03 g, 0.04 mmol) was dissolved in 2 M HCl in methanol and stirred for 4 h at rt. The solvent was evaporated and the crude material was purified by HPLC to afford the title compound (17 mg, 70% yield) as a brown solid. ¹H NMR (500 MHz, D₂O): δ 7.98 (d, 1H), 7.35 (d, 1H), 7.25 (s, 1H), 7.24 (s, 1H), 6.80 (d, 1H), 4.19-3.98 (m, 1H), 3.81-3.73 (m, 8H), 3.66-3.61 (m, 1H), 3.33-3.19 (m, 5H), 3.17-2.96 (m, 2H), 2.40-2.23 (m, 5H), 2.05-1.98 (m, 1H), 1.85-1.78 (m, 2H), 1.72 (s, 6H), 1.68-1.56 (m, 2H), 1.18-1.12 (m, 1H), 0.77 (d, 2H), 0.43 (d, 2H). LCMS [M+H] 605.5.

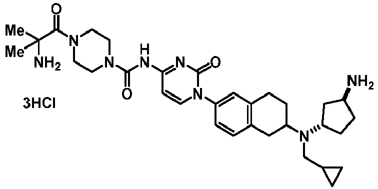

### 4-(2-Amino-2-methylpropanoyl)-N-(1-(6-(((1S,3S)-3-aminocyclopentyl)(cyclopropylmethyl)amino)-5,6,7,8-tetrahydronaphthalen-2-yl)-2-oxo-1,2-dihydropyrimidin-4-yl)piperazine-1-carboxamide hydrochloride salt(Compound 21)

Prepared as in Scheme 6 from *tert*-butyl (1-(4-((1-(6-(((1S,3S)-3-((*tert-*butoxycarbonyl)amino)cyclopentyl)amino)-5,6,7,8-tetrahydronaphthalen-2-yl)-2-oxo-1,2-dihydropyrimidin-4-yl)carbamoyl)piperazin-1-yl)-2-methyl-1-oxopropan-2-yl)carbamate and cyclopropanecarbaldehyde. ¹H NMR (500 MHz, D₂O): δ 7.97 (d, 1H), 7.37-7.35 (m, 1H), 7.24 (s, 1H), 7.23 (s, 1H), 6.80 (d, 1H), 4.27-4.19 (m, 1H), 4.06-3.95 (m, 1H), 3.94-3.86 (m, 1H), 3.83-3.63 (m, 8H), 3.38-3.17 (m, 3H), 3.12-2.93 (m, 3H), 2.51-2.29 (m, 5H), 2.06-1.91 (m, 2H), 1.83-1.71 (m, 1H), 1.72 (s, 6H), 1.22-1.15 (m, 1H), 0.78 (t, 2H), 0.42 (t, 2H). LCMS [M+H] 591.5.

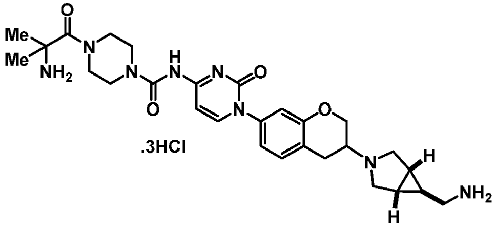

### exo-4-(2-Amino-2-methylpropanoyl)-N-(1-(3-(-6-(aminomethyl)-3-azabicyclo[3.1.0]hexan-3-yl)chroman-7-yl)-2-oxo-1,2-dihydropyrimidin-4-yl)piperazine-1-carboxamide hydrochloride salt (Compound 12)

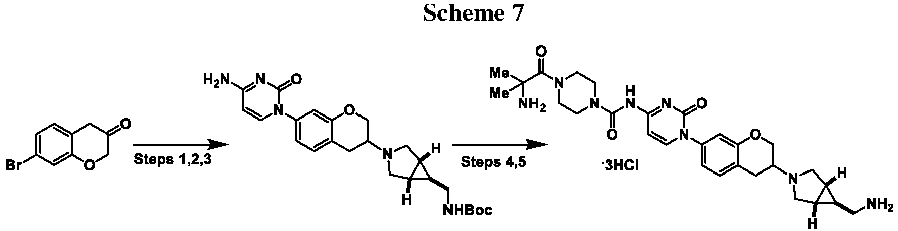

### Reagents: Step 1) exo-tert-butyl ((3-azabicyclo[3.1.0]hexan-6-yl)methyl)carbamate NaCNBH₃, DCE, rt, 16 h 2) B₂Pin₂, KOAc, PdCl₂(dppf), dioxane, 80°C, 16h 3) Cytosine, TMEDA, Cu(OAc)₂ H₂O, MeOH:H₂O (4:1), air, rt, 20 h (4) 1-(4-(2-((tert-butoxycarbonyl)amino)-2-methylpropanoyl)piperazine-1-carbonyl)-3-methyl-1H-imidazol-3-ium iodide, CH₃CN, 80°C, 16h (5) 4M HCl in dioxane, rt, 8h.

**Step 1: *exo-tert*-butyl ((3-(7-bromochroman-3-yl)-3-azabicyclo[3.1.0]hexan-6-yl)methyl) carbamate.** To a stirred solution of 7-bromochroman-3-one (0.1 g, 0.5 mmol) in DCE (5 mL) was added *exo-tert*-butyl ((3-azabicyclo[3.1.0]hexan-6-yl)methyl)carbamate (0.09 g, 0.5 mmol) and activated 4Å molecular sieves (0.05 g) at rt. The reaction mixture was stirred at rt for 2 h. NaCNBH₃ (0.04 g, 0.7 mmol) was added and the reaction mixture was stirred at rt for 16 h. The reaction mixture was filtered and the filtrate was poured into water (30 mL) and extracted with DCE (3 × 15 mL). The combined organic layers were dried over Na₂SO₄, filtered and concentrated under reduced pressure and purified by flash chromatography (20% EtOAc in hexane) to afford the title compound (0.1 g, 54%) as a yellow solid. ¹H NMR (400 MHz, MeOD) δ 6.96-6.94 (m, 2H), 6.88 (s, 1H), 4.24 (d,1H), 3.08 (t, 1H), 3.13 (t, 1H), 2.93-2.86 (m, 3H), 2.68-2.62 (m, 2H), 2.55-2.52 (m, 2H), 1.46 (s, 9H), 1.43-1.33 (m, 2H), 1.30-1.24 (m, 1H), 1.19-1.16 (m, 1H). LCMS [M+H] 423.2.

**Step 2: exo-*tert*-butyl-3-(7-(4,4,5,5-tetramethyl-1,3,2-dioxaborolan-2-yl)chroman-3-yl)-3-azabicyclo[3.1.0]hexan-6-yl)methyl)carbamate.** To a stirred a solution of *exo*-*tert*-butyl((3-(7-bromochroman-3-yl)-3-azabicyclo[3.1.0]hexan-6-yl)methyl)carbamate (0.1 g, 0.3 mmol) in dioxane (150 mL) was added B₂Pin₂ (0.12 g, 0.5 mmol) and KOAc (0.07 g, 0.7 mmol) at rt under N₂ atmosphere. The reaction mixture was degassed with N₂ for 20 min. PdCl₂(dppf) (0.02 g, 0.02 mmol) was added and heated at 80°C for 16 h. The reaction mixture was poured into water (30 mL) and extracted with EtOAc (3 × 15 mL). The combined organic layers were dried over Na₂SO₄, filtered and concentrated under reduced pressure to afford the title compound (0.2 g, quantitative) as brown sticky material. LCMS[M+H] 471.2.

**Step-3: tert-butyl ((exo)-3-(7-(4-amino-2-oxopyrimidin-1(2H)-yl)chroman-3-yl)-3-azabicyclo[3.1.0]hexan-6-yl)methyl)carbamate.** A solution of *exo-tert-*butyl ((3-(7-(4,4,5,5-tetramethyl-1,3,2-dioxaborolan-2-yl)chroman-3-yl)-3-azabicyclo[3.1.0]hexan-6-yl)methyl)carbamate (0.2 g, 0.4 mmol) and cytosine (0.05 g, 0.4 mmol) in MeOH:H₂O (6 mL, 4:1) was stirred at rt in open air for 30 min. TMEDA (0.07 mL, 0.5 mmol) and Cu(OAc)₂.H₂O (0.08 g, 0.4 mmol) were added and the reaction mixture and stirred in open air at rt for 20 h. The reaction mixture was concentrated under reduced pressure. The crude material was poured into water (30 mL) and extracted with EtOAc (3 × 15 mL). The combined organic phase was dried over Na₂SO₄, filtered and concentrated under reduced pressure and purified by flash chromatography (10% MeOH in DCM) to afford the title compound (0.045g, 24%) as brown solid. ¹H NMR: (400 MHz, DMSO-d₆): δ 7.53 (d, 1H), 7.19-7.07 (m, 2H), 6.76-6.70 (m, 2H), 6.67 (d, 1H), 5.80-5.72 (m, 1H), 4.21 (d, 1H), 3.79 (t, 1H), 3.06-2.99 (m, 2H), 2.90 (d, 2H), 2.76-2.73 (m, 2H), 2.66-2.63 (m, 2H), 1.35 (s, 9H), 1.30-1.28 (m, 3H), 1.27-1.22 (m, 1H), 1.06-1.04 (m, 1H). LCMS [M+H] 454.9.

**Step 4: *exo-tert*-butyl ((3-(7-(4-(4-(2-((tert-butoxycarbonyl)amino)-2-methylpropanoyl)piperazine-1-carboxamido)-2-oxopyrimidin-1(2H)-yl)chroman-3-yl)-3-azabicyclo[3.1.0]hexan-6-yl)methyl)carbamate.** To a stirred solution of tert-butyl ((exo)-3-(7-(4-amino-2-oxopyrimidin-1(2H)-yl)chroman-3-yl)-3-azabicyclo[3.1.0]hexan-6-yl)methyl)carbamate (0.05 g, 0.1 mmol) and 1-(4-(2-((*tert*-butoxycarbonyl)amino)-2-methyl propanoyl)piperazine-1-carbonyl)-3-methyl-1H-imidazol-3-ium iodide (0.08 g, 0.2 mmol) in acetonitrile (2 mL) was heated at 80°C for 16 h. The reaction mixture was concentrated under reduced pressure and crude was purified by flash chromatography (2% MeOH in DCM) to afford the title compound (0.08 g) as brown sticky material. LCMS[M+H] 751.

**Step 5: *exo*-4-(2-amino-2-methylpropanoyl)-N-(1-(3-(-6-(aminomethyl)-3-azabicyclo[3.1.0]hexan-3-yl)chroman-7-yl)-2-oxo-1,2-dihydropyrimidin-4-yl)piperazine-1-carboxamide hydrochloride salt.** To a stirred solution of ***exo**-* tert-butyl ((3-(7-(4-(4-(2-((tert-butoxycarbonyl)amino)-2-methylpropanoyl)piperazine-1-carboxamido)-2-oxopyrimidin-1 (2H)-yl)chroman-3-yl)-3-azabicyclo[3 .1.0]hexan-6-yl)methyl)carbamate (0.08 g, 0.1 mmol) in dioxane (1.0 mL) was added 4M HCl in dioxane (0.5 mL) at rt. The reaction mixture was stirred at rt for 8 h. The reaction mixture was concentrated under reduced pressure and purified by Prep-HPLC purification to afford the title compound (0.005 g, 8%) as a white solid. ¹H NMR: (400Mz, D₂O): δ 7.91 (d, 1H), 7.42 (d, 1H), 7.13 (q, 1H), 7.07 (d, 1H), 6.91 (d, 1H), 4.60-4.55 (m, 1H), 4.55-4.48(m, 1H), 4.13-4.04 (m, 2H), 3.84-3.79 (m, 8H),3.68-3.49 (m, 2H), 3.35-3.20 (m, 2H), 3.03 (d, 2H), 2.21-2.04 (m, 2H), 1.80 (s, 6H), 1.68-1.55 (m, 1H). LCMS[M+H] 551.3.

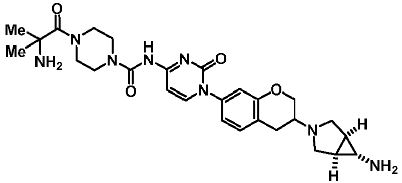

### exo-4-(2-Amino-2-methylpropanoyl)-N-(1-(3-(-6-amino-3-azabicyclo[3.1.0]hexan-3-yl)chroman-7-yl)-2-oxo-1,2-dihydropyrimidin-4-yl)piperazine-1-carboxamide hydrochloride salt (Compound 13)

Prepared in a similar fashion to Scheme 7 from 7-bromochroman-3-one and *exo-tert*-butyl (3-azabicyclo[3.1.0]hexan-6-yl)carbamate. ¹H NMR: (400MHz, D₂O): δ 7.71 (d, 1H), 7.20 (d, 1H), 6.91 (d, 1H), 6.85 (s, 1H), 6.69 (d, 1H), 4.37 (d, 1H), 4.21 (d, 1H), 3.90-3.84 (m, 2H), 3.62-3.50(m, 9H), 3.41-3.31(m, 3H), 3.04-2.91 (m, 2H), 2.32 (s, 2H), 1.58 (s, 6H). LCMS[M+H] 537.3.

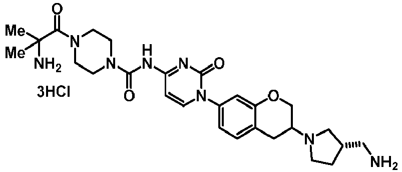

### 4-(2-Amino-2-methylpropanoyl)-N-(1-(3-((S)-3-(aminomethyl)pyrrolidin-1-yl)chroman-7-yl)-2-oxo-1,2-dihydropyrimidin-4-yl)piperazine-1-carboxamidehydrochloride salt (Compound 14)

Prepared in a similar fashion to Scheme 7 from 7-bromochroman-3-one and (R)-*tert*-butyl (pyrrolidin-3-ylmethyl)carbamate. ¹H NMR: (400 MHz, D2O): δ 7.70 (d, 1H), 7.21 (d, 1H), 6.91 (d, 1H), 6.86 (s, 1H), 6.69 (d, 1H), 4.47 (d, 2H), 4.24 (t, 2H), 3.87 (bs, 1H), 3.69-3.55(m, 7H), 3.45-3.34(m, 2H), 3.25-3.18 (m, 1H), 3.12-3.01 (m, 4H), 2.65-2.52 (m, 2H), 2.40-2.26 (m, 2H), 1.57 (s, 6H). LCMS[M+2H]/2 270.2.

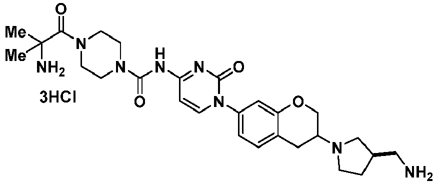

### 4-(2-Amino-2-methylpropanoyl)-N-(1-(3-((R)-3-(aminomethyl)pyrrolidin-1-yl)chroman-7-yl)-2-oxo-1,2-dihydropyrimidin-4-yl)piperazine-1-carboxamide hydrochloride salt (Compound 18)

Prepared in a similar fashion to Scheme 7 from 7-bromochroman-3-one and (S)-*tert*-butyl (pyrrolidin-3-ylmethyl)carbamate. ¹H NMR: (400 MHz, D₂O): δ 7.72 (d, 1H), 7.21 (d, 1H), 6.91 (d, 1H), 6.85 (s, 1H), 6.68 (d, 1H), 4.46 (d, 2H), 4.23 (t, 2H), 3.86 (bs, 2H), 3.61-3.50 (m, 8H), 3.07 (d, 2H), 3.15-2.95 (m, 4H), 2.41-2.26 (m, 2H), 1.56 (s, 6H). LCMS[M+H] 539.8.

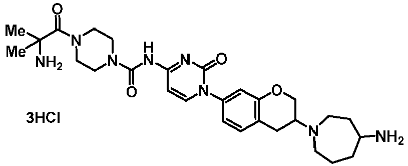

### 4-(2-Amino-2-methylpropanoyl)-N-(1-(3-(4-aminoazepan-1-yl)chroman-7-yl)-2-oxo-1,2-dihydropyrimidin-4-yl)piperazine-1-carboxamidehydrochloride salt (Compound 22)

Prepared in a similar fashion to Scheme 7 from 7-bromochroman-3-one and *tert-*butyl azepan-4-ylcarbamate. ¹H NMR: (400 MHz, D₂O): δ 7.76 (d, 1H), 7.25 (d, 1H), 6.95 (d, 1H), 6.89 (s, 1H), 6.73-6.71 (m, 1H), 4.56 (d, 2H), 4.32 (t, 1H), 3.96 (bs, 1H), 3.85-3.62(m, 4H), 3.60-3.29(m, 9H), 3.28-3.16 (m, 2H), 2.85 (d, 1H), 2.35-2.22 (m, 2H), 2.15-1.90 (m, 2H), 1.61 (s, 6H). LCMS[M+H] 553.5.

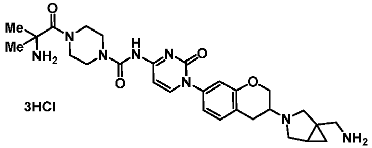

### 4-(2-Amino-2-methylpropanoyl)-N-(1-(3-(1-(aminomethyl)-3-azabicyclo[3.1.0]hexan-3-yl)chroman-7-yl)-2-oxo-1,2-dihydropyrimidin-4-yl)piperazine-1-carboxamide hydrochloride salt (Compound 23)

Prepared in a similar fashion to Scheme 7 from 7-bromochroman-3-one and *tert-*butyl ((3-azabicyclo[3.1.0]hexan-1-yl)methyl)carbamate. ¹H NMR: (400 MHz, D₂O): 7.76 (d, 1H), 7.19 (d, 1H), 6.90 (d, 1H), 6.84 (s, 1H), 6.66 (d, 1H), 4.32-422 (m, 3H), 3.86 (bs, 2H), 3.78-3.47 (m, 11H), 3.35-3.31(m, 3H), 3.12-2.80 (m, 3H), 1.92 (bs, 1H), 1.56 (s, 6H), 1.21-0.95 (m, 2H). LCMS[M+H] 551.3.

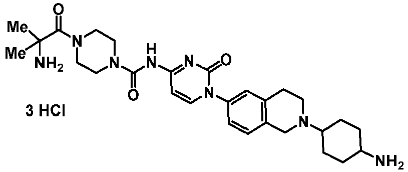

### 4-(2-Amino-2-methylpropanoyl)-N-(1-(2-(4-aminocyclohexyl)-1,2,3,4-tetrahydroisoquinolin-6-yl)-2-oxo-1,2-dihydropyrimidin-4-yl)piperazine-1-carboxamide hydrochloride salt (Compound 35)

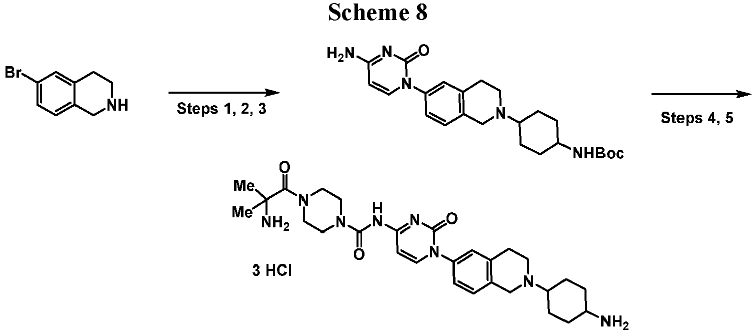

### Reagents: 1) tert-butyl (4-oxocyclohexyl)carbamate, Na(OAc)₃BH, DCE, rt. 16h 2) Pd(dppf)Cl₂·CH₂Cl₂, KOAc, B₂Pin₂, 1,4-Dioxane, 105 °C, 16h, 3) cytosine, Cu(OAc)₂·H₂O, TMEDA, MeOH:H₂O (4:1), rt. 48h 4) 1-(4-(2-((tert-butoxycarbonyl)amino)-2-methylpropanoyl)piperazine-1-carbonyl)-3-methyl-1H-imidazol-3-ium iodide, MeCN, 85 °C, 16h 5) HCl, MeOH, rt. 4h.

**Step 1: *tert*-butyl (4-(6-bromo-3,4-dihydroisoquinolin-2(1H)-yl)cyclohexyl)carbamate.** To a stirred solution of *tert*-butyl (4-oxocyclohexyl)carbamate (251 mg, 1.2 mmol) in DCE (10 mL), was added 6-bromo-1,2,3,4-tetrahydroisoquinoline (250 mg, 1.2 mmol) and Na(OAc)₃BH (375 mg, 1.8 mmol). The reaction was stirred at rt for 16h. The reaction mixture was diluted with CH₂Cl₂ and washed with saturated NaHCO₃ (1x15 mL). The aqueous layer was extracted with CH₂Cl₂ (2×20 mL) and the combined organics were dried over Na₂SO₄, concentrated under reduced pressure and purified by column chromatography (CH₂Cl₂:MeOH) to afford the desired product. LCMS[M+H] 411.1.

**Step 2 *tert*-butyl (4-(6-(4,4,5,5-tetramethyl-1,3,2-dioxaborolan-2-yl)-3,4-dihydroisoquinolin-2(1H)-yl)cyclohexyl)carbamate.** A flask containing *tert*-butyl (4-(6-bromo-3,4-dihydroisoquinolin-2(1H)-yl)cyclohexyl)carbamate (512 mg, 1.25 mmol), Pd(dppf)Cl₂·CH₂Cl₂ (30 mg, 0.03 mmol), KOAc (368 mg, 3.75 mmol) and B₂pin₂ (349 mg, 1.38 mmol) was evacuated and flushed with N₂. 1,4-Dioxane (10 mL) was added, and the reaction mixture was de-gassed and heated to 105°C for 16h. The reaction mixture was cooled to rt, diluted with EtOAc (30 mL) and filtered through a pad of Celite^{®}. The crude reaction mixture was purified by flash chromatography (EtOAc:Hex) to afford the desired product. LCMS[M+H] 457.8.

**Step 3: *tert*-butyl (4-(6-(4-amino-2-oxopyrimidin-1(2H)-yl)-3,4-dihydroisoquinolin-2(1H)-yl)cyclohexyl)carbamate.** A suspension of tert-butyl (4-(6-(4,4,5,5-tetramethyl-1,3,2-dioxaborolan-2-yl)-3,4-dihydroisoquinolin-2(1H)-yl)cyclohexyl)carbamate (500 mg, 1.1 mmol) and cytosine (122 mg, 1.1 mmol) in MeOH:H₂O (4:1, 100 mL) was stirred open to air at rt for 30 min. Cu(OAc)₂·H₂O (219 mg, 1.1 mmol) and TMEDA (0.20 mL, 1.3 mmol) were added and the reaction was stirred at rt for 24h. The reaction mixture was concentrated under reduced pressure and H₂O was added (150 mL). The solids were collected by filtration and washed with Et₂O (25 mL) and cold H₂O (25 mL) to afford the desired product. LCMS[M+H] 440.3.

**Step 4: *tert*-butyl (1-(4-((1-(2-(4-((*tert*-butoxycarbonyl)amino)cyclohexyl)-1,2,3,4-tetrahydroisoquinolin-6-yl)-2-oxo-1,2-dihydropyrimidin-4-yl)carbamoyl)piperazin-1-yl)-2-methyl-1-oxopropan-2-yl)carbamate.** To a solution of *tert*-butyl (4-(6-(4-amino-2-oxopyrimidin-1(2H)-yl)-3,4-dihydroisoquinolin-2(1H)-yl)cyclohexyl)carbamate (124 mg, 0.28 mmol) in MeCN (20 mL), was added 1-(4-(2-((*tert-*butoxycarbonyl)amino)-2-methylpropanoyl)piperazine-1-carbonyl)-3-methyl-1H-imidazol-3-ium iodide (200 mg, 0.39 mmol). The reaction mixture was heated to 85°C for 16h. The volatiles were removed under reduced pressure and the crude solid was dissolved in CH₂Cl₂ (100 mL), washed with H₂O (1x100 mL) and concentrated under reduced pressure. The crude solid was purified by flash chromatography (CH₂Cl₂:MeOH:NH₄OH) to afford the desired product.

**Step 5: 4-(2-amino-2-methylpropanoyl)-N-(1-(2-(4-aminocyclohexyl)-1,2,3,4-tetrahydroisoquinolin-6-yl)-2-oxo-1,2-dihydropyrimidin-4-yl)piperazine-1-carboxamide hydrochloride salt*****.** tert*-butyl (1-(4-((1-(2-(4-((*tert*-butoxycarbonyl)amino)cyclohexyl)-1,2,3,4-tetrahydroisoquinolin-6-yl)-2-oxo-1,2-dihydropyrimidin-4-yl)carbamoyl)piperazin-1-yl)-2-methyl-1-oxopropan-2-yl)carbamate was dissolved in a solution of HCl/MeOH (5 mL) and stirred for 4h. The volatiles were removed under reduced pressure and the crude solid was purified by reverse phase HPLC (H₂O:CH₃CN:TFA) and concentrated under reduced pressure. Addition and evaporation under reduced pressure with HCl/MeOH (3x15 mL) afforded the desired product. ¹H NMR (500 MHz, D₂O) δ 8.05 (d, 1H), 7.49-7.37 (m, 3H), 6.87 (dd, 1H), 4.64 (d, 2H), 3.99-3.49 (m, 10H), 3.44-3.23 (m, 3H), 2.47-2.28 (m, 2H), 2.26-2.11 (m, 2H), 2.03 (d, 2H), 1.87 (d, 1H), 1.78 (s, 6H), 1.73-1.59 (m, 1H). LCMS[M+H] 537.4.

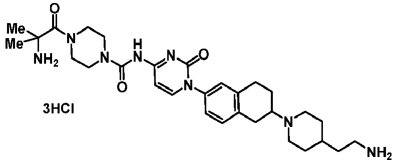

### 4-(2-Amino-2-methylpropanoyl)-N-(1-(6-(4-(2-aminoethyl)piperidin-1-yl)-5,6,7,8-tetrahydronaphthalen-2-yl)-2-oxo-1,2-dihydropyrimidin-4-yl)piperazine-1-carboxamide hydrochloride salt (Compound 42)

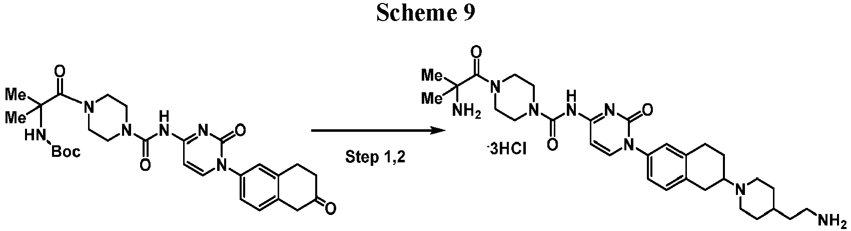

### Reagents: 1) DMP, CH₂Cl₂, rt, 2h 8) NaCNBH₃, DCM, 45°C, 16h 2) 4M HCl/dioxane, rt, 3h.

**Step 8: *tert-*butyl *(*1-(4-((1-(6-(4-(2-((*tert-*butoxycarbonyl)amino)ethyl)piperidin-1-yl)-5,6,7,8-tetrahydronaphthalen-2-yl)-2-oxo-1,2-dihydropyrimidin-4-yl)carbamoyl) piperazin-1-yl)-2-methyl-1-oxopropan-2-yl)carbamate.** To a stirred solution of *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl) propan-2-yl) carbamate (0.25 g, 0.45 mmol) and *tert*-butyl (2-(piperidin-4-yl)ethyl)carbamate (0.15 g, 0.67 mmol) in DCM (10 mL) was added NaBH₃CN (0.06 g, 0.9 mmol) at rt under N₂ atmosphere. The reaction mixture was stirred at 40°C for 16 h. The reaction mixture was concentrated under reduced pressure and purified by column chromatography (12% MeOH in DCM) to afford the title compound (0.1 g, 29%) as off white solid. LCMS[M+H] 765.6.

**Step 9: 4-(2-Amino-2-methylpropanoyl)-N-(1-(6-(4-(2-aminoethyl)piperidin-1-yl)-5,6,7,8-tetrahydronaphthalen-2-yl)-2-oxo-1,2-dihydropyrimidin-4-yl)piperazine-1-carboxamide hydrochloride salt.** To a stirred solution of *tert*-butyl (1-(4-((1-(6-(4-(2-((*tert-*butoxycarbonyl)amino)ethyl)piperidin-1-yl)-5, 6,7, 8-tetrahydronaphthalen-2-yl)-2-oxo-1,2-dihydropyrimidin-4-yl)carbamoyl)piperazin-1-yl)-2-methyl-1-oxopropan-2-yl)carbamate (0.1 g, 0.13 mmol) in dioxane (2 mL) was added 4M HCl in dioxane (2 mL) at rt. The reaction mixture was stirred at rt for 3h. The reaction mixture was concentrated under reduced pressure and purified by HPLC to afford the title compound (0.025 g, 33%) as a light yellow solid. ¹H NMR: (400 MHz, D₂O): δ 7.79 (d, 1H), 7.20 (d, 1H), 7.08 (d, 2H), 6.66 (d, 1H), 3.61-3.49 (m, 11H), 3.18-3.14 (m, 1H), 3.07-2.81 (m, 7H), 2.19 (bs, 1H), 1.94 (d, 2H), 1.83-1.78 (m, 1H), 1.63-1.51 (m, 9H), 1.43-1.34 (m, 2H). LCMS [M/2+H] 283.3.

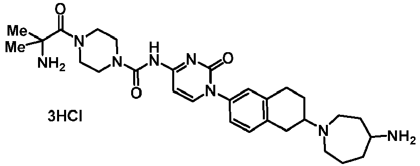

### 4-(2-Amino-2-methylpropanoyl)-N-(1-(7-(4-aminoazepan-1-yl)-5,6,7,8-tetrahydronaphthalen-2-yl)-2-oxo-1,2-dihydropyrimidin-4-yl)piperazine-1-carboxamide hydrochloride salt (Compound 43)

Prepared in a similar fashion to Scheme 9 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl azepan-4-ylcarbamate. ¹H NMR: (400 MHz, D₂O): LCMS [M/2+H] 276.3.

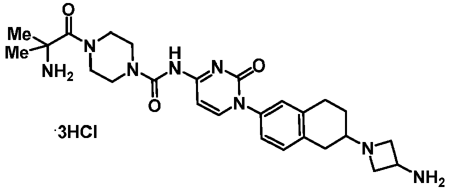

### 4-(2-Amino-2-methylpropanoyl)-N-(1-(7-(3-aminoazetidin-1-yl)-5,6,7,8-tetrahydronaphthalen-2-yl)-2-oxo-1,2-dihydropyrimidin-4-yl)piperazine-1-carboxamide hydrochloride salt (Compound 39)

Prepared in a similar fashion to Scheme 9 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl) propan-2-yl)carbamate and *tert*-butyl azetidin-3-ylcarbamate. ¹H NMR: (400 MHz, D₂O): δ 7.83 (d, 1H), 7.24 (d, 1H), 7.14 (d, 2H), 6.71 (d, 1H), 4.70-4.60 (m, 2H), 4.41 (s, 3H), 3.79 (bs, 2H), 3.66-3.54 (m, 7H), 3.24-3.20 (m, 1H), 2.87-2.75 (m, 3H), 2.17 (bs, 1H), 1.78-1.72 (m, 1H), 1.62 (s, 6H). LCMS [M+H] 509.5.

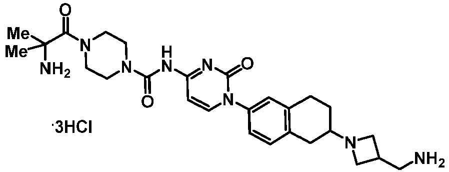

### 4-(2-Amino-2-methylpropanoyl)-N-(1-(7-(3-(aminomethyl)azetidin-1-yl)-5,6,7,8-tetrahydronaphthalen-2-yl)-2-oxo-1,2-dihydropyrimidin-4-yl)piperazine-1-carboxamide hydrochloride salt (Compound 40)

Prepared in a similar fashion to Scheme 9 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl) propan-2-yl)carbamate and *tert*-butyl (azetidin-3-ylmethyl)carbamate. ¹H NMR: (400 MHz, D₂O): δ 7.82 (d, 1H), 7.23 (d, 1H), 7.14 (d, 2H), 6.71 (d, 1H), 4.36 (t, 2H), 4.12-4.07 (m, 2H), 3.66-3.62 (m, 8H), 3.34 (d, 1H), 3.23-3.15 (m, 4H), 2.86 (d, 2H), 2.76-2.72 (m, 1H), 2.15 (bs, 1H), 1.70 (m, 1H), 1.62 (s, 6H). LCMS [M+H] 523.4.

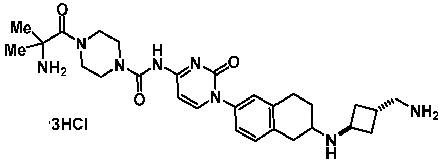

### trans-4-(2-Amino-2-methylpropanoyl)-N-(1-(7-((3-(aminomethyl)cyclobutyl)amino)-5,6,7,8-tetrahydronaphthalen-2-yl)-2-oxo-1,2-dihydropyrimidin-4-yl)piperazine-1-carboxamide hydrochloride salt (Compound 41)

Prepared in a similar fashion to Scheme 9 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl) propan-2-yl)carbamate and *tert*-butyl (((trans)-3-aminocyclobutyl)methyl)carbamate. ¹H NMR: (400 MHz, D₂O): δ 7.77 (d, 1H), 7.22 (d, 1H), 7.12 (s, 2H), 6.72 (d, 1H), 4.07 (d, 1H), 3.66-3.61 (m, 9H), 3.20-3.10 (m, 3H), 2.91-2.70 (m, 3H), 2.70-2.66 (m, 1H), 2.50-2.44 (m, 2H), 2.26-2.17 (m, 3H), 1.90-1.81 (m,1H), 1.62 (s, 6H). LCMS [M+H] 537.4.

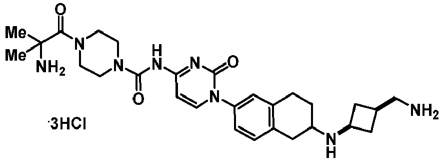

### cis-4-(2-Amino-2-methylpropanoyl)-N-(1-(7-((3-(aminomethyl)cyclobutyl)amino)-5,6,7,8-tetrahydronaphthalen-2-yl)-2-oxo-1,2-dihydropyrimidin-4-yl)piperazine-1-carboxamide hydrochloride salt (Compound 44)

Prepared in a similar fashion to Scheme 9 from *tert*-butyl (2-methyl-1-oxo-1-(4-((2-oxo-1-(6-oxo-5,6,7,8-tetrahydronaphthalen-2-yl)-1,2-dihydropyrimidin-4-yl)carbamoyl)piperazin-1-yl)propan-2-yl)carbamate and *tert*-butyl (((cis)-3-aminocyclobutyl)methyl)carbamate. ¹H NMR: (400 MHz, D₂O): δ 7.75 (d, 1H), 7.22 (d, 1H), 7.12 (s, 2H), 6.73 (d, 1H), 3.91 (bs, 1H), 3.67-3.61 (m, 8H), 3.31-3.15 (m, 3H), 3.03-2.88 (m, 4H), 2.57-2.45 (m, 3H), 2.31-2.18 (m, 1H), 2.09-1.96 (m, 2H), 1.90-1.83 (m,1H), 1.63 (s, 6H). LCMS [M/2+H] 269.3.

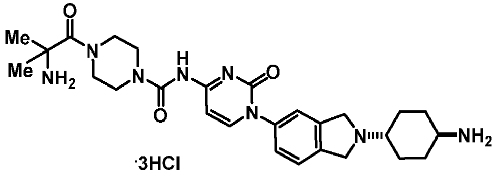

### 4-(2-Amino-2-methylpropanoyl)-N-(1-(2-((trans)-4-aminocyclohexyl)isoindolin-5-yl)-2-oxo-1,2-dihydropyrimidin-4-yl)piperazine-1-carboxamidehydrochloride salt (Compound 46)

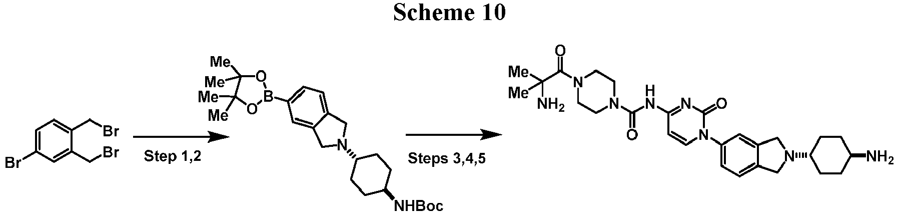

### Reagents: Step 1) tert-Butyl ((1r,4r)-4-aminocyclohexyl)carbamate, K₂CO₃, CH₃CN, 85°C, 16 h 2) B₂Pin₂, KOAc, PdCl₂(dppf), dioxane, 100°C, 16 h 3) Cytosine, TMEDA, Cu(OAc)₂.H₂O, MeOH:H₂O (4:1), air, rt, 16 h (4) 1-(4-(2-((tert-butoxycarbonyl)amino)-2-methylpropanoyl)piperazine-1-carbonyl)-3-methyl-1H-imidazol-3-ium iodide, CH₃CN, 80°C, 16h (5) AcCl, MeOH, rt, 4h.

**Step 1: *tert*-butyl ((trans)-4-(5-bromoisoindolin-2-yl)cyclohexyl)carbamate.** To a stirred solution of 4-bromo-1,2-bis(bromomethyl)benzene (0.31 g, 0.9 mmol) in CH₃CN (15 mL) was added *tert*-butyl ((trans)-4-aminocyclohexyl)carbamate (0.21 g, 1.0 mmol), and K₂CO₃ (0.27 g, 2.0 mmol) at rt. The reaction mixture was stirred at 85°C for 16h. The reaction mixture was filtered, and the filtrate was concentrated under reduced pressure. The residue was purified by flash chromatography (20% EtOAc in hexane) to afford the title compound (0.2 g, 56%) as a pale yellow solid.

**Step 2: *tert*-butyl ((trans)-4-(5-(4,4,5,5-tetramethyl-1,3,2-dioxaborolan-2-yl)isoindolin-2-yl)cyclohexyl)carbamate.** To a stirred a solution of *tert*-butyl ((*trans*)*-4-(5-*bromoisoindolin-2-yl)cyclohexyl)carbamate (0.1 g, 0.25 mmol) in dioxane (5 mL) was added B₂Pin₂ (0.08 g, 0.3 mmol) and KOAc (0.07 g, 0.8 mmol) at rt under N₂. The reaction mixture was degassed with N₂ for 20 min. and PdCl₂(dppf) (6.0 mg, 0.03 mmol) was added and the reaction mixture stirred at 100°C for 16h. The reaction mixture was filtered through celite, and the filtrate was concentrated under reduced pressure. The residue was purified by flash chromatography (75% EtOAc in hexane) to afford the title compound (0.08 g, 73%) as a brown colored solid.

**Step-3: *tert*-butyl ((trans)-4-(5-(4-amino-2-oxopyrimidin-1(2H)-yl)isoindolin-2-yl)cyclohexyl)carbamate.** A solution of *tert*-butyl ((trans)-4-(5-(4,4,5,5-tetramethyl-1,3,2-dioxaborolan-2-yl)isoindolin-2-yl)cyclohexyl)carbamate (80.0 mg, 0.18 mmol) and cytosine (20 mg, 0.18 mmol) in MeOH:H₂O (6 mL, 4:1) was stirred for 30 min. TMEDA (25.0 □L, 0.22 mmol) and Cu(OAc)₂.H₂O (36.0 mg, 0.18 mmol) were added and the reaction mixture was stirred in open air at rt for 16h. The reaction mixture was concentrated under reduced pressure. The crude material was poured into water (30 mL) and extracted with CHCl₃ (3x15 mL). The combined organic phase was dried over Na₂SO₄, filtered, and concentrated under reduced pressure and purified by flash chromatography (8:2:0.2 MeOH: DCM:NH₄OH) to afford the title compound (32 mg, 44%) as white colored solid.

**Step 4: *tert*-butyl (1-(4-((1-(2-((trans)-4-((*tert-*butoxycarbonyl)amino)cyclohexyl)isoindolin-5-yl)-2-oxo-1,2-dihydropyrimidin-4-yl)carbamoyl)piperazin-1-yl)-2-methyl-1-oxopropan-2-yl)carbamate.** To a stirred solution of *tert*-butyl ((trans)-4-(5-(4-amino-2-oxopyrimidin-1(2H)-yl)isoindolin-2-yl)cyclohexyl)carbamate (32 mg, 0.08 mmol) in acetonitrile (4 mL), was added 1-(4-(2-((*tert-*butoxycarbonyl)amino)-2-methyl propanoyl)piperazine-1-carbonyl)-3-methyl-1H-imidazol-3-ium iodide (80 mg, 0.15 mmol) and the reaction mixture stirred under N₂ at 80°C for 16 h. The reaction mixture was concentrated under reduced pressure and crude was purified by flash chromatography (DCM/MeOH) to afford the title compound (35 mg, 64%) as a pale yellow solid.

**Step 5: 4-(2-amino-2-methylpropanoyl)-N-(1-(2-((trans)-4-aminocyclohexyl)isoindolin-5-yl)-2-oxo-1,2-dihydropyrimidin-4-yl)piperazine-1-carboxamide hydrochloride salt.** The compound *tert*-butyl (1-(4-((1-(2-((trans)-4-((*tert-*butoxycarbonyl)amino)cyclohexyl)isoindolin-5-yl)-2-oxo-1,2-dihydropyrimidin-4-yl)carbamoyl)piperazin-1-yl)-2-methyl-1-oxopropan-2-yl)carbamate (30 mg, 0.04 mmol) was dissolved in freshly prepared methanolic HCl (4 mL) and stirred at rt for 4h. The reaction mixture was concentrated under reduced pressure and purified by RPHPLC. The desired fractions were collected and evaporated under reduced pressure. To the residue was added HCl in MeOH (2x5 mL) and the solvent evaporated to afford the title compound (11 mg, 50%) as a white solid. ¹H NMR: (400Mz, D₂O): δ 8.03 (d, 1H), 7.61 (d, 1H), 7.55 - 7.48 (m, 2H), 6.85 (d, 1H), 4.98 (d, 2H), 4.73 (d, 3H), 3.80 - 3.73 (m, 8H), 3.67 - 3.59 (m,1H), 3.36 - 3.26 (m, 1H), 2.42 (d, 2H), 2.27 (d, 2H), 1.75 (s, 6H), 1.74 - 1.56 (m, 4H). LCMS[M+H] 524.3.

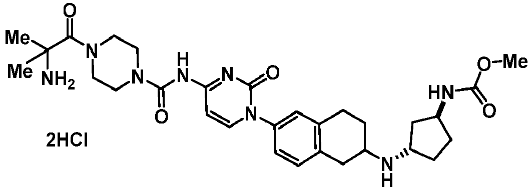

### Methyl ((1S,3S)-3-((6-(4-(4-(2-amino-2-methylpropanoyl)piperazine-1-carboxamido)-2-oxopyrimidin-1(2H)-yl)-1,2,3,4-tetrahydronaphthalen-2-yl)amino)cyclopentyl)carbamate hydrochloride salt (Compound 45)

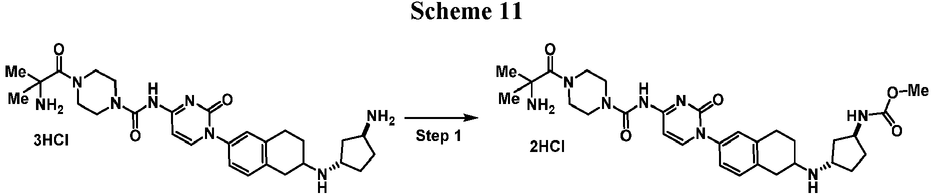

### Reagents: Step 1) NaHCO₃, methyl chloroformate, THF, H₂O, 0°C to rt, 2h.

**Step 1: Methyl ((1*S*,3*S*)-3-((6-(4-(4-(2-amino-2-methylpropanoyl)piperazine-1-carboxamido)-2-oxopyrimidin-1(2H)-yl)-1,2,3,4-tetrahydronaphthalen-2-yl)amino)cyclopentyl)carbamate:** 4-(2-Amino-2-methylpropanoyl)-N-(1-(6-(((1S,3S)-3-aminocyclopentyl)amino)-5,6,7,8-tetrahydronaphthalen-2-yl)-2-oxo-1,2-dihydropyrimidin-4-yl)piperazine-1-carboxamide (160 mg, 0.3 mmol) was dissolved in sat. aq. NaHCOs (2 ml) and THF (2 ml). Methyl chloroformate (28 mg, 0.3 mmol) was added at 0°C. The reaction mixture was warmed to rt and stirred for 2h. The solid was filtered and the residue was concentrated under reduced pressure. The residue was dissolved in MeOH (0.5 ml) and 2M HCl in ether (3 ml) was added and the resultant solid was filtered, washed with ether and dried under vacuum resulting in the title compound (51 mg, 31%) as a white colored solid. 1H NMR (500 MHz, D₂O) δ 8.10 (dd, 1H), 7.39 (d, 1H), 7.28 (dd, 2H), 6.82 (d, 1H), 4.20 - 4.12 (m, 1H), 4.06 (q, 1H), 3.79 (d, 9H), 3.68 (s, 3H), 3.39 (d, 1H), 3.08 - 2.95 (m, 3H), 2.36 (s, 2H), 2.22 (dd, 1H), 2.14 (td, 2H), 1.98 - 1.88 (m, 1H), 1.76 (d, 7H), 1.72 - 1.61 (m, 1H). LCMS[M+2H/2] = 298.3.

### Biological Examples

### Standard Microbiological Activity:

A certified BSL-2 laboratory was used for testing. Compounds were evaluated using the broth microdilution minimum inhibitory concentration (MIC) and minimum bactericidal concentration (MBC) assays defined by Clinical and Laboratory Standards Institute (CLSI) in the M26-A guideline against *S. aureus* (Sa), *E. coli* (Ec), *K. pneumoniae* (Kp), *A. baumannii* (Ab), *E. faecalis* (Ef) and *P. aeruginosa* (Pa).

***E. coli* S30 extract:** Inhibition of bacterial protein synthesis was determined using the E. coli S30 Extract System for Circular DNA (Promega catalog #L-2010) and Luciferase Assay Reagent (Promega catalog #E1500) with slight modifications to a published protocol. Fyfe, C., Sutcliffe, J.A. and Grossman, T.H. (2012) "Development and characterization of a Pseudomonas aeruginosa in vitro coupled transcription-translation assay system for evaluation of translation inhibitors" J. Microbiol. Methods 90(3), 256-261.

Compounds were serial diluted in 0.5 mL microcentrifuge tubes by mixing and transferring 50 µL from the highest concentration to 50 µL of water, mixing and transferring 50 µL of this 2-fold dilution to 50 µL of water. This mixing and transferring was repeated so that there are a total of 8 tubes with serial dilutions of compound at 10x the desired screening concentration that are ultimately diluted to 1x by the addition of S30 luciferase synthesis mixture. Serial dilutions of compounds were added (2 µL) to wells in a black round bottom 96-well plate. Water (2 µL) was used as a "no inhibitor" control in 4 wells/plate. No DNA control reaction mixture (20 µL; see below) was used as a control in 4 wells/plate for background luminescence. S30 luciferase synthesis mixture (18 µL; see below) was added to wells with compounds or water mixture and incubated at 37 °C for 1 hour. Reactions were stopped by transferring to 4 °C refrigerator for 5 minutes then 25 µL of luciferase activity mix was added. Luminescence was measured using a BioTek Synergy HTX plate reader. % Inhibition was determined relative to no inhibitor controls.

### S30 luciferase synthesis mixture:

445 µL S30 extract, circular
712 µL S30 Premix without amino acids
4.45 µL pBESTluc^{™} DNA (1 µg/ µL)
78 µL complete amino acid mixture
267 µL water

### No DNA control:

20 µL S30 extract, circular
32 µL S30 Premix without amino acids
7 µL complete amino acid mixture
21 µL water

### Rabbit Reticulocyte lysate

Inhibition of eukaryotic protein synthesis was determined using the Rabbit Reticulocyte Lysate System, Nuclease-Treated from Promega (catalog #L-4960) with slight modifications to the manufacturer's protocol. Compounds were serial diluted in 0.5 mL microcentrifuge tubes by mixing and transferring 50 µL from the highest concentration to 50 µL of water, mixing and transferring 50 µL of this 2-fold dilution to 50 µL of water. This mixing and transferring was repeated so that there are a total of 8 tubes with serial dilutions of compound at 10x the desired screening concentration that are ultimately diluted to 1x by the addition of rabbit reticulocyte luciferase synthesis mixture. Serial dilutions of compounds were added (2.5 µL) to wells in a black round bottom 96-well plate. Water (2.5 µL) was used as a "no inhibitor" control in 4 wells/plate. No RNA control reaction mixture (2 µL; see below) was used as a control in 4 wells/plate for background luminescence. Rabbit reticulocyte luciferase synthesis mixture (22.5 µL; see below) was added to wells with compounds or water mixture and incubated at 30 °C for 90 minutes. Luciferase assay reagent (25 µL) was added with luminescence measured using a BioTek Synergy HTX plate reader. % Inhibition was determined relative to no inhibitor controls.

### Rabbit reticulocyte luciferase synthesis mixture:

1,000 µL rabbit reticulocyte lysate
5.7 µL Luciferase Control RNA (1 µg/ µL)
26 µL complete amino acid mixture
395 µL water

### No RNA Control

70 µL rabbit reticulocyte lysate
2 µL complete amino acid mixture
28 µL water

### Minimum Inhibitory Concentration (MIC)

MICs were determined using the Clinical Laboratory and Standards Institute (CLSI) Broth Microdilution Method with slight modification. Clinical and Laboratory Standards Institute (2012). "Methods for dilution antimicrobial susceptibility tests for bacteria that grow aerobically; approved standard, 9th ed. M07-A9. Clinical and Laboratory Standards Institute, Wayne, PA." Serial two-fold dilutions of compounds are prepared in sterile clear roundbottom 96-well plates.

To prepare microdilution trays, two-fold dilutions of antimicrobial agent are prepared in growth medium: Cation-Adjusted Mueller-Hinton Broth (CAMHB), or CAMHB supplemented with sodium bicarbonate (6.25 or 25 mM final concentration prepared from a 1.0M stock solution) or CAMHB supplemented with heat inactivated human serum (Fisher Cat. # BP2657100) 0-50% by adding 200 µL of the highest concentration to be tested (64 µg/mL, for example) in row A, mixing and transferring 100 µL from row A to 100 µL growth medium in row B, then repeating the mixing and transferring through row H of the 96-well plate, discarding the excess 100 µL remaining. This slight modification to the CLSI protocol enables evaluation of MICs for 3 compounds per plate in triplicate, albeit with only 8 compound dilutions (CLSI protocol enables 2 compounds in triplicate with 10 dilutions). Bacterial suspensions are added to a final concentration of 5 × 10⁴ CFU/well by adding 5 µL of a 1:10 dilution of a 0.5 McFarland suspension (1 × 10⁸ CFU/mL) for each bacterium evaluated. Bacterial suspensions were prepared using the growth method described by CLSI. Well-isolated colonies (3-5 from an agar plate) were selected using a sterile loop and used to inoculate a tube containing 4 mL of CAMHB. The cultures are incubated at 35 ± 2°C until it achieves or exceeds the turbidity of the 0.5 McFarland standard, determined by measuring A₆₀₀ₙₘ (usually two to six hours). When growth exceeds a 0.5 McFarland standard, the turbidity is adjusted with broth to be equivalent to a 0.5 McFarland standard.

Data for compounds is provided in Table 6. An IC₅₀ value (µM) that is 1 µM or greater (% inhibition is ≤ 50% @ 1 µM) is designated by a "+". An IC₅₀ value that is 0.5 µM or greater and less than 1 µM (% inhibition is > 50% and ≤ 90% @ 1 µM) is designated by a "++". An IC₅₀ value that is less than 0.5 µM (% inhibition is > 90% @ 1 µM) is designated by "+++". An MIC value (µg/mL) that is 32 µg/mL or greater is designated by a "+". An MIC value (µg/mL) that is 8 µg/mL or greater and less than 32 µg/mL is designated by a "++". An MIC value (µg/mL) that is less than 8 µg/mL is designated by "+++". "NA" means not available.

**Table 6. Biological Activity of Compounds of Formula I or a pharmaceutically acceptable salt thereof**

| **No.** | **Sa + bicarb 25** | **Sa + bicarb 6.25** | **E.coli + bicarb 25** | **E. coli + bicarb 6.25** | **Kp (1705) +bicarb 25** | **Kp (1705) +bicarb 6.25** | **Kp (060) +bicarb 25** | **Kp (060) +bicarb 6.25** |
|---|---|---|---|---|---|---|---|---|
| 1 | NA | NA | NA | NA | NA | NA | NA | NA |
| 2 | NA | NA | NA | +++ | NA | NA | NA | NA |
| 3 | NA | NA | NA | NA | NA | NA | NA | NA |
| 4 | NA | NA | NA | NA | NA | NA | NA | NA |
| 5 | NA | NA | NA | NA | NA | NA | NA | NA |
| 6 | +++ | NA | +++ | NA | +++ | NA | +++ | NA |
| 7 | +++ | NA | +++ | NA | ++ | NA | ++ | NA |
| 8 | +++ | NA | +++ | NA | +++ | NA | +++ | NA |
| 9 | +++ | NA | +++ | +++ | +++ | ++ | +++ | ++ |
| 10 | +++ | NA | +++ | NA | +++ | NA | +++ | NA |
| 11 | +++ | NA | +++ | NA | ++ | NA | ++ | NA |
| 12 | ++ | NA | +++ | NA | + | NA | + | NA |
| 13 | ++ | NA | ++ | NA | ++ | NA | ++ | NA |
| 14 | +++ | NA | +++ | NA | ++ | NA | ++ | NA |
| 15 | +++ | NA | +++ | NA | +++ | NA | +++ | NA |
| 16 | +++ | NA | +++ | NA | +++ | NA | +++ | NA |
| 17 | +++ | NA | +++ | NA | ++ | NA | +++ | NA |
| 18 | +++ | NA | +++ | NA | ++ | NA | ++ | NA |
| 19 | +++ | NA | +++ | NA | +++ | NA | +++ | NA |
| 20 | +++ | NA | +++ | NA | +++ | NA | +++ | NA |
| 21 | +++ | NA | +++ | NA | +++ | NA | +++ | NA |
| 22 | NA | ++ | NA | +++ | NA | + | NA | + |
| 23 | NA | + | NA | ++ | NA | + | NA | + |
| 24 | NA | + | NA | +++ | NA | + | NA | + |
| 25 | NA | +++ | NA | +++ | NA | + | NA | + |
| 26 | NA | +++ | NA | +++ | NA | + | NA | + |
| 27 | NA | +++ | NA | +++ | NA | ++ | NA | ++ |
| 28 | NA | +++ | NA | +++ | NA | ++ | NA | ++ |
| 29 | NA | +++ | NA | +++ | NA | + | NA | + |
| 30 | NA | +++ | NA | +++ | NA | + | NA | + |
| 31 | NA | +++ | NA | +++ | NA | ++ | NA | ++ |
| 32 | NA | +++ | NA | +++ | NA | ++ | NA | ++ |
| 33 | NA | ++ | NA | +++ | NA | + | NA | + |
| 34 | NA | +++ | NA | +++ | NA | + | NA | + |
| 35 | NA | +++ | NA | +++ | NA | + | NA | ++ |
| 36 | NA | ++ | NA | +++ | NA | + | NA | ++ |
| 37 | NA | ++ | NA | +++ | NA | + | NA | ++ |
| 38 | NA | ++ | NA | +++ | NA | + | NA | ++ |
| 39 | NA | ++ | NA | ++ | NA | + | NA | + |
| 40 | NA | +++ | NA | +++ | NA | ++ | NA | + |
| 41 | NA | ++ | NA | +++ | NA | + | NA | + |
| 42 | NA | ++ | NA | +++ | NA | + | NA | + |
| 43 | +++ | +++ | +++ | +++ | NA | +++ | +++ | +++ |
| 44 | NA | + | NA | ++ | NA | + | NA | + |
| 45 | NA | + | NA | + | NA | + | NA | + |
| 46 | +++ | NA | +++ | NA | ++ | NA | +++ | NA |

**Table 6.Continued. Biological Activity of Compounds of Formula I or a pharmaceutically acceptable salt thereof**

| **No.** | **Pa +bicarb 25** | **Pa +bicarb 6.25** | **Ab +bicarb 25** | **Ab +bicarb 6.25** | **Ef +bicarb 25** | **Ef +bicarb 6.25** | **S30 IC₅₀ (uM)** | **retic. IC₅₀ (uM)** |
|---|---|---|---|---|---|---|---|---|
| 1 | NA | NA | NA | NA | NA | NA | + | + |
| 2 | NA | NA | NA | NA | NA | NA | +++ | + |
| 3 | NA | NA | NA | NA | NA | NA | +++ | + |
| 4 | ++ | NA | NA | NA | NA | NA | +++ | + |
| 5 | NA | NA | NA | NA | NA | NA | +++ | + |
| 6 | + | NA | + | NA | +++ | NA | +++ | + |
| 7 | + | NA | + | NA | +++ | NA | +++ | + |
| 8 | ++ | NA | ++ | NA | +++ | NA | +++ | + |
| 9 | + | NA | + | NA | +++ | NA | +++ | + |
| 10 | + | NA | + | NA | +++ | NA | +++ | + |
| 11 | + | NA | + | NA | +++ | NA | ++ | + |
| 12 | + | NA | + | NA | + | NA | + | + |
| 13 | ++ | NA | ++ | NA | ++ | NA | + | + |
| 14 | ++ | NA | ++ | NA | ++ | NA | + | + |
| 15 | ++ | NA | ++ | NA | +++ | NA | +++ | + |
| 16 | ++ | NA | ++ | NA | +++ | NA | +++ | + |
| 17 | ++ | NA | ++ | NA | +++ | NA | ++ | + |
| 18 | ++ | NA | ++ | NA | ++ | NA | + | + |
| 19 | ++ | NA | ++ | NA | +++ | NA | +++ | + |
| 20 | ++ | NA | ++ | NA | +++ | NA | +++ | + |
| 21 | ++ | NA | ++ | NA | +++ | NA | +++ | + |
| 22 | NA | + | NA | + | NA | + | ++ | + |
| 23 | NA | + | NA | + | NA | + | + | + |
| 24 | NA | + | NA | + | NA | + | +++ | + |
| 25 | NA | + | NA | + | NA | + | +++ | + |
| 26 | NA | + | NA | + | NA | + | +++ | + |
| 27 | NA | + | NA | + | NA | +++ | +++ | + |
| 28 | NA | + | NA | + | NA | ++ | +++ | + |
| 29 | NA | + | NA | + | NA | ++ | +++ | + |
| 30 | NA | + | NA | + | NA | ++ | +++ | + |
| 31 | NA | + | NA | + | NA | ++ | +++ | + |
| 32 | NA | + | NA | + | NA | ++ | +++ | + |
| 33 | NA | + | NA | + | NA | + | +++ | + |
| 34 | NA | + | NA | + | NA | + | +++ | + |
| 35 | NA | + | NA | + | NA | + | +++ | + |
| 36 | NA | + | NA | + | NA | + | +++ | +++ |
| 37 | NA | + | NA | + | NA | + | +++ | + |
| 38 | NA | + | NA | + | NA | + | +++ | + |
| 39 | NA | + | NA | + | NA | + | ++ | + |
| 40 | NA | + | NA | + | NA | ++ | +++ | + |
| 41 | NA | + | NA | + | NA | + | +++ | + |
| 42 | NA | + | NA | + | NA | + | + | + |
| 43 | ++ | + | ++ | + | +++ | + | +++ | + |
| 44 | NA | + | NA | + | NA | + | + | + |
| 45 | NA | + | NA | + | NA | + | + | + |
| 46 | ++ | NA | ++ | NA | ++ | NA | +++ | + |

The foregoing disclosure has been described in some detail by way of illustration and example, for purposes of clarity and understanding. The invention has been described with reference to various specific and preferred embodiments and techniques. However, it should be understood that many variations and modifications can be made while remaining within the scope of the invention. It will be obvious to one of skill in the art that changes and modifications can be practiced within the scope of the appended claims. Therefore, it is to be understood that the above description is intended to be illustrative and not restrictive. The scope of the invention should, therefore, be determined not with reference to the above description, but should instead be determined with reference to the following appended claims.

## Claims

1. A compound of formula I: 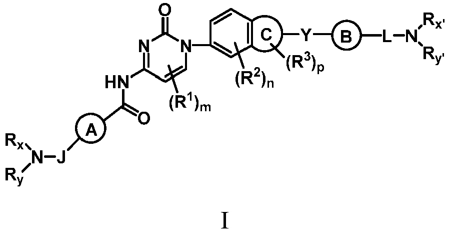 or a single stereoisomer or mixture of stereoisomers thereof, or a pharmaceutically acceptable salt thereof, wherein:
ring A is a 3-8 membered monocyclic heterocycloalkylene optionally substituted with up to three substituents selected from the group consisting of C₁-C₆ alkyl, C₁-C₆ alkoxy, halo, CN, C₁-C₆ haloalkyl, phenyl, OH, NH₂, NH(C₁-C₆ alkyl), N(C₁-C₆ alkyl)₂, COOH, COO(Ci-C₆ alkyl), CONH₂, CONH(C₁-C₆ alkyl), CON(C₁-C₆ alkyl)₂, and oxo;
J is C₁-C₆ alkylene or C₃-C₈ cycloalkylene, either of which is optionally substituted with halo, OH, or C₁-C₆ alkoxy, wherein up to two methylene units of the C₁-C₆ alkylene are optionally and independently replaced with O, S, SO, SO₂, or C=O;
Rₓ, R_{y}, R_{x'}, and R_{y'} are each independently H, C₁-C₆ alkyl, or an amino protecting group;
Y is a bond or C₁-C₆ alkylene optionally substituted with OH, NH₂, CN, halo, or Ci-C₆ alkoxy, wherein up to two methylene units of the C₁-C₆ alkylene are optionally and independently replaced by O, NH, N-(C₁-C₆ alkyl), N-(C₁-C₆ hydroxyalkyl), N-(C₁-C₆ haloalkyl), N-(C₁₋₆ alkylene-C₃₋₈ cycloalkyl), NH(C=O), N-(C₁₋₆ alkyl)(C=O), or (C=O);
ring B is a 3-8 membered monocyclic cycloalkylene, 3-8 membered monocyclic heterocycloalkylene, 6-12 membered bicyclic cycloalkylene, or a 6-12 membered bicyclic heterocycloalkylene, each of which is optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and Ci-C₆ hydroxyalkyl;
L is a bond or a C₁-C₆ alkylene, wherein up to two methylene units of the C₁-C₆ alkylene may be independently replaced with O, NH, (C=O), NH(C=O), N-(C₁₋₆ alkyl)(C=O), (C=NH), NH(C=N), or N-(C₁₋₆ alkyl);
ring C, together with the phenyl ring to which it is fused, forms an 8-12 membered bicyclic arylene or an 8-12 membered bicyclic heteroarylene, wherein the bicyclic heteroarylene has 1-3 heteroatoms independently selected from N, O, or S;
R¹, R², and R³ are each independently selected from the group consisting of C₁-C₆ alkyl, halo, CN, OH, NH₂, NH(C₁-C₆ alkyl), N(C₁-C₆ alkyl)₂, COO(C₁-C₆ alkyl), CONH₂, C₁-C₆ haloalkyl, oxo, and C₁-C₆ alkoxy; and
m, n, and p are each independently 0, 1, 2, or 3.

2. The compound of claim 1, or a single stereoisomer or mixture of stereoisomers thereof, or a pharmaceutically acceptable salt thereof, wherein ring A is a 5-6 membered monocyclic heterocycloalkylene optionally substituted with up to three substituents selected from the group consisting of C₁-C₆ alkyl, C₁-C₆ alkoxy, halo, CN, C₁-C₆ haloalkyl, phenyl, OH, NH₂, and oxo;
such as wherein ring A is 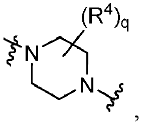 wherein each R⁴ is independently selected from the group consisting of C₁-C₆ alkyl, C₁-C₆ alkoxy, halo, CN, C₁-C₆ haloalkyl, phenyl, OH, NH₂, and oxo, wherein q is 0, 1, or 2;
such as wherein ring A is

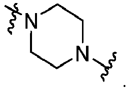

3. The compound of claim 1 of claim 2, or a single stereoisomer or mixture of stereoisomers thereof, or a pharmaceutically acceptable salt thereof, wherein J is C₁-C₆ alkylene optionally substituted with halo, OH, or C₁-C₆ alkoxy, wherein up to two methylene units of the C₁-C₆ alkylene are optionally and independently replaced with O, S, SO, SO₂, or C=O;
such as wherein J is C₁-C₆ alkylene optionally substituted with OH, wherein one methylene unit of the C₁-C₆ alkylene is replaced with C=O;
such as wherein J is

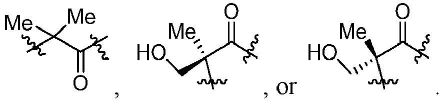

4. The compound of any one of claims 1-3, or a single stereoisomer or mixture of stereoisomers thereof, or a pharmaceutically acceptable salt thereof, wherein Rₓ and R_{y} are H;
such as wherein 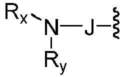 is 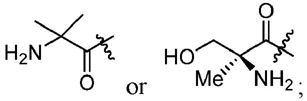
such as wherein 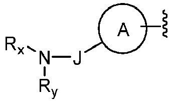 is

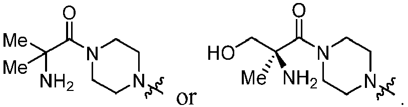

5. The compound of any one of claims 1-4, or a single stereoisomer or mixture of stereoisomers thereof, or a pharmaceutically acceptable salt thereof, wherein 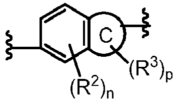 is selected from the group consisting of 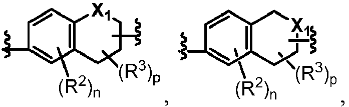 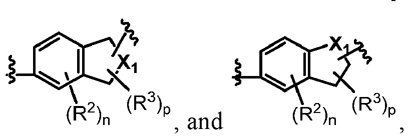 wherein each X₁ is independently selected from CH₂, CH, O, S, SO, SO₂, N, and NH, and wherein R² and R³ are each independently C₁-C₆ alkyl, halo, or C₁-C₆ haloalkyl, wherein n and p are each independently 0, 1, or 2;
such as wherein 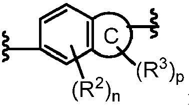 is selected from the group consisting of 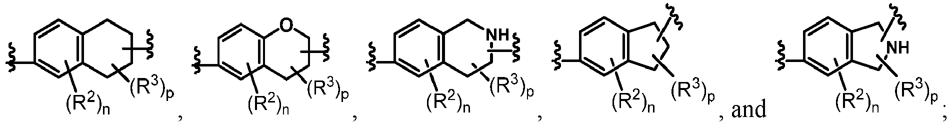
such as wherein 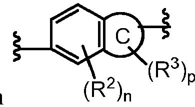 is selected from the group consisting of

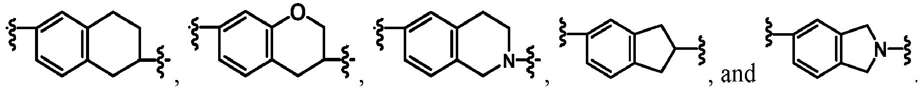

6. The compound of any one of claims 1-5, or a single stereoisomer or mixture of stereoisomers thereof, or a pharmaceutically acceptable salt thereof, wherein Y is a bond;
or wherein Y is C₁-C₃ alkylene optionally substituted with OH, NH₂, halo, or C₁-C₆ alkoxy, and wherein one methylene unit of the C₁-C₃ alkylene is optionally replaced by O, NH, N-(C₁-C₆ alkyl), N-(C₁-C₆ hydroxyalkyl), N-(C₁-C₆ haloalkyl), N-(C₁₋₆ alkylene-C₃₋₈ cycloalkyl), NH(C=O), N-(C₁₋₆ alkyl)(C=O), or (C=O);
or wherein Y is selected from the group consisting of O, NH, NH-C₁₋₂ alkylene, N(Ci-C₆ alkyl), N(C₁-C₆ hydroxyalkyl), N(C₁-C₆ haloalkyl), N(C₁₋₆ alkylene-C₃₋₈cycloalkyl), NH(C=O), N(C₁₋₆ alkyl) (C=O), and (C=O); such as wherein Y is selected from the group consisting of -NH-, -NMe-, -NEt-, -NH-CH₂-, and -N(CH₂-cyclopropyl)-;
or wherein 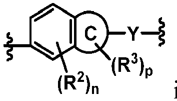 is selected from the group consisting of 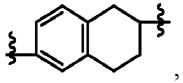 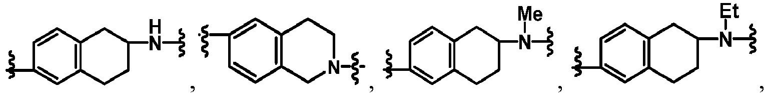 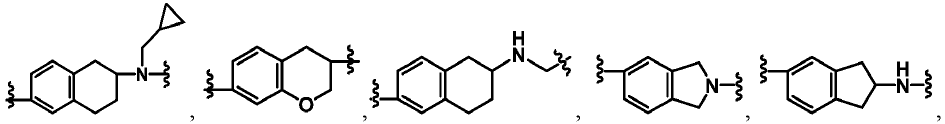 and

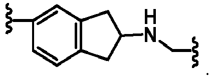

7. The compound of any one of claims 1-6, or a single stereoisomer or mixture of stereoisomers thereof, or a pharmaceutically acceptable salt thereof, wherein ring B is a 3-8 membered monocyclic cycloalkylene optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and Ci-C₆ hydroxyalkyl; such as wherein ring B is a 4-6 membered monocyclic cycloalkylene optionally substituted with up to three substituents selected from the group consisting of C₁-C₆ alkyl, C₁-C₆ alkoxy, halo, CN, C₁-C₆ haloalkyl, OH, and C₁-C₆ hydroxyalkyl;
or wherein B is a 3-8 membered monocyclic heterocycloalkylene optionally substituted with up to three substituents selected from the group consisting of C₁-C₆ alkyl, C₁-C₆ alkoxy, halo, CN, C₁-C₆ haloalkyl, OH, COOH, COO(C₁-C₆ alkyl), CONH₂, CONH(C₁-C₆ alkyl), CON(C₁-C₆ alkyl)₂, and C₁-C₆ hydroxyalkyl; such as wherein ring B is a 4-7 membered monocyclic heterocycloalkylene optionally substituted with up to three substituents selected from the group consisting of C₁-C₆ alkyl, C₁-C₆ alkoxy, halo, CN, C₁-C₆ haloalkyl, OH, and C₁-C₆ hydroxyalkyl, and wherein B contains up to 2 nitrogen atoms;
or wherein ring B is a 6-10 membered bicyclic cycloalkylene optionally substituted with up to three substituents selected from the group consisting of C₁-C₆ alkyl, C₁-C₆ alkoxy, halo, C₁-C₆ haloalkyl, OH, and C₁-C₆ hydroxyalkyl; such as wherein ring B is a 6-10 membered fused, spiro, or bridged bicyclic cycloalkylene;
or wherein ring B is a 6-12 membered bicyclic heterocycloalkylene optionally substituted with up to three substituents selected from the group consisting of C₁-C₆ alkyl, C₁-C₆ alkoxy, halo, C₁-C₆ haloalkyl, OH, and C₁-C₆ hydroxyalkyl; such as wherein ring B is a 6-9 membered fused, spiro, or bridged bicyclic heterocycloalkylene containing up to 3 nitrogen atoms; such as a 6-9 membered fused, spiro, or bridged bicyclic heterocycloalkylene containing up to 2 nitrogen atoms;
or wherein ring B is selected from the group consisting of:

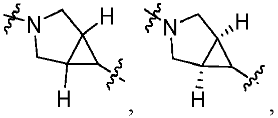

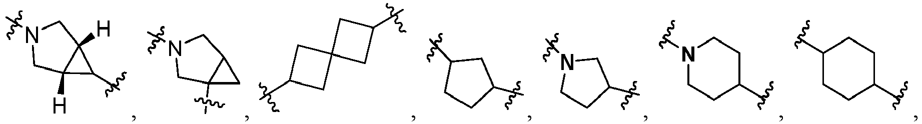

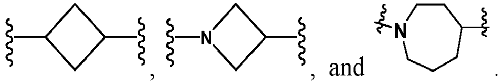

8. The compound of any one of claims 1-7, or a single stereoisomer or mixture of stereoisomers thereof, or a pharmaceutically acceptable salt thereof, wherein L is a bond;
or wherein L is C₁-C₆ alkylene, wherein up to two methylene units of the C₁-C₆ alkylene are optionally and independently replaced with O, NH, (C=O), NH(C=O), N-(C₁₋₆ alkyl)(C=O), (C=NH), NH(C=N), or N-(C₁₋₆ alkyl); such as wherein L is C₁-C₆ alkylene; such as wherein L is -CH₂- or -CH₂CH₂-.

9. The compound of any one of claims 1-8, or a single stereoisomer or mixture of stereoisomers thereof, or a pharmaceutically acceptable salt thereof, wherein R_{x'} and R_{y'} are each independently selected from the group consisting of H, Boc, and methoxycarbonyl;
such as wherein 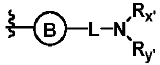 is selected from the group consisting of

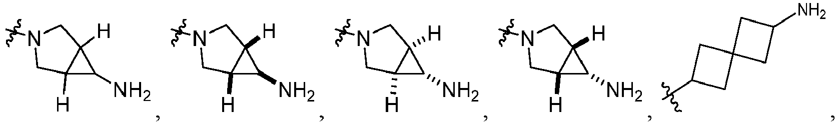

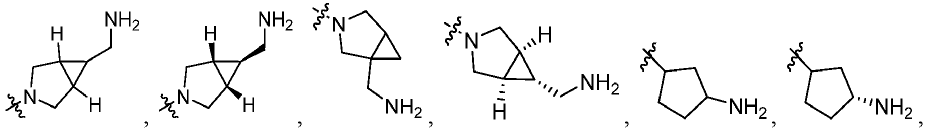

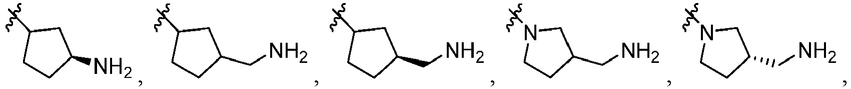

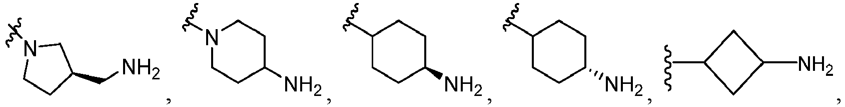

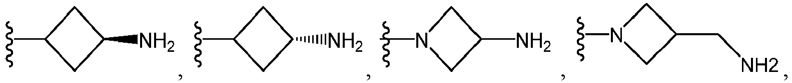

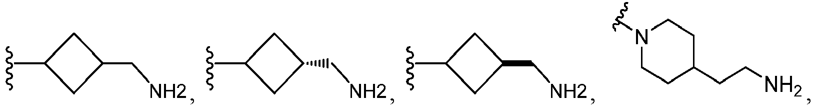

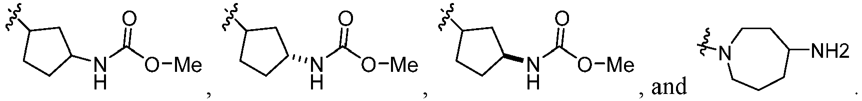

10. The compound of any one of claims 1-9, or a single stereoisomer or mixture of stereoisomers thereof, or a pharmaceutically acceptable salt thereof, wherein R¹, R², and R³ are each independently selected from the group consisting of C₁-C₆ alkyl, halo, C₁-C₆ haloalkyl, oxo, and C₁-C₆ alkoxy, and m, n, and p are each independently 0, 1, or 2;
such as wherein R¹, R², and R³ are each independently C₁-C₆ alkyl, halo, oxo, or Ci-C₆ haloalkyl, and m, n, and p are each independently 0 or 1.

11. The compound of any one of claims 1-10, or a single stereoisomer or mixture of stereoisomers thereof, or a pharmaceutically acceptable salt thereof, wherein the compound is a compound of formula IA: 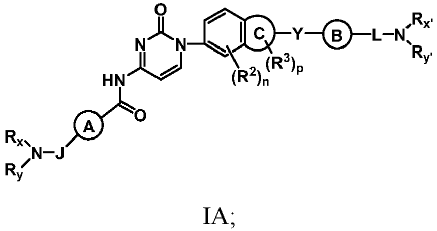
or a compound of formula IA-1: 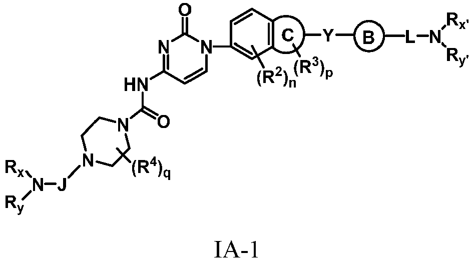 wherein each R⁴ is independently selected from the group consisting of H, C₁-C₆ alkyl, C₁-C₆ alkoxy, halo, CN, C₁-C₆ haloalkyl, OH, NH₂, and oxo, and wherein q is 0, 1, 2, or 3;
or a compound of formula IA-2: 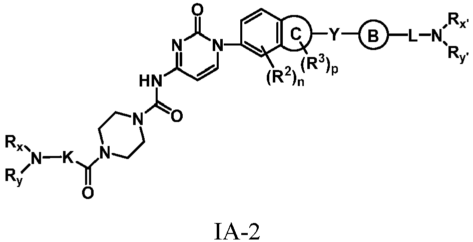 wherein K is C₁-C₄ alkylene optionally substituted with halo, hydroxyl or C₁-C₆ alkoxy group;
or a compound of formula IA-3: 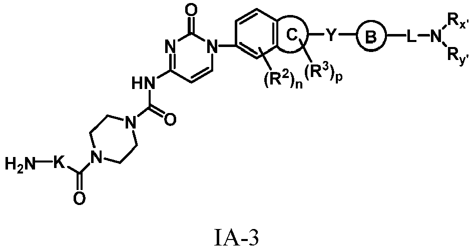 wherein K is C₁-C₃ alkylene optionally substituted with hydroxyl;
or a compound of formula IA-4a or IA-4b: 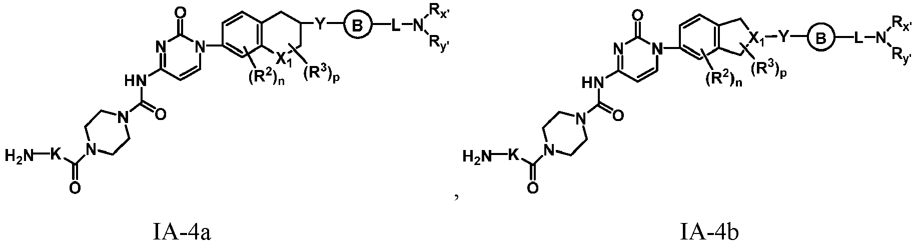 wherein each Xi is independently selected from the group consisting of CH₂, CH, O, S, N, and NH;
or a compound of formula IA-5a or IA-5b: 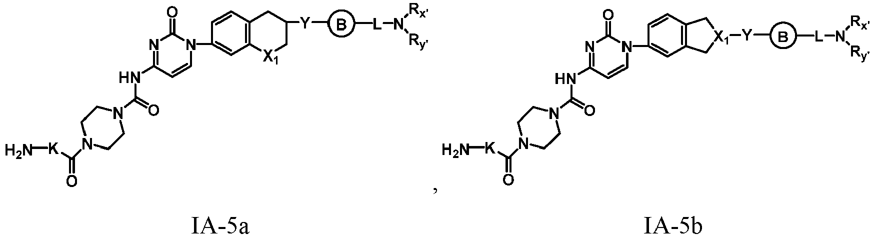 wherein each Xi is independently selected from the group consisting of CH₂, CH, NH, N, and O;
or a compound of formula IA-6a or IA-6b: 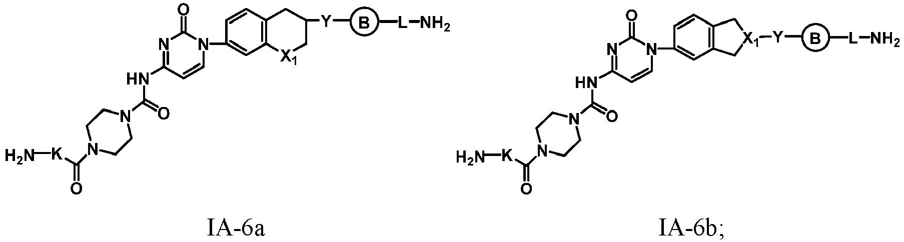
or a compound selected from the group consisting of formula IA-7a, formula IA-7b, formula IA-7c, formula IA-7d, formula IA-7e, and formula IA-7f: 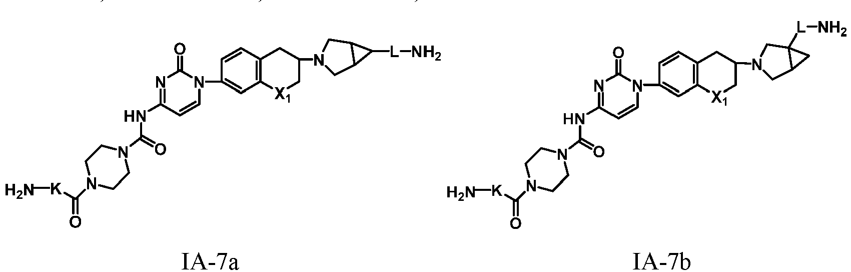 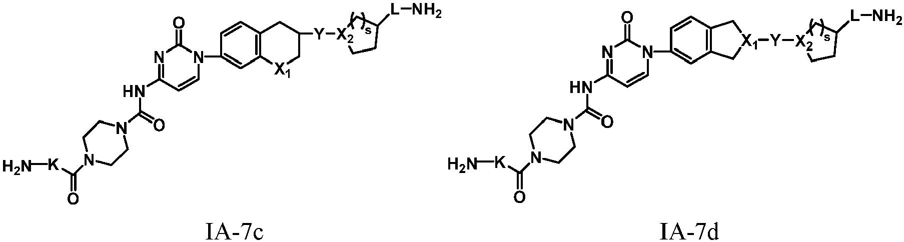 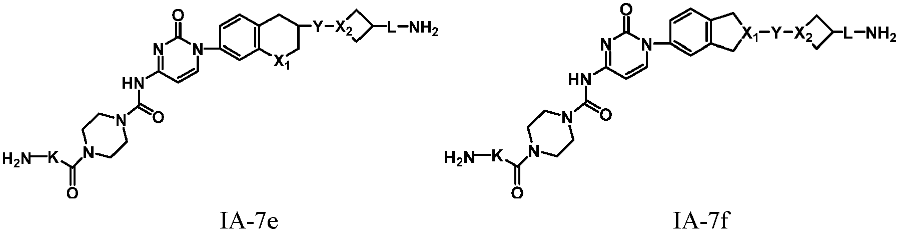 wherein each X₂ is independently CH or N, and each s is independently 1, 2, or 3.

12. The compound of claim 11, or a single stereoisomer or mixture of stereoisomers thereof, or a pharmaceutically acceptable salt thereof, wherein K in any of formulae IA-2, IA-3, IA-4a, IA-4b, IA-5a, IA-5b, IA-6a, IA-6b, IA-7a, IA-7b, IA-7c, IA-7d, IA-7e, or IA-7f is 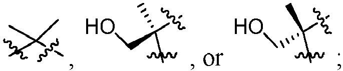 and/or
wherein Y is a bond, NH, NH-(C₁-C₆ alkylene)-, N-(C₁-C₆ alkyl), or N-(C₁-C₆ alkylene-Cs-Cs cycloalkyl); and/or
wherein L is a bond or C₁-C₆ alkylene.

13. A compound according to claim 1 which is listed in Table 1 or Table 2, or a pharmaceutically acceptable salt thereof:
**Table 1**
| **No.** | **Free Base Structure** |
|---|---|
| 1 | 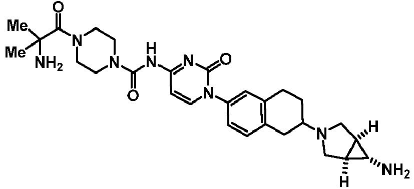 |
| 2 | 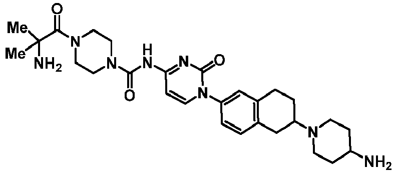 |
| 3 | 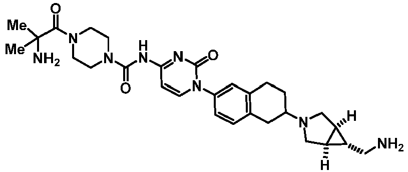 |
| 4 | 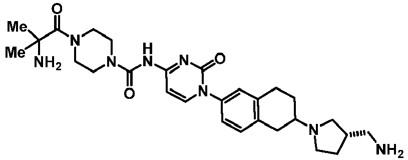 |
| 5 | 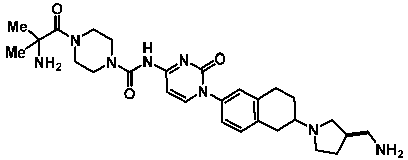 |
| 6 | 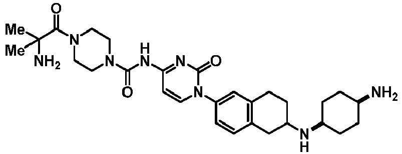 |
| 7 | 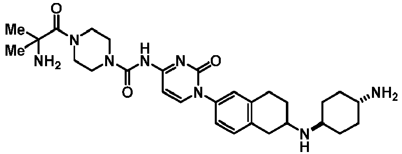 |
| 8 | 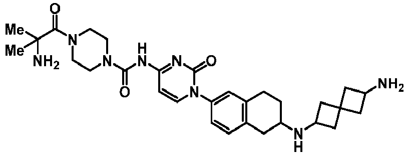 |
| 9 | 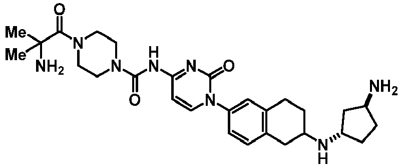 |
| 10 | 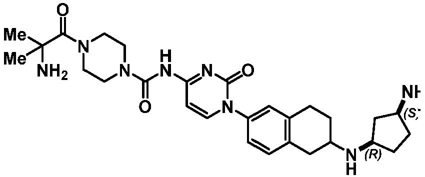 |
| 11 | 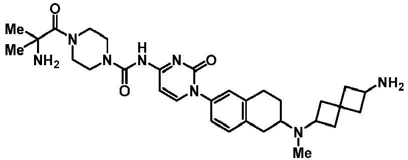 |
| 12 | 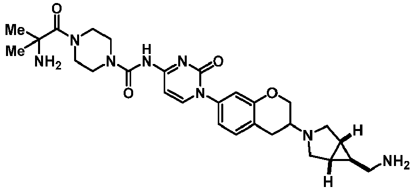 |
| 13 | 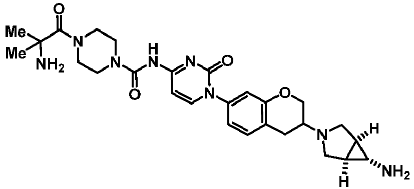 |
| 14 | 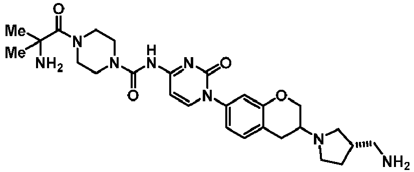 |
| 15 | 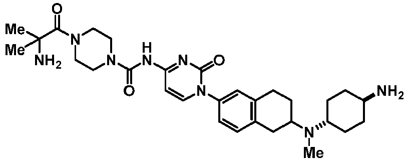 |
| 16 | 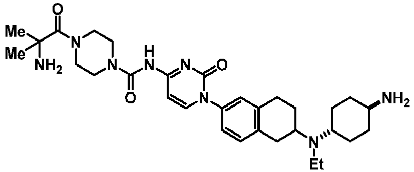 |
| 17 | 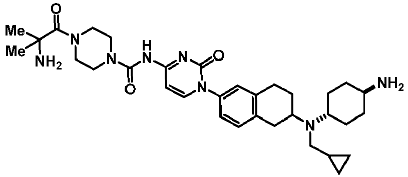 |
| 18 | 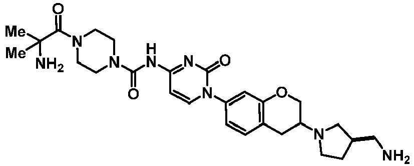 |
| 19 | 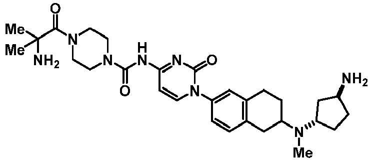 |
| 20 | 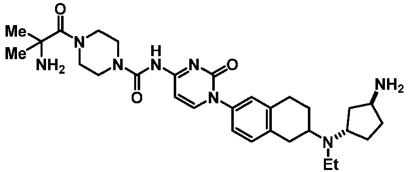 |
| 21 | 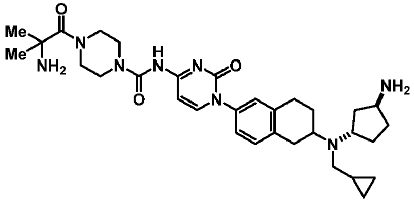 |
| 22 | 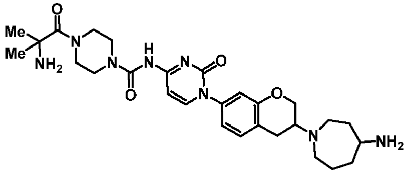 |
| 23 | 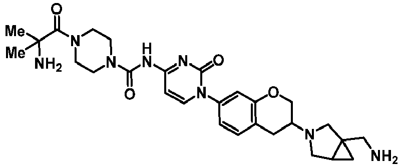 |
| 24 | 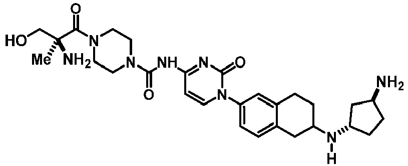 |
| 25 | 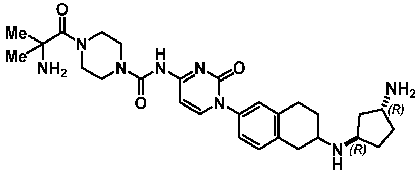 |
| 26 | 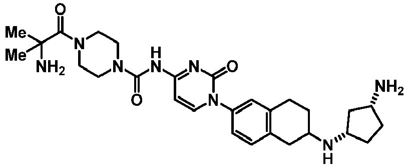 |
| 27 | 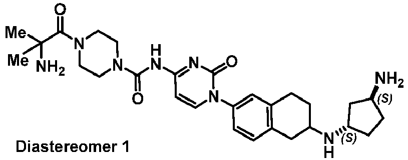 |
| 28 | 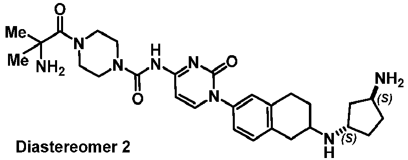 |
**Table 2**
| **No.** | **Free Base Structure** |
|---|---|
| 29 | 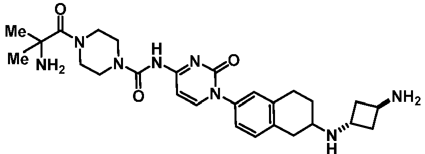 |
| 30 | 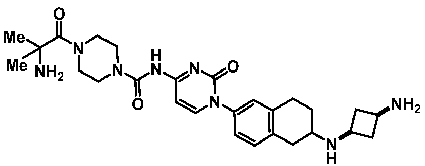 |
| 31 | 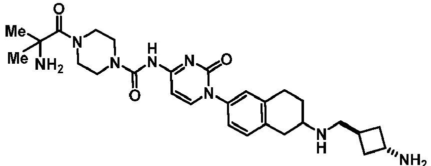 |
| 32 | 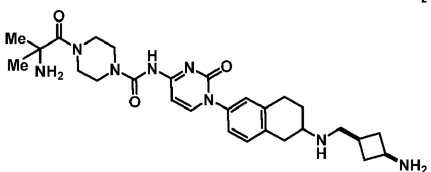 |
| 33 | 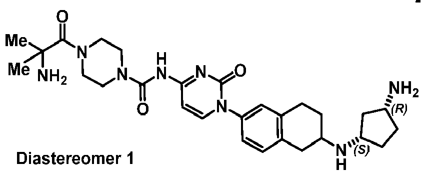 |
| 34 | 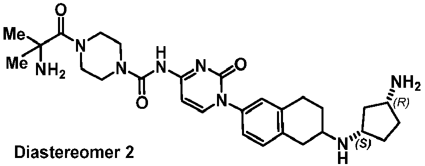 |
| 35 | 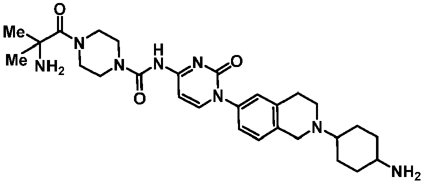 |
| 36 | 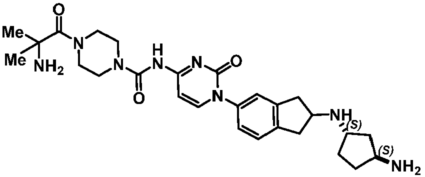 |
| 37 | 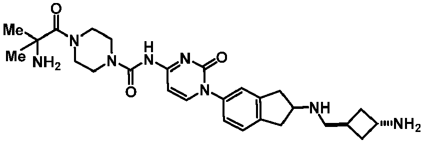 |
| 38 | 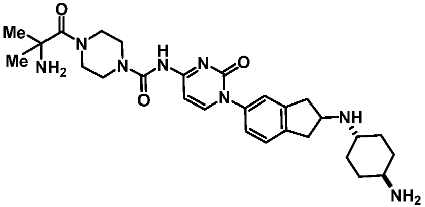 |
| 39 | 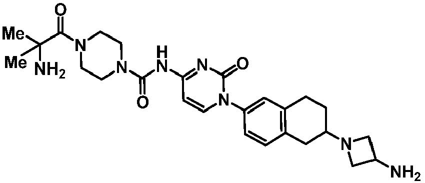 |
| 40 | 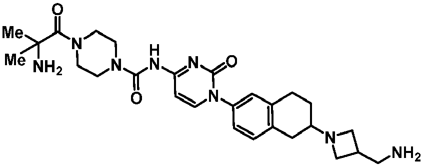 |
| 41 | 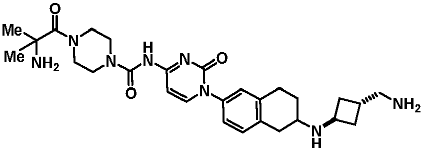 |
| 42 | 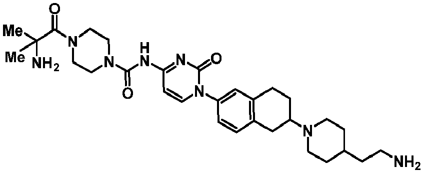 |
| 43 | 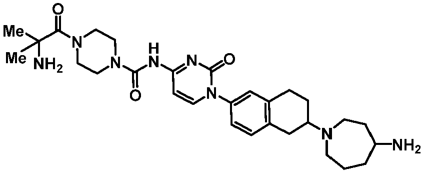 |
| 44 | 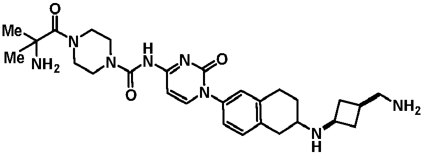 |
| 45 | 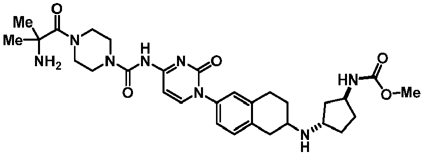 |
| 46 | 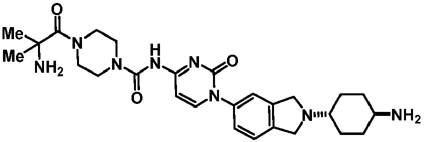 |

14. A pharmaceutical composition comprising a compound of any one of claims 1-13 or a pharmaceutically acceptable salt thereof and a pharmaceutically acceptable excipient.

15. A compound for use in a method of treating a bacterial infection in a patient in need of such treatment, wherein the method comprises administering the compound of any one of claims 1-13 or a pharmaceutically acceptable salt thereof or the pharmaceutical composition of claim 14.

16. The compound for use of claim 15, wherein the bacterial infection is caused by a bacterium including gram positive and gram negative bacteria.

17. A process for preparing a compound of formula I of claim 1: 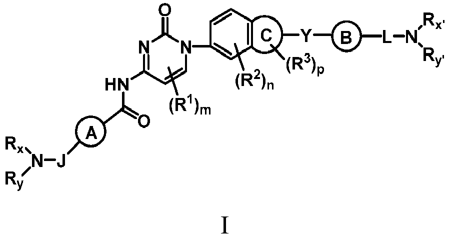 or a single stereoisomer or mixture of stereoisomers thereof, or a pharmaceutically acceptable salt thereof, the process comprising:
combining a compound of formula A: 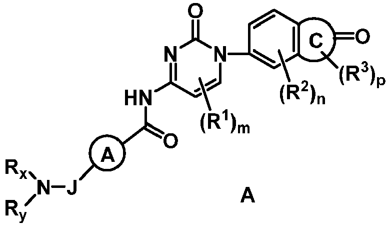 with a compound of:
formula B' 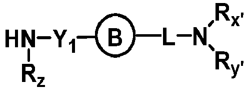 (B') or formula C 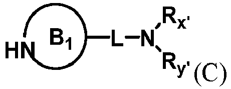 under a reductive amination condition to provide the compound of formula I,
wherein ring A, ring B, ring C, J, L, R¹, R², R³, Rₓ, R_{y}, R_{X'}, R_{y'}, m, n, and p are as defined in any one of claims 1-12;
ring Bi is a nitrogen containing 3-8 membered monocyclic heterocycloalkylene, or a nitrogen containing 6-12 membered bicyclic heterocycloalkylene, each of which is optionally and independently substituted with up to three substituents selected from the group consisting of C₁-C₆ alkyl, C₁-C₆ alkoxy, halo, CN, C₁-C₆ haloalkyl, OH, COO(C₁-C₆ alkyl), CONH₂, CONH(C₁-C₆ alkyl), CON(C₁-C₆ alkyl)₂, and C₁-C₆ hydroxyalkyl;
Y is a bond or C₁-C₆ alkylene optionally substituted with OH, NH₂, CN, halo, or Ci-C₆ alkoxy, wherein one methylene unit of the C₁-C₆ alkylene is optionally replaced by NH, N-(C₁-C₆ alkyl), N-(C₁-C₆ hydroxyalkyl), N-(C₁-C₆ haloalkyl), or N-(C₁-C₆ alkylene-Cs-Cs cycloalkyl);
Yi is a bond or C₁-C₅ alkylene optionally substituted with OH, NH₂, CN, halo, or Ci-C₆ alkoxy; and
R_{z} is H, C₁-C₆ alkyl, C₁-C₆ hydroxyalkyl, C₁-C₆ haloalkyl, or C₁-C₆ alkylene-Cs-Cs cycloalkyl.

18. A process for preparing a compound of formula ID: 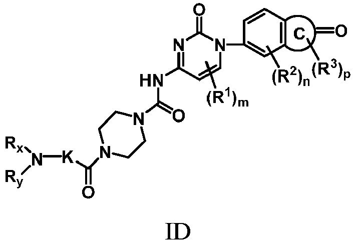 or a pharmaceutically acceptable salt thereof, the process comprising:
coupling a compound of formula E 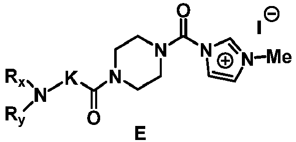
with a compound of formula F 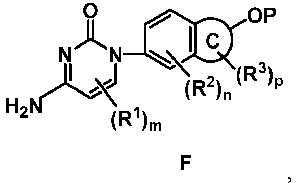 wherein ring C, K, R¹, R², R³, Rₓ, R_{y}, m, n, and p are as defined in any one of claims 1-12; and
P is a hydroxyl protecting group.

19. The process of claim 18, further comprising the step of removing the hydroxyl protecting group to provide a compound of formula IE 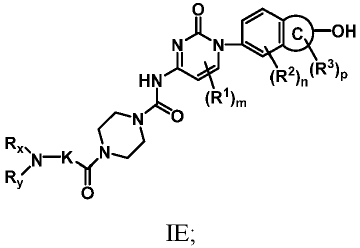 optionally further comprising the step of oxidizing the hydroxyl group to provide the compound of formula ID.

20. A compound of formula ID: 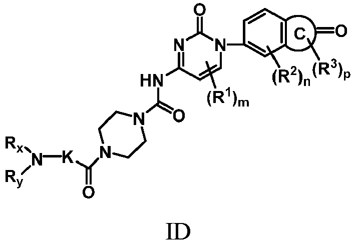 or a single stereoisomer or mixture of stereoisomers thereof, or a pharmaceutically acceptable salt thereof, wherein C, K, R¹, R², R³, Rₓ, R_{y}, m, n, and p are as defined in any one of claims 1-12.

21. A compound of formula II: 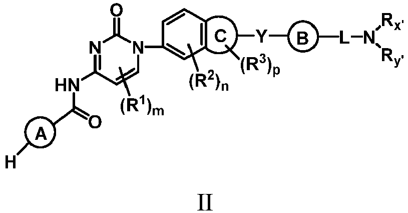 or a single stereoisomer or mixture of stereoisomers thereof, or a pharmaceutically acceptable salt thereof, wherein ring A, ring B, ring C, L, R¹, R², R³, R_{X'}, R_{y'}, m, n, and p are as defined in any one of claims 1-12.

22. The compound of claim 21 or a pharmaceutically acceptable salt thereof, wherein the compound is selected from the compounds listed in Table 3:
**Table 3**
| **Compound Structure** | **Compound Structure** |
|---|---|
| 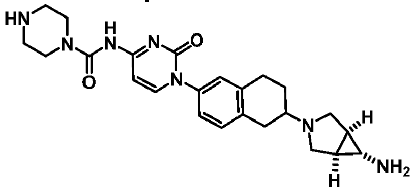 | 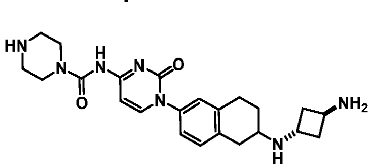 |
| 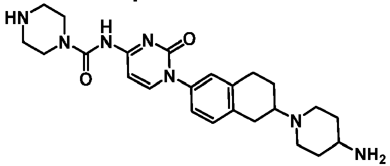 | 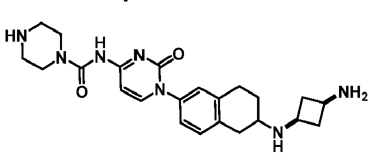 |
| 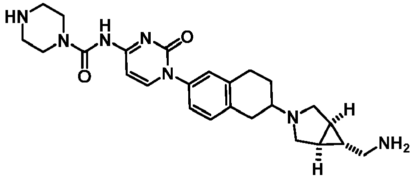 | 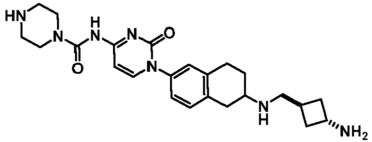 |
| 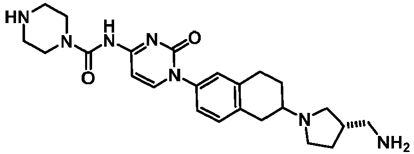 | 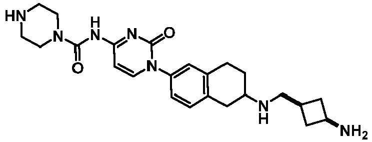 |
| 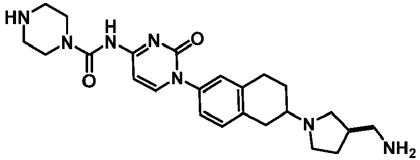 | 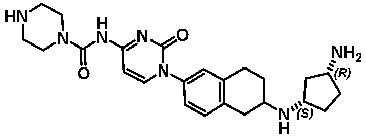 |
| 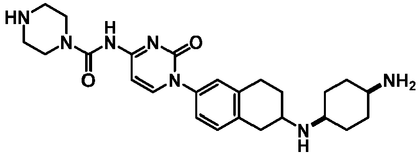 | 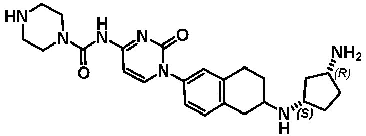 |
| 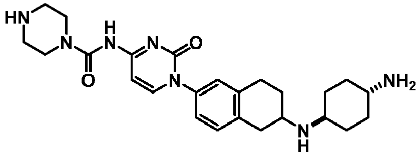 | 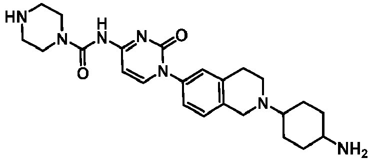 |
| 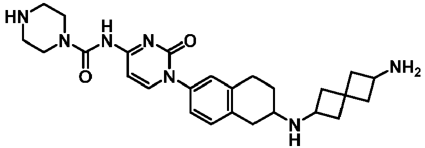 | 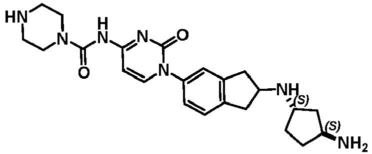 |
| 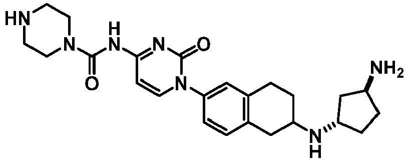 | 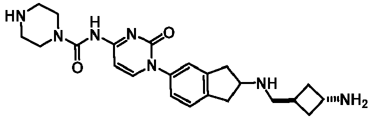 |
| 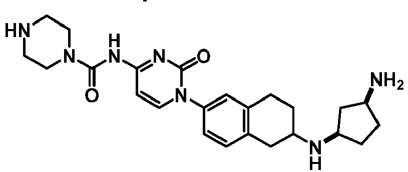 | 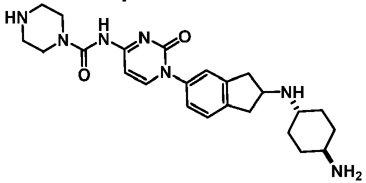 |
| 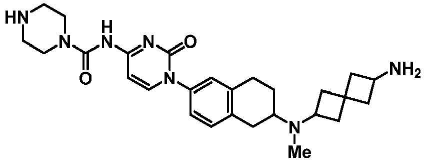 | 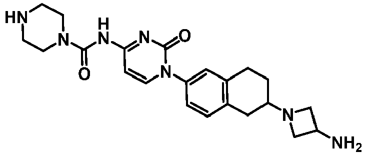 |
| 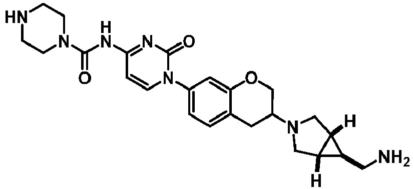 | 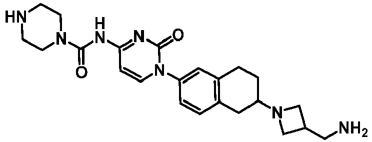 |
| 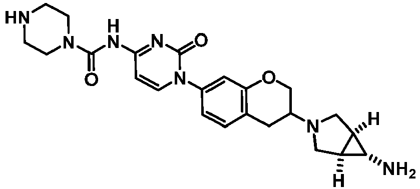 | 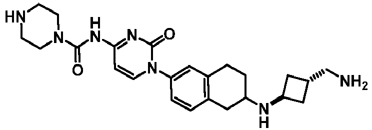 |
| 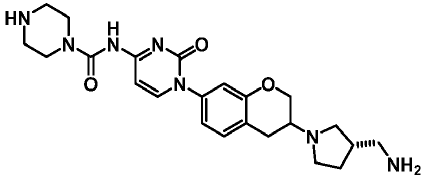 | 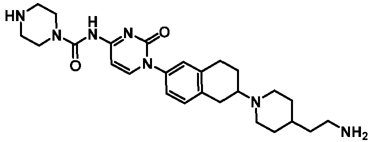 |
| 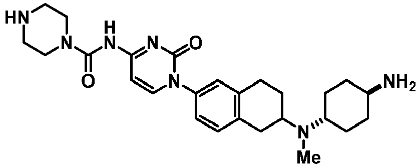 | 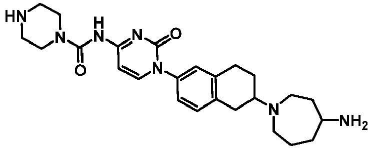 |
| 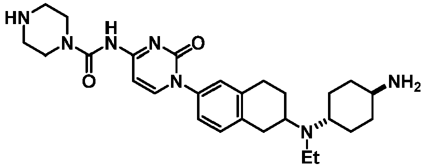 | 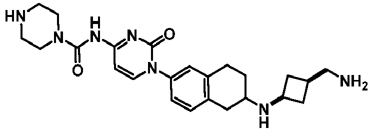 |
| 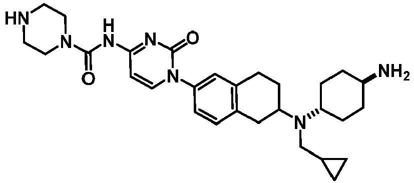 | 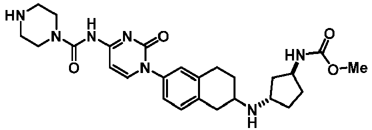 |
| 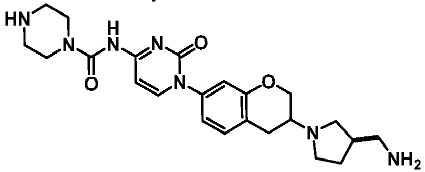 | 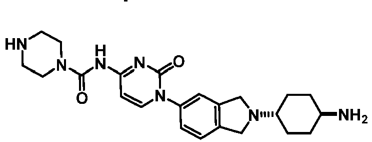 |
| 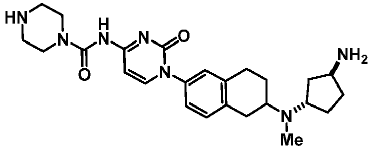 | 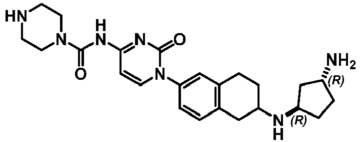 |
| 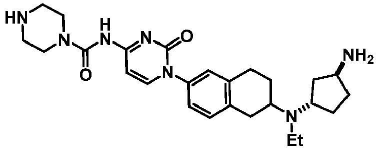 | 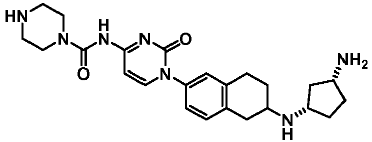 |
| 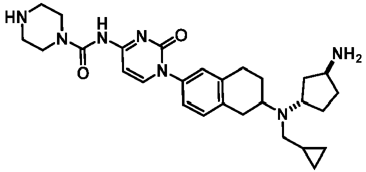 | 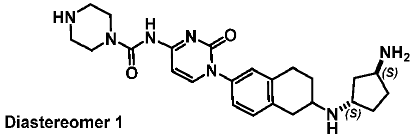 |
| 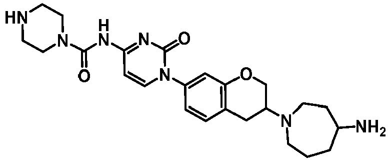 | 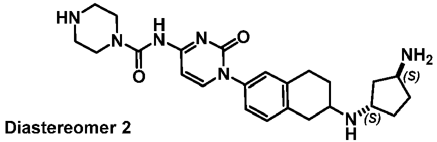 |
| 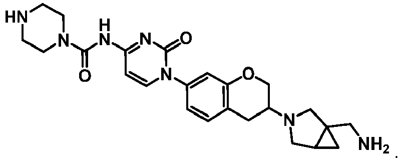 | |

23. A compound of formula III 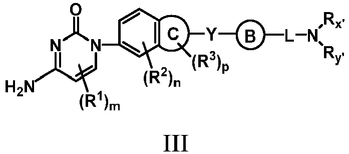 or a single stereoisomer or mixture of stereoisomers thereof, or a pharmaceutically acceptable salt thereof, wherein ring B, ring C, L, R¹, R², R³, R_{X'}, R_{y'}, m, n, and p are as defined in any one of claims 1-12.

24. The compound of claim 23 or a pharmaceutically acceptable salt thereof, wherein the compound is selected from the compounds listed in Table 4:
**Table 4**
| **Compound Structure** | **Compound Structure** |
|---|---|
| 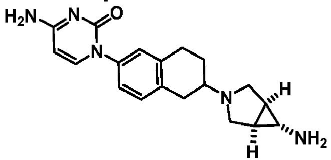 | 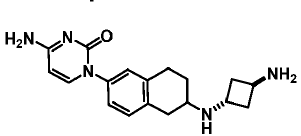 |
| 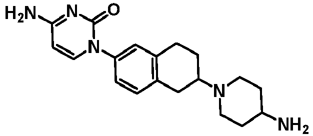 | 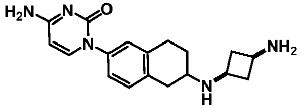 |
| 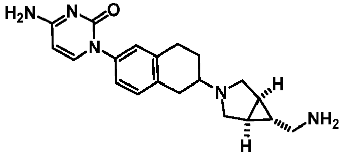 | 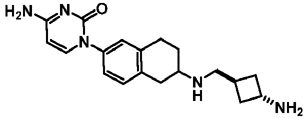 |
| 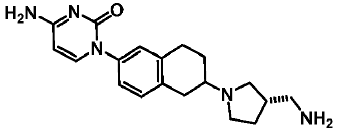 | 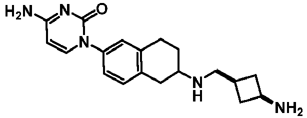 |
| 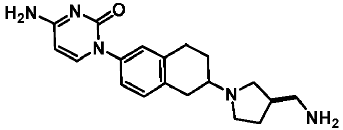 | 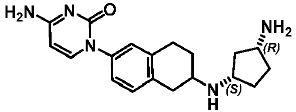 |
| 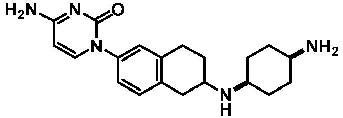 | 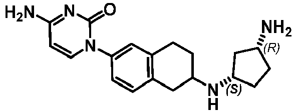 |
| 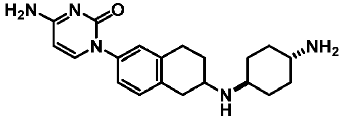 | 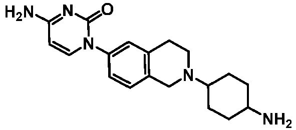 |
| 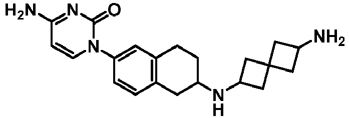 | 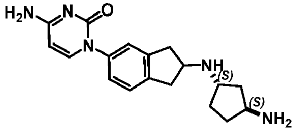 |
| 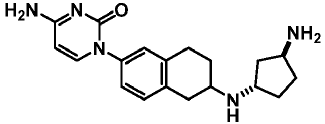 | 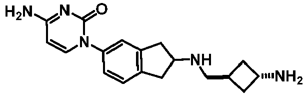 |
| 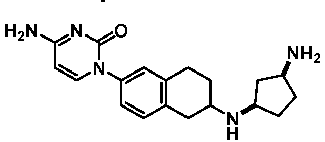 | 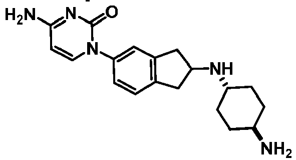 |
| 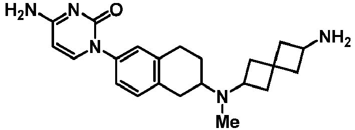 | 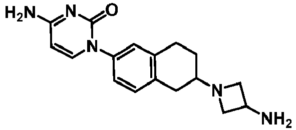 |
| 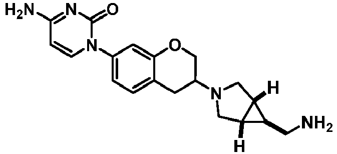 | 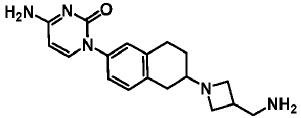 |
| 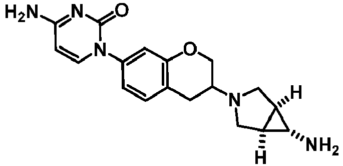 | 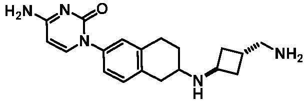 |
| 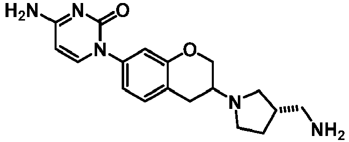 | 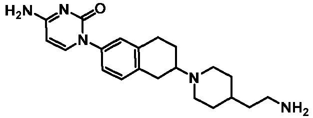 |
| 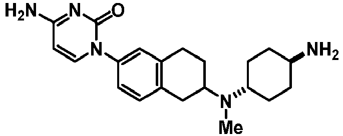 | 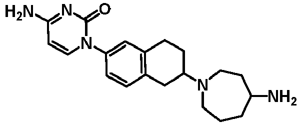 |
| 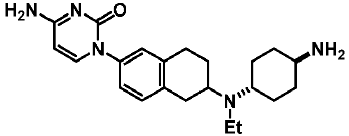 | 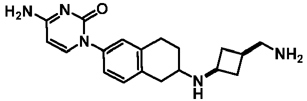 |
| 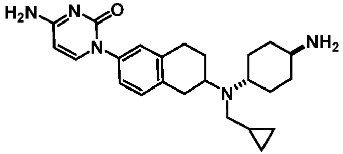 | 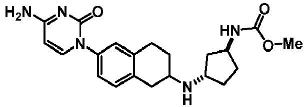 |
| 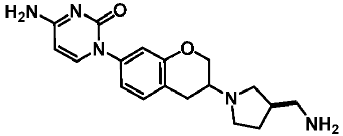 | 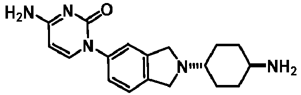 |
| 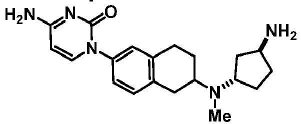 | 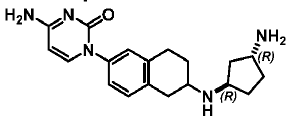 |
| 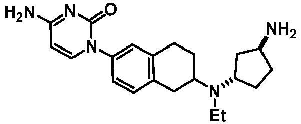 | 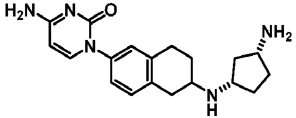 |
| 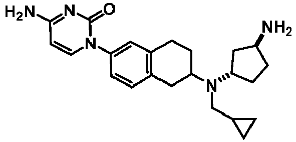 | 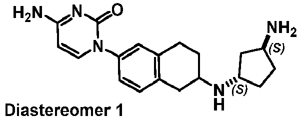 |
| 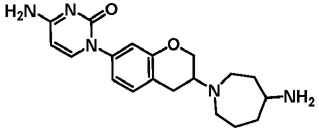 | 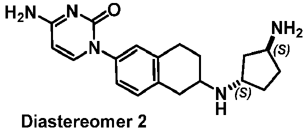 |
| 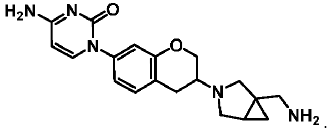 | |

## Patentansprüche

1. Eine Verbindung der Formel I: 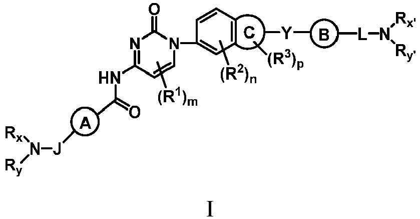 oder ein einzelnes Stereoisomer oder eine Mischung von Stereoisomeren davon oder ein pharmazeutisch akzeptables Salz davon, wobei:
Ring A ein 3- bis 8-gliedriges monocyclisches Heterocycloalkylen ist, das optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, Phenyl, OH, NH₂, NH(C₁-C₆-Alkyl), N(C₁-C₆-Alkyl)₂, COOH, COO(C₁-C₆-Alkyl), CONH₂, CONH(C₁-C₆-Alkyl), CON(C₁-C₆-Alkyl)₂ und Oxo, substituiert ist;
J C₁-C₆-Alkylen oder Cs-Cs-Cycloalkylen ist, von denen jedes optional mit Halogen, OH oder C₁-C₆-Alkoxy substituiert ist, wobei bis zu zwei Methyleneinheiten des C₁-C₆-Alkylens optional und unabhängig durch O, S, SO, SO₂ oder C=O ersetzt sind;
Rₓ, R_{y}, Rₓ, und R_{y'} jeweils unabhängig H, C₁-C₆-Alkyl oder eine AminoSchutzgruppe sind;
Y eine Bindung oder C₁-C₆-Alkylen ist, die optional mit OH, NH₂, CN, Halogen oder C₁-C₆-Alkoxy substituiert ist, wobei bis zu zwei Methyleneinheiten des C₁-C₆-Alkylens optional und unabhängig durch O, NH, N-(C₁-C₆-Alkyl), N-(C₁-C₆-Hydroxyalkyl), N-(C₁-C₆-Halogenalkyl), N-(C₁₋₆-Alkylen-C₃₋₈-cycloalkyl), NH(C=O), N-(C₁-₆-Alkyl)(C=O) oder (C=O) ersetzt sind;
Ring B ein 3- bis 8-gliedriges monocyclisches Cycloalkylen, 3- bis 8-gliedriges monocyclisches Heterocycloalkylen, 6- bis 12-gliedriges bicyclisches Cycloalkylen oder ein 6- bis 12-gliedriges bicyclisches Heterocycloalkylen ist, von denen jedes optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, OH, COOH, COO(C₁-C₆-Alkyl), CONH₂, CONH(C₁-C₆-Alkyl), CON(C₁-C₆-Alkyl)₂ und C₁-C₆-Hydroxyalkyl, substituiert ist;
L eine Bindung oder ein C₁-C₆-Alkylen ist, wobei bis zu zwei Methyleneinheiten des C₁-C₆-Alkylens unabhängig durch O, NH, (C=O), NH(C=O), N-(Ci-₆-Alkyl)(C=O), (C=NH), NH(C=N) oder N-(C₁₋₆-Alkyl) ersetzt sein können;
Ring C, zusammen mit dem Phenylring, an den er kondensiert ist, ein 8- bis 12-gliedriges bicyclisches Arylen oder ein 8- bis 12-gliedriges bicyclisches Heteroarylen bildet, wobei das bicyclische Heteroarylen 1-3 Heteroatome aufweist, die unabhängig aus N, O oder S ausgewählt sind;
R¹, R² und R³ jeweils unabhängig aus der Gruppe ausgewählt sind, die aus C₁-C₆-Alkyl, Halogen, CN, OH, NH₂, NH(C₁-C₆-Alkyl), N(C₁-C₆-Alkyl)₂, COO(C₁-C₆-Alkyl), CONH₂, C₁-C₆-Halogenalkyl, Oxo und C₁-C₆-Alkoxy besteht; und
m, n und p jeweils unabhängig 0, 1, 2 oder 3 sind.

2. Verbindung nach Anspruch 1 oder ein einzelnes Stereoisomer oder eine Mischung von Stereoisomeren davon oder ein pharmazeutisch akzeptables Salz davon, wobei Ring A ein 5- bis 6-gliedriges monocyclisches Heterocycloalkylen ist, das optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, Phenyl, OH, NH₂ und Oxo, substituiert ist;
wie etwa wobei Ring A 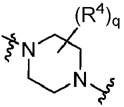 ist, wobei jedes R⁴ unabhängig aus der Gruppe, bestehend aus C₁-C₆-Alkyl, C₁-C₆-Alkoxy, Halogen, CN, C₁-C₆-Halogenalkyl, Phenyl, OH, NH₂ und Oxo, ausgewählt ist, wobei q 0, 1 oder 2 ist;
wie etwa wobei Ring A 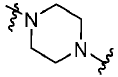 ist.

3. Verbindung nach Anspruch 1 oder Anspruch 2 oder ein einzelnes Stereoisomer oder eine Mischung von Stereoisomeren davon oder ein pharmazeutisch akzeptables Salz davon, wobei J C₁-C₆-Alkylenist, das optional mit Halogen, OH oder C₁-C₆-Alkoxy substituiert ist, wobei bis zu zwei Methyleneinheiten des C₁-C₆-Alkylens optional und unabhängig durch O, S, SO, SO₂ oder C=O ersetzt sind;
wie etwa wobei J C₁-C₆-Alkylen ist, das optional mit OH substituiert ist, wobei eine Methyleneinheit des C₁-C₆-Alkylens durch C=O ersetzt ist;
wie etwa wobei J 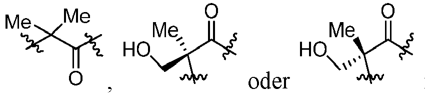 ist.

4. Verbindung nach einem der Ansprüche 1-3 oder ein einzelnes Stereoisomer oder eine Mischung von Stereoisomeren davon oder ein pharmazeutisch akzeptables Salz davon, wobei Rₓ und R_{y} H sind;
wie etwa wobei 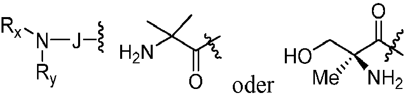 ist;
wie etwa wobei 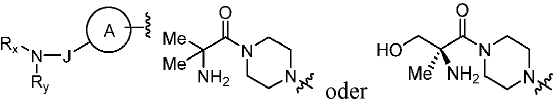 ist.

5. Verbindung nach einem der Ansprüche 1-4 oder ein einzelnes Stereoisomer oder eine Mischung von Stereoisomeren davon oder ein pharmazeutisch akzeptables Salz davon, wobei 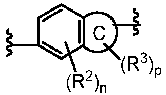 aus der Gruppe, bestehend aus 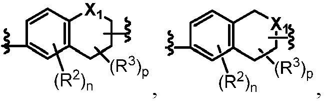 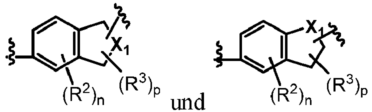 ausgewählt ist, wobei jedes X₁ unabhängig aus CH₂, CH, O, S, SO, SO₂, N und NH ausgewählt ist und wobei R² und R³ jeweils unabhängig Ci-C₆-Alkyl, Halogen oder C₁-C₆-Halogenalkyl sind, wobei n und p jeweils unabhängig 0, 1 oder 2 sind;
wie etwa wobei 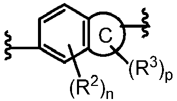 aus der Gruppe, bestehend aus 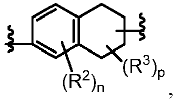 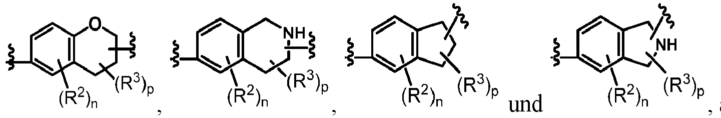 ausgewählt ist;
wie etwa wobei 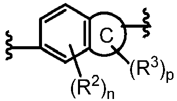 aus der Gruppe, bestehend aus 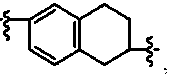 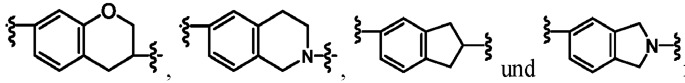 m ausgewählt ist.

6. Verbindung nach einem der Ansprüche 1-5 oder ein einzelnes Stereoisomer oder eine Mischung von Stereoisomeren davon oder ein pharmazeutisch akzeptables Salz davon, wobei Y eine Bindung ist;
oder wobei Y C₁-C₃-Alkylen ist, das optional mit OH, NH₂, Halogen oder C₁-C₆-Alkoxy substituiert ist, und wobei eine Methyleneinheit des C₁-C₃-Alkylens optional durch O, NH, N-(C₁-C₆-Alkyl), N-(C₁-C₆-Hydroxyalkyl), N-(C₁-C₆-Halogenalkyl), N-(C₁₋₆-Alkylen-C₃₋₈-cycloalkyl), NH(C=O), N-(C₁₋₆-Alkyl)(C=O) oder (C=O) ersetzt ist;
oder wobei Y aus der Gruppe, bestehend aus O, NH, NH-C₁₋₂-Alkylen, N(C₁-C₆-Alkyl), N(C₁-C₆-Hydroxyalkyl), N(C₁-C₆-Halogenalkyl), N(C₁₋₆-Alkylen-C₃₋₈-cycloalkyl), NH(C=O), N(C₁₋₆-Alkyl) (C=O) und (C=O), ausgewählt ist; wie etwa wobei Y aus der Gruppe, bestehend aus -NH-, -NMe-, -NEt-, -NH-CH₂-, und -N(CH₂-cyclopropyl)-, ausgewählt ist;
oder wobei 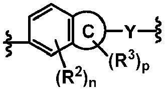 aus der Gruppe, bestehend aus 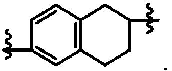 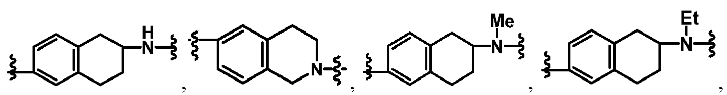 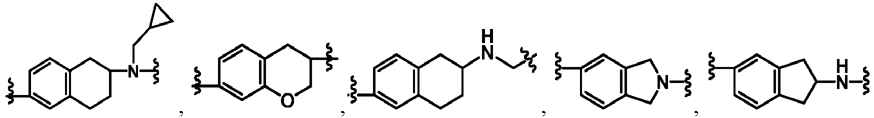 und 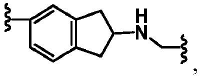 ausgewählt ist.

7. Verbindung nach einem der Ansprüche 1-6 oder ein einzelnes Stereoisomer oder eine Mischung von Stereoisomeren davon oder ein pharmazeutisch akzeptables Salz davon, wobei Ring B ein 3- bis 8-gliedriges monocyclisches Cycloalkylen ist, das optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, OH, COOH, COO(C₁-C₆-Alkyl), CONH₂, CONH(C₁-C₆-Alkyl), CON(C₁-C₆-Alkyl)₂ und C₁-C₆-Hydroxyalkyl, substituiert ist; wie etwa wobei Ring B ein 4- bis 6-gliedriges monocyclisches Cycloalkylen ist, das optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, OH und C₁-C₆-Hydroxyalkyl, substituiert ist;
oder wobei B ein 3- bis 8-gliedriges monocyclisches Heterocycloalkylen ist, das optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, OH, COOH, COO(C₁-C₆-Alkyl), CONH₂, CONH(C₁-C₆-Alkyl), CON(C₁-C₆-Alkyl)₂ und C₁-C₆-Hydroxyalkyl, substituiert ist; wie etwa wobei Ring B ein 4- bis 7-gliedriges monocyclisches Heterocycloalkylen ist, das optional mit bis zu drei Substituenten, ausgewählt aus der Gruppe, bestehend aus C₁-C₆-Alkyl, C₁-C₆-Alkoxy, Halogen, CN, C₁-C₆-Halogenalkyl, OH und C₁-C₆-Hydroxyalkyl, substituiert ist, und wobei B bis zu 2 Stickstoffatome enthält;
oder wobei Ring B ein 6- bis 10-gliedriges bicyclisches Cycloalkylen ist, das optional mit bis zu drei Substituenten, ausgewählt aus der Gruppe, bestehend aus C₁-C₆-Alkyl, C₁-C₆-Alkoxy, Halogen, C₁-C₆-Halogenalkyl, OH und C₁-C₆-Hydroxyalkyl, substituiert ist; wie etwa wobei Ring B ein 6- bis 10-gliedriges kondensiertes, Spiro- oder verbrücktes bicyclisches Cycloalkylen ist;
oder wobei Ring B ein 6- bis 12-gliedriges bicyclisches Heterocycloalkylen ist, das optional mit bis zu drei Substituenten, ausgewählt aus der Gruppe, bestehend aus C₁-C₆-Alkyl, C₁-C₆-Alkoxy, Halogen, C₁-C₆-Halogenalkyl, OH und C₁-C₆-Hydroxyalkyl, substituiert ist; wie etwa wobei Ring B ein 6- bis 9-gliedriges kondensiertes, Spiro- oder verbrücktes bicyclisches Heterocycloalkylen ist, das bis zu 3 Stickstoffatome enthält; wie etwa ein 6- bis 9-gliedriges kondensiertes, Spiro- oder verbrücktes bicyclisches Heterocycloalkylen, das bis zu 2 Stickstoffatome enthält;
oder wobei Ring B aus der Gruppe, bestehend aus: 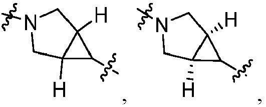 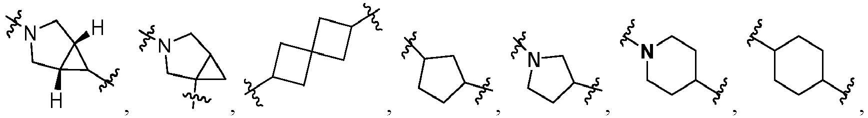 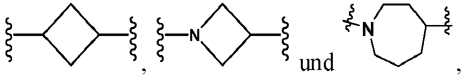 ausgewählt ist.

8. Verbindung nach einem der Ansprüche 1-7 oder ein einzelnes Stereoisomer oder eine Mischung von Stereoisomeren davon oder ein pharmazeutisch akzeptables Salz davon, wobei L eine Bindung ist;
oder wobei L C₁-C₆-Alkylen ist, wobei bis zu zwei Methyleneinheiten des C₁-C₆-Alkylens optional und unabhängig durch O, NH, (C=O), NH(C=O), N-(C₁₋₆-Alkyl)(C=O), (C=NH), NH(C=N) oder N-(C₁₋₆-Alkyl) ersetzt sind; wie etwa wobei L C₁-C₆-Alkylen ist; wie etwa wobei L -CH₂- oder -CH₂CH₂- ist.

9. Verbindung nach einem der Ansprüche 1-8 oder ein einzelnes Stereoisomer oder eine Mischung von Stereoisomeren davon oder ein pharmazeutisch akzeptables Salz davon, wobei R_{x'} und R_{y'} jeweils unabhängig aus der Gruppe, bestehend aus H, Boc und Methoxycarbonyl, ausgewählt sind;
wie etwa wobei 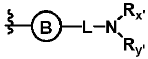 aus der Gruppe, bestehend aus 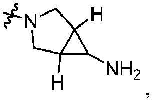 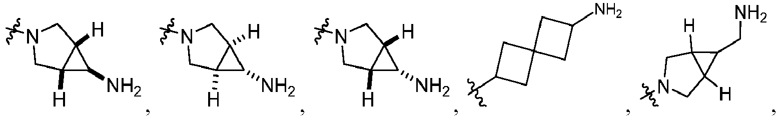 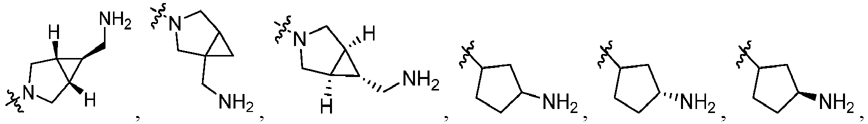 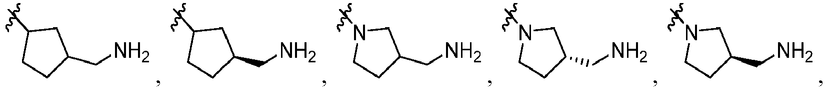 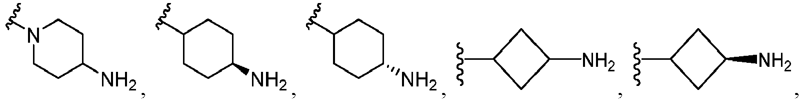 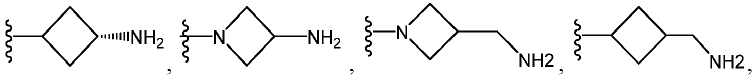 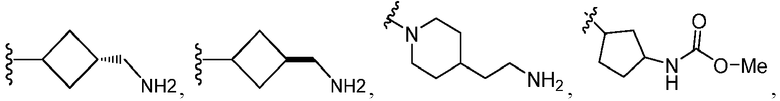 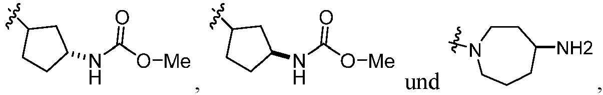 ausgewählt ist.

10. Verbindung nach einem der Ansprüche 1-9 oder ein einzelnes Stereoisomer oder eine Mischung von Stereoisomeren davon oder ein pharmazeutisch akzeptables Salz davon, wobei R¹, R² und R³ jeweils unabhängig aus der Gruppe, bestehend aus C₁-C₆-Alkyl, Halogen, Ci-C₆-Halogenalkyl, Oxo und C₁-C₆-Alkoxy, ausgewählt sind und m, n, und p jeweils unabhängig 0, 1 oder 2 sind;
wie etwa wobei R¹, R² und R³ jeweils unabhängig C₁-C₆-Alkyl, Halogen, Oxo oder C₁-C₆-Halogenalkyl sind und m, n und p jeweils unabhängig 0 oder 1 sind.

11. Verbindung nach einem der Ansprüche 1-10 oder ein einzelnes Stereoisomer oder eine Mischung von Stereoisomeren davon oder ein pharmazeutisch akzeptables Salz davon, wobei die Verbindung Folgendes ist: eine Verbindung der Formel IA 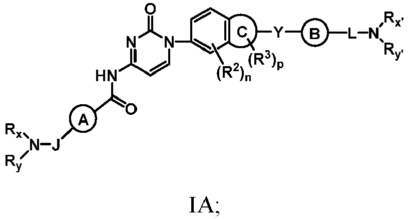
oder eine Verbindung der Formel IA-1: 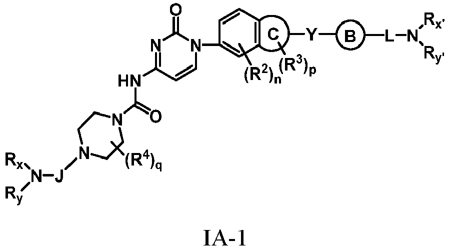 wobei jedes R⁴ unabhängig aus der Gruppe, bestehend aus H, C₁-C₆-Alkyl, C₁-C₆-Alkoxy, Halogen, CN, C₁-C₆-Halogenalkyl, OH, NH₂ und Oxo, ausgewählt ist und wobei q 0, 1, 2 oder 3 ist;
oder eine Verbindung der Formel IA-2: 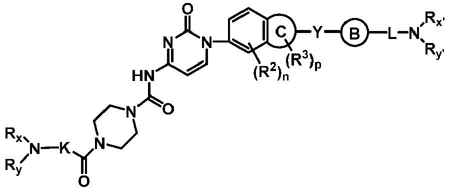 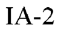 wobei K C₁-C₄-Alkylen ist, das optional mit einer Halogen-, Hydroxyl- oder C₁-C₆-AlkoxyGruppe substituiert ist;
oder eine Verbindung der Formel IA-3: 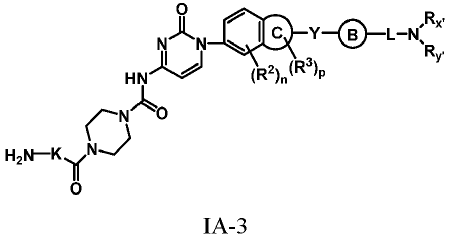 wobei K C₁-C₃-Alkylen ist, das optional mit Hydroxyl substituiert ist;
oder eine Verbindung der Formel IA-4a oder IA-4b: 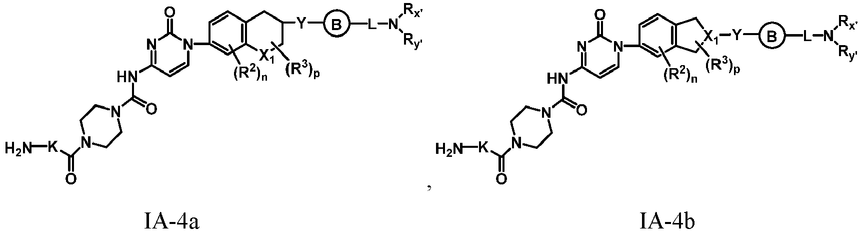 wobei jedes X₁ unabhängig aus der Gruppe, bestehend aus CH₂, CH, O, S, N und NH, ausgewählt ist;
oder eine Verbindung der Formel IA-5a oder IA-5b: 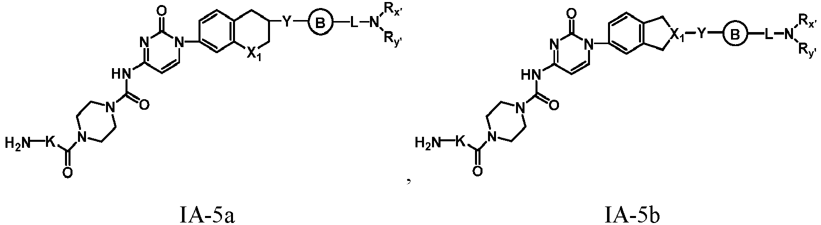 wobei jedes X₁ unabhängig aus der Gruppe, bestehend aus CH₂, CH, NH, N und O, ausgewählt ist;
oder eine Verbindung der Formel IA-6a oder IA-6b: 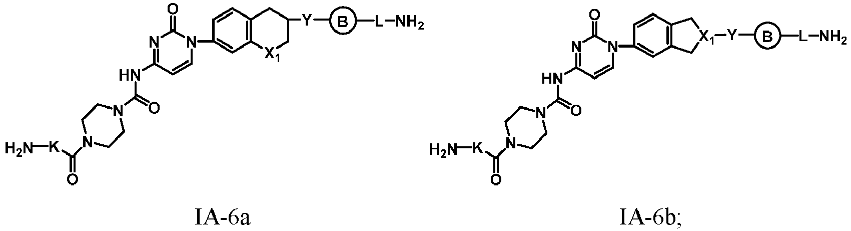
oder eine Verbindung, die aus der Gruppe, bestehend aus Formel IA-7a, Formel IA-7b, Formel IA-7c, Formel IA-7d, Formel IA-7e und Formel IA-7f, ausgewählt ist: 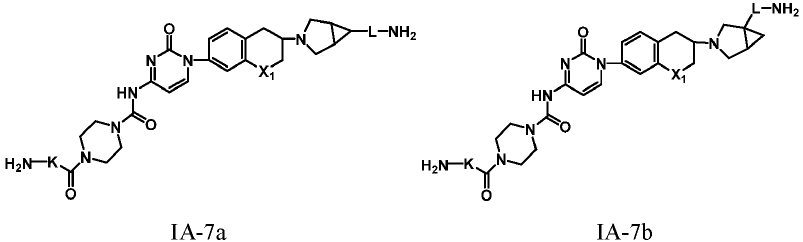 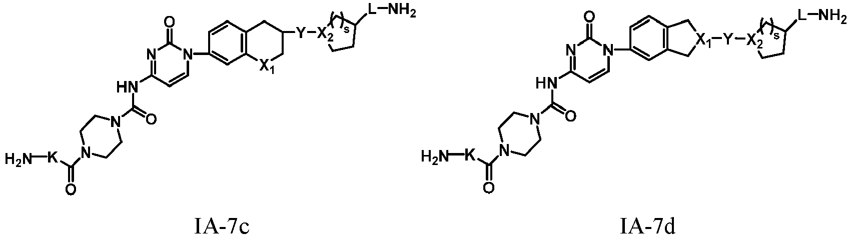 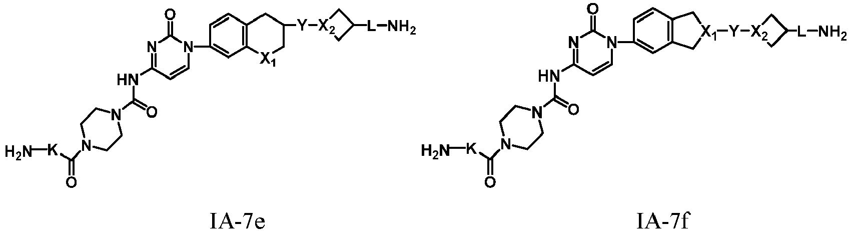 wobei jedes X₂ unabhängig CH oder N ist und jedes s unabhängig 1, 2 oder 3 ist.

12. Verbindung nach Anspruch 11 oder ein einzelnes Stereoisomer oder eine Mischung von Stereoisomeren davon oder ein pharmazeutisch akzeptables Salz davon, wobei K in einer beliebigen der Formeln IA-2, IA-3, IA-4a, IA-4b, IA-5a, IA-5b, IA-6a, IA-6b, IA-7a, IA-7b, IA-7c, IA-7d, IA-7e oder IA-7f 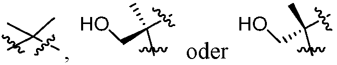 ist; und/oder
wobei Y eine Bindung, NH, NH-(C₁-C₆-Alkylen)-, N-(C₁-C₆-Alkyl) oder N-(C₁-C₆-Alkylen-C₃-C₈-cycloalkyl) ist; und/oder
wobei L eine Bindung oder C₁-C₆-Alkylen ist.

13. Eine Verbindung nach Anspruch 1, die in Tabelle 1 oder Tabelle 2 aufgeführt ist, oder ein pharmazeutisch akzeptables Salz davon:
**Tabelle 1**
| **Nr.** | **Freie Basenstruktur** |
|---|---|
| 1 | 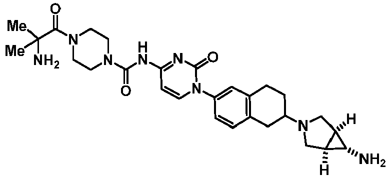 |
| 2 | 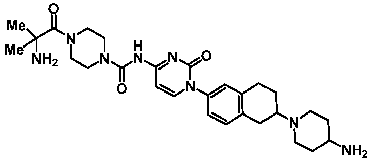 |
| 3 | 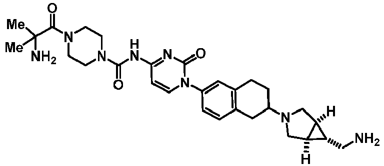 |
| 4 | 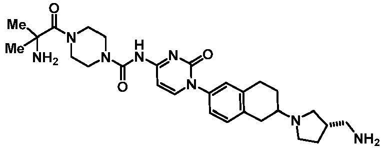 |
| 5 | 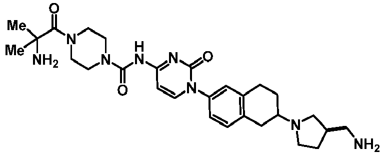 |
| 6 | 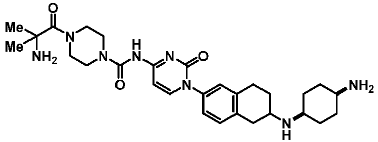 |
| 7 | 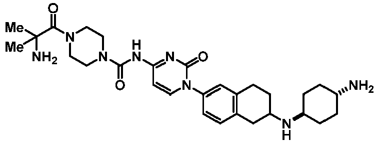 |
| 8 | 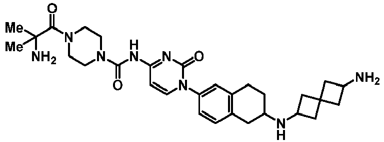 |
| 9 | 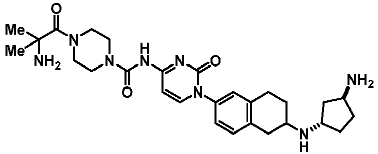 |
| 10 | 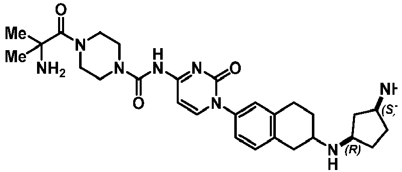 |
| 11 | 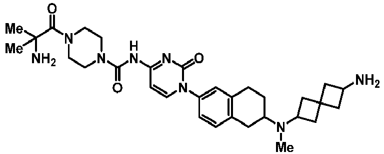 |
| 12 | 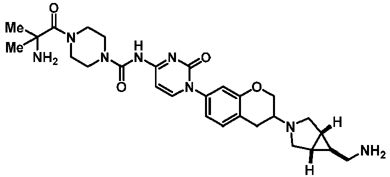 |
| 13 | 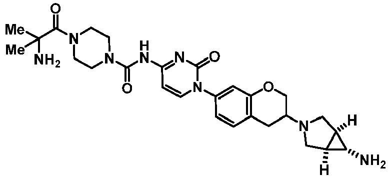 |
| 14 | 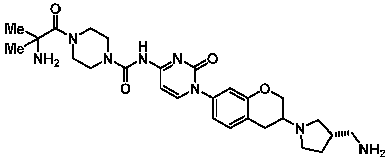 |
| 15 | 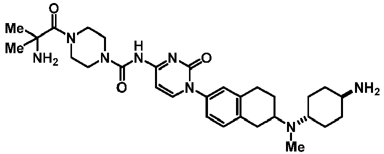 |
| 16 | 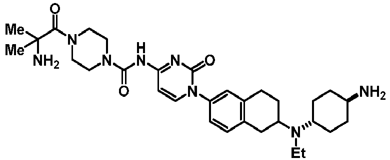 |
| 17 | 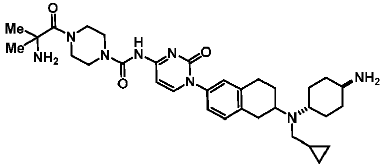 |
| 18 | 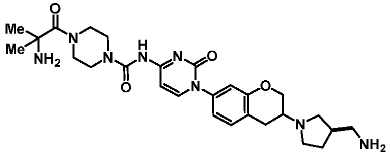 |
| 19 | 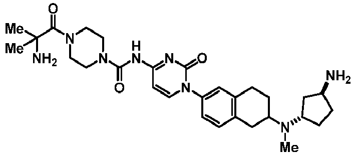 |
| 20 | 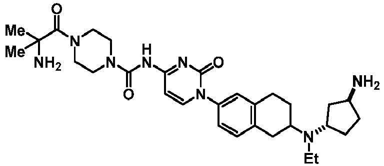 |
| 21 | 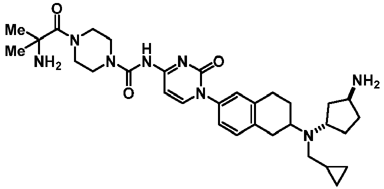 |
| 22 | 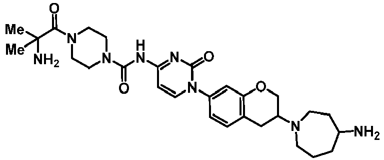 |
| 23 | 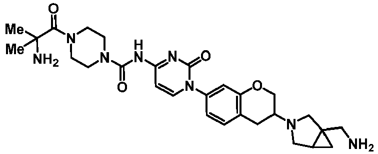 |
| 24 | 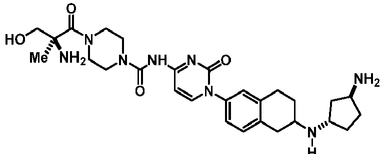 |
| 25 | 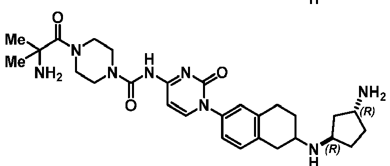 |
| 26 | 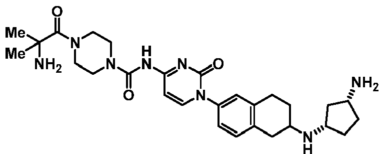 |
| 27 | 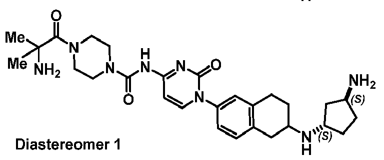 |
| 28 | 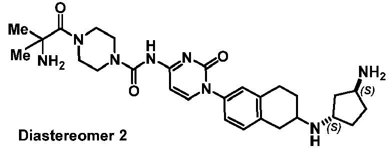 |
**Tabelle 2**
| **Nr**. | **Freie Basenstruktur** |
|---|---|
| 29 | 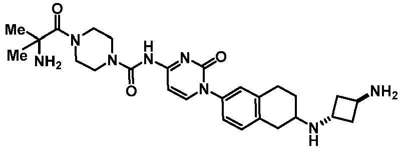 |
| 30 | 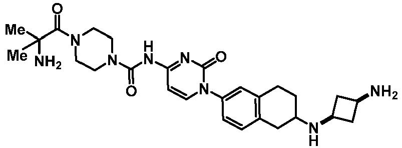 |
| 31 | 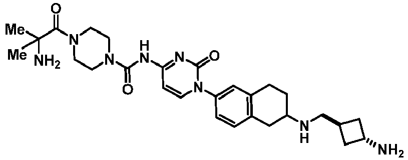 |
| 32 | 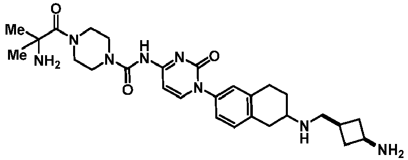 |
| 33 | 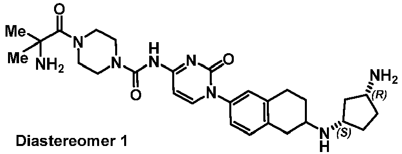 |
| 34 | 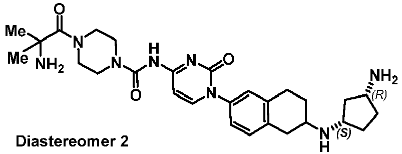 |
| 35 | 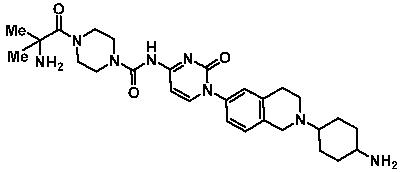 |
| 36 | 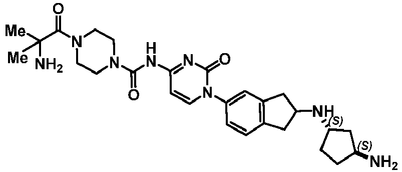 |
| 37 | 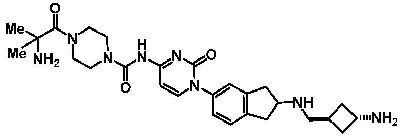 |
| 38 | 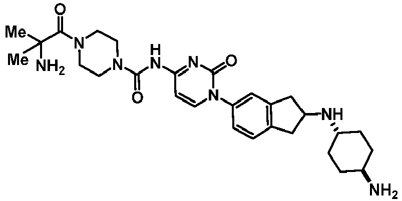 |
| 39 | 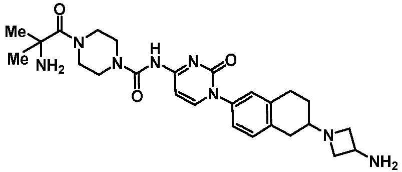 |
| 40 | 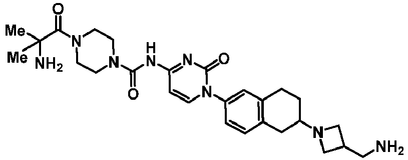 |
| 41 | 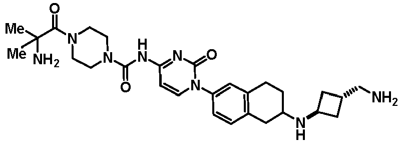 |
| 42 | 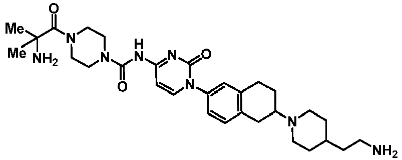 |
| 43 | 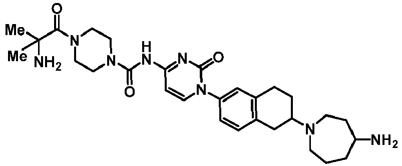 |
| 44 | 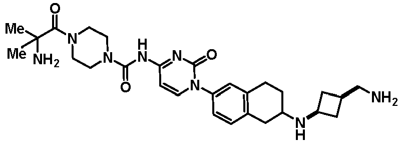 |
| 45 | 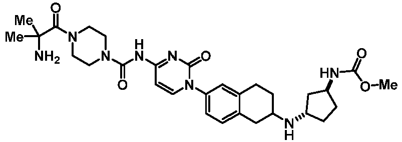 |
| 46 | 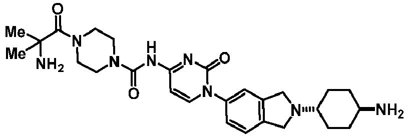 |

14. Eine pharmazeutische Zusammensetzung, die eine Verbindung nach einem der Ansprüche 1-13 oder ein pharmazeutisch akzeptables Salz davon und einen pharmazeutisch akzeptablen Hilfsstoff umfasst.

15. Eine Verbindung zur Verwendung in einem Verfahren zur Behandlung einer bakteriellen Infektion bei einem Patienten, der eine solche Behandlung benötigt, wobei das Verfahren das Verabreichen der Verbindung nach einem der Ansprüche 1-13 oder eines pharmazeutisch akzeptablen Salzes davon oder der pharmazeutischen Zusammensetzung nach Anspruch 14 umfasst.

16. Verbindung zur Verwendung nach Anspruch 15, wobei die bakterielle Infektion durch ein Bakterium, einschließlich grampositiver und gramnegativer Bakterien, verursacht wird.

17. Ein Prozess zur Herstellung einer Verbindung der Formel I nach Anspruch 1: 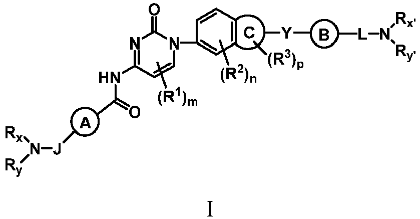 oder eines einzelnen Stereoisomers oder einer Mischung von Stereoisomeren davon oder eines pharmazeutisch akzeptablen Salzes davon, wobei der Prozess Folgendes umfasst:
Kombinieren einer Verbindung der Formel A: 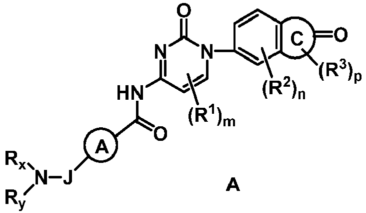 mit einer Verbindung der: 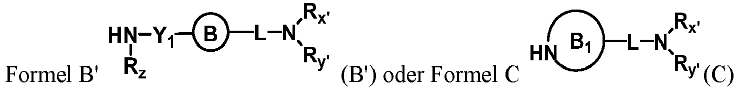 unter einer reduzierenden Aminationsbedingung, um die Verbindung der Formel I bereitzustellen,
wobei Ring A, Ring B, Ring C, J, L, R¹, R², R³, Rₓ, R_{y}, R_{x'}, R_{y'}, m, n und p wie in einem der Ansprüche 1-12 definiert sind;
Ring B₁ ein stickstoffhaltiges 3- bis 8-gliedriges monocyclisches Heterocycloalkylen oder ein stickstoffhaltiges 6- bis 12-gliedriges bicyclisches Heterocycloalkylen ist, von denen jedes optional und unabhängig mit bis zu drei Substituenten, ausgewählt aus der Gruppe, bestehend aus C₁-C₆-Alkyl, C₁-C₆-Alkoxy, Halogen, CN, C₁-C₆-Halogenalkyl, OH, COO(Ci-C₆-Alkyl), CONH₂, CONH(C₁-C₆-Alkyl), CON(C₁-C₆-Alkyl)₂ und C₁-C₆-Hydroxyalkyl, substituiert ist;
Y eine Bindung oder ein C₁-C₆-Alkylen ist, das optional mit OH, NH₂, CN, Halogen oder C₁-C₆-Alkoxy substituiert ist, wobei eine Methyleneinheit des C₁-C₆-Alkylens optional durch NH, N-(C₁-C₆-Alkyl), N-(C₁-C₆-Hydroxyalkyl), N-(C₁-C₆-Halogenalkyl) oder N-(C₁-C₆-Alkylen-C₃-C₈-cycloalkyl) ersetzt ist;
Yi eine Bindung oder ein C₁-C₅-Alkylen ist, das optional mit OH, NH₂, CN, Halogen oder C₁-C₆-Alkoxy substituiert ist; und
R_{z} H, C₁-C₆-Alkyl, C₁-C₆-Hydroxyalkyl, C₁-C₆-Halogenalkyl oder C₁-C₆-Alkylen-C₃-Cs-cycloalkyl ist.

18. Ein Prozess zur Herstellung einer Verbindung der Formel ID: 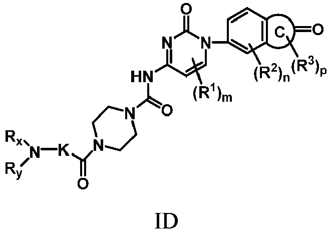 oder eines pharmazeutisch akzeptablen Salzes davon, wobei der Prozess Folgendes umfasst:
Koppeln einer Verbindung der Formel E 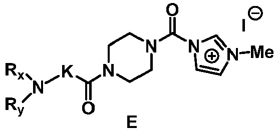
mit einer Verbindung der Formel F 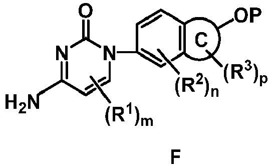 wobei Ring C, K, R¹, R², R³, Rₓ, R_{y}, m, n und p wie in einem der Ansprüche 1-12 definiert sind; und
P eine Hydroxyl-Schutzgruppe ist.

19. Prozess nach Anspruch 18, der ferner den Schritt des Entfernens der Hydroxyl-Schutzgruppe umfasst, um eine Verbindung der Formel IE bereitzustellen 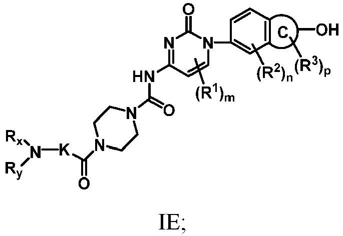 der optional ferner den Schritt des Oxidierens der Hydroxylgruppe umfasst, um die Verbindung der Formel ID bereitzustellen.

20. Eine Verbindung der Formel ID: 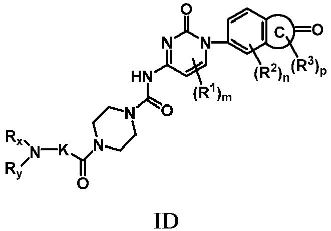 oder ein einzelnes Stereoisomer oder eine Mischung von Stereoisomeren davon oder ein pharmazeutisch akzeptables Salz davon, wobei C, K, R¹, R², R³, Rₓ, R_{y}, m, n und p wie in einem der Ansprüche 1-12 definiert sind.

21. Eine Verbindung der Formel II: 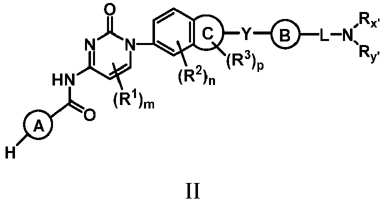 oder ein einzelnes Stereoisomer oder eine Mischung von Stereoisomeren davon oder ein pharmazeutisch akzeptables Salz davon, wobei Ring A, Ring B, Ring C, L, R¹, R², R³, R_{x'}, R_{y'}, m, n und p wie in einem der Ansprüche 1-12 definiert sind.

22. Verbindung nach Anspruch 21 oder ein pharmazeutisch akzeptables Salz davon, wobei die Verbindung aus den in Tabelle 3 aufgeführten Verbindungen ausgewählt ist:
**Tabelle 3**
| **Struktur der Verbindung** | **Struktur der Verbindung** |
|---|---|
| 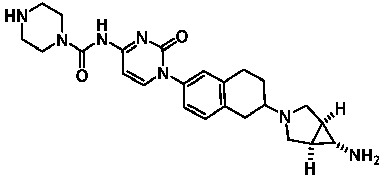 | 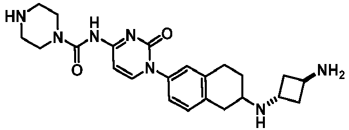 |
| 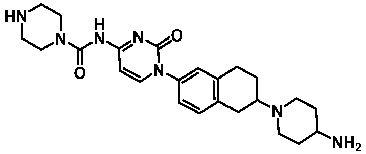 | 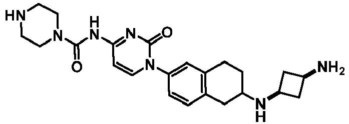 |
| 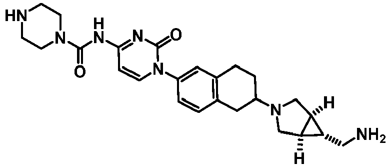 | 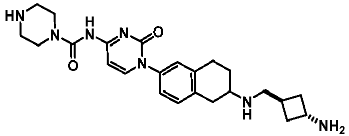 |
| 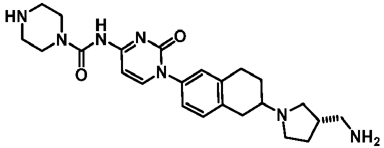 | 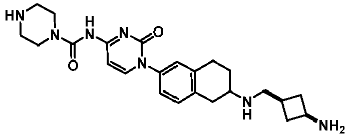 |
| 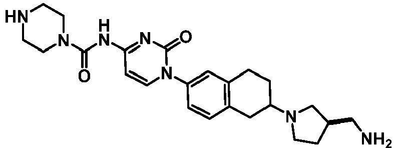 | 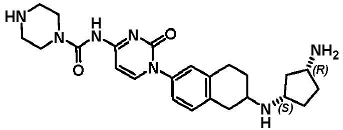 |
| 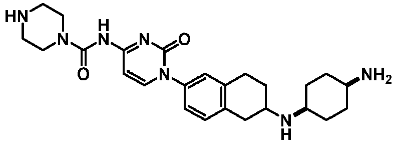 | 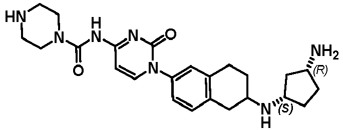 |
| 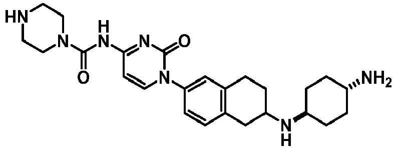 | 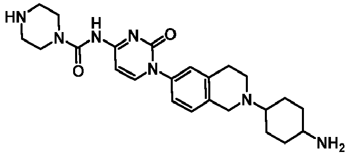 |
| 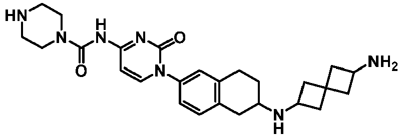 | 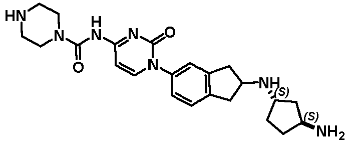 |
| 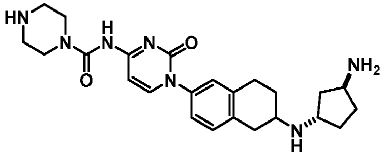 | 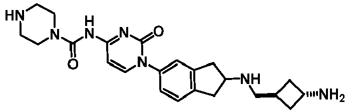 |
| 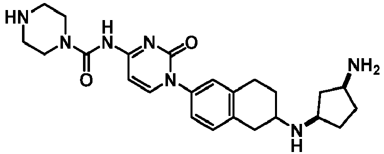 | 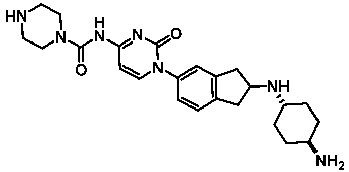 |
| 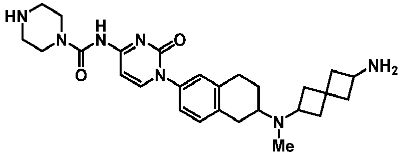 | 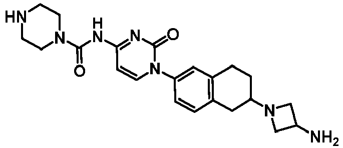 |
| 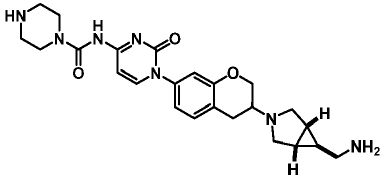 | 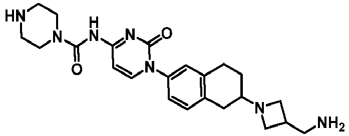 |
| 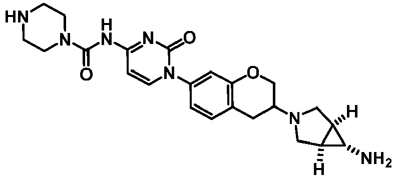 | 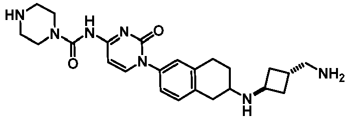 |
| 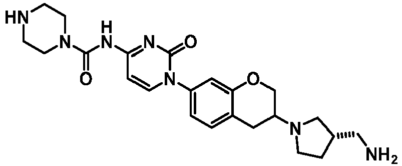 | 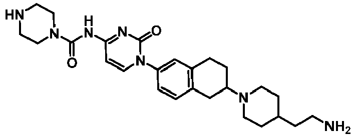 |
| 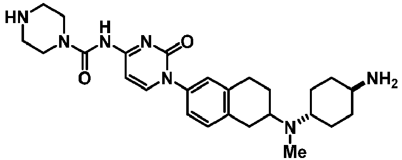 | 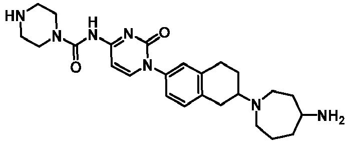 |
| 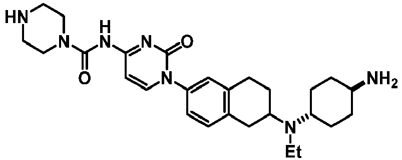 | 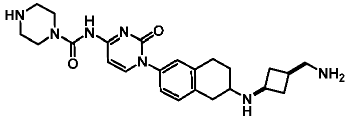 |
| 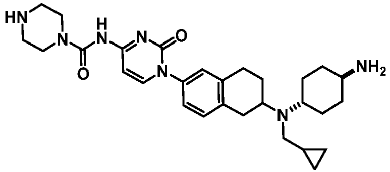 | 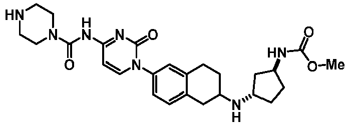 |
| 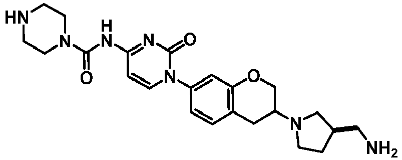 | 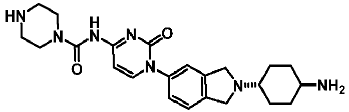 |
| 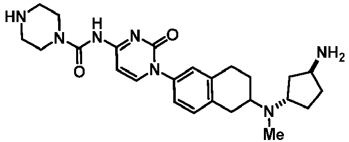 | 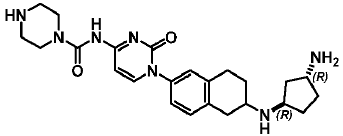 |
| 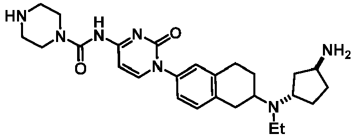 | 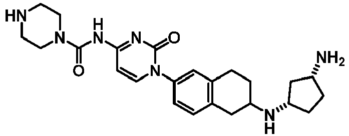 |
| 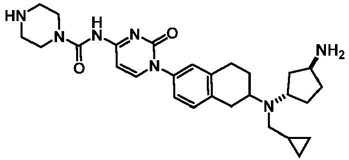 | 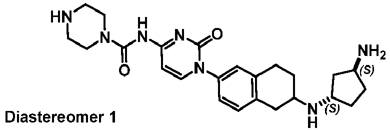 |
| 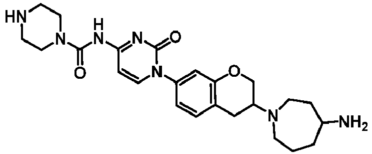 | 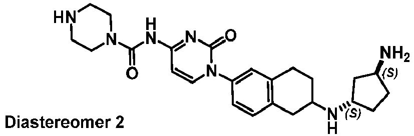 |
| 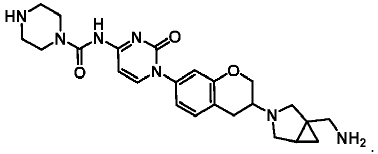 | |

23. Eine Verbindung der Formel III 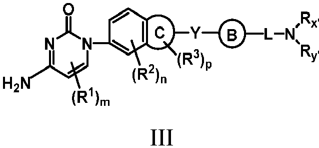 oder ein einzelnes Stereoisomer oder eine Mischung von Stereoisomeren davon oder ein pharmazeutisch akzeptables Salz davon, wobei Ring B, Ring C, L, R¹, R², R³, R_{x'}, R_{y'}, m, n und p wie in einem der Ansprüche 1-12 definiert sind.

24. Verbindung nach Anspruch 23 oder ein pharmazeutisch akzeptables Salz davon, wobei die Verbindung aus den in Tabelle 4 aufgeführten Verbindungen ausgewählt ist:
**Tabelle 4**
| **Struktur der Verbindung** | **Struktur der Verbindung** |
|---|---|
| 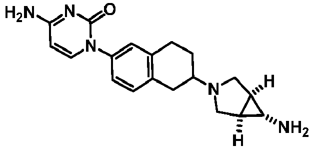 | 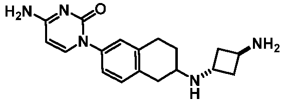 |
| 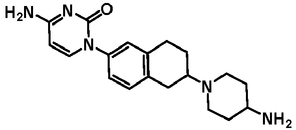 | 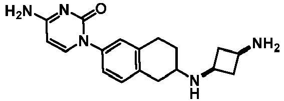 |
| 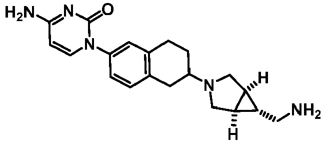 | 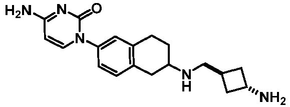 |
| 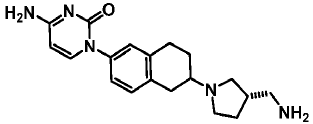 | 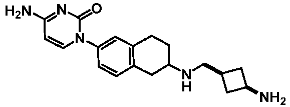 |
| 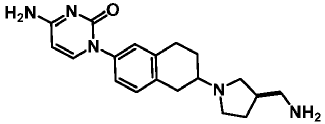 | 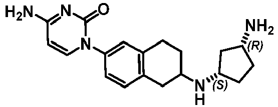 |
| 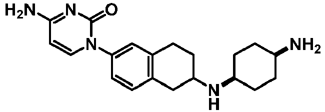 | 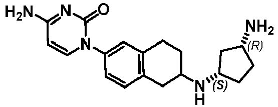 |
| 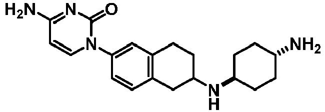 | 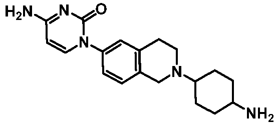 |
| 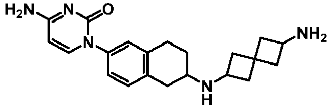 | 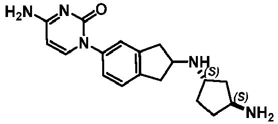 |
| 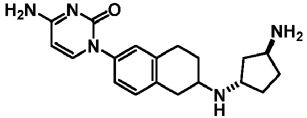 | 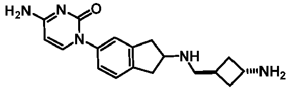 |
| 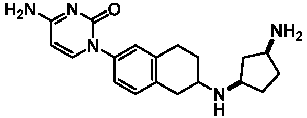 | 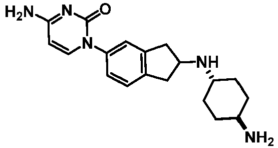 |
| 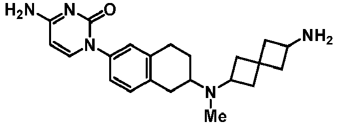 | 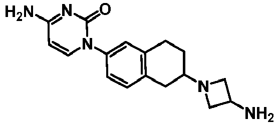 |
| 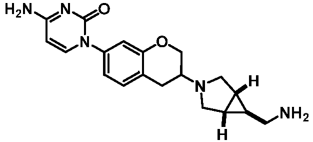 | 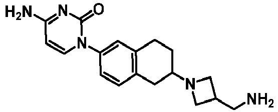 |
| 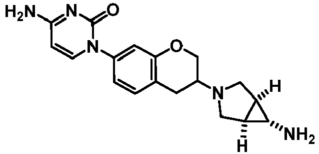 | 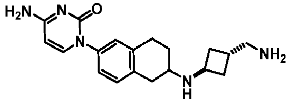 |
| 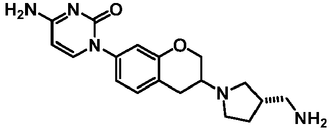 | 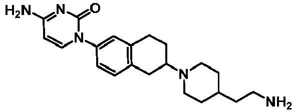 |
| 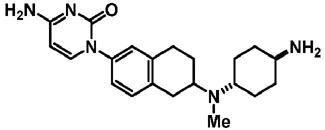 | 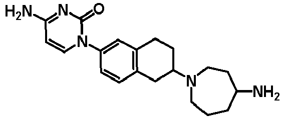 |
| 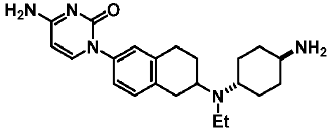 | 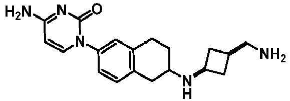 |
| 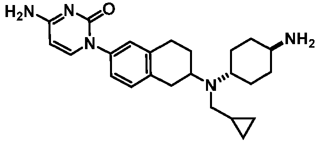 | 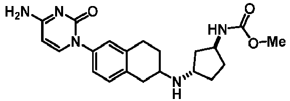 |
| 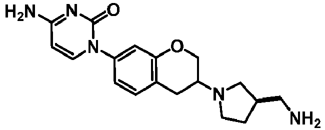 | 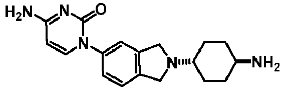 |
| 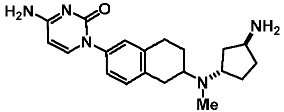 | 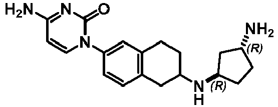 |
| 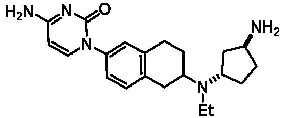 | 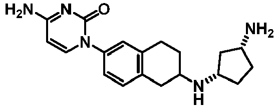 |
| 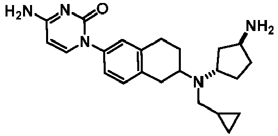 | 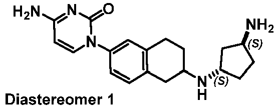 |
| 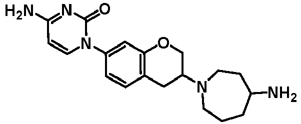 | 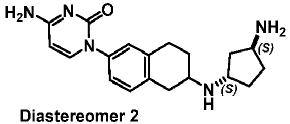 |
| 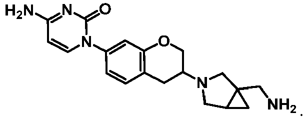 | |

## Revendications

1. Composé de formule I : 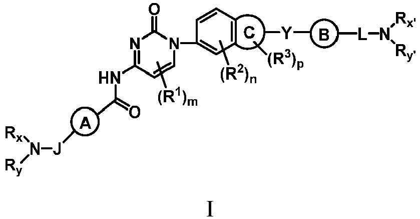 ou un stéréoisomère unique ou mélange de stéréoisomères de celui-ci, ou un sel pharmaceutiquement acceptable de celui-ci, dans lequel :
le noyau A est un hétérocycloalkylène monocyclique de 3 à 8 éléments facultativement substitué par jusqu'à trois substituants choisis parmi le groupe constitué par un alkyle en C₁-C₆, un alcoxy en C₁-C₆, un halo, CN, un haloalkyle en C₁-C₆, un phényle, OH, NH₂, NH(alkyle en C₁-C₆), N(alkyle en C₁-C₆)₂, COOH, COO(alkyle en C₁-C₆), CONH₂, CONH(alkyle en C₁-C₆), CON(alkyle en C₁-C₆)₂, et un oxo ;
J est un alkylène en C₁-C₆ ou un cycloalkylène en C₃-C₈, dont l'un ou l'autre est facultativement substitué par un halo, OH, ou un alcoxy en Ci-Ce, dans lequel jusqu'à deux unités de méthylène de l'alkylène en C₁-C₆ sont facultativement et indépendamment remplacées par O, S, SO, SO₂, ou C=O ;
Rₓ, R_{y}, R_{x'}, et R_{y'} sont chacun indépendamment H, un alkyle en C₁-C₆, ou un groupement protecteur d'amines ;
Y est une liaison ou un alkylène en C₁-C₆ facultativement substitué par OH, NH₂, CN, halo, ou un alcoxy en Ci-Ce, dans lequel jusqu'à deux unités de méthylène de l'alkylène en C₁-C₆ sont facultativement et indépendamment remplacées par O, NH, N-(alkyle en C₁-C₆), N-(hydroxyalkyle en C₁-C₆), N-(haloalkyle en C₁-C₆), N-(alkylène en C₁₋₆-cycloalkyle en aC₃₋₈), NH(C=O), N-(alkyle en C₁₋₆)(C=O), ou (C=O) ;
le noyau B est un cycloalkylène monocyclique de 3 à 8 éléments, un hétérocycloalkylène monocyclique de 3 à 8 éléments, un cycloalkylène bicyclique de 6 à 12 éléments, ou un hétérocycloalkylène bicyclique de 6 à 12 éléments, dont chacun d'eux est facultativement substitué par jusqu'à trois substituants choisis parmi le groupe constitué par un alkyle en C₁-C₆, un alcoxy en C₁-C₆, un halo, CN, un haloalkyle en C₁-C₆, OH, COOH, COO(alkyle en C₁-C₆), CONH₂, CONH(alkyle en C₁-C₆), CON(alkyle en C₁-C₆)₂, et un hydroxyalkyle en C₁-C₆ ;
L est une liaison ou un alkylène en C₁-C₆, dans lequel jusqu'à deux unités de méthylène de l'alkylène en C₁-C₆ peuvent être indépendamment remplacées par O, NH, (C=O), NH(C=O), N-(alkyle en C₁-₆)(C=O), (C=NH), NH(C=N), ou N-(alkyle en C₁-₆) ;
le noyau C, ensemble avec le noyau phényle avec lequel il est condensé, forme un arylène bicyclique de 8 à 12 éléments ou un hétéroarylène bicyclique de 8 à 12 éléments, dans lequel l'hétéroarylène bicyclique a 1 à 3 hétéroatomes indépendamment choisis parmi N, O, ou S ;
R¹, R², et R³ sont chacun indépendamment choisis parmi le groupe constitué par un alkyle en C₁-C₆, un halo, CN, OH, NH₂, NH(alkyle en C₁-C₆), N(alkyle en C₁-C₆)₂, COO(alkyle en C₁-C₆), CONH₂, un haloalkyle en C₁-C₆, un oxo, et un alcoxy en C₁-C₆ ; et
m, n, et p valent chacun indépendamment 0, 1, 2, ou 3.

2. Composé selon la revendication 1, ou un stéréoisomère unique ou mélange de stéréoisomères de celui-ci, ou un sel pharmaceutiquement acceptable de celui-ci, dans lequel le noyau A est un hétérocycloalkylène monocyclique de 5 à 6 éléments facultativement substitué par jusqu'à trois substituants choisis parmi le groupe constitué par un alkyle en C₁-C₆, un alcoxy en C₁-C₆, un halo, CN, un haloalkyle en C₁-C₆, un phényle, OH, NH₂, et un oxo ;
tel que dans lequel le noyau A est 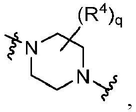 dans lequel chaque R⁴ est indépendamment choisi parmi le groupe constitué par un alkyle en C₁-C₆, un alcoxy en C₁-C₆, un halo, CN, un haloalkyle en C₁-C₆, un phényle, OH, NH₂, et un oxo, dans lequel q vaut 0, 1, ou 2 ;
tel que dans lequel le noyau A est

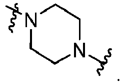

3. Composé selon la revendication 1 ou la revendication 2, ou un stéréoisomère unique ou mélange de stéréoisomères de celui-ci, ou un sel pharmaceutiquement acceptable de celui-ci, dans lequel J est un alkylène en C₁-C₆ facultativement substitué par un halo, OH, ou un alcoxy en C₁-C₆, dans lequel jusqu'à deux unités de méthylène de l'alkylène en C₁-C₆ sont facultativement et indépendamment remplacées par O, S, SO, SO₂, ou C=O ;
tel que dans lequel J est un alkylène en C₁-C₆ facultativement substitué par OH, dans lequel une unité de méthylène de l'alkylène en C₁-C₆ est remplacée par C=O ;
tel que dans lequel J est

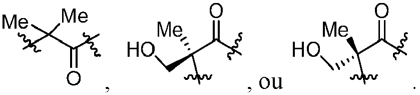

4. Composé selon l'une quelconque des revendications 1 à 3, ou un stéréoisomère unique ou mélange de stéréoisomères de celui-ci, ou un sel pharmaceutiquement acceptable de celui-ci, dans lequel Rₓ et R_{y} sont H ;
tel que dans lequel 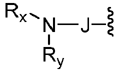 est 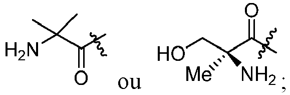
tel que dans lequel 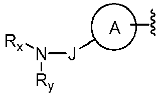 est

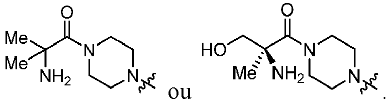

5. Composé selon l'une quelconque des revendications 1 à 4, ou un stéréoisomère unique ou mélange de stéréoisomères de celui-ci, ou un sel pharmaceutiquement acceptable de celui-ci, dans lequel 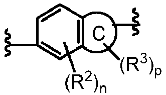 est choisi parmi le groupe constitué par 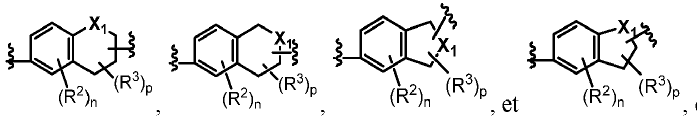 dans lequel chaque Xi est indépendamment choisi parmi CH₂, CH, O, S, SO, SO₂, N, et NH, et dans lequel R² et R³ sont chacun indépendamment un alkyle en C₁-C₆, un halo, ou un haloalkyle en C₁-C₆, dans lequel n et p valent chacun indépendamment 0, 1, ou 2 ;
tel que dans lequel 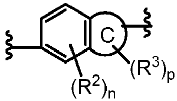 est choisi parmi le groupe constitué par 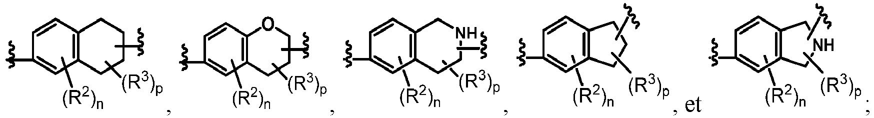
tel que dans lequel 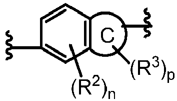 est choisi parmi le groupe constitué par

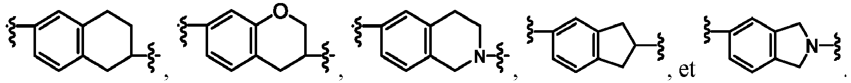

6. Composé selon l'une quelconque des revendications 1 à 5, ou un stéréoisomère unique ou mélange de stéréoisomères de celui-ci, ou un sel pharmaceutiquement acceptable de celui-ci, dans lequel Y est une liaison ;
ou dans lequel Y est un alkylène en C₁-C₃ facultativement substitué par OH, NH₂, un halo, ou un alcoxy en C₁-C₆, et dans lequel une unité de méthylène de l'alkylène en C₁-C₃ est facultativement remplacée par O, NH, N-(alkyle en C₁-C₆), N-(hydroxyalkyle en C₁-C₆), N-(haloalkyle en C₁-C₆), N-(alkylène en C₁₋₆-cycloalkyle en C₃₋₈), NH(C=O), N-(alkyle en C₁₋₆)(C=O), ou (C=O) ;
ou dans lequel Y est choisi parmi le groupe constitué par O, NH, NH-alkylène en C₁₋₂, N(alkyle en C₁-C₆), N(hydroxyalkyle en C₁-C₆), N(haloalkyle en C₁-C₆), N(alkylène en C₁₋₆-cycloalkyle en C₃₋₈), NH(C=O), N(alkyle en C₁₋₆) (C=O), et (C=O) ; tel que dans lequel Y est choisi parmi le groupe constitué par -NH-, -NMe-, -NEt-, -NH-CH₂-, et -N(CH₂-cyclopropyle)- ;
ou dans lequel 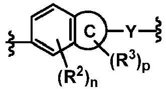 est choisi parmi le groupe constitué par 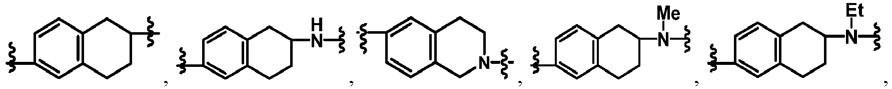 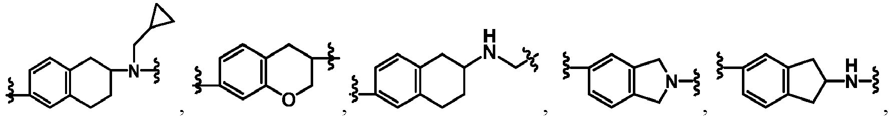 et

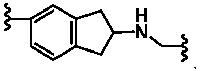

7. Composé selon l'une quelconque des revendications 1 à 6, ou un stéréoisomère unique ou mélange de stéréoisomères de celui-ci, ou un sel pharmaceutiquement acceptable de celui-ci, dans lequel le noyau B est un cycloalkylène monocyclique de 3 à 8 éléments facultativement substitué par jusqu'à trois substituants choisis parmi le groupe constitué par un alkyle en C₁-C₆, un alcoxy en C₁-C₆, un halo, CN, un haloalkyle en C₁-C₆, OH, COOH, COO(alkyle en C₁-C₆), CONH₂, CONH(alkyle en C₁-C₆), CON(alkyle en C₁-C₆)₂, et un hydroxyalkyle en Ci-Ce ; tel que dans lequel le noyau B est un cycloalkylène monocyclique de 4 à 6 éléments facultativement substitué par jusqu'à trois substituants choisis parmi le groupe constitué par un alkyle en C₁-C₆, un alcoxy en C₁-C₆, un halo, CN, un haloalkyle en C₁-C₆, OH, et un hydroxyalkyle en Ci-Ce ;
ou dans lequel B est un hétérocycloalkylène monocyclique de 3 à 8 éléments facultativement substitué par jusqu'à trois substituants choisis parmi le groupe constitué par un alkyle en C₁-C₆, un alcoxy en C₁-C₆, un halo, CN, un haloalkyle en C₁-C₆, OH, COOH, COO(alkyle en C₁-C₆), CONH₂, CONH(alkyle en C₁-C₆), CON(alkyle en C₁-C₆)₂, et un hydroxyalkyle en C₁-C₆ ; tel que
dans lequel le noyau B est un hétérocycloalkylène monocyclique de 4 à 7 éléments facultativement substitué par jusqu'à trois substituants choisis parmi le groupe constitué par un alkyle en C₁-C₆, un alcoxy en C₁-C₆, un halo, CN, un haloalkyle en C₁-C₆, OH, et un hydroxyalkyle en Ci-Ce, et dans lequel B contient jusqu'à 2 atomes d'azote ;
ou dans lequel le noyau B est un cycloalkylène bicyclique de 6 à 10 éléments facultativement substitué par jusqu'à trois substituants choisis parmi le groupe constitué par un alkyle en C₁-C₆, un alcoxy en C₁-C₆, un halo, un haloalkyle en C₁-C₆, OH, et un hydroxyalkyle en Ci-Ce ; tel que dans lequel le noyau B est un cycloalkylène bicyclique condensé, spiro, ou ponté de 6 à 10 éléments ;
ou dans lequel le noyau B est un hétérocycloalkylène bicyclique de 6 à 12 éléments facultativement substitué par jusqu'à trois substituants choisis parmi le groupe constitué par un alkyle en C₁-C₆, un alcoxy en C₁-C₆, un halo, un haloalkyle en C₁-C₆, OH, et un hydroxyalkyle en Ci-Ce ; tel que dans lequel le noyau B est un hétérocycloalkylène bicyclique condensé, spiro, ou ponté de 6 à 9 éléments contenant jusqu'à 3 atomes d'azote ; tel qu'un hétérocycloalkylène bicyclique condensé, spiro, ou ponté de 6 à 9 éléments contenant jusqu'à 2 atomes d'azote ;
ou dans lequel le noyau B est choisi parmi le groupe constitué par :

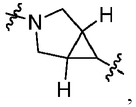

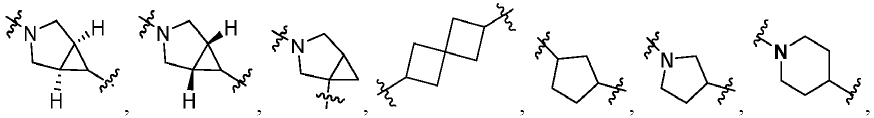

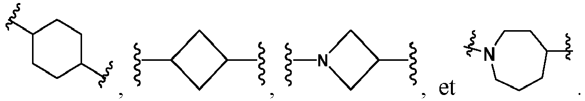

8. Composé selon l'une quelconque des revendications 1 à 7, ou un stéréoisomère unique ou mélange de stéréoisomères de celui-ci, ou un sel pharmaceutiquement acceptable de celui-ci, dans lequel L est une liaison ;
ou dans lequel L est un alkylène en C₁-C₆, dans lequel jusqu'à deux unités de méthylène de l'alkylène en C₁-C₆ sont facultativement et indépendamment remplacées par O, NH, (C=O), NH(C=O), N-(alkyle en C₁-C₆)(C=O), (C=NH), NH(C=N), ou N-(alkyle en C₁-C₆) ; tel que dans lequel L est un alkylène en C₁-C₆ ; tel que dans lequel L est -CH₂- ou-CH₂CH₂-.

9. Composé selon l'une quelconque des revendications 1 à 8, ou un stéréoisomère unique ou mélange de stéréoisomères de celui-ci, ou un sel pharmaceutiquement acceptable de celui-ci, dans lequel R_{x'} et R_{y'} sont chacun indépendamment choisis parmi le groupe constitué par H, Boc, et un méthoxycarbonyle ;
tel que dans lequel 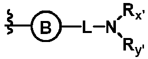 est choisi parmi le groupe constitué par

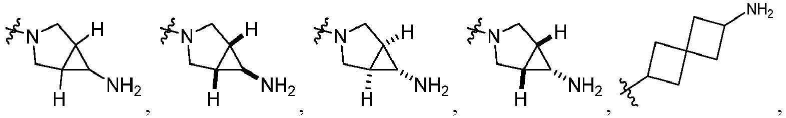

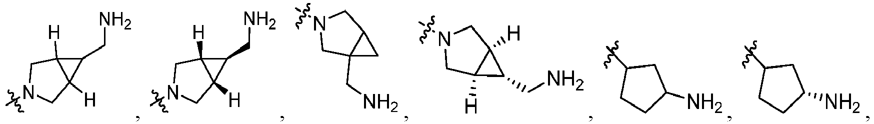

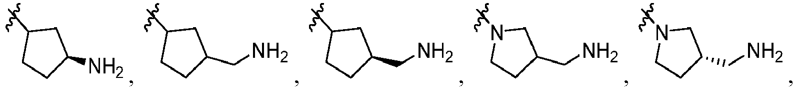

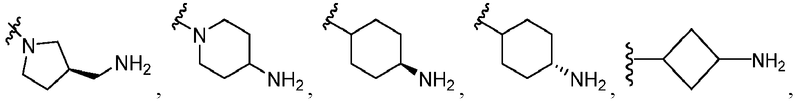

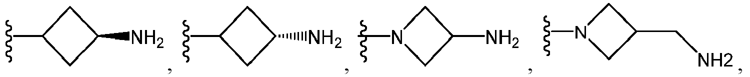

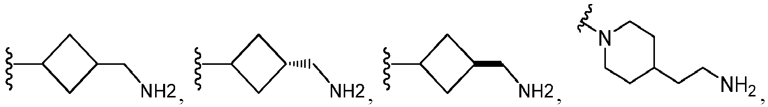

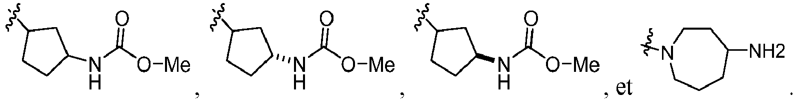

10. Composé selon l'une quelconque des revendications 1 à 9, ou un stéréoisomère unique ou mélange de stéréoisomères de celui-ci, ou un sel pharmaceutiquement acceptable de celui-ci, dans lequel R¹, R², et R³ sont chacun indépendamment choisis parmi le groupe constitué par un alkyle en C₁-C₆, un halo, un haloalkyle en C₁-C₆, un oxo, et un alcoxy en C₁-C₆, et m, n, et p valent chacun indépendamment 0, 1, ou 2 ;
tel que dans lequel R¹, R², et R³ sont chacun indépendamment un alkyle en C₁-C₆, un halo, un oxo, ou un haloalkyle en C₁-C₆, et m, n, et p valent chacun indépendamment 0 ou 1.

11. Composé selon l'une quelconque des revendications 1 à 10, ou un stéréoisomère unique ou mélange de stéréoisomères de celui-ci, ou un sel pharmaceutiquement acceptable de celui-ci, dans lequel le composé est un composé de formule IA : 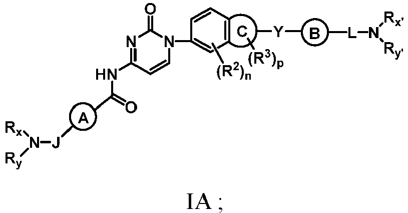
ou un composé de formule IA-1 : 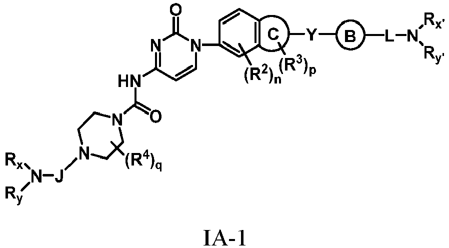 dans lequel chaque R⁴ est indépendamment choisi parmi le groupe constitué par H, un alkyle en C₁-C₆, un alcoxy en C₁-C₆, un halo, CN, un haloalkyle en C₁-C₆, OH, NH₂, et un oxo, et dans lequel q vaut 0, 1, 2, ou 3 ;
ou un composé de formule IA-2 : 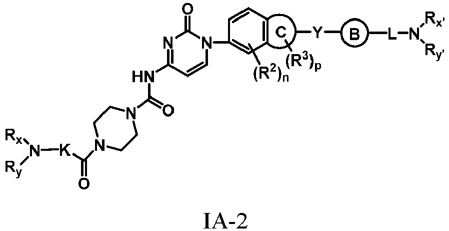 dans lequel K est un alkylène en C₁-C₄ facultativement substitué par un halo, un hydroxyle ou un groupe alcoxy en C₁-C₆ ;
ou un composé de formule IA-3 : 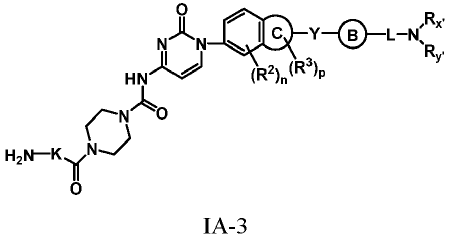 dans lequel K est un alkylène en C₁-C₃ facultativement substitué par un hydroxyle ;
ou un composé de formule IA-4a ou IA-4b : 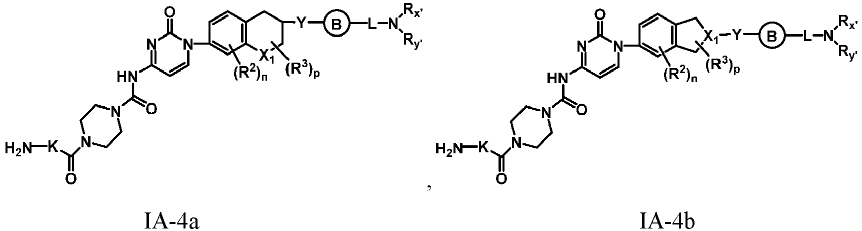 dans lequel chaque X₁ est indépendamment choisi parmi le groupe constitué par CH₂, CH, O, S, N, et NH ;
ou un composé de formule IA-5a ou IA-5b : 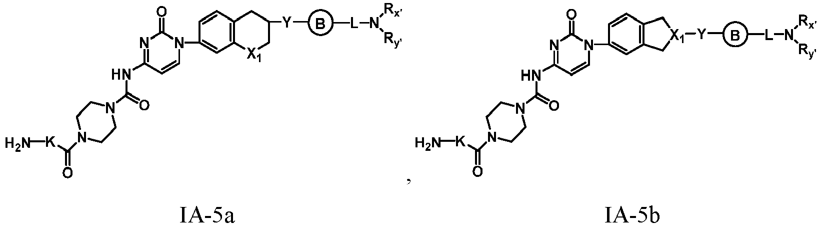 dans lequel chaque Xi est indépendamment choisi parmi le groupe constitué par CH₂, CH, NH, N, et O ;
ou un composé de formule IA-6a ou IA-6b : 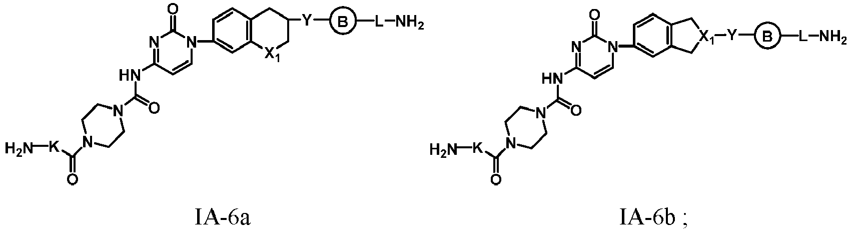
ou un composé choisi parmi le groupe constitué par la formule IA-7a, la formule IA-7b, la formule IA-7c, la formule IA-7d, la formule IA-7e, et la formule IA-7f : 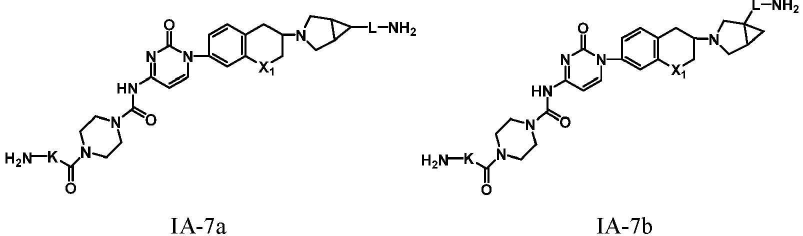 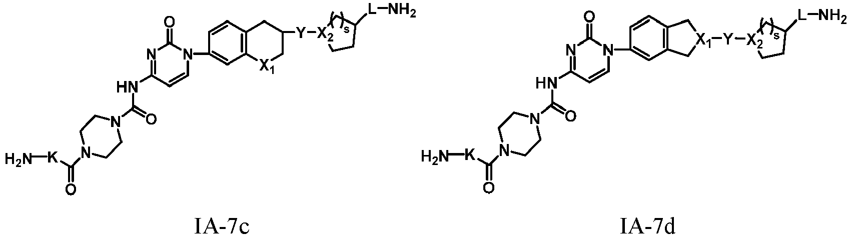 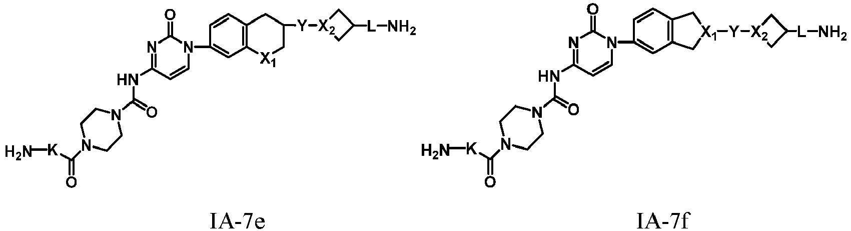 dans lequel chaque X₂ est indépendamment CH ou N, et chaque s est indépendamment 1, 2, ou 3.

12. Composé selon la revendication 11, ou un stéréoisomère unique ou mélange de stéréoisomères de celui-ci, ou un sel pharmaceutiquement acceptable de celui-ci, dans lequel K dans l'une quelconque des formules IA-2, IA-3, IA-4a, IA-4b, IA-5a, IA-5b, IA-6a, IA-6b, IA-7a, IA-7b, IA-7c, IA-7d, IA-7e, ou IA-7f est 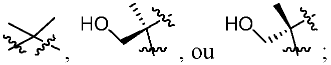 et/ou
dans lequel Y est une liaison, NH, NH-(alkylène en C₁-C₆)-, N-(alkyle en C₁-C₆), ou N-(alkylène en C₁-C₆-cycloalkyle en C₃-C₈) ; et/ou
dans lequel L est une liaison ou un alkylène en C₁-C₆.

13. Composé selon la revendication 1 qui est énuméré dans le Tableau 1 ou le Tableau 2, ou un sel pharmaceutiquement acceptable de celui-ci :
**Tableau 1**
| **No.** | **Structure de base libre** |
|---|---|
| 1 | 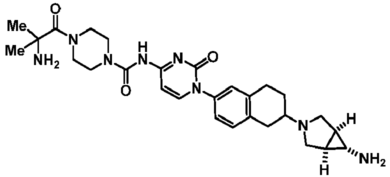 |
| 2 | 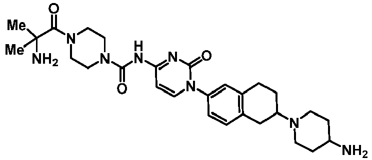 |
| 3 | 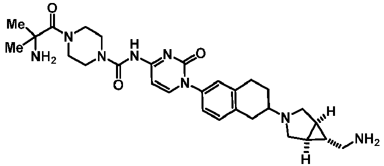 |
| 4 | 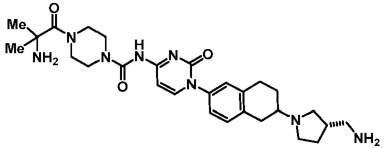 |
| 5 | 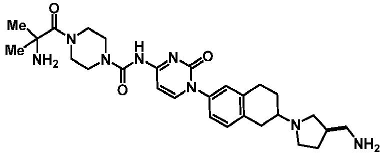 |
| 6 | 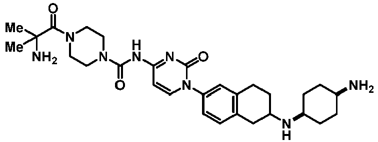 |
| 7 | 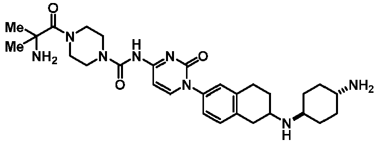 |
| 8 | 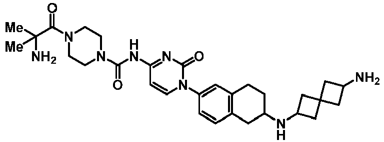 |
| 9 | 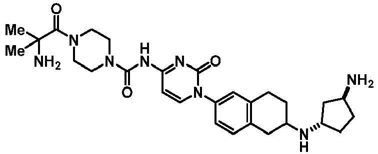 |
| 10 | 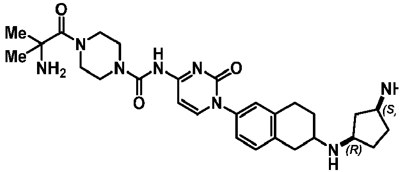 |
| 11 | 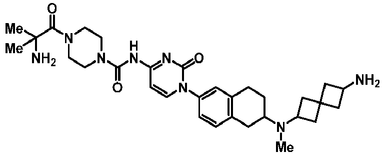 |
| 12 | 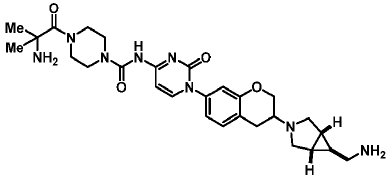 |
| 13 | 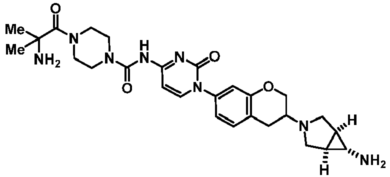 |
| 14 | 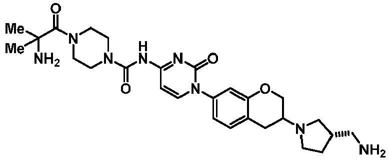 |
| 15 | 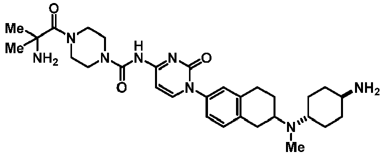 |
| 16 | 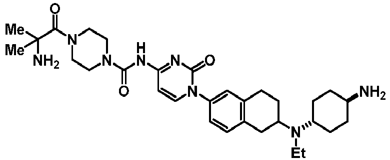 |
| 17 | 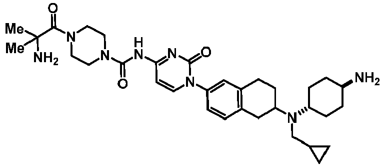 |
| 18 | 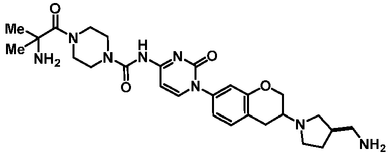 |
| 19 | 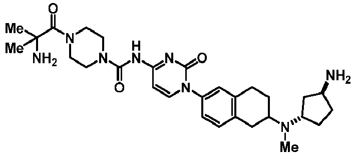 |
| 20 | 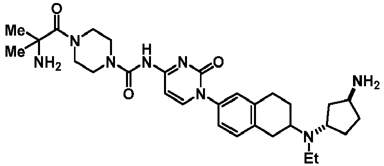 |
| 21 | 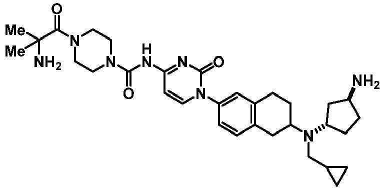 |
| 22 | 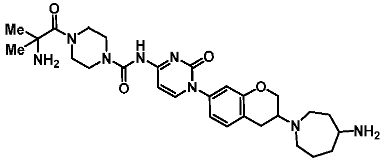 |
| 23 | 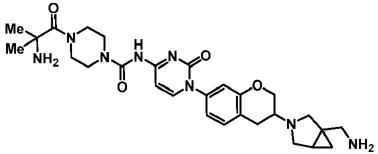 |
| 24 | 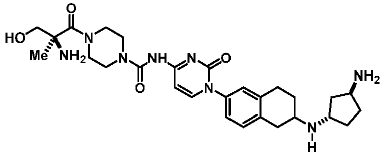 |
| 25 | 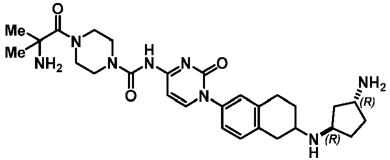 |
| 26 | 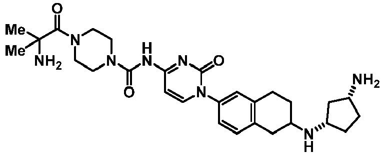 |
| 27 | 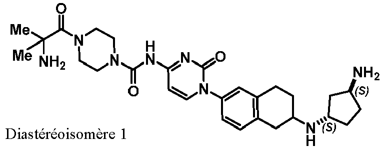 |
| 28 | 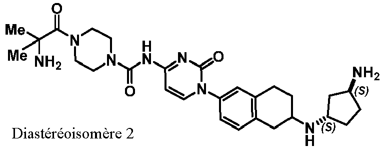 |
**Tableau 2**
| **No.** | **Structure de base libre** |
|---|---|
| 29 | 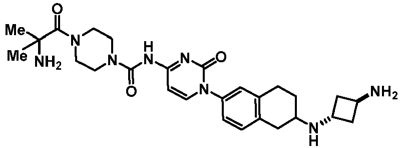 |
| 30 | 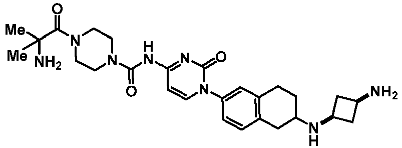 |
| 31 | 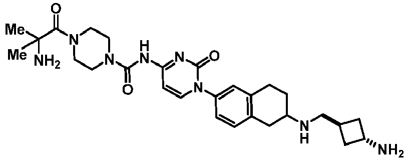 |
| 32 | 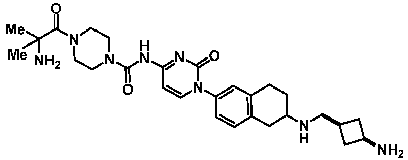 |
| 33 | 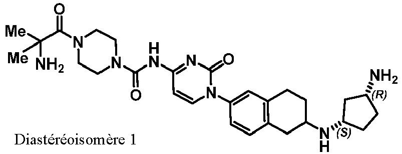 |
| 34 | 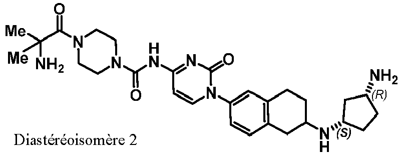 |
| 35 | 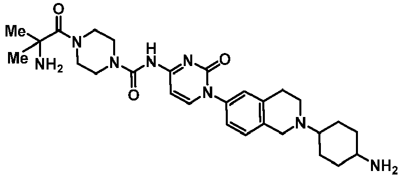 |
| 36 | 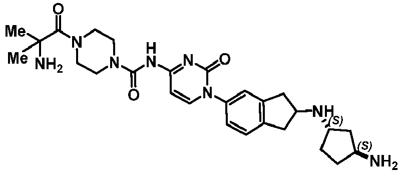 |
| 37 | 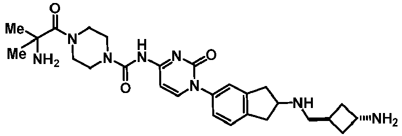 |
| 38 | 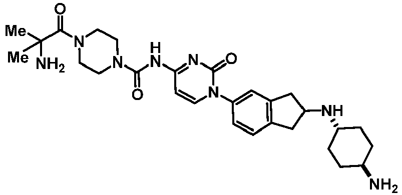 |
| 39 | 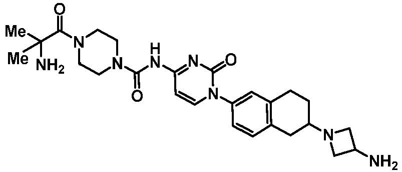 |
| 40 | 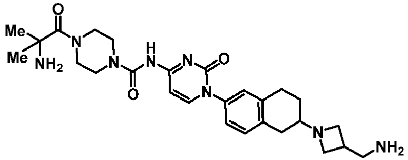 |
| 41 | 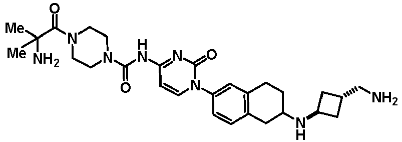 |
| 42 | 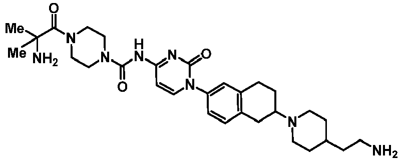 |
| 43 | 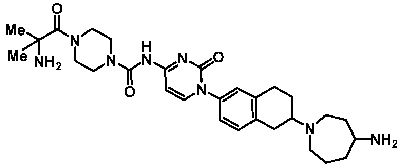 |
| 44 | 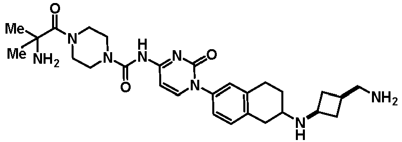 |
| 45 | 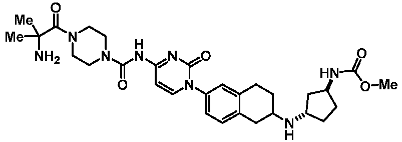 |
| 46 | 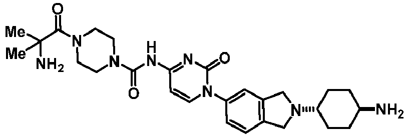 |

14. Composition pharmaceutique comprenant un composé selon l'une quelconque des revendications 1 à 13 ou un sel pharmaceutiquement acceptable de celui-ci et un excipient pharmaceutiquement acceptable.

15. Composé destiné à être utilisé dans un procédé de traitement d'une infection bactérienne chez un patient ayant besoin d'un tel traitement, dans lequel le procédé comprend l'administration du composé selon l'une quelconque des revendications 1 à 13 ou d'un sel pharmaceutiquement acceptable de celui-ci ou de la composition pharmaceutique selon la revendication 14.

16. Composé destiné à être utilisé selon la revendication 15, dans lequel l'infection bactérienne est causée par une bactérie incluant des bactéries à Gram positif et à Gram négatif.

17. Processus destiné à préparer un composé de formule I selon la revendication 1 : 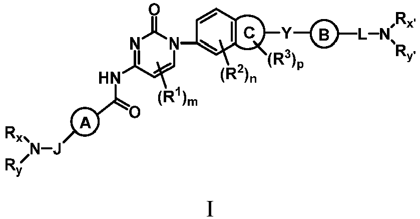 ou un stéréoisomère unique ou mélange de stéréoisomères de celui-ci, ou un sel pharmaceutiquement acceptable de celui-ci, le processus comprenant :
la combinaison d'un composé de formule A : 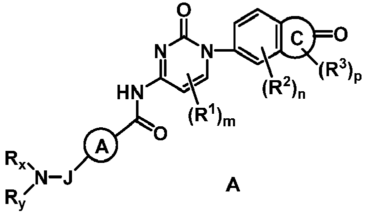 avec un composé de : 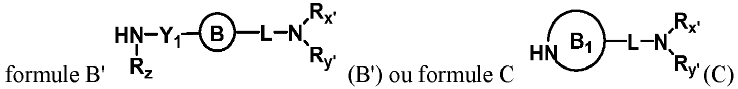 dans une condition d'amination réductrice pour fournir le composé de formule I,
dans lequel le noyau A, le noyau B, le noyau C, J, L, R¹, R², R³, Rₓ, R_{y}, R_{x'}, R_{y'}, m, n, et p sont tels que définis dans l'une quelconque des revendications 1 à 12 ;
le noyau B₁ est un hétérocycloalkylène monocyclique de 3 à 8 éléments contenant de l'azote, ou un hétérocycloalkylène bicyclique de 6 à 12 éléments contenant de l'azote, dont chacun est facultativement et indépendamment substitué par jusqu'à trois substituants choisis parmi le groupe constitué par un alkyle en C₁-C₆, un alcoxy en C₁-C₆, un halo, CN, un haloalkyle en C₁-C₆, OH, COO(alkyle en C₁-C₆), CONH₂, CONH(alkyle en C₁-C₆), CON(alkyle en C₁-C₆)₂, et un hydroxyalkyle en C₁-C₆ ;
Y est une liaison ou un alkylène en C₁-C₆ facultativement substitué par OH, NH₂, CN, halo, ou un alcoxy en C₁-C₆, dans lequel une unité de méthylène de l'alkylène en C₁-C₆ est facultativement remplacée par NH, N-(alkyle en C₁-C₆), N-(hydroxyalkyle en C₁-C₆), N-(haloalkyle en C₁-C₆), ou N-(alkylène en C₁-C₆-cycloalkyle en C₃-C₈) ;
Y₁ est une liaison ou un alkylène en C₁-C₅ facultativement substitué par OH, NH₂, CN, un halo, ou un alcoxy en Ci-Ce ; et
R_{z} est H, un alkyle en C₁-C₆, un hydroxyalkyle en C₁-C₆, un haloalkyle en C₁-C₆, ou un alkylène en C₁-C₆-cycloalkyle en C₃-C₈.

18. Processus destiné à préparer un composé de formule ID : 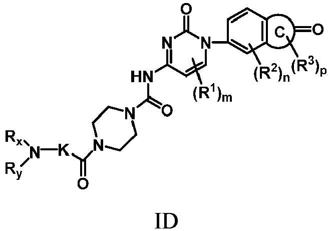 ou un sel pharmaceutiquement acceptable de celui-ci, le processus comprenant :
le couplage d'un composé de formule E 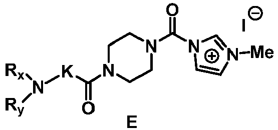
avec un composé de formule F 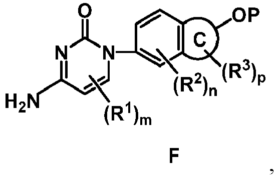 dans lequel le noyau C, K, R¹, R², R³, Rₓ, R_{y}, m, n, et p sont tels que définis dans l'une quelconque des revendications 1 à 12 ; et
P est un groupe protecteur d'hydroxyle.

19. Processus selon la revendication 18, comprenant en outre l'étape de retrait du groupe protecteur d'hydroxyle pour fournir un composé de formule IE 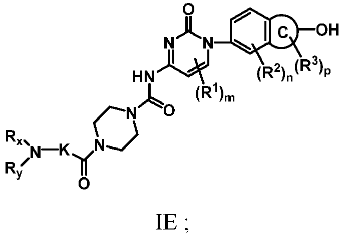 comprenant facultativement en outre l'étape d'oxydation du groupe hydroxyle pour fournir le composé de formule ID.

20. Composé de formule ID : 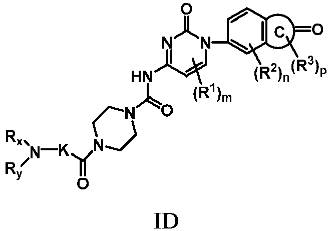 ou un stéréoisomère unique ou mélange de stéréoisomères de celui-ci, ou un sel pharmaceutiquement acceptable de celui-ci, dans lequel C, K, R¹, R², R³, Rₓ, R_{y}, m, n, et p sont tels que définis dans l'une quelconque des revendications 1 à 12.

21. Composé de formule II : 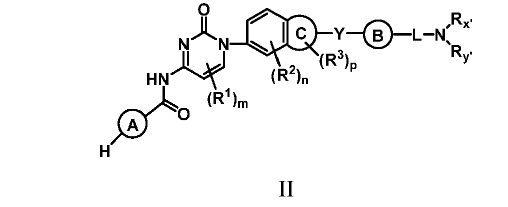 ou un stereoisomere unique ou mélange de stereoisomeres de celui-ci, ou un sel pharmaceutiquement acceptable de celui-ci, dans lequel le noyau A, le noyau B, le noyau C, L, R¹, R², R³, R_{x'}, R_{y'}, m, n, et p sont tels que définis dans l'une quelconque des revendications 1 à 12.

22. Composé selon la revendication 21 ou un sel pharmaceutiquement acceptable de celui-ci, dans lequel le composé est choisi parmi les composés énumérés dans le Tableau 3 :
**Tableau 3**
| **Structure de composé** | **Structure de composé** |
|---|---|
| 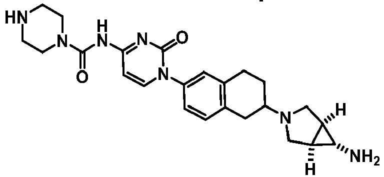 | 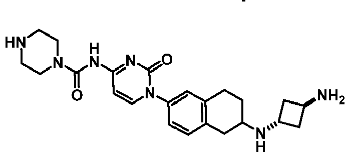 |
| 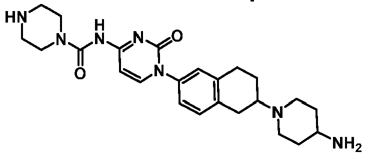 | 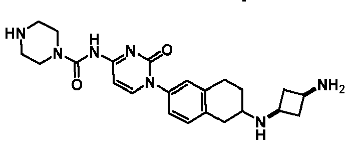 |
| 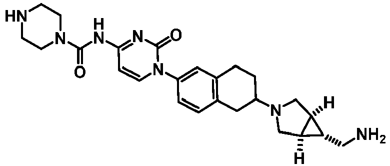 | 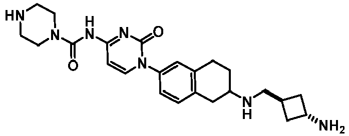 |
| 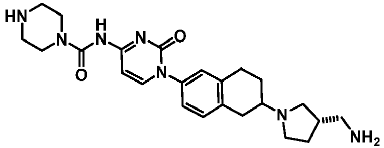 | 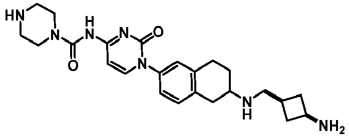 |
| 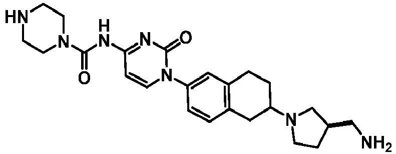 | 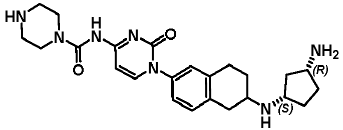 |
| 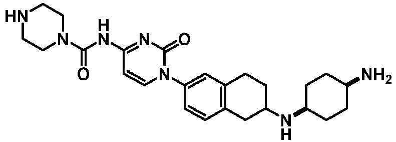 | 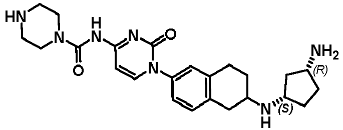 |
| 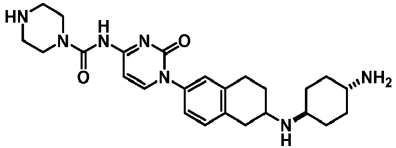 | 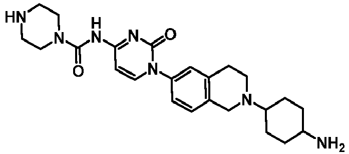 |
| 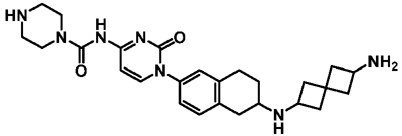 | 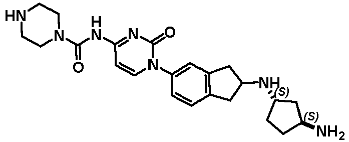 |
| 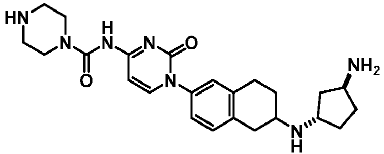 | 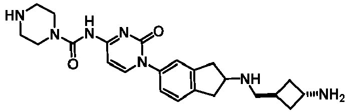 |
| 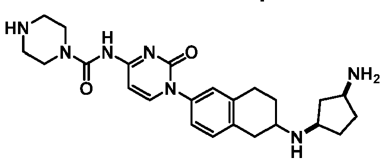 | 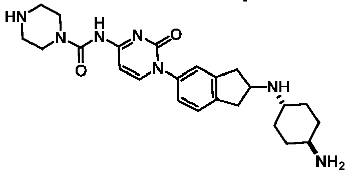 |
| 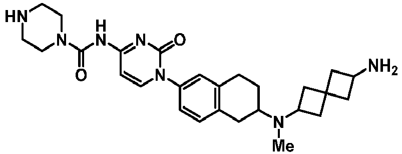 | 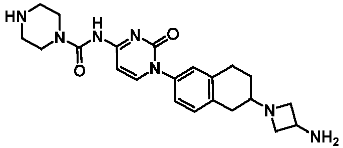 |
| 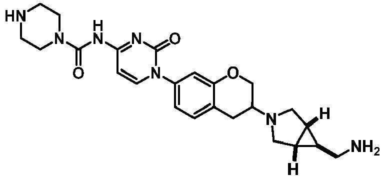 | 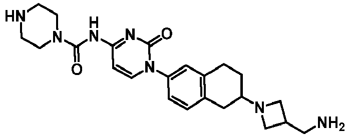 |
| 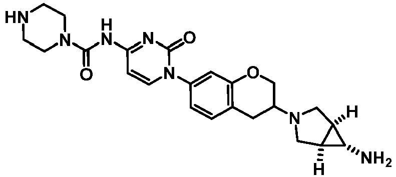 | 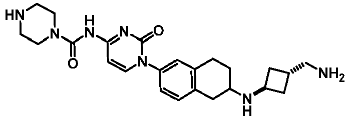 |
| 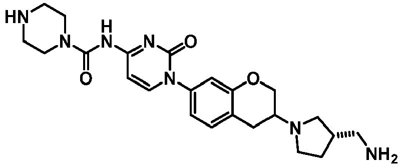 | 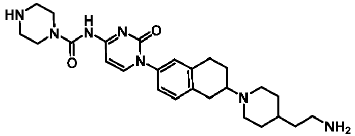 |
| 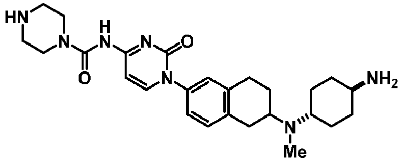 | 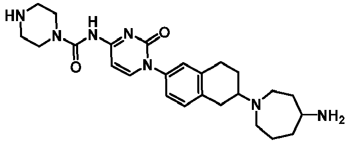 |
| 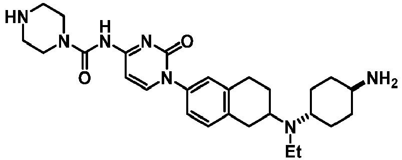 | 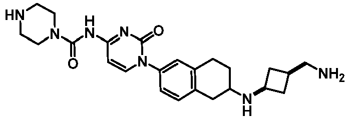 |
| 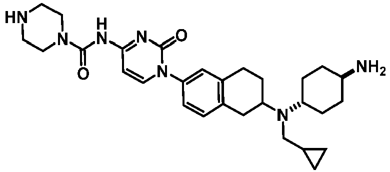 | 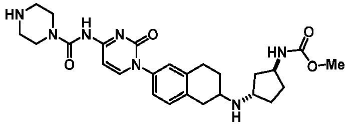 |
| 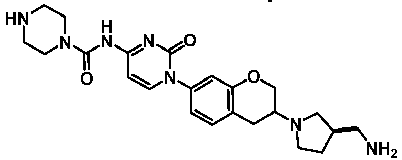 | 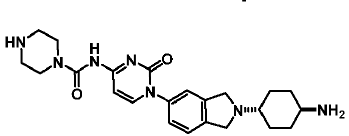 |
| 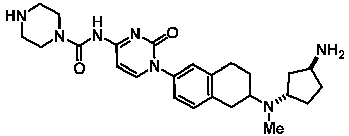 | 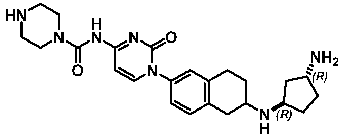 |
| 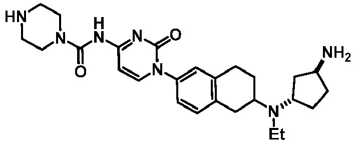 | 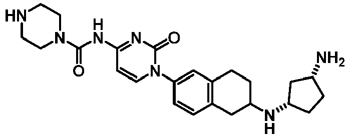 |
| 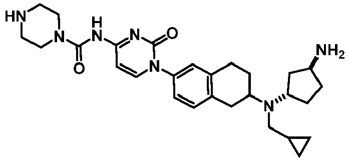 | 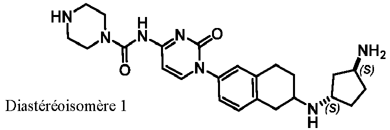 |
| 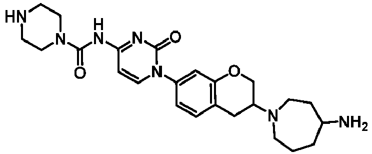 | 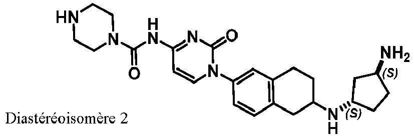 |
| 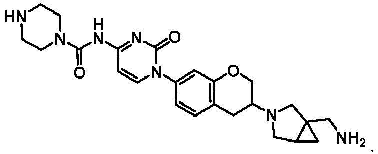 | |

23. Composé de formule III 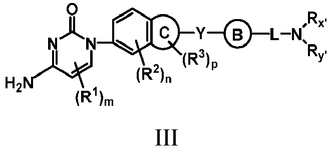 ou un stéréoisomère unique ou mélange de stéréoisomères de celui-ci, ou un sel pharmaceutiquement acceptable de celui-ci, dans lequel le noyau B, le noyau C, L, R¹, R², R³, R_{x'}, R_{y'}, m, n, et p sont tels que définis dans l'une quelconque des revendications 1 à 12.

24. Composé selon la revendication 23 ou un sel pharmaceutiquement acceptable de celui-ci, dans lequel le composé est choisi parmi les composés énumérés dans le Tableau 4 :
**Tableau 4**
| **Structure de composé** | **Structure de composé** |
|---|---|
| 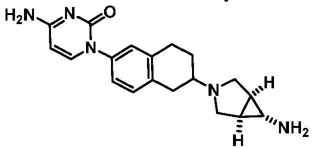 | 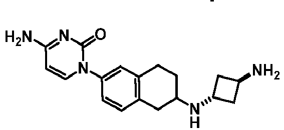 |
| 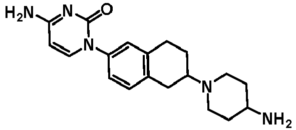 | 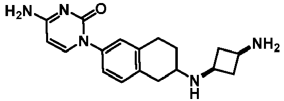 |
| 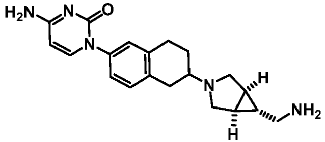 | 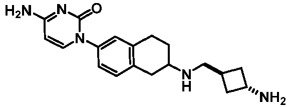 |
| 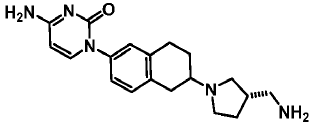 | 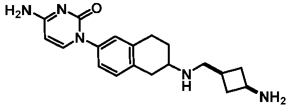 |
| 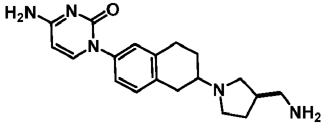 | 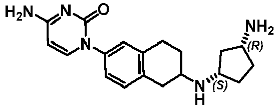 |
| 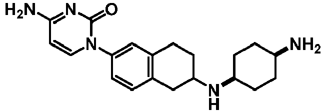 | 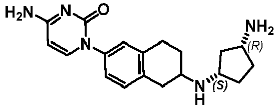 |
| 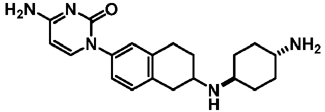 | 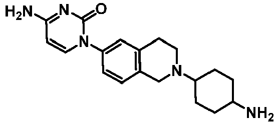 |
| 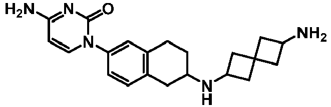 | 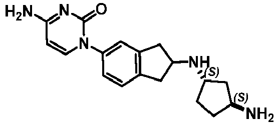 |
| 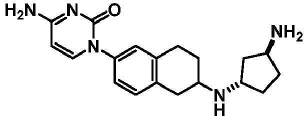 | 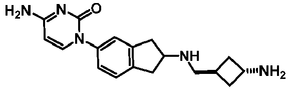 |
| 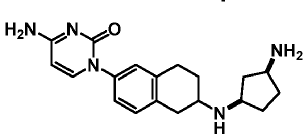 | 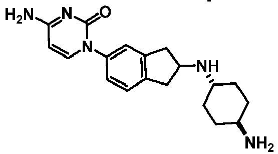 |
| 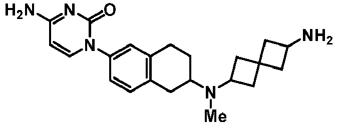 | 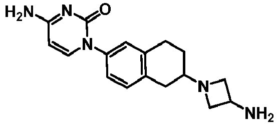 |
| 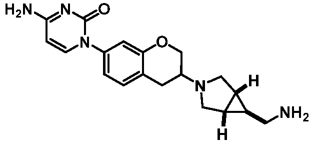 | 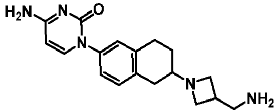 |
| 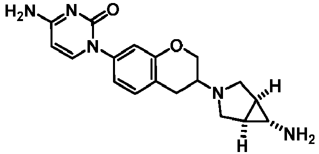 | 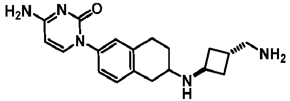 |
| 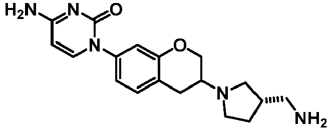 | 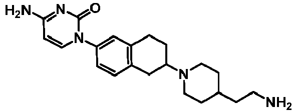 |
| 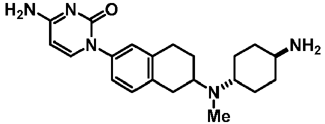 | 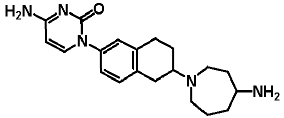 |
| 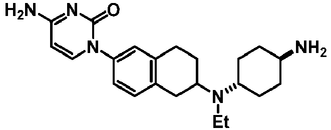 | 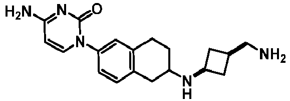 |
| 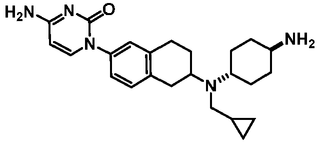 | 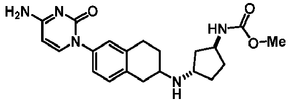 |
| 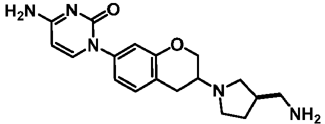 | 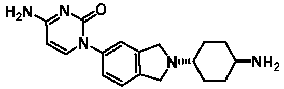 |
| 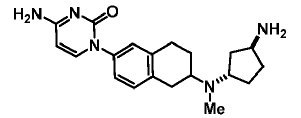 | 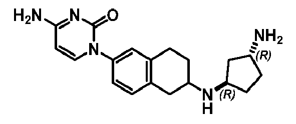 |
| 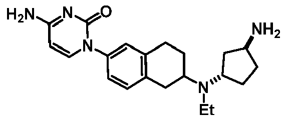 | 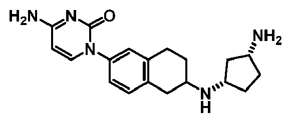 |
| 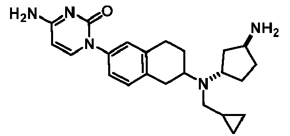 | 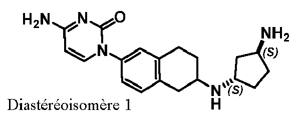 |
| 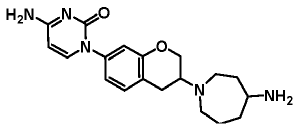 | 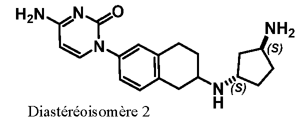 |
| 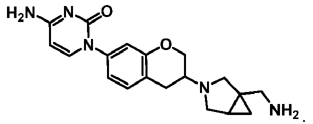 | |
